# EUROPEAN PATENT SPECIFICATION

(11) **EP 2 771 340 B1**
(45) Date of publication and mention of the grant of the patent: **13.04.2016**
(21) Application number: 12775026.3
(22) Date of filing: 22.10.2012
(51) Int. Cl.: C07D 471/04, C07D 519/00, A61K 31/4745, A61K 31/4748, A61P 35/00, A61P 9/00, A61P 25/00

(54) **6-(4-HYDROXY-PHENYL)-1H-PYRAZOLO[3,4-B]PYRIDINE-4-CARBOXYLIC ACID AMIDE DERIVATIVES AS KINASE INHIBITORS**
6-(4-HYDROXY-PHENYL)-1H-PYRAZOLO[3,4-B]PYRIDIN-4-CARBONSÄUREAMIDDERIVATE ALS KINASEINHIBITOREN
DÉRIVÉS D'AMIDE DE L'ACIDE 6-(4-HYDROXY-PHÉNYL)-1H-PYRAZOLO[3,4-B]PYRIDINE-4-CARBOXYLIQUE EN TANT QU'INHIBITEURS DE KINASES

(30) Priority: 25.10.2011 EP 11306377
(43) Date of publication of application: 03.09.2014
(73) Proprietor: SANOFI, 75008 Paris (FR)
(72) Inventor: Plettenburg, Oliver, 65926 Frankfurt am Main (DE); Loehn, Matthias, 65926 Frankfurt am Main (DE); Mendez-Perez, Maria, 65926 Frankfurt am Main (DE); Hachtel, Stephanie, 65926 Frankfurt am Main (DE); Podeschwa, Michael, 65926 Frankfurt am Main (DE); Kannt, Aimo, 65926 Frankfurt am Main (DE); Ivashchenko, Yuri, 65926 Frankfurt am Main (DE); Bjergarde, Kirsten, Bridgewater, New Jersey 08807 (US)
(74) Representative: Wingefeld, Renate
(86) International application number: PCT/EP2012/070843
(87) International publication number: WO 2013/060636

(56) References cited:
- WO-A1-2008/144253
- WO-A1-2010/129668
- WO-A2-2010/011772

## Description

The present invention relates to 1 H-pyrazolo[3,4-b]pyridine compounds of the formula I, 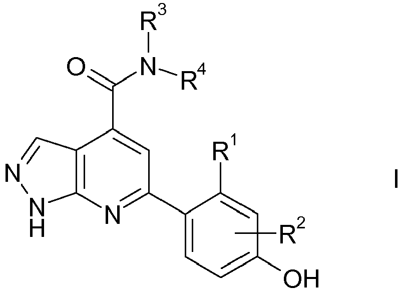 in which R¹, R², R³ and R⁴ are defined as indicated below. The compounds of the formula I are kinase inhibitors, and are useful for the treatment of diseases associated with diabetes and diabetic complications, e.g., diabetic nephropathy, diabetic neuropathy and diabetic retinopathy, for example. The invention furthermore relates to the use of compounds of the formula I, in particular as active ingredients in pharmaceuticals, and pharmaceutical compositions comprising them.

Protein kinase C (PKC) comprises a family of several related isoenzymes that function as serine/threonine kinases. PKC plays an important role in intercellular and intracellular signaling, gene expression, and in the control of cell differentiation and growth. Currently, at least ten isoforms of PKC are known which are different in regulation, tissue distribution, and enzymatic specificity (Newton AC. Regulation of the ABC kinases by phosphorylation: protein kinase C as a paradigm. Biochem J 2003; 370(Pt 2):361-371; Newton AC. Protein kinase C: poised to signal. Am J Physiol Endocrinol Metab 2010; 298(3):E395-E402; Nishizuka Y. Studies and prospectives of the protein kinase c family for cellular regulation. Cancer 1989; 63(10):1892-1903; Nishizuka Y. The Albert Lasker Medical Awards. The family of protein kinase C for signal transduction. JAMA 1989; 262(13):1826-1833). The PKC family of isoenzymes are grouped into three subclasses based on the domain composition of the regulatory moiety: (1) conventional PKCs (alpha, beta-II, and beta-I), (2) novel PKCs (delta, epsilon, gamma, eta and theta) and (3) atypical PKCs (zeta and iota/lambda) (Newton AC. Regulation of the ABC kinases by phosphorylation: protein kinase C as a paradigm. Biochem J 2003; 370(Pt 2):361-371; Mellor H, Parker PJ. The extended protein kinase C superfamily. Biochem J 1998; 332 (Pt 2):281-292). PKC is a membrane-associated enzyme that is regulated by several distinct factors, such as membrane phospholipids, calcium, and membrane lipids, e.g. diacylglycerol (Newton AC. Regulation of the ABC kinases by phosphorylation: protein kinase C as a paradigm. Biochem J 2003; 370(Pt 2):361-371; Newton AC. Protein kinase C: poised to signal. Am J Physiol Endocrinol Metab 2010; 298(3):E395-E402; Mellor H, Parker PJ. The extended protein kinase C superfamily. Biochem J 1998; 332 (Pt 2):281-292; Kishimoto A, Kikkawa U, Ogita K, Shearman MS, Nishizuka Y. The protein kinase C family in the brain: heterogeneity and its implications. Ann N Y Acad Sci 1989; 568: 181-186; Nishizuka Y. Calcium, phospholipid turnover and transmembrane signalling. Philos Trans R Soc Lond B Biol Sci 1983; 302(1108):101-112.). All PKC isoforms have an autoinhibitory pseudosubstrate sequence that is N-terminal to the C1 domain, which functions as a diacylglycerol sensor. Atypical PKCs have a diacylglycerol non-responsive C1 domain. Conventional PKCs have a C2 domain that serves as a Ca²⁺-regulated phospholipid-binding module. The C2 domain in novel PKCs binds neither Ca²⁺ nor membrane phospholipids. Based on the structural differences conventional PKCs require membrane phospholipids, calcium and diacylglycerol for complete activation. Novel PKCs do not require calcium but diacylglycerol for activation. The zeta and iota/lambda forms of PKC are independent of both calcium and diacylglycerol for their activation (Newton AC. Regulation of the ABC kinases by phosphorylation: protein kinase C as a paradigm. Biochem J 2003; 370(Pt 2):361-371; Newton AC. Lipid activation of protein kinases. J Lipid Res 2009; 50 Suppl:S266-S271). PKC is involved in the regulation of smooth muscle contractility. Upon stimulation PKC phosphorylates the regulatory myosin light chain (MLC₂₀) and inhibits the myosin associated phosphatase (MYPT). Phosphorylation of MLC₂₀ and inhibition of MYPT leads to an increased activity of the acto-myosin complex and to vasoconstriction in different vascular beds, e.g. resistance-sized, retinal, cerebral, coronary, conduit arteries and veins (Merkel LA, Rivera LM, Colussi DJ, Perrone MH. Protein kinase C and vascular smooth muscle contractility: effects of inhibitors and down-regulation. J Pharmacol Exp Ther 1991; 257(1):134-140; Sehic E, Malik KU. Influence of protein kinase C activators on vascular tone and adrenergic neuroeffector events in the isolated rat kidney. J Pharmacol Exp Ther 1989; 251(2):634-639.). Overexpressed or overactivated PKC detrimentally affects heart function. Upon activation PKC affects the intracellular calcium homeostasis which results in reduced myocardial contractility and relaxation of the myocardium. Overall this effect leads to myocardial contractile insufficiency (Connelly KA, Kelly DJ, Zhang Y, Prior DL, Advani A, Cox AJ, Thai K, Krum H, Gilbert RE. Inhibition of protein kinase C-beta by ruboxistaurin preserves cardiac function and reduces extracellular matrix production in diabetic cardiomyopathy. Circ Heart Fail 2009; 2(2):129-137). Moreover, activated PKC mediates organ damage during end-organ injuries, e.g. during ischemia in heart (Connelly KA, Kelly DJ, Zhang Y, Prior DL, Advani A, Cox AJ, Thai K, Krum H, Gilbert RE. Inhibition of protein kinase C-beta by ruboxistaurin preserves cardiac function and reduces extracellular matrix production in diabetic cardiomyopathy. Circ Heart Fail 2009; 2(2):129-137; Hambleton M, Hahn H, Pleger ST, Kuhn MC, Klevitsky R, Carr AN, Kimball TF, Hewett TE, Dorn GW, Koch WJ, Molkentin JD. Pharmacological- and gene therapy-based inhibition of protein kinase Calpha/beta enhances cardiac contractility and attenuates heart failure. Circulation 2006; 114(6): 574-582) or kidney (Tuttle KR. Protein kinase C-beta inhibition for diabetic kidney disease. Diabetes Res Clin Pract 2008; 82 Suppl 1:S70-S74; Anderson PW, McGill JB, Tuttle KR. Protein kinase C beta inhibition: the promise for treatment of diabetic nephropathy. Curr Opin Nephrol Hypertens 2007; 16(5):397-402). PKC and especially the PKC-beta II isoform is overexpressed or overactivated in diabetes in various different types of tissue and exerts its deleterious effect to the cells, tissues and end-organs, e.g. kidney (Tuttle KR. Protein kinase C-beta inhibition for diabetic kidney disease. Diabetes Res Clin Pract 2008; 82 Suppl 1:S70-S74; Anderson PW, McGill JB, Tuttle KR. Protein kinase C beta inhibition: the promise for treatment of diabetic nephropathy. Curr Opin Nephrol Hypertens 2007; 16(5):397-402; Tuttle KR, Bakris GL, Toto RD, McGill JB, Hu K, Anderson PW. The effect of ruboxistaurin on nephropathy in type 2 diabetes. Diabetes Care 2005; 28(11):2686-2690; Kelly DJ, Zhang Y, Hepper C, Gow RM, Jaworski K, Kemp BE, Wilkinson-Berka JL, Gilbert RE. Protein kinase C beta inhibition attenuates the progression of experimental diabetic nephropathy in the presence of continued hypertension. Diabetes 2003; 52(2):512-518), heart (Connelly KA, Kelly DJ, Zhang Y, Prior DL, Advani A, Cox AJ, Thai K, Krum H, Gilbert RE. Inhibition of protein kinase C-beta by ruboxistaurin preserves cardiac function and reduces extracellular matrix production in diabetic cardiomyopathy. Circ Heart Fail 2009; 2(2):129-137; Guo M, Wu MH, Korompai F, Yuan SY. Upregulation of PKC genes and isozymes in cardiovascular tissues during early stages of experimental diabetes. Physiol Genomics 2003; 12(2):139-146), or in tissues like the retina (Aiello LP, Clermont A, Arora V, Davis MD, Sheetz MJ, Bursell SE. Inhibition of PKC beta by oral administration of ruboxistaurin is well tolerated and ameliorates diabetes-induced retinal hemodynamic abnormalities in patients. Invest Ophthalmol Vis Sci 2006; 47(1):86-92; Aiello LP. The potential role of PKC beta in diabetic retinopathy and macular edema. Surv Ophthalmol 2002; 47 Suppl 2:S263-S269; Kimura M, Ishizawa M, Miura A, Itaya S, Kanoh Y, Yasuda K, Uno Y, Morita H, Ishizuka T. Platelet protein kinase C isoform content in type 2 diabetes complicated with retinopathy and nephropathy. Platelets 2001; 12(3):138-143) or neuronal tissue (Krishnan ST, Rayman G. New treatments for diabetic neuropathy: symptomatic treatments. Curr Diab Rep 2003; 3(6):459-467; Kim H, Sasaki T, Maeda K, Koya D, Kashiwagi A, Yasuda H. Protein kinase Cbeta selective inhibitor LY333531 attenuates diabetic hyperalgesia through ameliorating cGMP level of dorsal root ganglion neurons. Diabetes 2003; 52(8):2102-2109; Cotter MA, Jack AM, Cameron NE. Effects of the protein kinase C beta inhibitor LY333531 on neural and vascular function in rats with streptozotocin-induced diabetes. Clin Sci (Lond) 2002; 103(3):311-321; Nakamura J, Kato K, Hamada Y, Nakayama M, Chaya S, Nakashima E, Naruse K, Kasuya Y, Mizubayashi R, Miwa K, Yasuda Y, Kamiya H, lenaga K, Sakakibara F, Koh N, Hotta N. A protein kinase C-beta-selective inhibitor ameliorates neural dysfunction in streptozotocin-induced diabetic rats. Diabetes 1999; 48(10):2090-2095) or in platelets (Assert R, Scherk G, Bumbure A, Pirags V, Schatz H, Pfeiffer AF. Regulation of protein kinase C by short term hyperglycaemia in human platelets in vivo and in vitro. Diabetologia 2001; 44(2):188-195; Bynagari-Settipalli YS, Chari R, Kilpatrick L, Kunapuli SP. Protein kinase C - possible therapeutic target to treat cardiovascular diseases. Cardiovasc Hematol Disord Drug Targets 2010; 10(4):292-308; Kimura M, Ishizawa M, Miura A, Itaya S, Kanoh Y, Yasuda K, Uno Y, Morita H, Ishizuka T. Platelet protein kinase C isoform content in type 2 diabetes complicated with retinopathy and nephropathy. Platelets 2001; 12(3):138-143; Oskarsson HJ, Hofmeyer TG, Coppey L, Yorek MA. Effect of protein kinase C and phospholipase A2 inhibitors on the impaired ability of human diabetic platelets to cause vasodilation. Br J Pharmacol 1999; 127(4):903-908) or induces endothelial dysfunction (Chiasson VL, Quinn MA, Young KJ, Mitchell BM. Protein kinase Cbetall-mediated phosphorylation of endothelial nitric oxide synthase threonine 495 mediates the endothelial dysfunction induced by FK506 (tacrolimus). J Pharmacol Exp Ther 2011; 337(3):718-723; Xu Y, Wang S, Feng L, Zhu Q, Xiang P, He B. Blockade of PKC-beta protects HUVEC from advanced glycation end products induced inflammation. Int Immunopharmacol 2010; 10(12):1552-1559; Geraldes P, King GL. Activation of protein kinase C isoforms and its impact on diabetic complications. Circ Res 2010; 106(8):1319-1331; Nacci C, Tarquinio M, Montagnani M. Molecular and clinical aspects of endothelial dysfunction in diabetes. Intern Emerg Med 2009; 4(2):107-116). Furthermore, it has been suggested that PKC signalling is involved in tumour formation (Gonelli A, Mischiati C, Guerrini R, Voltan R, Salvadori S, Zauli G. Perspectives of protein kinase C (PKC) inhibitors as anti-cancer agents. Mini Rev Med Chem 2009; 9(4):498-509; Ali AS, Ali S, EI-Rayes BF, Philip PA, Sarkar FH. Exploitation of protein kinase C: a useful target for cancer therapy. Cancer Treat Rev 2009; 35(1):1-8), e.g. in hematological tumours (Mischiati C, Melloni E, Corallini F, Milani D, Bergamini C, Vaccarezza M. Potential role of PKC inhibitors in the treatment of hematological malignancies. Curr Pharm Des 2008; 14(21):2075-2084; Cheson BD, Zwiebel JA, Dancey J, Murgo A. Novel therapeutic agents for the treatment of myelodysplastic syndromes. Semin Oncol 2000; 27(5):560-577; Deng X, Kornblau SM, Ruvolo PP, May WS, Jr. Regulation of Bcl2 phosphorylation and potential significance for leukemic cell chemoresistance. J Natl Cancer Inst Monogr 2001;(28):30-37), in glioma formation (Baltuch GH, Dooley NP, Villemure JG, Yong VW. Protein kinase C and growth regulation of malignant gliomas. Can J Neurol Sci 1995; 22(4):264-271; Blobe GC, Obeid LM, Hannun YA. Regulation of protein kinase C and role in cancer biology. Cancer Metastasis Rev 1994; 13(3-4):411-431; Bredel M, Pollack IF. The role of protein kinase C (PKC) in the evolution and proliferation of malignant gliomas, and the application of PKC inhibition as a novel approach to anti-glioma therapy. Acta Neurochir (Wien) 1997; 139(11):1000-1013), in gastric and intestinal cancer (Atten MJ, Godoy-Romero E, Attar BM, Milson T, Zopel M, Holian O. Resveratrol regulates cellular PKC alpha and delta to inhibit growth and induce apoptosis in gastric cancer cells. Invest New Drugs 2005; 23(2):111-119; Fahrmann M. Targeting protein kinase C (PKC) in physiology and cancer of the gastric cell system. Curr Med Chem 2008; 15(12):1175-1191), in skin cancer (Birt DF, Yaktine A, Duysen E. Glucocorticoid mediation of dietary energy restriction inhibition of mouse skin carcinogenesis. J Nutr 1999; 129 (2S Suppl):571 S-574S; Birt DF, Przybyszewski J, Wang W, Stewart J, Liu Y. Identification of molecular targets for dietary energy restriction prevention of skin carcinogenesis: an idea cultivated by Edward Bresnick. J Cell Biochem 2004; 91(2):258-264), lung cancer (Herbst RS, Oh Y, Wagle A, Lahn M. Enzastaurin, a protein kinase Cbeta- selective inhibitor, and its potential application as an anticancer agent in lung cancer. Clin Cancer Res 2007; 13(15 Pt 2):s4641-s4646; Herbst RS. Targeted therapy in non-small-cell lung cancer. Oncology (Williston Park) 2002; 16(9 Suppl 9):19-24) and others. PKC is an important signal transducer of events in autoimmune responses, e.g. in T-cell (Birchall AM, Bishop J, Bradshaw D, Cline A, Coffey J, Elliott LH, Gibson VM, Greenham A, Hallam TJ, Harris W, . Ro 32-0432, a selective and orally active inhibitor of protein kinase C prevents T-cell activation. J Pharmacol Exp Ther 1994; 268(2):922-929; Isakov N, Altman A. Protein kinase C(theta) in T cell activation. Annu Rev Immunol 2002; 20:761-794) or B-cell (Shinohara H, Kurosaki T. Comprehending the complex connection between PKCbeta, TAK1, and IKK in BCR signaling. Immunol Rev 2009; 232(1):300-318; Venkataraman C, Chen XC, Na S, Lee L, Neote K, Tan SL. Selective role of PKCbeta enzymatic function in regulating cell survival mediated by B cell antigen receptor cross-linking. Immunol Lett 2006; 105(1):83-89) linked autoimmune signalling, and in inflammatory processes. The above mentioned effects of the PKC-mediated signalling leads to induction or promotion of the progression of asthma (Boschelli DH. Small molecule inhibitors of PKCTheta as potential antiinflammatory therapeutics. Curr Top Med Chem 2009; 9(7):640-654), chronic obstructive pulmonary disease (Mercer BA, D'Armiento JM. Emerging role of MAP kinase pathways as therapeutic targets in COPD. Int J Chron Obstruct Pulmon Dis 2006; 1(2):137-150; Adcock IM, Chung KF, Caramori G, Ito K. Kinase inhibitors and airway inflammation. Eur J Pharmacol 2006; 533(1-3):118-132; Dempsey EC, Cool CD, Littler CM. Lung disease and PKCs. Pharmacol Res 2007; 55(6):545-559; Ishii M, Kurachi Y. Muscarinic acetylcholine receptors. Curr Pharm Des 2006; 12(28):3573-3581; Medina-Tato DA, Watson ML, Ward SG. Leukocyte navigation mechanisms as targets in airway diseases. Drug Discov Today 2006; 11 (19-20):866-879), pulmonary hypertension (Agbani EO, Coats P, Mills A, Wadsworth RM. Peroxynitrite stimulates pulmonary artery endothelial and smooth muscle cell proliferation: involvement of ERK and PKC. Pulm Pharmacol Ther 2011; 24(1):100-109; Littler CM, Wehling CA, Wick MJ, Fagan KA, Cool CD, Messing RO, Dempsey EC. Divergent contractile and structural responses of the murine PKC-epsilon null pulmonary circulation to chronic hypoxia. Am J Physiol Lung Cell Mol Physiol 2005; 289(6):L1083-L1093), retinopathy, like retinal ischemia and neovascularization (Galvez MI. Protein kinase C inhibitors in the treatment of diabetic retinopathy. Review. Curr Pharm Biotechnol 2011; 12(3):386-391; Schwartz SG, Flynn HW, Jr., Aiello LP. Ruboxistaurin mesilate hydrate for diabetic retinopathy. Drugs Today (Barc) 2009; 45(4):269-274), nephropathy, including hypertension-induced (Kelly DJ, Edgley AJ, Zhang Y, Thai K, Tan SM, Cox AJ, Advani A, Connelly KA, Whiteside CI, Gilbert RE. Protein kinase C-beta inhibition attenuates the progression of nephropathy in non-diabetic kidney disease. Nephrol Dial Transplant 2009; 24(6):1782-1790; Hayashi K, Wakino S, Ozawa Y, Homma K, Kanda T, Okubo K, Takamatsu I, Tatematsu S, Kumagai H, Saruta T. Role of protein kinase C in Ca channel blocker-induced renal arteriolar dilation in spontaneously hypertensive rats--studies in the isolated perfused hydronephrotic kidney. Keio J Med 2005; 54(2):102-108; Kelly DJ, Zhang Y, Hepper C, Gow RM, Jaworski K, Kemp BE, Wilkinson-Berka JL, Gilbert RE. Protein kinase C beta inhibition attenuates the progression of experimental diabetic nephropathy in the presence of continued hypertension. Diabetes 2003; 52(2):512-518), non-hypertension-induced, and diabetic nephropathies (Danis RP, Sheetz MJ. Ruboxistaurin: PKC-beta inhibition for complications of diabetes. Expert Opin Pharmacother 2009; 10(17):2913-2925; Tuttle KR. Protein kinase C-beta inhibition for diabetic kidney disease. Diabetes Res Clin Pract 2008; 82 Suppl 1:S70-S74), renal failure (Danis RP, Sheetz MJ. Ruboxistaurin: PKC-beta inhibition for complications of diabetes. Expert Opin Pharmacother 2009; 10(17):2913-2925; Yamagishi S, Fukami K, Ueda S, Okuda S. Molecular mechanisms of diabetic nephropathy and its therapeutic intervention. Curr Drug Targets 2007; 8(8):952-959) and myocardial infarction (Byna-gari-Settipalli YS, Chari R, Kilpatrick L, Kunapuli SP. Protein kinase C - possible therapeutic target to treat cardiovascular diseases. Cardiovasc Hematol Disord Drug Targets 2010; 10(4):292-308; Rohilla A, Singh G, Singh M, Bala kP. Possible involvement of PKC-delta in the abrogated cardioprotective potential of ischemic preconditioning in hyperhomocysteinemic rat hearts. Biomed Pharmacother 2010; 64(3):195-202; Liu Q, Chen X, Macdonnell SM, Kranias EG, Lorenz JN, Leitges M, Houser SR, Molkentin JD. Protein kinase C{alpha}, but not PKC{beta} or PKC {gamma}, regulates contractility and heart failure susceptibility: implications for ruboxistaurin as a novel therapeutic approach. Circ Res 2009; 105(2):194-200; Yonezawa T, Kurata R, Kimura M, Inoko H. PKC delta and epsilon in drug targeting and therapeutics. Recent Pat DNA Gene Seq 2009; 3(2):96-101) cardiac hypertrophy and failure (Ferreira JC, Brum PC, Mochly-Rosen D. betaIIPKC and epsilonPKC isozymes as potential pharmacological targets in cardiac hypertrophy and heart failure. J Mol Cell Cardiol 2010; Palaniyandi SS, Sun L, Ferreira JC, Mochly-Rosen D. Protein kinase C in heart failure: a therapeutic target? Cardiovasc Res 2009; 82(2):229-239), coronary heart disease, artherosclerosis, restenosis (Ding RQ, Tsao J, Chai H, Mochly-Rosen D, Zhou W. Therapeutic potential for protein kinase C inhibitor in vascular restenosis. J Cardiovasc Pharmacol Ther 2011; 16(2):160-167; Schleicher E, Friess U. Oxidative stress, AGE, and atherosclerosis. Kidney Int Suppl 2007;(106):S17-S26), diabetes, diabetic complications, glucose utilization and metabolic syndrome (Bynagari-Settipalli YS, Chari R, Kilpatrick L, Kunapuli SP. Protein kinase C - possible therapeutic target to treat cardiovascular diseases. Cardiovasc Hematol Disord Drug Targets 2010; 10(4):292-308; Geraldes P, King GL. Activation of protein kinase C isoforms and its impact on diabetic complications. Circ Res 2010; 106(8):1319-1331; Danis RP, Sheetz MJ. Ruboxistaurin: PKC-beta inhibition for complications of diabetes. Expert Opin Pharmacother 2009; 10(17):2913-2925), immune diseases (Baier G, Wagner J. PKC inhibitors: potential in T cell-dependent immune diseases. Curr Opin Cell Biol 2009; 21(2):262-267; Mecklenbrauker I, Saijo K, Zheng NY, Leitges M, Tarakhovsky A. Protein kinase Cdelta controls self-antigen-induced B-cell tolerance. Nature 2002; 416(6883):860-865; Wilkinson SE, Hallam TJ. Protein kinase C: is its pivotal role in cellular activation over-stated? Trends Pharmacol Sci 1994; 15(2):53-57; Costello R, Mawas C, Olive D. Differential immuno-suppressive effects of metabolic inhibitors on T-lymphocyte activation. Eur Cytokine Netw 1993; 4(2):139-146), like psoriasis (Sommerer C, Zeier M. AEB071--a promising immunosuppressive agent. Clin Transplant 2009; 23 Suppl 21:15-18; Rasmussen HH, Celis JE. Evidence for an altered protein kinase C (PKC) signaling pathway in psoriasis. J Invest Dermatol 1993; 101(4):560-566; Fisher GJ, Tavakkol A, Leach K, Burns D, Basta P, Loomis C, Griffiths CE, Cooper KD, Reynolds NJ, Elder JT, . Differential expression of protein kinase C isoenzymes in normal and psoriatic adult human skin: reduced expression of protein kinase C-beta II in psoriasis. J Invest Dermatol 1993; 101 (4):553-559), rheumatoid athritis (Healy AM, Izmailova E, Fitzgerald M, Walker R, Hattersley M, Silva M, Siebert E, Terkelsen J, Picarella D, Pickard MD, LeClair B, Chandra S, Jaffee B. PKC-theta-deficient mice are protected from Th1-dependent antigen-induced arthritis. J Immunol 2006; 177(3):1886-1893; Ji JD, Tassiulas I, Park-Min KH, Aydin A, Mecklenbrauker I, Tarakhovsky A, Pricop L, Salmon JE, Ivashkiv LB. Inhibition of interleukin 10 signaling after Fc receptor ligation and during rheumatoid arthritis. J Exp Med 2003; 197(11):1573-1583; Kehlen A, Thiele K, Riemann D, Langner J. Expression, modulation and signalling of IL-17 receptor in fibroblast-like synoviocytes of patients with rheumatoid arthritis. Clin Exp Immunol 2002; 127(3):539-546), or other autoimmune disorders (Zanin-Zhorov A, Dustin ML, Blazar BR. PKC-theta function at the immunological synapse: prospects for therapeutic targeting. Trends Immunol 2011; 32(8):358-363), central nervous system disorders (Liang J, Takeuchi H, Jin S, Noda M, Li H, Doi Y, Kawanokuchi J, Sonobe Y, Mizuno T, Suzumura A. Glutamate induces neurotrophic factor production from microglia via protein kinase C pathway. Brain Res 2010; 1322:8-23; Bastianetto S, Zheng WH, Quirion R. Neuroprotective abilities of resveratrol and other red wine constituents against nitric oxide-related toxicity in cultured hippocampal neurons. Br J Pharmacol 2000; 131(4):711-720), cerebral ischemia or cerebral vasospasm (Bu X, Zhang N, Yang X, Liu Y, Du J, Liang J, Xu Q, Li J. Proteomic analysis of cPKCbetall-interacting proteins involved in HPC-induced neuroprotection against cerebral ischemia of mice. J Neurochem 2011; 117(2):346-356), neuropathies and pain, e.g. neuropathic pain (Nakajima A, Tsuboi Y, Suzuki I, Honda K, Shinoda M, Kondo M, Matsuura S, Shibuta K, Yasuda M, Shimizu N, Iwata K. PKCgamma in Vc and C1/C2 is involved in trigeminal neuropathic pain. J Dent Res 2011; 90(6):777-781; Malmberg AB, Chen C, Tonegawa S, Basbaum AI. Preserved acute pain and reduced neuropathic pain in mice lacking PKCgamma. Science 1997; 278(5336):279-283), cancer development and progression, neoplasia where inhibition of protein kinase C has been shown to inhibit tumor cell growth and metastasis (Kim J, Thorne SH, Sun L, Huang B, Mochly-Rosen D. Sustained inhibition of PKCalpha reduces intravasation and lung seeding during mammary tumor metastasis in an in vivo mouse model. Oncogene 2011; 30(3):323-333; Spindler KL, Lindebjerg J, Lahn M, Kjaer-Frifeldt S, Jakobsen A. Protein kinase C-beta II (PKC-beta II) expression in patients with colorectal cancer. Int J Colorectal Dis 2009; 24(6):641-645; Guo K, Li Y, Kang X, Sun L, Cui J, Gao D, Liu Y. Role of PKCbeta in hepatocellular carcinoma cells migration and invasion in vitro: a potential therapeutic target. Clin Exp Metastasis 2009; 26(3):189-195), angiogenesis (Nakamura S, Chikaraishi Y, Tsuruma K, Shimazawa M, Hara H. Ruboxistaurin, a PKCbeta inhibitor, inhibits retinal neovascularization via suppression of phosphorylation of ERK1/2 and Akt. Exp Eye Res 2010; 90(1):137-145; Ali AS, Ali S, EI-Rayes BF, Philip PA, Sarkar FH. Exploitation of protein kinase C: a useful target for cancer therapy. Cancer Treat Rev 2009; 35(1):1-8; Tekle C, Giovannetti E, Sigmond J, Graff JR, Smid K, Peters GJ. Molecular pathways involved in the synergistic interaction of the PKC beta inhibitor enzastaurin with the antifolate pemetrexed in non-small cell lung cancer cells. Br J Cancer 2008; 99(5):750-759; Mischiati C, Melloni E, Corallini F, Milani D, Bergamini C, Vaccarezza M. Potential role of PKC inhibitors in the treatment of hematological malignancies. Curr Pharm Des 2008; 14(21):2075-2084), platelet disorders leading to thrombosis (Gilio K, Harper MT, Cosemans JM, Konopatskaya O, Munnix IC, Prinzen L, Leitges M, Liu Q, Molkentin JD, Heemskerk JW, Poole AW. Functional divergence of platelet protein kinase C (PKC) isoforms in thrombus formation on collagen. J Biol Chem 2010; 285(30):23410-23419; Chari R, Getz T, Nagy B, Jr., Bhavaraju K, Mao Y, Bynagari YS, Murugappan S, Nakayama K, Kunapuli SP. Protein kinase C[delta] differentially regulates platelet functional responses. Arterioscler Thromb Vasc Biol 2009; 29(5):699-705; Nagy B, Jr., Bhavaraju K, Getz T, Bynagari YS, Kim S, Kunapuli SP. Impaired activation of platelets lacking protein kinase C-theta isoform. Blood 2009; 113(11):2557-2567; Harper MT, Poole AW. Isoform-specific functions of protein kinase C: the platelet paradigm. Biochem Soc Trans 2007; 35(Pt 5):1005-1008; Strehl A, Munnix IC, Kuijpers MJ, van der Meijden PE, Cosemans JM, Feijge MA, Nieswandt B, Heemskerk JW. Dual role of platelet protein kinase C in thrombus formation: stimulation of pro-aggregatory and suppression of procoagulant activity in platelets. J Biol Chem 2007; 282(10):7046-7055; London FS. The protein kinase C inhibitor R0318220 potentiates thrombin-stimulated platelet-supported prothrombinase activity. Blood 2003; 102(7):2472-2481; Wheeler-Jones CP, Patel Y, Kakkar VV, Krishnamurthi S. Translocation of protein kinase C (PKC) in stimulated platelets: a role for aggregation in PKC degradation. Br J Pharmacol 1989; 98 Suppl:845P), and leukocyte aggregation (Hu H, Zhang W, Li N. Glycoprotein IIb/IIIa inhibition attenuates platelet-activating factor-induced platelet activation by reducing protein kinase C activity. J Thromb Haemost 2003; 1(8):1805-1812; Kotovuori A, Pessa-Morikawa T, Kotovuori P, Nortamo P, Gahmberg CG. ICAM-2 and a peptide from its binding domain are efficient activators of leukocyte adhesion and integrin affinity. J Immunol 1999; 162(11):6613-6620; Lorenz HM, Lagoo AS, Hardy KJ. The cell and molecular basis of leukocyte common antigen (CD45)-triggered, lymphocyte function-associated antigen-1-/intercellular adhesion molecule-1-dependent, leukocyte adhesion. Blood 1994; 83(7):1862-1870).

Until now, mainly staurosporine derivatives have been described as PKC inhibitors in the prior art, for example Ruboxistaurin (e.g. EP 657458), Enzastaurin (e.g. WO 9517182), Midostaurin (e.g. EP 296110) or Sotrastaurin (e.g. WO 2002038561). Only very few PKCß inhibitors, which are not derived from staurosporine have been described, such as 3-amido-pyrrolo[3,4-c]pyrazole-5(1H, 4H,6H)carbaldehydes in WO 2008125945. However, there continues to be a need for further effective low molecular weight PKCß inhibitors, in particular in view of safety and selectivity. The present invention satisfies this need by providing the pyrazolo[3,4-b]pyridine compounds of the formula I.

Pyrazolo[3,4-b]pyridine derivatives which are useful for pharmaceutical applications, have already been disclosed, for example in WO 2005028480 (Neurogen Corp. and Aventis Pharmaceuticals Inc.), in WO 2005009389 (Exelixis Inc.) or in WO 2000058307 (Neurogen Corp.).

Accordingly, a subject of the present invention is a compound of the formula I, 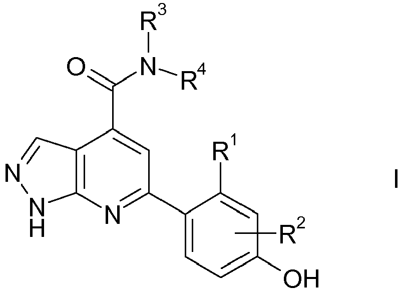 wherein
- R¹: is H, halogen or (C₁-C₄)-alkyl;
- R²: is H, halogen or (C₁-C₄)-alkyl;
- R³: is H or (C₁-C₄)-alkyl;
- R⁴: is
a) (C₀-C₆)-alkyl which is mono-substituted by
   i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
      ia) F,
      ib) (C₁-C₄)-alkyl, which is unsubstituted or one to fivefold substituted by F,
      ic) O-(C₁-C₄)-alkyl, which is unsubstituted or one to fivefold substituted by F,
      id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
      ie) (C₁-C₄)-alkylene-phenyl, which is unsubstituted or one to fivefold substituted by F,
      if) (C₃-C₈)- cycloalkyl,
      ig) oxo (=O), and
      ih) (CO)-(C₁-C₄)-alkyl,
         and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
   ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
      iia) F,
      iib) (C₁-C₄)-alkyl, wherein (C₁-C₄)-alkyl is unsubstituted or one to fivefold substituted by F,
      iic) O-(C₁-C₄)-alkyl, which is unsubstituted or one to fivefold substituted by F,
      iid) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F, and
      iie) (C₁-C₄)-alkylene-phenyl, which is unsubstituted or one to fivefold substituted by F;
      or
   iii) NH₂, NH(C₁-C₆)-alkyl, or N((C₁-C₆)-alkyl)₂,
      and wherein (C₁-C₆)-alkyl can be further mono-substituted by phenyl, phenylene-(C₁-C₄)-alkyl or phenylene-O-(C₁-C₄)-alkyl;
b) a bicyclic (C₆-C₁₁)-cycloalkyl group, which is mono-substituted by (C₀-C₂)-alkylene-NH₂, (C₀-C₂)-alkylene-NH-(C₁-C₄)-alkyl, (C₀-C₂)-alkylene-N((C₁-C₄)-alkyl)₂;
c) a fused bicyclic (C₆-C₁₀)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F;
d) a spiro bicylic (C₇C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
e) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
f) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 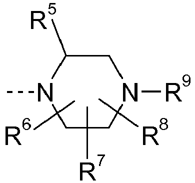 wherein
   - R⁵: is H or (C₀-C₆)-alkyl, which is unsubstituted or one to fivefold substituted by F, or mono-substituted by a substituent selected from the group consisting of
   i) O-(C₁-C₄)-alkyl, which is unsubstituted or one to fivefold substituted by F,
   ii) (C₃-C₆)-cycloalkyl,
   iii) phenyl, which is unsubstituted or substituted by one to two identical or different substituents selected from the group consisting o fhalogen, CF₃, O-(C₁-C₄)-alkyl, OCF₃, and (C₁-C₄)-alkyl,
   iv) a 5- to 6-membered monocyclic heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl,
   v) benzo[1,3]dioxole, and
   vi) CO-O-(C₁-C₄)-alkyl or CO- NH-(C₁-C₆)-alkyl;
   - R⁶: is H or (C₀-C₆)-alkyl, which is unsubstituted or one to fivefold substituted by F or mono-substituted by a substituent selected from the group consisting of
   i) CO-O-(C₁-C₄)-alkyl;
   ii) (C₃-C₆)-cycloalkyl,
   iii) phenyl, which is unsubstituted or substituted by one to two identical or different substituents selected from the group consisting of halogen, CF₃, O-(C₁-C₄)-alkyl, OCF₃, and (C₁-C₄)-alkyl,
   iv) O-(C₁-C₄)-alkyl, which is unsubstituted or one to fivefold substituted by F, and
   v) a 5- to 6-membered monocyclic heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl;
   - R⁷: is H, (C₁-C₆)-alkyl, which is unsubstituted or one to fivefold substituted by F, or phenyl;
   - R⁸: is H, (C₁-C₆)-alkyl or oxo (=O);
   - R⁹: is H, (C₁-C₆)-alkyl, (C₃-C₆)-cycloalkyl or CO-R¹⁰, where (C₁-C₆)-alkyl is unsubstituted or one to fivefold substituted by F, or mono-substituted by a substituent selected from the group consisting of O-(C₁-C₄)-alkyl, SO₂-(C₁-C₄)-alkyl, phenyl, a 5- to 6-membered monocyclic heterocyclic ring comprising one heteroatom selected from the group consisting of nitrogen and oxygen, a 5- to 6-membered monocyclic heteroaromatic ring comprising one heteroatom selected from the group consisting of nitrogen and oxygen; wherein
   R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen, wherein phenyl can be further mono-substituted by (C₁-C₄)-alkyl or O-(C₁-C₄)-alkyl;
b) a four to seven membered monocyclic heterocycloalkyl group containing a nitrogen atom, which is attached via said nitrogen and which is mono-substituted by (C₀-C₆)-alkylene-NH₂, (C₀-C₆)-alkylene-NH-(C₁-C₄)-alkyl, (C₀-C₆)-alkylene-NH-phenyl, (C₀-C₆)-alkylene-N((C₁-C₄)-alkyl)₂, (C₀-C₆)-alkylene-N((C₁-C₄)-alkyl)(phenyl), (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, which is unsubstituted or one to fivefold substituted by F, CO-O-(C₁-C₄)-alkyl or phenyl, and wherein said azetidinyl, pyrroldinyl and piperidyl group can be further mono-substituted by (C₁-C₆)-alkyl, which is unsubstituted or one to fivefold substituted by F;
c) a 1,4-diazepanyl, which is unsubstituted or mono-substituted by a substituent selected from the group consisting of
   i) (C₁-C₆)-alkyl, wherein (C₁-C₆)-alkyl is unsubstituted or one to fivefold substituted by F,
   ii) CO-(C₁-C₆)-alkyl, wherein (C₁-C₆)-alkyl is unsubstituted or one to fivefold substituted by F,
   ii) CO-phenyl, and
   iv) CO-pyridyl;
d) a fused bicyclic (C₆-C₁₀)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or mono- or di-substituted by F or (C₁-C₄)-alkyl, which is unsubstituted or one to fivefold substituted by F;
f) a bridged bicyclic (C₇-C₉)-heterocycloalkyl group containing one nitrogen atom, which is attached via said nitrogen atom, which is mono-substituted by (C₀-C₂)-alkylene-NH₂, (C₀-C₂)-alkylene-NH-(C₁-C₄)-alkyl, (C₀-C₂)-al kylene-N((C₁-C₄)-alkyl)₂
g) a bridged bicyclic (C₇-C₉)-heterocycloalkyl group containing two nitrogen atoms, which is attached via a nitrogen atom; and which is unsubstitued or substituted by one to four identical or different substituents selected from the group consisting of F, OH, and (C₁-C₄)-alkyl, which is unsubstituted or one to fivefold substituted by F; or
h) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
in any of its stereoisomeric forms, or a mixture of stereoisomeric forms in any ratio, or a physiologically acceptable salt thereof, or a physiologically acceptable solvate of any of them.

In another group of embodiments
- R¹: is H, halogen or (C₁-C₄)-alkyl;
- R²: is H or halogen;
- R³: is H or (C₁-C₄)-alkyl;
- R⁴: is
a) (C₀-C₄)-alkyl which is mono-substituted by
   i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
      ia) F,
      ib) (C₁-C₄)-alkyl, which is unsubstituted or one to fivefold substituted by F,
      ic) O-(C₁-C₄)-alkyl, which is unsubstituted or one to fivefold substituted by F,
      id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
      ie) (C₁-C₄)-alkylene-phenyl, which is unsubstituted or one to fivefold substituted by F,
      if) (C₃-C₈)- cycloalkyl,
      ig) oxo (=O), and
      ih) (CO)-(C₁-C₄)-alkyl,
         and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
   ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
      iia) F,
      iib) (C₁-C₄)-alkyl, wherein (C₁-C₄)-alkyl is unsubstituted or one to fivefold substituted by F;
b) a spiro bicylic (C₇-C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
c) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
d) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 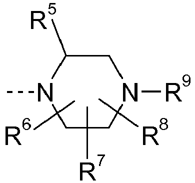 wherein
   - R⁵: is H or (C₁-C₆)-alkyl, which is unsubstituted or one to fivefold substituted by F, or mono-substituted by a substituent selected from the group consisting of
   i) O-(C₁-C₄)-alkyl,
   ii) (C₃-C₆)-cycloalkyl,
   iii) phenyl,
   iv) a 5- to 6-membered monocyclic heteroaromatic ring comprising one heteroatom selected from the group consisting of nitrogen, oxygen, and sulphur;
   - R⁶: is H or (C₁-C₆)-alkyl, which is unsubstituted or one to fivefold substituted by F;
   - R⁷: is H, (C₁-C₆)-alkyl;
   - R⁸: is H, (C₁-C₆)-alkyl or oxo (=O);
   - R⁹: is H, (C₁-C₆)-alkyl, (C₃-C₆)-cycloalkyl or CO-R¹⁰, where (C₁-C₆)-alkyl is unsubstituted or one to fivefold substituted by F, or mono-substituted O-(C₁-C₄)-alkyl ; wherein
   R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen;
b) a four to seven membered monocyclic heterocycloalkyl group containing one nitrogen atom, which is attached via said nitrogen and which is mono-substituted by (C₀-C₆)-alkylene-NH₂, (C₀-C₆)-alkylene-NH-(C₁-C₄)-alkyl, (C₀-C₆)-alkylene-N((C₁-C₄)-alkyl)₂, (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, and wherein said azetidinyl, pyrroldinyl and piperidyl group can be further mono-substituted by (C₁-C₆)-alkyl,;
c) a 1,4-diazepanyl, which is unsubstituted or mono-substituted by a substituent selected from the group consisting of
   i) (C₁-C₆)-alkyl, wherein (C₁-C₆)-alkyl is unsubstituted or one to fivefold substituted by F,
   ii) CO-(C₁-C₆)-alkyl, wherein (C₁-C₆)-alkyl is unsubstituted or one to fivefold substituted by F;
d) a fused bicyclic (C₆-C₁₀)-heterocycloalkyl group containing two nitrogen atoms, which is attached via a nitrogen atom, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further oxygen atom, wherein said heterocycloalkyl group is unsubstituted or mono- or di-substituted by F or (C₁-C₄)-alkyl, which is unsubstituted or one to fivefold substituted by F;
   or
f) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F.

In another group of embodiments
- R¹: is H or halogen;
- R²: is H;
- R³: is H or (C₁-C₄)-alkyl;
- R⁴: is
a) (C₀-C₄)-alkyl which is mono-substituted by
   i) a 3- to 8-membered monocyclic heterocycle comprising a ring nitrogen, which is unsubstituted or substituted by one to four identical or different substituents selected from the group consisting of (C₁-C₄)-alkyl, O-(C₁-C₄)-alkyl, (C₁-C₄)-alkylene-phenyl, (C₁-C₄)-alkylene-pyridyl, (C₃-C₈)-cycloalkyl, oxo (=O), and (CO)-(C₁-C₄)-alkyl;
   ii) (C₃-C₈)-cycloalkyl which is monosubstituted by NH₂;
b) a spiro bicylic (C₉)-heterocycloalkyl group containing one nitrogen atom, which is attached via a carbon atom;
c) a brigded bicyclic (C₈)-heterocycloalkyl group containing one nitrogen atom, which is attached via a carbon atom;
d) a tricyclic (C₁₄)-heterocycloalkyl group containing one nitrogen atom, which is attached via a carbon atom and which consists of a spiro bicyclic ring with an additional fused phenyl ring;
or
R³ and R⁴ together with the N-atom carrying them denote
a) a 1,4-piperazinyl of the formula 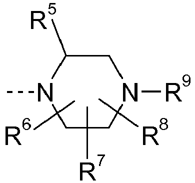 wherein
   - R⁵: is H or (C₁-C₆)-alkyl;
   - R⁶: is H or (C₁-C₆)-alkyl;
   - R⁷: is H or (C₁-C₆)-alkyl;
   - R⁸: is H or oxo (=O);
   - R⁹: is H, (C₁-C₆)-alkyl, (C₃-C₆)-cycloalkyl or CO-R¹⁰, where (C₁-C₆)-alkyl is unsubstituted or mono-substituted by O-(C₁-C₄)-alkyl;
   - R¹⁰: is (C₁-C₄)-alkyl, (C₃-C₆)-cycloalkyl, a 5- to 6-membered aromatic or aliphatic heterocyclic ring comprising one heteroatom selected from nitrogen or oxygen;
b) 1-azetidinyl, which is mono-substituted by NH₂;
c) 1-pyrrolidinyl, which is mono-substituted by NH₂, NH-(C₁-C₄)-alkyl, NH(C₁-C₄)-alkyl)₂, (C₀-C₂)-alkylene-pyrrolidinyl or (C₀-C₂)-piperidinyl which is unsubstituted or monosubstituted by (C₁-C₄)-alkyl;
d) 1-piperidyl, which is mono-substituted by NH₂;
e) 1,4-diazepanyl of the formula 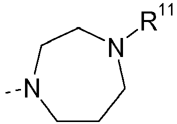 wherein
   - R¹¹: is H, (C₁-C₆)-alkyl or CO-(C₁-C₄)-alkyl;
f) a fused bicyclic (C₈-C₁₀)-heterocycloalkyl group containing two nitrogen atoms, which is attached via a nitrogen;
g) a spiro bicylic (C₈-C₁₁)-heterocylcoalkyl group containing two nitrogen atoms, which is attached via a nitrogen atom and which can contain one further oxygen atom;
h) a tricyclic (C₁₄)-heterocycloalkyl group containing two nitrogen atoms, which is attached via a nitrogen atom and which consists of a spiro bicyclic ring with an additional fused phenyl ring.

In onother group of embodiments
- R¹: is H or F;
- R²: is H;
- R³: is H or CH₃;
- R⁴: is
a) (C₀-C₁)-alkyl which is mono-substituted by
   i) azetidyl, pyrrolidinyl or piperidyl, which are unsubstituted or substituted by one to four identical or different substituents selected from the group consisting of CH₃, C₂H₅, O-CH₃, methylene-phenyl, methylene-pyridyl, cyclohexyl, oxo (=O), and (CO)-CH₃;
   ii) (C₃-C₆)-cycloalkyl which is monosubstituted by NH₂;
b) a spiro bicyclic ring 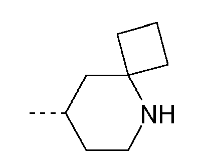
c) a bridged bicyclic ring selected from the group consisting of 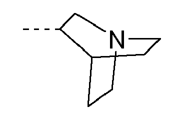
d) a spiro bicyclic ring with a fused ring selected from the group consisting of 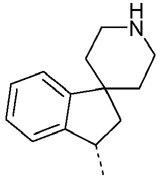
or
R³ and R⁴ together with the N-atom carrying them denote
a) a 1,4-piperazinyl of the formula 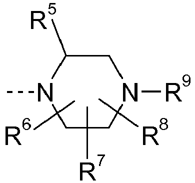 wherein
   - R⁵: is H or CH₃;
   - R⁶: is H or CH₃;
   - R⁷: is H or (C₁-C₄)-alkyl;
   - R⁸: is H or oxo (=O);
   - R⁹: is H, (C₁-C₃)-alkyl, cycloheptyl or CO-R¹⁰, where (C₁-C₃)-alkyl is unsubstituted or mono-substituted by O-CH₃;
   - R¹⁰: is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 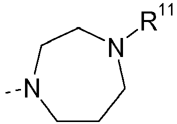 wherein
   - R¹¹: is H, ethyl or CO-CH₃;
f) a fused bicyclic ring selected from the group consisting of 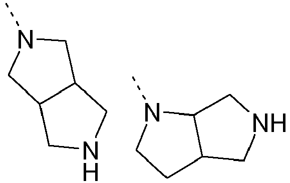
g) a spiro bicyclic ring selected from the group consisting of 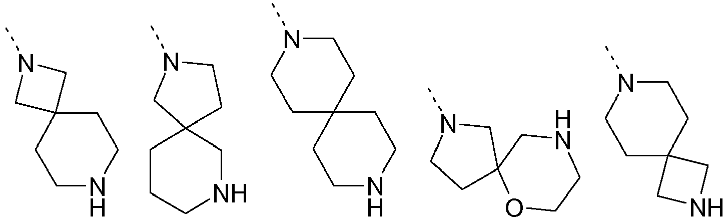 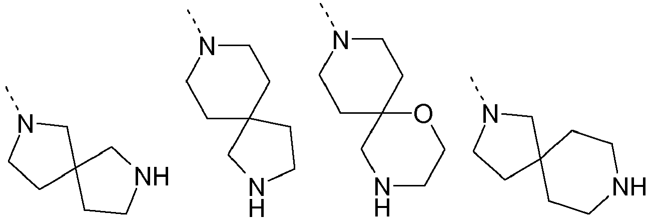 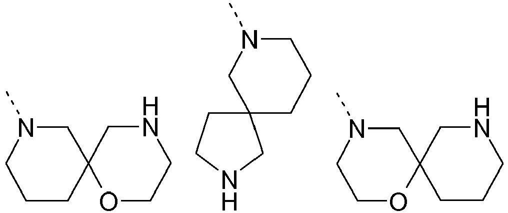
h) a bridged bicyclic ring selected from the group consisting of 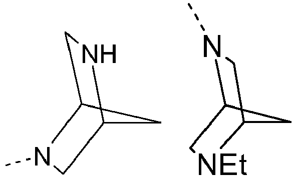
i) a spiro bicyclic ring with a fused ring selected from the group consisting of

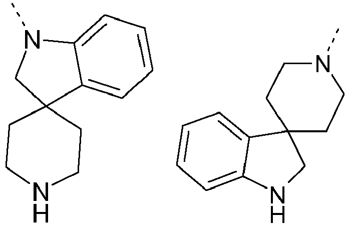

Another group of embodiments are compounds of the formula I wherein
- R¹: is H, halogen or (C₁-C₄)-alkyl; or R¹ is H or halogen; or R¹ is H, F, Cl or CH₃, or R¹ is H, F or Cl, or R¹ is H or F; or R¹ is H.

Another group of embodiments are compounds of the formula I wherein
- R²: is H, halogen or (C₁-C₄)-alkyl; or R² is H or halogen; or R² is H, F, Cl or CH₃, or R² is H, F or Cl, or R² is H or F, or R² is H.

Another group of embodiments are compounds of the formula I wherein
- R³: is H or (C₁-C₄)-alkyl; or R³ is H or CH₃; or R³ is H.

Another group of embodiments are compounds of the formula I wherein
- R⁴: is
a) (C₀-C₆)-alkyl which is mono-substituted by
   i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
      ia) F,
      ib) (C₁-C₄)-alkyl, which is unsubstituted or one to fivefold substituted by F,
      ic) O-(C₁-C₄)-alkyl, which is unsubstituted or one to fivefold substituted by F,
      id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
      ie) (C₁-C₄)-alkylene-phenyl, which is unsubstituted or one to fivefold substituted by F,
      if) (C₃-C₈)- cycloalkyl,
      ig) oxo (=O), and
      ih) (CO)-(C₁-C₄)-alkyl,
         and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
   ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
      iia) F,
      iib) (C₁-C₄)-alkyl, wherein (C₁-C₄)-alkyl is unsubstituted or one to fivefold substituted by F,
      iic) O-(C₁-C₄)-alkyl, which is unsubstituted or one to fivefold substituted by F,
      iid) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F, and
      iie) (C₁-C₄)-alkylene-phenyl, which is unsubstituted or one to fivefold substituted by F;
      or
   iii) NH₂, NH(C₁-C₆)-alkyl, or N((C₁-C₆)-alkyl)₂,
      and wherein (C₁-C₆)-alkyl can be further mono-substituted by phenyl, phenylene-(C₁-C₄)-alkyl or phenylene-O-(C₁-C₄)-alkyl;
b) a bicyclic (C₆-C₁₁)-cycloalkyl group, which is mono-substituted by (C₀-C₂)-alkylene-NH₂, (C₀-C₂)-alkylene-NH-(C₁-C₄)-alkyl, (C₀-C₂)-alkylene-N((C₁-C₄)-alkyl)₂;
c) a fused bicyclic (C₆-C₁₀)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F;
d) a spiro bicylic (C₇-C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
e) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
f) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or R⁴ is
a) (C₀-C₄)-alkyl which is mono-substituted by
   i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
      ia) F,
      ib) (C₁-C₄)-alkyl, which is unsubstituted or one to fivefold substituted by F,
      ic) O-(C₁-C₄)-alkyl, which is unsubstituted or one to fivefold substituted by F,
      id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
      ie) (C₁-C₄)-alkylene-phenyl, which is unsubstituted or one to fivefold substituted by F,
      if) (C₃-C₈)- cycloalkyl,
      ig) oxo (=O), and
      ih) (CO)-(C₁-C₄)-alkyl,
         and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
   ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
      iia) F,
      iib) (C₁-C₄)-alkyl, wherein (C₁-C₄)-alkyl is unsubstituted or one to fivefold substituted by F;
b) a spiro bicylic (C₇-C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
c) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
d) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or R⁴ is
a) (C₀-C₄)-alkyl which is mono-substituted by
   i) a 3- to 8-membered monocyclic heterocycle comprising a ring nitrogen, which is unsubstituted or substituted by one to four identical or different substituents selected from the group consisting of (C₁-C₄)-alkyl, O-(C₁-C₄)-alkyl, (C₁-C₄)-alkylene-phenyl, (C₁-C₄)-alkylene-pyridyl, (C₃-C₈)-cycloalkyl, oxo (=O), and (CO)-(C₁-C₄)-alkyl;
   ii) (C₃-C₈)-cycloalkyl which is monosubstituted by NH₂;
b) a spiro bicylic (C₉)-heterocycloalkyl group containing one nitrogen atom, which is attached via a carbon atom;
c) a brigded bicyclic (C₈)-heterocycloalkyl group containing one nitrogen atom, which is attached via a carbon atom;
d) a tricyclic (C₁₄)-heterocycloalkyl group containing one nitrogen atom, which is attached via a carbon atom and which consists of a spiro bicyclic ring with an additional fused phenyl ring;
or R⁴ is
a) (C₀-C₁)-alkyl which is mono-substituted by
   i) azetidyl, pyrrolidinyl or piperidyl, which are unsubstituted or substituted by one to four identical or different substituents selected from the group consisting of CH₃, C₂H₅, O-CH₃, methylene-phenyl, methylene-pyridyl, cyclohexyl, oxo (=O), and (CO)-CH₃;
   ii) (C₃-C₆)-cycloalkyl which is monosubstituted by NH₂;
b) a spiro bicyclic ring 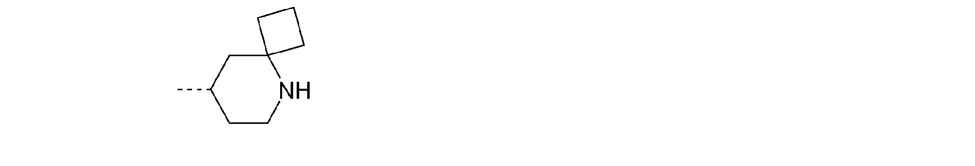
c) a bridged bicyclic ring selected from the group consisting of 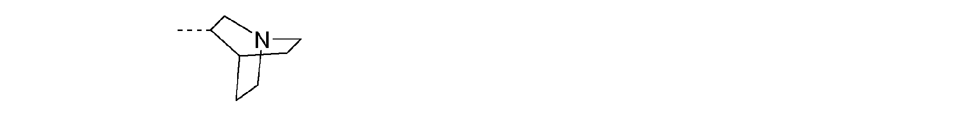
d) a spiro bicyclic ring with a fused ring selected from the group consisting of 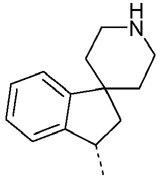
or R⁴ is
a) methylene which is mono-substituted by
   i) azetidyl or pyrrolidinyl, which are attached by a carbon atom and which are unsubstituted or substituted by oxo (=O);
   ii) cyclohexyl which is monosubstituted by NH₂;
b) azetidyl which is attached by a carbon atom and which is unsubstituted or substituted by CH_{3;}
c) pyrrolidinyl, which is attached by a carbon atom and which is unsubstituted or substituted by O-CH₃;
d) piperidyl, which is attached by a carbon atom and which is unsubstituted or substituted by one to four identical or different substituents selected from the group consisting of CH₃, C₂H₅, methylene-phenyl, methylene-pyridyl, cyclohexyl, oxo (=O), and (CO)-CH₃;
e) (C₃-C₆)-cycloalkyl which is monosubstituted by NH₂;
f) a spiro bicyclic ring 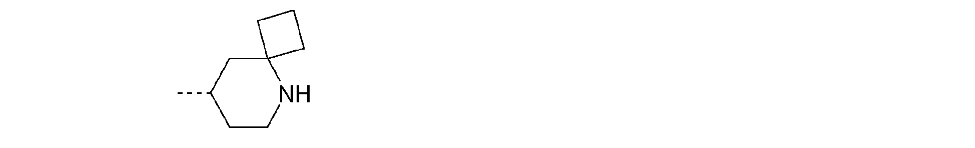
g) a bridged bicyclic ring selected from the group consisting of 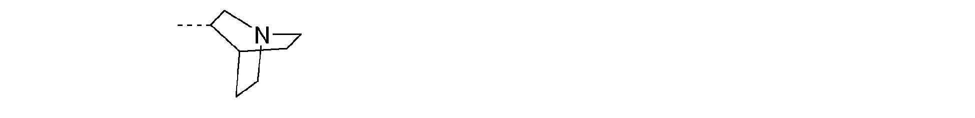
h) a spiro bicyclic ring with a fused ring selected from the group consisting of 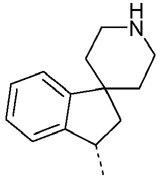
or R⁴ is
a) methylene which is mono-substituted by
   i) 3-azetidyl;
   ii) cyclohexyl which is monosubstituted by NH₂;
b) azetidyl which is attached by a carbon atom;
c) pyrrolidinyl, which is attached by a carbon atom;
d) piperidyl, which is attached by a carbon atom and which is unsubstituted or substituted by one substituent selected from the group consisting of CH₃, C₂H₅, methylene-phenyl, methylene-pyridyl, and cyclohexyl;
e) cyclohexyl which is monosubstituted by NH₂;
f) a spiro bicyclic ring 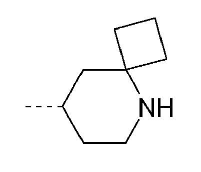
or R⁴ is
a) methylene which is mono-substituted by
   i) 3-azetidyl;
   ii) 4-amino-cyclohexyl;
b) 3-azetidyl;
c) 3-pyrrolidinyl;
d) 3-piperidyl or 4-piperidyl, which are unsubstituted or substituted at the nitrogen atom by one substituent selected from the group consisting of CH₃, C₂H₅, methylene-phenyl, methylene-pyridyl, and cyclohexyl;
e) 3-amino-cyclohexyl or 4-amino-cylohexyl;
f) a spiro bicyclic ring

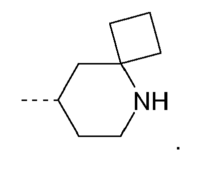

Another group of embodiments are compounds of the formula I wherein
R³ and R⁴ together with the N-atom carrying them denote
a) a 1,4-piperazinyl of the formula 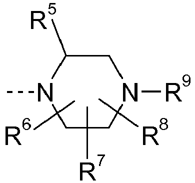 wherein
   - R⁵: is H or (C₀-C₆)-alkyl, which is unsubstituted or one to fivefold substituted by F, or mono-substituted by a substituent selected from the group consisting of
   i) O-(C₁-C₄)-alkyl, which is unsubstituted or one to fivefold substituted by F,
   ii) (C₃-C₆)-cycloalkyl,
   iii) phenyl, which is unsubstituted or substituted by one to two identical or different substituents selected from the group consisting o fhalogen, CF₃, O-(C₁-C₄)-alkyl, OCF₃, and (C₁-C₄)-alkyl,
   iv) a 5- to 6-membered monocyclic heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl,
   v) benzo[1,3]dioxole, and
   vi) CO-O-(C₁-C₄)-alkyl or CO- NH-(C₁-C₆)-alkyl;
   - R⁶: is H or (C₀-C₆)-alkyl, which is unsubstituted or one to fivefold substituted by F or mono-substituted by a substituent selected from the group consisting of
   i) CO-O-(C₁-C₄)-alkyl;
   ii) (C₃-C₆)-cycloalkyl,
   iii) phenyl, which is unsubstituted or substituted by one to two identical or different substituents selected from the group consisting of halogen, CF₃, O-(C₁-C₄)-alkyl, OCF₃, and (C₁-C₄)-alkyl,
   iv) O-(C₁-C₄)-alkyl, which is unsubstituted or one to fivefold substituted by F, and
   v) a 5- to 6-membered heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl;
   - R⁷: is H, (C₁-C₆)-alkyl, which is unsubstituted or one to fivefold substituted by F, or phenyl;
   - R⁸: is H, (C₁-C₆)-alkyl or oxo (=O);
   - R⁹: is H, (C₁-C₆)-alkyl, (C₃-C₆)-cycloalkyl or CO-R¹⁰, where (C₁-C₆)-alkyl is unsubstituted or one to fivefold substituted by F, or mono-substituted by a substituent selected from the group consisting of O-(C₁-C₄)-alkyl, SO₂-(C₁-C₄)-alkyl, phenyl, a 5- to 6-membered monocyclic heterocyclic ring comprising one heteroatom selected from the group consisting of nitrogen and oxygen, a 5- to 6-membered monocyclic heteroaromatic ring comprising one heteroatom selected from the group consisting of nitrogen and oxygen; wherein
   R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen, wherein phenyl can be further mono-substituted by (C₁-C₄)-alkyl or O-(C₁-C₄)-alkyl;
b) a four to seven membered monocyclic heterocycloalkyl group containing a nitrogen atom, which is attached via said nitrogen and which is mono-substituted by (C₀-C₆)-alkylene-NH₂, (C₀-C₆)-alkylene-NH-(C₁-C₄)-alkyl, (C₀-C₆)-alkylene-NH-phenyl, (C₀-C₆)-alkylene-N((C₁-C₄)-alkyl)₂, (C₀-C₆)-alkylene-N((C₁-C₄)-alkyl)(phenyl), (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, which is unsubstituted or one to fivefold substituted by F, CO-O-(C₁-C₄)-alkyl or phenyl, and wherein said azetidinyl, pyrroldinyl and piperidyl group can be further mono-substituted by (C₁-C₆)-alkyl, which is unsubstituted or one to fivefold substituted by F;
c) a 1,4-diazepanyl, which is unsubstituted or mono-substituted by a substituent selected from the group consisting of
   i) (C₁-C₆)-alkyl, wherein (C₁-C₆)-alkyl is unsubstituted or one to fivefold substituted by F,
   ii) CO-(C₁-C₆)-alkyl, wherein (C₁-C₆)-alkyl is unsubstituted or one to fivefold substituted by F,
   ii) CO-phenyl, and
   iv) CO-pyridyl;
d) a fused bicyclic (C₆-C₁₀)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or mono- or di-substituted by F or (C₁-C₄)-alkyl, which is unsubstituted or one to fivefold substituted by F;
f) a bridged bicyclic (C₇-C₉)-heterocyc!oa!ky! group containing one nitrogen atom, which is attached via said nitrogen atom, which is mono-substituted by (C₀-C₂)-alkylene-NH₂, (C₀-C₂)-alkylene-NH-(C₁-C₄)-alkyl, (C₀-C₂)-alkylene-N((C₁-C₄)-alkyl)₂
g) a bridged bicyclic (C₇-C₉)-heterocycloalkyl group containing two nitrogen atoms, which is attached via a nitrogen atom; and which is unsubstitued or substituted by one to four identical or different substituents selected from the group consisting of F, OH, and (C₁-C₄)-alkyl, which is unsubstituted or one to fivefold substituted by F; or
h) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 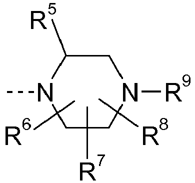 wherein
   - R⁵: is H or (C₁-C₆)-alkyl, which is unsubstituted or one to fivefold substituted by F, or mono-substituted by a substituent selected from the group consisting of
   i) O-(C₁-C₄)-alkyl,
   ii) (C₃-C₆)-cycloalkyl,
   iii) phenyl,
   iv) a 5- to 6-membered monocyclic heteroaromatic ring comprising one heteroatom selected from the group consisting of nitrogen, oxygen, and sulphur;
   - R⁶: is H or (C₁-C₆)-alkyl, which is unsubstituted or one to fivefold substituted by F;
   - R⁷: is H, (C₁-C₆)-alkyl;
   - R⁸: is H, (C₁-C₆)-alkyl or oxo (=O);
   - R⁹: is H, (C₁-C₆)-alkyl, (C₃-C₆)-cycloalkyl or CO-R¹⁰, where (C₁-C₆)-alkyl is unsubstituted or one to fivefold substituted by F, or mono-substituted O-(C₁-C₄)-alkyl ; wherein
   R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen;
b) a four to seven membered monocyclic heterocycloalkyl group containing one nitrogen atom, which is attached via said nitrogen and which is mono-substituted by (C₀-C₆)-aikyiene-NH₂, (C₀-C₆)-alkylene-NH-(C₁-C₄)-alkyl, (C₀-C₆)-alkylene-N((C₁-C₄)-alkyl)₂, (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, and wherein said azetidinyl, pyrroldinyl and piperidyl group can be further mono-substituted by (C₁-C₆)-alkyl,;
c) a 1,4-diazepanyl, which is unsubstituted or mono-substituted by a substituent selected from the group consisting of
   i) (C₁-C₆)-alkyl, wherein (C₁-C₆)-alkyl is unsubstituted or one to fivefold substituted by F,
   ii) CO-(C₁-C₆)-alkyl, wherein (C₁-C₆)-alkyl is unsubstituted or one to fivefold substituted by F;
d) a fused bicyclic (C₆-C₁₀)-heterocycloalkyl group containing two nitrogen atoms, which is attached via a nitrogen atom, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further oxygen atom, wherein said heterocycloalkyl group is unsubstituted or mono- or di-substituted by F or (C₁-C₄)-alkyl, which is unsubstituted or one to fivefold substituted by F;
   or
f) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 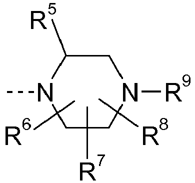 wherein
   - R⁵: is H or (C₁-C₆)-alkyl;
   - R⁶: is H or (C₁-C₆)-alkyl;
   - R⁷: is H or (C₁-C₆)-alkyl;
   - R⁸: is H or oxo (=O);
   - R⁹: is H, (C₁-C₆)-alkyl, (C₃-C₆)-cycloalkyl or CO-R¹⁰, where (C₁-C₆)-alkyl is unsubstituted or mono-substituted by O-(C₁-C₄)-alkyl;
   - R¹⁰: is (C₁-C₄)-alkyl, (C₃-C₆)-cycloalkyl, a 5- to 6-membered aromatic or aliphatic heterocyclic ring comprising one heteroatom selected from nitrogen or oxygen;
b) 1-azetidinyl, which is mono-substituted by NH₂;
c) 1-pyrrolidinyl, which is mono-substituted by NH₂, NH-(C₁-C₄)-alkyl, NH(C₁-C₄)-alkyl)₂, (C₀-C₂)-alkylene-pyrrolidinyl or (C₀-C₂)-piperidinyl which is unsubstituted or monosubstituted by (C₁-C₄)-alkyl;
d) 1-piperidyl, which is mono-substituted by NH₂;
e) 1,4-diazepanyl of the formula 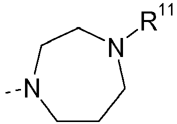 wherein
   - R¹¹: is H, (C₁-C₆)-alkyl or CO-(C₁-C₄)-alkyl;
f) a fused bicyclic (C₈-C₁₀)-heterocycloalkyl group containing two nitrogen atoms, which is attached via a nitrogen;
g) a spiro bicylic (C₈-C₁₁)-heterocylcoalkyl group containing two nitrogen atoms, which is attached via a nitrogen atom and which can contain one further oxygen atom;
h) a tricyclic (C₁₄)-heterocycloalkyl group containing two nitrogen atoms, which is attached via a nitrogen atom and which consists of a spiro bicyclic ring with an additional fused phenyl ring;
or
R³ and R⁴ together with the N-atom carrying them denote
a) a 1,4-piperazinyl of the formula 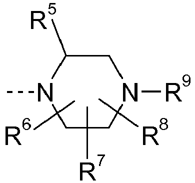 wherein
   - R⁵: is H or CH₃;
   - R⁶: is H or CH₃;
   - R⁷: is H or (C₁-C₄)-alkyl;
   - R⁸: is H or oxo (=O);
   - R⁹: is H, (C₁-C₃)-alkyl, cycloheptyl or CO-R¹⁰, where (C₁-C₃)-alkyl is unsubstituted or mono-substituted by O-CH₃;
   - R¹⁰: is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 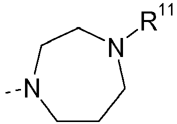 wherein
   - R¹¹: is H, ethyl or CO-CH₃;
f) a fused bicyclic ring selected from the group consisting of 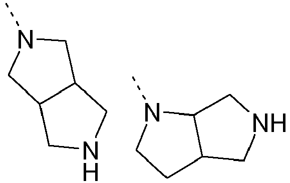
g) a spiro bicyclic ring selected from the group consisting of 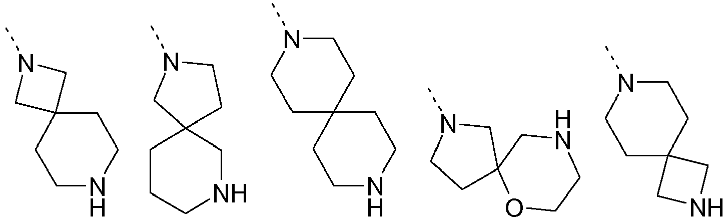 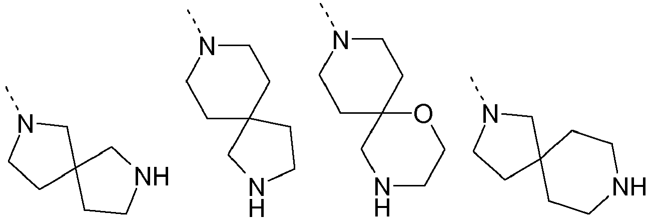 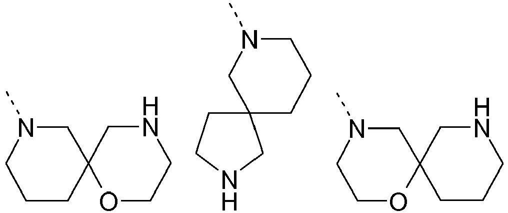
h) a bridged bicyclic ring selected from the group consisting of 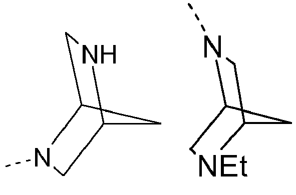
i) a spiro bicyclic ring with a fused ring selected from the group consisting of 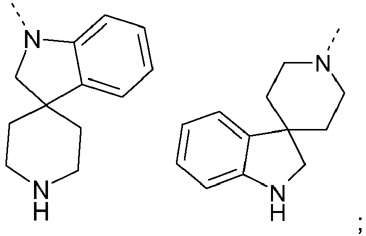
or
R³ and R⁴ together with the N-atom carrying them denote
a) a 1,4-piperazinyl of the formula 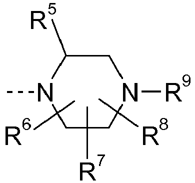 wherein
   - R⁵: is H or CH₃;
   - R⁶: is H or CH₃;
   - R⁷: is H or (C₁-C₄)-alkyl;
   - R⁸: is H or oxo (=O);
   - R⁹: is H, (C₁-C₃)-alkyl, cycloheptyl or CO-R¹⁰, where (C₁-C₃)-alkyl is unsubstituted or mono-substituted by O-CH₃;
   - R¹⁰: is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 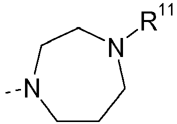 wherein
   - R¹¹: is H, ethyl or CO-CH₃;
f) a fused bicyclic ring selected from the group consisting of 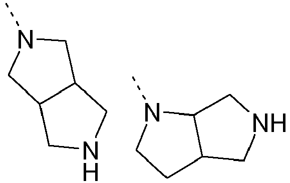
g) a spiro bicyclic ring selected from the group consisting of 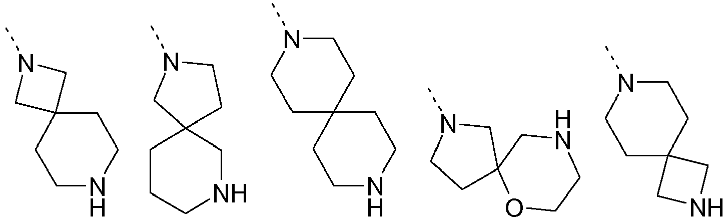 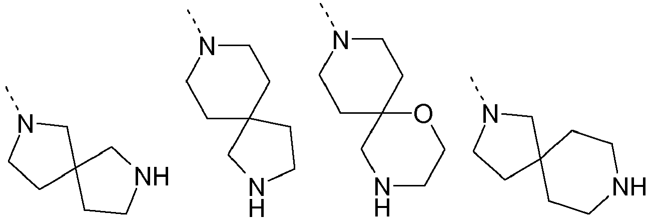 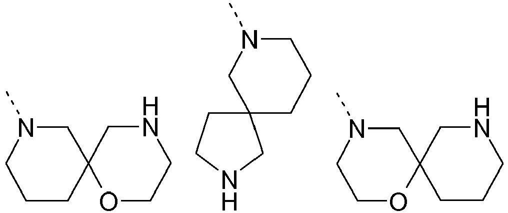
h) a bridged bicyclic ring selected from the group consisting of 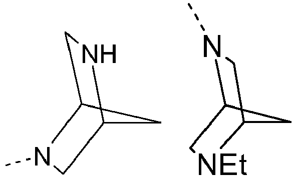
i) a spiro bicyclic ring with a fused ring selected from the group consisting of 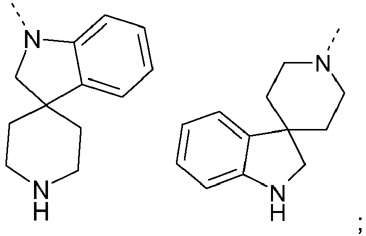
or
R³ and R⁴ together with the N-atom carrying them denote
a) a 1,4-piperazinyl of the formula 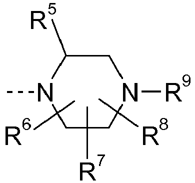 wherein
   - R⁵: is H or CH₃;
   - R⁶: is H or CH₃;
   - R⁷: is H or (C₁-C₄)-alkyl;
   - R⁸: is H;
   - R⁹: is H or (C₁-C₃)-alkyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 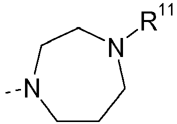 wherein
   - R¹¹: is H or ethyl;
f) a fused bicyclic ring selected from the group consisting of 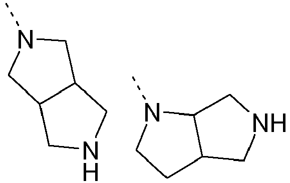
g) a spiro bicyclic ring selected from the group consisting of 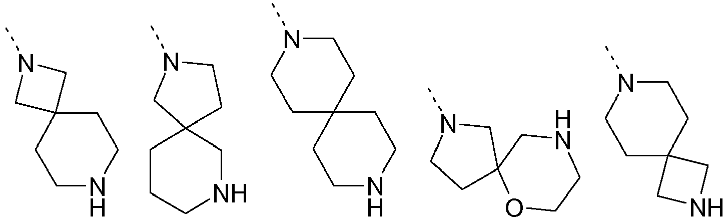 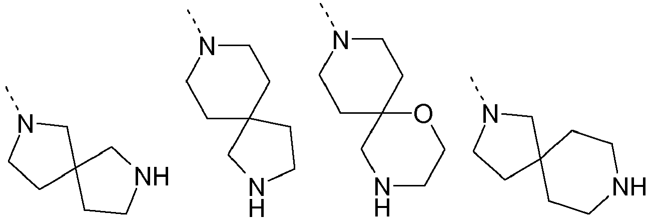 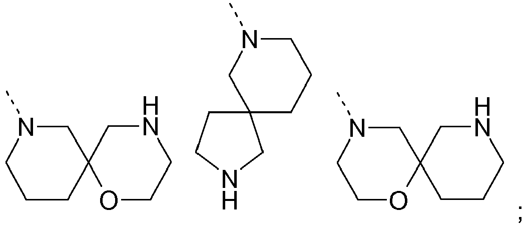
or
R³ and R⁴ together with the N-atom carrying them denote
a) a 1,4-piperazinyl of the formula 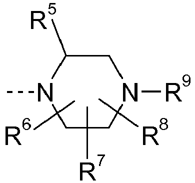 wherein
   - R⁵: is H or CH₃;
   - R⁶: is H or CH₃;
   - R⁷: is H or iso-propyl;
   - R⁸: is H;
   - R⁹: is H or (C₁-C₃)-alkyl;
b) 3-amino-1-azetidinyl;
c) 1-pyrrolidinyl, which is mono-substituted in 3-position by NH₂, NH(CH₃), NH(CH₃)₂, or 1-piperidinyl-4-methyl;
d) 4-amino-1-piperidyl;
e) a 1,4-diazepanyl of the formula 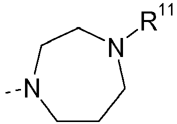 wherein
   - R¹¹: is H or ethyl;
f) a fused bicyclic ring selected from the group consisting of 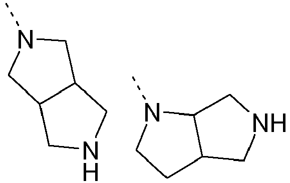
g) a spiro bicyclic ring selected from the group consisting of

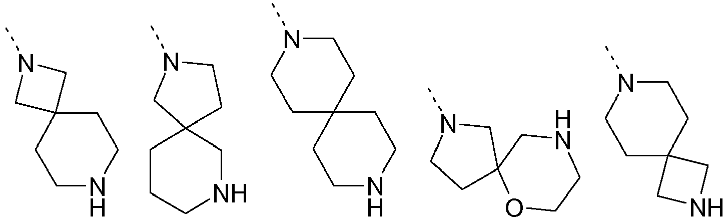

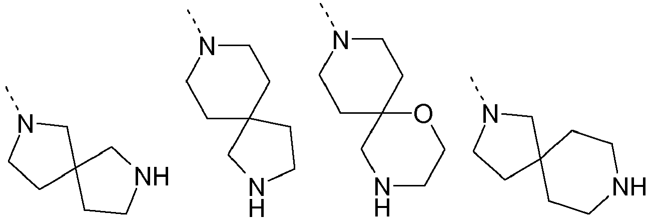

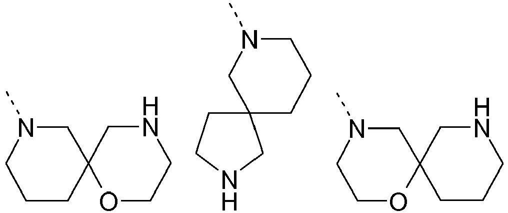

In another group of embodiments
- R¹: is H or F;
- R²: is H;
- R³: is H or CH₃;
- R⁴: is
a) methylene which is mono-substituted by
   i) azetidyl or pyrrolidinyl, which are attached by a carbon atom and which are unsubstituted or substituted by oxo (=O);
   ii) cyclohexyl which is monosubstituted by NH₂;
b) azetidyl which is attached by a carbon atom and which is unsubstituted or substituted by CH_{3;}
c) pyrrolidinyl, which is attached by a carbon atom and which is unsubstituted or substituted by O-CH₃;
d) piperidyl, which is attached by a carbon atom and which is unsubstituted or substituted by one to four identical or different substituents selected from the group consisting of CH₃, C₂H₅, methylene-phenyl, methylene-pyridyl, cyclohexyl, oxo (=O), and (CO)-CH₃;
e) (C₃-C₆)-cycloalkyl which is monosubstituted by NH_{2;}
f) a spiro bicyclic ring 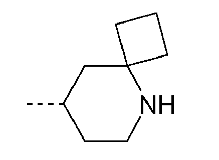
g) a bridged bicyclic ring selected from the group consisting of 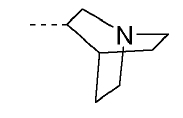
h) a spiro bicyclic ring with a fused ring selected from the group consisting of 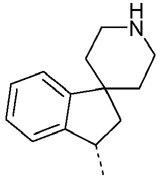
or
R³ and R⁴ together with the N-atom carrying them denote
a) a 1,4-piperazinyl of the formula 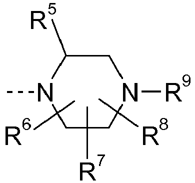 wherein
   - R⁵: is H or CH₃;
   - R⁶: is H or CH₃;
   - R⁷: is H or (C₁-C₄)-alkyl;
   - R⁸: is H or oxo (=O);
   - R⁹: is H, (C₁-C₃)-alkyl, cycloheptyl or CO-R¹⁰, where (C₁-C₃)-alkyl is unsubstituted or mono-substituted by O-CH₃;
   - R¹⁰: is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 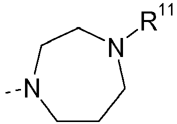 wherein
   - R¹¹: is H, ethyl or CO-CH₃;
f) a fused bicyclic ring selected from the group consisting of 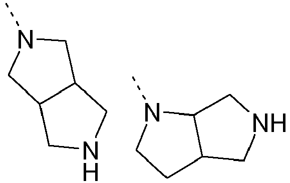
g) a spiro bicyclic ring selected from the group consisting of 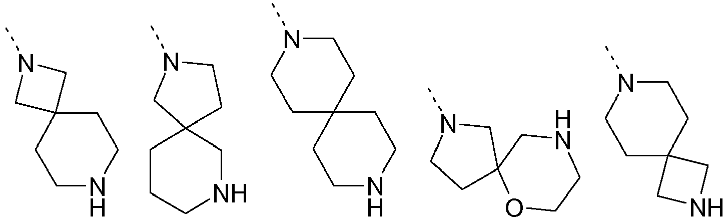 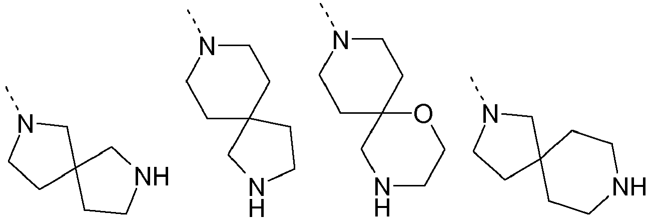 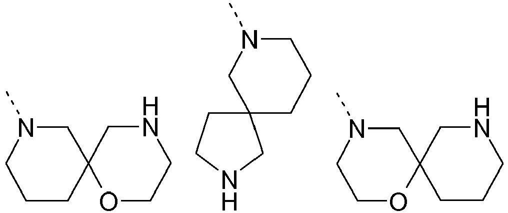
h) a bridged bicyclic ring selected from the group consisting of 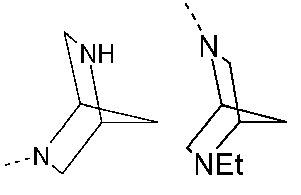
i) a spiro bicyclic ring with a fused ring selected from the group consisting of

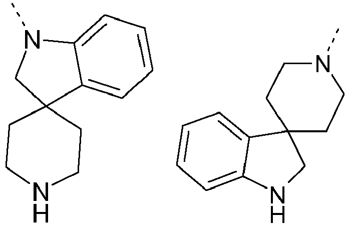

In another group of embodiments
- R¹: is H or F;
- R²: is H;
- R³: is H or CH₃;
- R⁴: is
a) methylene which is mono-substituted by
   i) 3-azetidyl;
   ii) cyclohexyl which is monosubstituted by NH₂;
b) azetidyl which is attached by a carbon atom;
c) pyrrolidinyl, which is attached by a carbon atom;
d) piperidyl, which is attached by a carbon atom and which is unsubstituted or substituted by one substituent selected from the group consisting of CH₃, C₂H₅, methylene-phenyl, methylene-pyridyl, and cyclohexyl;
e) cyclohexyl which is monosubstituted by NH_{2;}
f) a spiro bicyclic ring 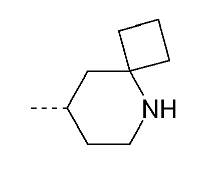
or
R³ and R⁴ together with the N-atom carrying them denote
a) a 1,4-piperazinyl of the formula 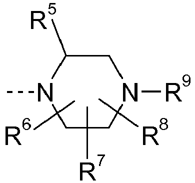 wherein
   - R⁵: is H or CH₃;
   - R⁶: is H or CH₃;
   - R⁷: is H or (C₁-C₄)-alkyl;
   - R⁸: is H;
   - R⁹: is H or (C₁-C₃)-alkyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 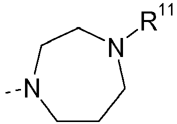 wherein
   - R¹¹: is H or ethyl;
f) a fused bicyclic ring selected from the group consisting of 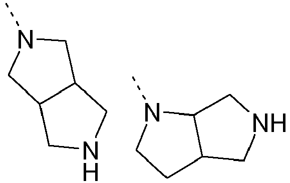
g) a spiro bicyclic ring selected from the group consisting of

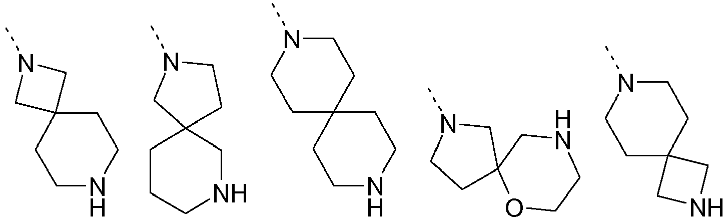

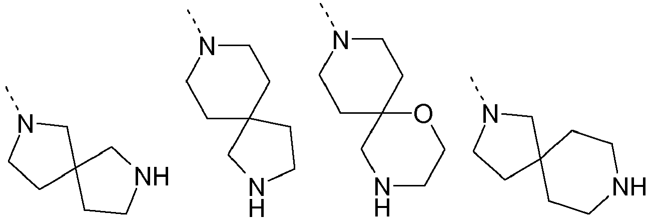

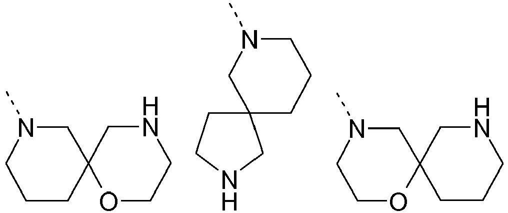

In another group of embodiments
- R¹: is H;
- R²: is H;
- R³: is H;
- R⁴: is
a) methylene which is mono-substituted by
   i) 3-azetidyl;
   ii) 4-amino-cyclohexyl;
b) 3-azetidyl_{;}
c) 3-pyrrolidinyl;
d) 3-piperidyl or 4-piperidyl, which are unsubstituted or substituted at the nitrogen atom by one substituent selected from the group consisting of CH₃, C₂H₅, methylene-phenyl, methylene-pyridyl, and cyclohexyl;
e) 3-amino-cyclohexyl or 4-amino-cylohexyl_{;}
f) a spiro bicyclic ring 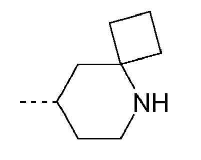
or
R³ and R⁴ together with the N-atom carrying them denote
a) a 1,4-piperazinyl of the formula 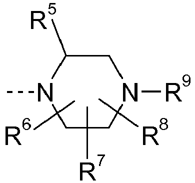 wherein
   - R⁵: is H or CH₃;
   - R⁶: is H or CH₃;
   - R⁷: is H or iso-propyl;
   - R⁸: is H;
   - R⁹: is H or (C₁-C₃)-alkyl;
b) 3-amino-1-azetidinyl;
c) 1-pyrrolidinyl, which is mono-substituted in 3-position by NH₂, NH(CH₃), NH(CH₃)₂, or 1-piperidinyl-4-methyl;
d) 4-amino-1-piperidyl;
e) a 1,4-diazepanyl of the formula 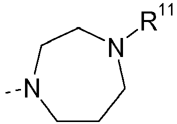 wherein
   - R¹¹: is H or ethyl;
f) a fused bicyclic ring selected from the group consisting of 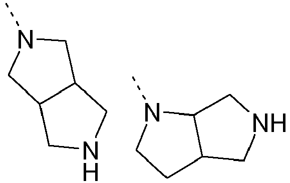
g) a spiro bicyclic ring selected from the group consisting of

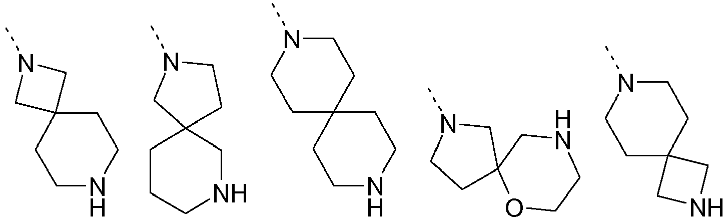

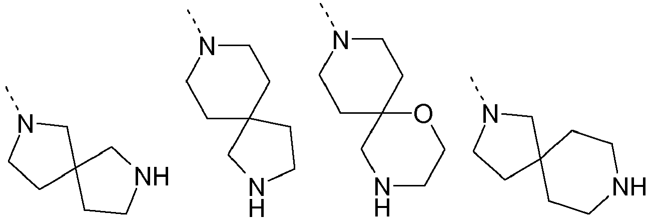

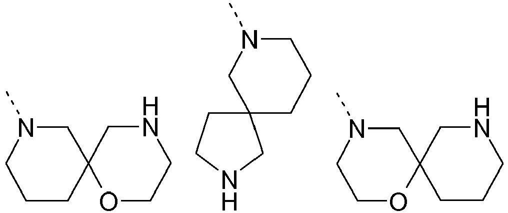

In another embodiment compounds of the formula I are encompassed selected from the group consisting of

| | |
|---|---|
| 1 | 6-(4-Hydroxy-phenyl)-1H-pyrazolo[3,4-b]pyridine-4-carboxylic acid (2,2,6,6-tetramethyl-piperidin-4-yl)-amide |
| 2 | 6-(4-Hydroxy-phenyl)-1H-pyrazolo[3,4-b]pyridine-4-carboxylic acid piperidin-4-ylamide |
| 3 | (3-Amino-piperidin-1-yl)-[6-(4-hydroxy-phenyl)-1H-pyrazolo[3,4-b]pyridin-4-yl]-methanone |
| 4 | [6-(4-Hydroxy-phenyl)-1H-pyrazolo[3,4-b]pyridin-4-yl]-piperazin-1-yl-methanone |
| 5 | 6-(4-Hydroxy-phenyl)-1 H-pyrazolo[3,4-b]pyridine-4-carboxylic acid pyrrolidin-3-ylamide |
| 6 | 6-(4-Hydroxy-phenyl)-1H-pyrazolo[3,4-b]pyridine-4-carboxylic acid ((3S,4S)-4-methoxy-pyrrolidin-3-yl)-amide |
| 7 | [1,4]Diazepan-1-yl-[6-(4-hydroxy-phenyl)-1H-pyrazolo[3,4-b]pyridin-4-yl]-methanone |
| 8 | 6-(4-Hydroxy-phenyl)-1H-pyrazolo[3,4-b]pyridine-4-carboxylic acid (cis-4-amino-cyclohexyl)-amide |
| 9 | 6-(4-Hydroxy-phenyl)-1H-pyrazolo[3,4-b]pyridine-4-carboxylic acid (trans-4-amino-cyclohexyl)-amide |
| 10 | (2,2-Dimethyl-piperazin-1-yl)-[6-(4-hydroxy-phenyl)-1H-pyrazolo[3,4-b]pyridin-4-yl]-methanone |
| 11 | 6-(4-Hydroxy-phenyl)-1H-pyrazolo[3,4-b]pyridine-4-carboxylic acid (trans-4-amino-cyclohexylmethyl)-amide |
| 12 | 6-(4-Hydroxy-phenyl)-1H-pyrazolo[3,4-b]pyridine-4-carboxylic acid (cis-4-amino-cyclohexylmethyl)-amide |
| 13 | 6-(4-Hydroxy-phenyl)-1H-pyrazolo[3,4-b]pyridine-4-carboxylic acid (azetidin-3-ylmethyl)-amide |
| 14 | 6-(4-Hydroxy-phenyl)-1H-pyrazolo[3,4-b]pyridine-4-carboxylic acid (3-amino-cyclobutyl)-amide |
| 15 | [6-(4-Hydroxy-phenyl)-1H-pyrazolo[3,4-b]pyridin-4-yl]-(5-isopropyl-2,2-dimethyl-piperazin-1-yl)-methanone |
| 16 | ((R)-3-Amino-pyrrolidin-1-yl)-[6-(4-hydroxy-phenyl)-1H-pyrazolo[3,4-b]pyridin-4-yl]-methanone |
| 17 | ((S)-3-Amino-pyrrolidin-1-yl)-[6-(4-hydroxy-phenyl)-1H-pyrazolo[3,4-b]pyridin-4-yl]-methanone |
| 18 | 6-(4-Hydroxy-phenyl)-1H-pyrazolo[3,4-b]pyridine-4-carboxylic acid-(R)-(1-Aza-bicyclo[2.2.2]oct-3-yl)amide |
| 19 | 6-(4-Hydroxy-phenyl)-1H-pyrazolo[3,4-b]pyridine-4-carboxylic acid (R)-piperidin-3-ylamide |
| 20 | (2,7-Diaza-spiro[3.5]non-2-yl)-[6-(4-hydroxy-phenyl)-1H-pyrazolo[3,4-b]pyridin-4-yl]-methanone |
| 21 | (2,7-Diaza-spiro[3.5]non-7-yl)-[6-(4-hydroxy-phenyl)-1H-pyrazolo[3,4-b]pyridin-4-yl]-methanone |
| 22 | (2,8-Diaza-spiro[4.5]dec-2-yl)-[6-(4-hydroxy-phenyl)-1H-pyrazolo[3,4-b]pyridin-4-yl]-methanone |
| 23 | (2,7-Diaza-spiro[4.5]dec-2-yl)-[6-(4-hydroxy-phenyl)-1H-pyrazolo[3,4-b]pyridin-4-yl]-methanone |
| 24 | (2,7-Diaza-spiro[4.5]dec-7-yl)-[6-(4-hydroxy-phenyl)-1H-pyrazolo[3,4-b]pyridin-4-yl]-methanone |
| 25 | (2,7-Diaza-spiro[4.4]non-2-yl)-[6-(4-hydroxy-phenyl)-1H-pyrazolo[3,4-b]pyridin-4-yl]-methanone |
| 26 | [6-(4-Hydroxy-phenyl)-1H-pyrazolo[3,4-b]pyridin-4-yl]-(1-oxa-4,9-diaza-spiro[5.5]undec-9-yl)-methanone |
| 27 | [6-(4-Hydroxy-phenyl)-1H-pyrazolo[3,4-b]pyridin-4-yl]-(1-oxa-4,8-diaza-spiro[5.5]undec-4-yl)-methanone |
| 28 | [6-(4-Hydroxy-phenyl)-1H-pyrazolo[3,4-b]pyridin-4-yl]-(1-oxa-4,8-diaza-spiro[5.5]undec-8-yl)-methanone |
| 29 | 6-(4-Hydroxy-phenyl)-1H-pyrazolo[3,4-b]pyridine-4-carboxylic acid (trans-4-methoxy-pyrrolidin-3-yl)-amide |
| 30 | 6-(4-Hydroxy-phenyl)-1H-pyrazolo[3,4-b]pyridine-4-carboxylic acid (trans-3-amino-cyclobutyl)-amide |
| 31 | [6-(4-Hydroxy-phenyl)-1H-pyrazolo[3,4-b]pyridin-4-yl]-(6-oxa-2,9-diaza-spiro[4.5]dec-2-yl)-methanone |
| 32 | 6-(4-Hydroxy-phenyl)-1H-pyrazolo[3,4-b]pyridine-4-carboxylic acid (5-aza-spiro[3.5]non-8-yl)-amide |
| 33 | 6-(4-Hydroxy-phenyl)-1H-pyrazolo[3,4-b]pyridine-4-carboxylic acid (3-amino-cyclohexyl)-amide |
| 34 | (Hexahydro-pyrrolo[3,4-b]pyrrol-1-yl)-[6-(4-hydroxy-phenyl)-1H-pyrazolo[3,4-b]pyridin-4-yl]-methanone |
| 35 | 6-(4-Hydroxy-phenyl)-1H-pyrazolo[3,4-b]pyridine-4-carboxylic acid azetidin-3-ylamide |
| 36 | (3-Amino-azetidin-1-yl)-[6-(4-hydroxy-phenyl)-1 H-pyrazolo[3,4-b]pyridin-4-yl]-methanone |
| 37 | [6-(4-Hydroxy-phenyl)-1H-pyrazolo[3,4-b]pyridin-4-yl]-(3-methyl-piperazin-1-yl)-methanone |
| 38 | (4-Amino-piperidin-1-yl)-[6-(4-hydroxy-phenyl)-1 H-pyrazolo[3,4-b]pyridin-4-yl]-methanone |
| 39 | [6-(4-Hydroxy-phenyl)-1H-pyrazolo[3,4-b]pyridin-4-yl]-(3-methylamino-pyrrolidin-1-yl)-methanone |
| 40 | 6-(4-Hydroxy-phenyl)-1H-pyrazolo[3,4-b]pyridine-4-carboxylic acid-(S)-(1-Aza-bicyclo[2.2.2]oct-3-yl)amide |
| 41 | 6-(4-Hydroxy-phenyl)-1H-pyrazolo[3,4-b]pyridine-4-carboxylic acid (1-methyl-piperidin-3-yl)-amide |
| 42 | 6-(4-Hydroxy-phenyl)-1H-pyrazolo[3,4-b]pyridine-4-carboxylic acid methyl-piperidin-4-yl-amide |
| 43 | (2,8-Diaza-spiro[4.5]dec-8-yl)-[6-(4-hydroxy-phenyl)-1H-pyrazolo[3,4-b]pyridin-4-yl]-methanone |
| 44 | (3,9-Diaza-spiro[5.5]undec-3-yl)-[6-(4-hydroxy-phenyl)-1H-pyrazolo[3,4-b]pyridin-4-yl]-methanone |
| 45 | (3-Amino-pyrrolidin-1-yl)-[6-(4-hydroxy-phenyl)-1 H-pyrazolo[3,4-b]pyridin-4-yl]-methanone |
| 46 | (Hexahydro-pyrrolo[3,4-c]pyrrol-2-yl)-[6-(4-hydroxy-phenyl)-1H-pyrazolo[3,4-b]pyridin-4-yl]-methanone |
| 47 | 2,5-Diaza-bicyclo[2.2.1]hept-2-yl-[6-(4-hydroxy-phenyl)-1H-pyrazolo[3,4-b]pyridin-4-yl]-methanone |
| 48 | 6-(4-Hydroxy-phenyl)-1H-pyrazolo[3,4-b]pyridine-4-carboxylic acid (2,3-dihydro-spiro[1H-indene-1,4'-piperidin]-3-yl)-amide |
| 49 | 6-(4-Hydroxy-phenyl)-1H-pyrazolo[3,4-b]pyridine-4-carboxylic acid (1,2-dihydrospiro[3H-indole-3,4'-piperidin]-1'-yl)-amide |
| 50 | 6-(4-hydroxy-phenyl)-1H-pyrazolo[3,4-b]pyridine-4-carboxylic acid (2,3-dihydrospiro[1H-indene-1,4'-piperidin]-3-yl)-amide |
| 51 | 6-(4-Hydroxy-phenyl)-1H-pyrazolo[3,4-b]pyridine-4-carboxylic acid (trans-2-amino-cyclopropyl)-amide |
| 52 | [6-(4-Hydroxy-phenyl)-1H-pyrazolo[3,4-b]pyridin-4-yl]-(4-methyl-piperazin-1-yl)-methanone |
| 53 | 1-{4-[6-(4-Hydroxy-phenyl)-1H-pyrazolo[3,4-b]pyridine-4-carbonyl]-piperazin-1-yl}-ethanone |
| 54 | [6-(4-Hydroxy-phenyl)-1 H-pyrazolo[3,4-b]pyridin-4-yl]-((S)-2-pyrrolidin-1-ylmethyl-pyrrolidin-1-yl)-methanone |
| 55 | ((S)-3-Dimethylamino-pyrrolidin-1-yl)-[6-(4-hydroxy-phenyl)-1H-pyrazolo[3,4-b]pyridin-4-yl]-methanone |
| 56 | [6-(4-Hydroxy-phenyl)-1H-pyrazolo[3,4-b]pyridin-4-yl]-(4-isopropyl-piperazin-1-yl)-methanone |
| 57 | (4-Cyclopropanecarbonyl-piperazin-1-yl)-[6-(4-hydroxy-phenyl)-1H-pyrazolo[3,4-b]pyridin-4-yl]-methanone |
| 58 | (4-Cycloheptyl-piperazin-1-yl)-[6-(4-hydroxy-phenyl)-1H-pyrazolo[3,4-b]pyridin-4-yl]-methanone |
| 59 | 1-{4-[6-(4-Hydroxy-phenyl)-1H-pyrazolo[3,4-b]pyridine-4-carbonyl]-[1,4]diazepan-1-yl}-ethanone |
| 60 | 6-(4-Hydroxy-phenyl)-1H-pyrazolo[3,4-b]pyridine-4-carboxylic acid (1-ethyl-piperidin-3-yl)-amide |
| 61 | [6-(4-Hydroxy-phenyl)-1H-pyrazolo[3,4-b]pyridin-4-yl]-[4-(tetrahydrofuran-2-carbonyl)-piperazin-1-yl]-methanone |
| 62 | 6-(4-Hydroxy-phenyl)-1H-pyrazolo[3,4-b]pyridine-4-carboxylic acid (1-methyl-azetidin-3-yl)-amide |
| 63 | 6-(4-Hydroxy-phenyl)-1H-pyrazolo[3,4-b]pyridine-4-carboxylic acid (5-oxo-pyrrolidin-2-ylmethyl)-amide |
| 64 | (4-Ethyl-[1,4]diazepan-1-yl)-[6-(4-hydroxy-phenyl)-1H-pyrazolo[3,4-b]pyridin-4-yl]-methanone |
| 65 | [6-(4-Hydroxy-phenyl)-1H-pyrazolo[3,4-b]pyridin-4-yl]-[3-(4-methyl-piperidin-1-yl)-pyrrolidin-1-yl]-methanone |
| 66 | 6-(4-Hydroxy-phenyl)-1H-pyrazolo[3,4-b]pyridine-4-carboxylic acid (1-methyl-piperidin-4-yl)-amide |
| 67 | 6-(4-Hydroxy-phenyl)-1H-pyrazolo[3,4-b]pyridine-4-carboxylic acid (1-cyclohexyl-piperidin-4-yl)-amide |
| 68 | 6-(4-Hydroxy-phenyl)-1H-pyrazolo[3,4-b]pyridine-4-carboxylic acid (1-acetyl-piperidin-4-yl)-amide |
| 69 | 6-(4-Hydroxy-phenyl)-1H-pyrazolo[3,4-b]pyridine-4-carboxylic acid ((R)-6-oxo-piperidin-3-yl)-amide |
| 70 | (5-Ethyl-2,5-diaza-bicyclo[2.2.1]hept-2-yl)-[6-(4-hydroxy-phenyl)-1H-pyrazolo[3,4-b]pyridin-4-yl]-methanone |
| 71 | [6-(4-Hydroxy-phenyl)-1H-pyrazolo[3,4-b]pyridin-4-yl]-[4-(2-methoxy-ethyl)-piperazin-1-yl]-methanone |
| 72 | 6-(4-Hydroxy-phenyl)-1H-pyrazolo[3,4-b]pyridine-4-carboxylic acid (1-aza-bicyclo[2.2.2]oct-3-yl)-amide |
| 73 | 6-(4-Hydroxy-phenyl)-1H-pyrazolo[3,4-b]pyridine-4-carboxylic acid methyl-(1-methyl-piperidin-4-yl)-amide |
| 74 | [6-(4-Hydroxy-phenyl)-1H-pyrazolo[3,4-b]pyridin-4-yl]-[4-(pyridine-3-carbonyl)-piperazin-1-yl]-methanone |
| 75 | 6-(4-Hydroxy-phenyl)-1H-pyrazolo[3,4-b]pyridine-4-carboxylic acid (1-pyridin-4-ylmethyl-piperidin-4-yl)-amide |
| 76 | 4-[6-(4-Hydroxy-phenyl)-1H-pyrazolo[3,4-b]pyridine-4-carbonyl]-1-methyl-piperazin-2-one |
| 77 | 6-(4-Hydroxy-phenyl)-1H-pyrazolo[3,4-b]pyridine-4-carboxylic acid ((R)-1-benzyl-piperidin-3-yl)-amide |
| 78 | 6-(4-Hydroxy-phenyl)-1H-pyrazolo[3,4-b]pyridine-4-carboxylic acid (1-pyridin-3-ylmethyl-piperidin-4-yl)-amide |

Structural elements such as groups, substituents, hetero ring members, numbers or other features, for example alkyl groups, groups like R¹, R², R³ etc., which can occur several times in the compounds of the formula I, can all independently of one another have any of the indicated meanings and can in each case be identical to or different from one another. For example, the alkyl groups in a dialkylamino group can be identical or different.

As used here, the terms "including" and "comprising" are used in their open, nonlimiting sense. As used herein, the terms "(C₁-C₈)" or "(C₅-C₈)" and so forth, refer to moieties having 1 to 8 or 5 to 8 carbon atoms, respectively. Within terms like "(C₀-C₆)-alkyl" or "(C₀-C₆)-alkylen" "C₀-alkyl" or "(C₀)-alkylen" refer to a bond, or in case of an unsubstituted "(C₀)-alkyl" it refers to a hydrogen.

The term "alkyl", as used herein, refers to saturated, monovalent hydrocarbon radicals. The term "alkenyl", as used herein, refers to monovalent hydrocarbon radicals, which contain at least one carbon-carbon double bond, wherein each double bond can have E- or Z-configuration. The term "alkinyl", as used herein, refers to monovalent hydrocarbon radicals, which contain at least one carbon-carbon triple bond. The alkyl, alkenyl and alkynyl groups can be linear, i.e. straight-chain, or branched. This also applies when they are part of other groups, for example alkyloxy groups (= alkoxy groups, O-alkylgroups), alkyloxycarbonyl groups or alkyl-substituted amino groups, or when they are substituted. Depending on the respective definition, the number of carbon atoms in an alkyl group can be 1, 2, 3, 4, 5, 6, 7 or 8, or 1, 2, 3, 4, 5 or 6, or 1, 2, 3 or 4, or 1, 2 or 3. Examples of alkyl are methyl, ethyl, propyl including n-propyl and isopropyl, butyl including n-butyl, sec-butyl, isobutyl and tert-butyl, pentyl including n-pentyl, 1-methylbutyl, isopentyl, neopentyl and tert-pentyl, hexyl including n-hexyl, 3,3-dimethylbutyl and isohexyl, heptyl and octyl. Double bonds and triple bonds in alkenyl groups and alkynyl groups can be present in any positions. In one embodiment of the invention, alkenyl groups contain one double bond and alkynyl groups contain one triple bond. In one embodiment of the invention, an alkenyl group or alkynyl group contains at least three carbon atoms and is bonded to the remainder of the molecule via a carbon atom which is not part of a double bond or triple bond. Examples of alkenyl and alkynyl are ethenyl, prop-1-enyl, prop-2-enyl (= allyl), but-2-enyl, 2-methylprop-2-enyl, 3-methylbut-2-enyl, hex-3-enyl, hex-4-enyl, prop-2-ynyl (= propargyl), but-2-ynyl, but-3-ynyl, hex-4-ynyl or hex-5-ynyl. Substituted alkyl groups, alkenyl groups and alkynyl groups can be substituted in any positions, provided that the respective compound is sufficiently stable and is suitable for the desired purpose such as use as a drug substance. The prerequisite that a specific group and a compound of the formula I are sufficiently stable and suitable for the desired purpose such as use as a drug substance, applies in general with respect to the definitions of all groups in the compounds of the formula I.

Independently of one another and independently of any other substituents, alkyl groups, divalent alkyl groups, alkenyl groups, alkynyl groups, cycloalkyl groups and heterocycloalkyl groups are optionally substituted by one or more fluorine substituents which can be located in any positions, i.e., the said groups can be unsubstituted by fluorine substituents or substituted by fluorine substituents, for example by 1, 2, 3, 4, 5, 6, 7, 8, 9, 10, 11, 12 or 13, or by 1, 2, 3, 4, 5, 6, 7, 8 or 9, or by 1, 2, 3, 4, 5, 6 or 7, or by 1, 2, 3, 4 or 5, or by 1, 2 or 3, or by 1 or 2, fluorine substituents. Examples of fluorine-substituted said groups are trifluoromethyl, 2-fluoroethyl, 2,2,2-trifluoroethyl, pentafluoroethyl, 3,3,3-trifluoropropyl, 2,2,3,3,3-pentafluoropropyl, 4,4,4-trifluorobutyl, heptafluoroisopropyl, -CHF-, -CF₂-, -CF₂-CH₂-, -CH₂-CF₂-, -CF₂-CF₂-, -CF(CH₃)-, -C(CF₃)₂-, -C(CH₃)₂-CF₂-, -CF₂-C(CH₃)₂-, 1-fluoro-cyclopropyl, 2,2-difluorocyclopropyl, 3,3-difluorocyclobutyl, 1-fluorocyclohexyl, 4,4-difluorocyclohexyl, 3,3,4,4,5,5-hexafluorocyclohexyl. Examples of alkyloxy groups in which the alkyl moiety is fluorine-substituted, are trifluoromethoxy, 2,2,2-trifluoro-ethoxy, pentafluoroethoxy and 3,3,3-trifluoropropoxy.

The term "alkanediyl" or "alkylene" ", as used herein, refers to saturated, divalent hydrocarbon radicals. The term "alkenediyl", as used herein, refers to divalent hydrocarbon radicals, which contain at least one carbon-carbon double bond, wherein each double bond can have E- or Z-configuration. The term "alkindiyl", as used herein, refers to divalent hydrocarbon radicals, which contain at least one carbon-carbon triple bond. As far as applicable, the preceding explanations regarding alkyl, alkenyl and alkinyl groups apply correspondingly to alkanediyl, alkendiyl and alkindiyl groups, which thus can likewise be linear and branched. Examples of divalent alkyl groups are -CH₂- (= methylene), -CH₂-CH₂-, -CH₂-CH₂-CH₂-, -CH₂-CH₂-CH₂-CH₂-, -CH(CH₃)-,-C(CH₃)₂-, -CH(CH₃)-CH₂-, -CH₂-CH(CH₃)-, -C(CH₃)₂-CH₂-, -CH₂-C(CH₃)₂-.

The term "cycloalkyl", as used herein, unless otherwise indicated, refers to a monovalent radical of a saturated or partially saturated hydrocarbon ring system, which can be monocyclic, bicyclic or tricyclic, i.e. which can contain one, two or three rings. The bicyclic or tricyclic ring system can be a fused ring system, in which two adjacent rings share two adjacent carbon atoms. The bicyclic or tricyclic ring system can be a spiro ring system or a di-spiro-ringsystem, in which two adjacent rings share a single carbon atom. The tricyclic ring system can also be a bicyclic spiro ring system, to which another ring is fused, that means that the latter ring and the ring in the spiro ring system, to which it is attached, share two adjacent carbon atoms; herein the latter ring can be an aromatic, saturated or partially saturated ring. The bicyclic or tricyclic system can also be a non-fused or bridged ring system, in which two adjacent rings share two non-adjacent carbon atoms. The bicyclic or tricyclic ring can be attached by any ring atom except a spiro- or a bridgehead atom.

In a monocyclic cycloalkyl group the number of ring carbon atoms can be 3, 4, 5, 6, 7 or 8. In one embodiment of the invention, the number of ring carbon atoms in a cycloalkyl group, independently of the number of ring carbon atoms in any other cycloalkyl group, is 3, 4, 5 or 6, in another embodiment 3, 4 or 5, in another embodiment 3 or 4, in another embodiment 3, in another embodiment 5, 6 or 7, in another embodiment 5 or 6, in another embodiment 6 or 7, in another embodiment 5, in another embodiment 6. Examples of cycloalkyl groups are cyclopropyl, cyclobutyl, cyclopentyl, cyclohexyl, cycloheptyl and cyclooctyl.

In a bicyclic cycloalkyl group the number of ring carbon atoms can be 6, 7, 8, 9, 10, 11 or 12. In one embodiment of the invention, the number of ring carbon atoms in a bicyclic cycloalkyl group can be 7, 8, 9, 10 or 11, in another embodiment 8, 9 or 10. In a tricyclic cycloalkyl group the number of ring carbon atoms can be 7, 8, 9, 10, 11, 12, 13, 14 or 15. In one embodiment of the invention, the number of ring carbon atoms in a tricyclic cycloalkyl group can be 10, 11 or 12.

Exemplary bicyclic or tricyclic fused ring cycloalkyls are derived from, but not limited to, the following ring systems: bicyclo[3.1.0]hexane, bicyclo[4.1.0]heptane, bicycle-[5.1.0]octane, bicyclo[3.2.0]heptane, bicyclo[4.2.0]octane, octahydro-pentalene, octa-hydro-indene, decahydro-azulene, decahydro-naphthalene, decahydro-benzo-cycloheptene, dodecahydro-heptalene, 1,2,3,3a,4,6a-hexahydro-pentalene, 1,2,3,4-tetrahydro-pentalene, 2,3,3a,4,5,7a-hexahydro-1 H-indene, 2,3,3a,4,7,7a-hexahydro-1H-indene, 3a,4,5,6,7,7a-hexahydro-1H-indene, 4,5,6,7-tetrahydro-1H-indene, indane, 1,2,3,4,4a,5,6,8a-octahydro-naphthalene, 1,2,3,4,4a,5,8,8a-octahydro-naphthalene, 1,2,4a,5,8,8a-hexahydro-naphthalene, 1,4,4a,5,8,8a-hexahydro-naphthalene, 1,2,3,4-tetrahydro-naphthalene, 2,3,4,4a,5,6,9,9a-octahydro-1 H-benzocycloheptene, 2,3,4,4a,5,9a-hexahydro-1 H-benzocycloheptene, 4,4a,5,6,7,8,9,9a-octahydro-1H-benzocycloheptene, 6,7,8,9-tetrahydro-5H-benzocycloheptene, 1,2,3,4,5,5a,6,7,8,10a-decahydro-heptalene, dodecahydro-as-indacene and 2,3,3a,4,9,9a-hexahydro-1 H-cyclopenta[b]naphthalene:

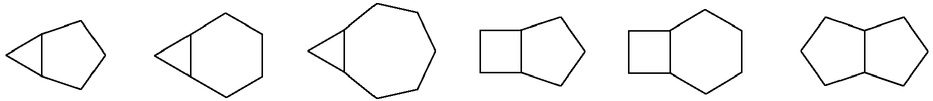

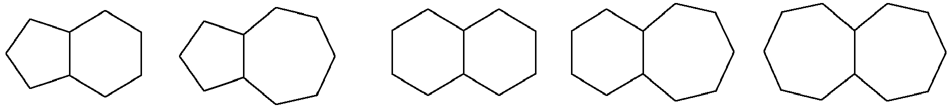

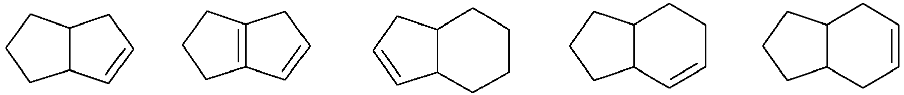

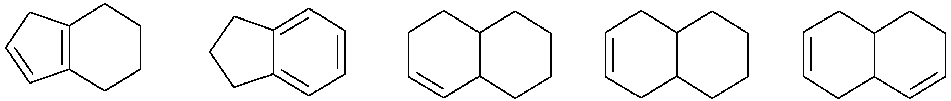

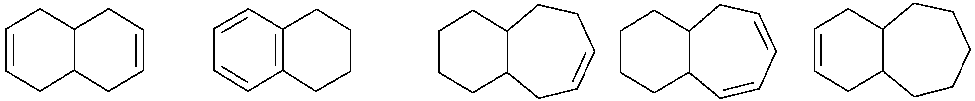

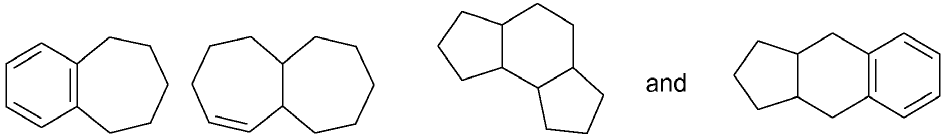

Exemplary bicyclic or tricyclic spiro ring cycloalkyls are derived from, but not limited to, the following ring systems: spiro[2.4]heptane, spiro[2.5]octane, spiro[2.6]nonane, spiro[3.3]heptane, spiro[3.4]octane, spiro[3.5]nonane, spiro[3.6]decane, spiro[4.4]nonane, spiro[4.5]decane, spiro[4.6]undecane, spiro[5.5]undecane, spiro[5.6]dodecane, spiro[6.6]tridecane, dispiro[2.2.4.2]dodecane, dispiro[2.2.3.2]undecane, dispiro-[2.1.4.2]undecane and spiro[5.5]undec-2-ene:

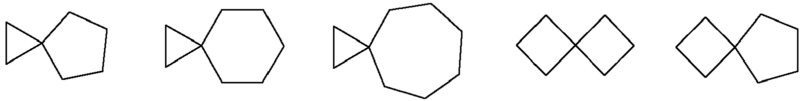

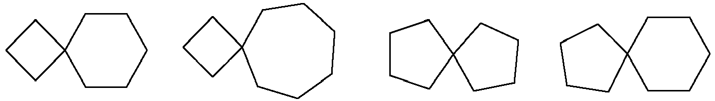

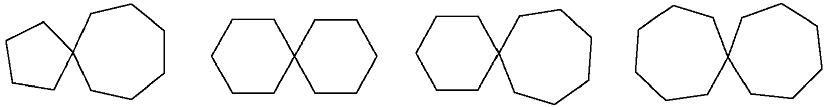

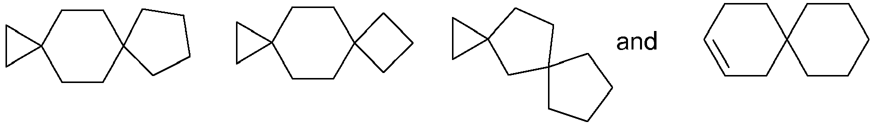

Exemplary cycloalkyls, in which a ring is fused to one ring of a bicyclic spiro system, are derived from, but not limited to, the following ring systems:

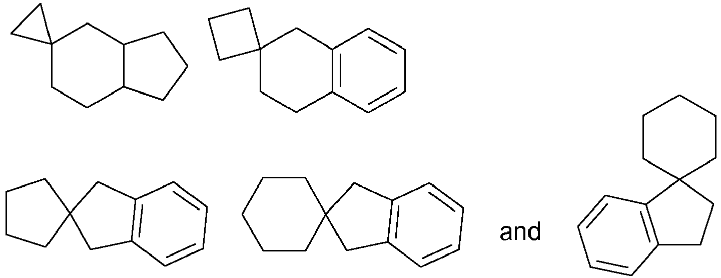

Exemplary non-fused or bridged bicyclic or tricyclic ring cycloalkyls are derived from, but not limited to, the following ring systems: bicyclo[2.2.1]heptane, bicyclo[2.2.2]octane, bicyclo[3.2.1]octane, bicyclo[3.2.2]nonane and adamantane.

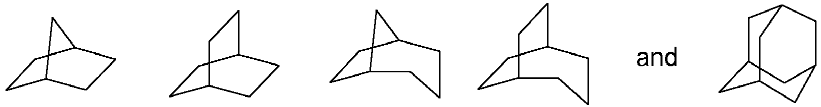

The term "heterocycloalkyl" or "heterocyclyl", as used herein, unless otherwise indicated, refers to a cycloalkyl as defined above, in which 1, 2, 3 or 4 carbon atoms are replaced by nitrogen, oxygen or sulfur atoms, provided that a spiro atom is always a carbon atom and a bridgehead atom is either a carbon or a nitrogen atom and provided that the heterocycloalkyl system is stable and suitable as a subgroup for the desired purpose of the compound of the formula I such as use as a drug substance. Depending on the definition of the respective heterocyclic group, in one embodiment of the invention the number of ring heteroatoms which can be present in a heterocyclic group, independently of the number of ring heteroatoms in any other heterocyclic group, is 1, 2, 3 or 4, in another embodiment 1, 2 or 3, in another embodiment 1 or 2, in another embodiment 2, in another embodiment 1, wherein the ring heteroatoms can be identical or different. The heterocycloalkyl group can be attached by any ring carbon atom or saturated ring nitrogen atom, with the exeption of spiro- or bridgehead atoms. A ring sulfur atom in a heterocycloalkyl group can carry zero, one or two oxo groups, it is a non-oxidized sulfur atom S in case it does not carry any oxo group, or it is an S(O) group (= sulfoxide group, S-oxide group) in case it carries one oxo group, or it is an S(O)₂ group (= sulfone group, S,S-dioxide group) in case it carries two oxo groups.

Exemplary monocyclic heterocycloalkyls are derived from, but not limited to, the following ring systems: aziridine, oxirane, azetidine, oxetane, pyrrolidine, tetrahydrofurane, tetrahydrothiophene, 4,5-dihydrothiazole, piperidine, piperazine, morpholine, thiomorpholine, tetrahydropyran, 1,4-dioxane, 1,4-oxathiane, 1,2,3,6-tetrahydropyridine, azepane, 2,3,4,7-tetrahydro-1H-azepine, 2,7-dihydro-1H-azepine, 1,4-diazepane, 1,4-oxazepane, 1,4-thiazepane and 1,4-dioxepane:

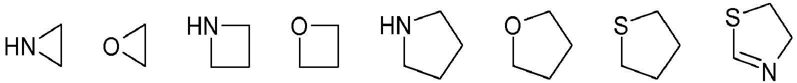

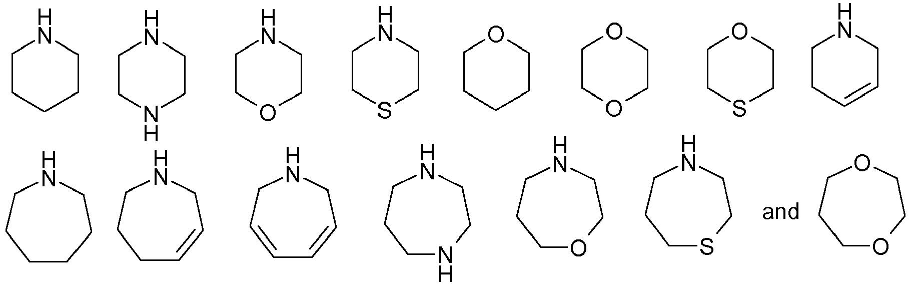

In one embodiment monocyclic heterocycloalkyls are derived from azetidine, pyrrolidine, piperidine, piperazine, morpholine or 1,4-diazepane:

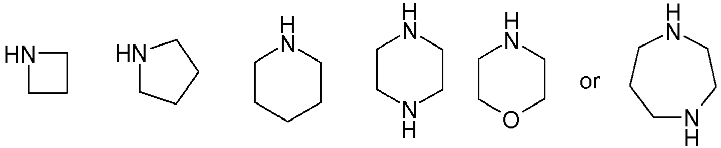

Exemplary bicyclic fused ring heterocycloalkyls are derived from, but not limited to, the following ring systems: 3-aza-bicyclo[3.1.0]hexane, 2-aza-bicyclo[4.1.0]heptane, 2-oxa-5-aza-bicyclo[5.1.0]octane, 3-aza-bicyclo[3.2.0]heptane, 2-aza-bicyclo[4.2.0]octane, octahydro-pyrrolo[3,4-c]pyrrole, octahydro-pyrrolo[3,4-b]pyrrole, octahydro-pyrrolo[3,4-b]pyridine, octahydro-thieno[3,4-b]pyrazine, octahydro-furo[3,4-b]pyridine, octahydro-cyclopenta[1,4]oxazine, octahydro-pyrrolo[1,2-a]pyrimidine, octahydro-pyrrolo[1,2-a]pyrazine, octahydro-cyclopenta[e][1,4]oxazepine, decahydro-quinoxaline, decahydro-[1,6]naphthyridine, octahydro-benzo[1,4]oxazine, octahydro-benzo-[1,4]thiazine, octahydro-pyrido[1,2-a]pyrazine, octahydro-pyrano[3,2-b]pyridine, decahydro-1-oxa-9-aza-benzocycloheptene, 1,2,3,3a,6,6a-hexahydro-cyclopenta[b]pyrrole, 5,6-dihydro-4H-cyclopenta[b]thiophene, 2,3,4,4a,7,7a-hexahydro-1H-[2]pyrindine, 2,4a,5,6,7,7a-hexahydro-1H-[1]pyrindine, 2,3,3a,4,7,7a-hexahydro-1H-indole, 1,2,3,4-tetrahydro-quinoxaline, 4,5,6,7-tetrahydro-benzofuran, benzo[1,3]dioxole, 3,4,4a,7,8,8a-hexahydro-2H-benzo[1,4]oxazine, 1,2,3,4,4a,5,8,8a-octahydro-quinoxaline, 4a,5,8,8a-tetrahydro-2H-thiopyrano[3,2-b]pyridine and 1,2,3,4-tetrahydro-[1,5]naphthyridine:

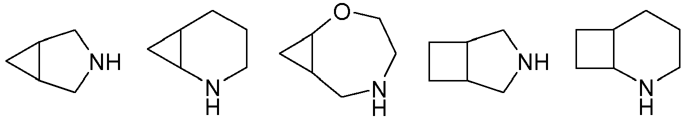

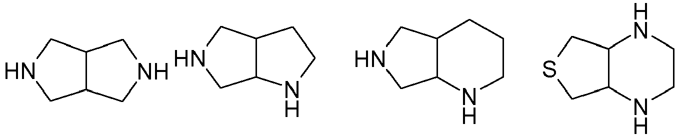

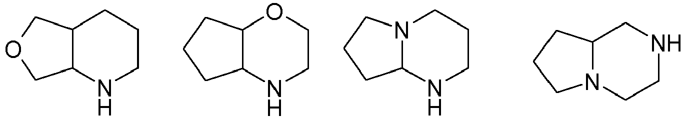

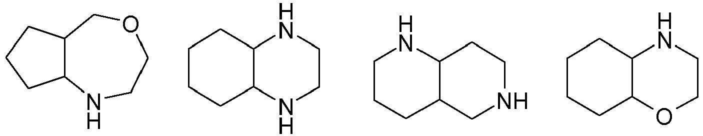

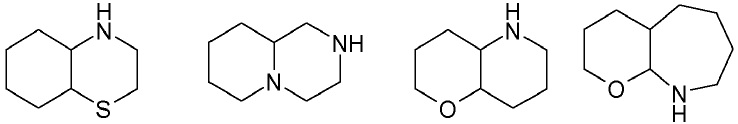

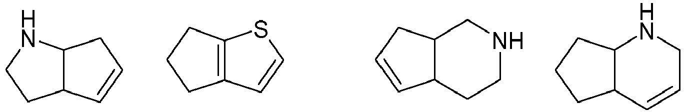

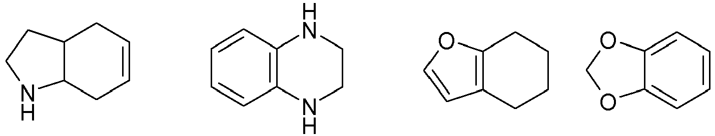

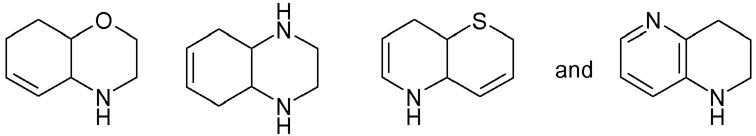

In one embodiment bicyclic fused ring heterocycloalkyls are derived from 3-aza-bicyclo[3.1.0]hexane, octahydro-pyrrolo[3,4-c]pyrrole, octahydro-pyrrolo[3,4-b]pyrrole, octahydro-thieno[3,4-b]pyrazine, octahydro-pyrrolo[1,2-a]pyrazine, decahydro-quinoxaline, octahydro-pyrido[1,2-a]pyrazine or decahydro-[1,6]naphthyridine:

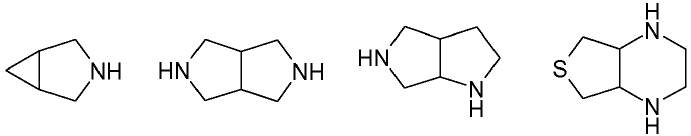

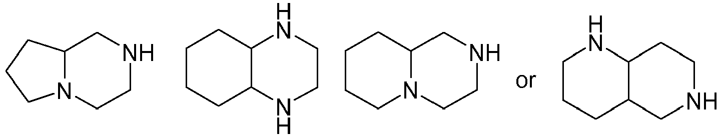

Exemplary bicyclic or tricyclic spiro ring heterocycloalkyls are derived from, but not limited to, the following ring systems:4-aza-spiro[2.4]heptane, 5-aza-spiro[3.4]octane, 1-aza-spiro[4.4]nonane, 7-oxa-1-aza-spiro[4.4]nonane, 7-thia-1-aza-spiro[4.4]nonane, 4-aza-spiro[2.5]octane, 5-aza-spiro[2.5]octane, 6-aza-spiro[2.5]octane, 5-aza-spiro[3.5]nonane, 6-aza-spiro[3.5]nonane, 7-aza-spiro[3.5]nonane, 4,7-diaza-spiro[2.5]octane, 5,8-diaza-spiro[3.5]nonane, 6,9-diaza-spiro[4.5]decane, 1,4-diaza-spiro[5.5]undecane, 2-oxa-6,9-diaza-spiro[4.5]decane, 2-oxa-6-aza-spiro[4.5]decane, 2,7-diaza-spiro[3.5]nonane, 3,9-diaza-spiro[5.5]undecane, 1-oxa-4,9-diaza-spiro-[5.5]undecane, 1-oxa-4,8-diaza-spiro[5.5]undecane, 1-thia-4,9-diaza-spiro[5.5]undecane, 1-thia-4,8-diaza-spiro[5.5]undecane, 4,8-diaza-spiro[2.6]nonane, 5,8-diaza-spiro[3.6]decane, 2-aza-spiro[5.5]undec-7-ene, 6,9-diaza-spiro[4.6]undecane, 4-aza-dispiro[2.1.4.2]undecane, 4,10-diaza-dispiro[2.2.3.2]undecane and 4,11-diaza-dispiro[2.2.4.2]dodecane:

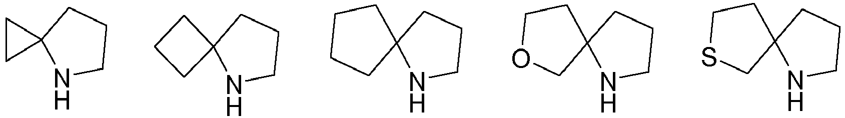

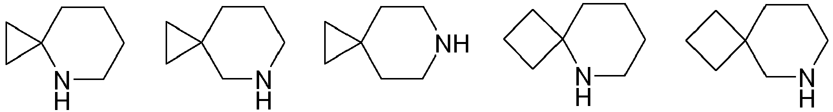

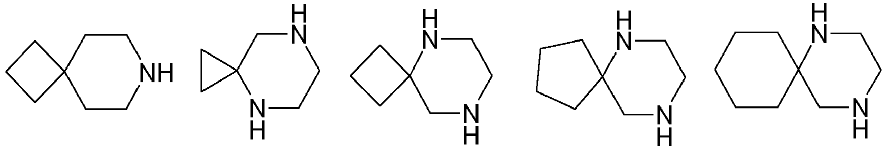

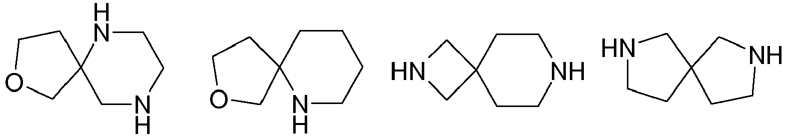

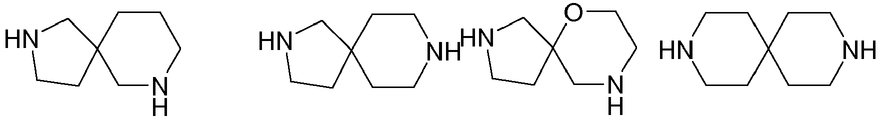

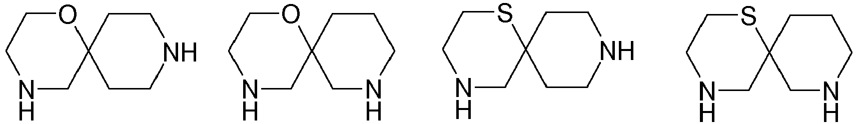

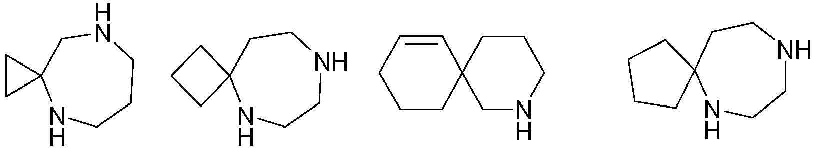

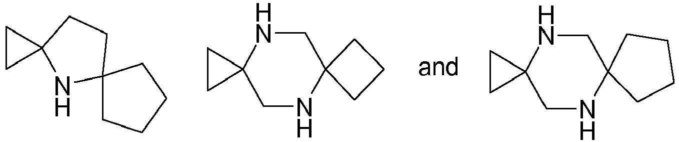

In one embodiment bicyclic or tricyclic spiro ring heterocycloalkyls are derived from 4,7-diaza-spiro[2.5]octane, 6-aza-spiro[3.5]nonane, 5,8-diaza-spiro[3.5]nonane, 6,9-diaza-spiro[4.5]decane, 2,7-diaza-spiro[3.5]nonane, 2,7-diaza-spiro[4.4]nonane, 2,7-diaza-spiro[4.5]decane, 2,8-diaza-spiro[4.5]decane, 6-oxa-2,9-diaza-spiro[4.5]decane, 3,9-diaza-spiro[5.5]undecane, 1-oxa-4,9-diaza-spiro[5.5]undecane or 1-oxa-4,8-diaza-spiro[5.5]undecane: 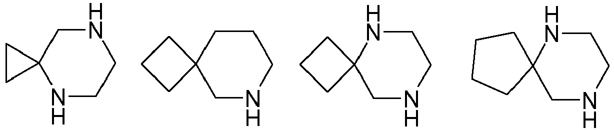 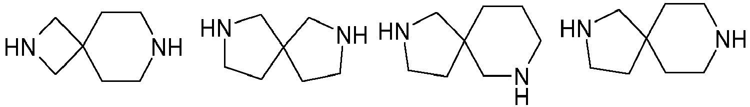 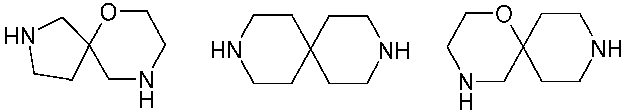 or

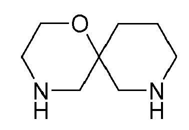

Exemplary heterocycloalkyls, in which a ring is fused to one ring of a bicyclic spiro system, are derived from, but not limited to, the following ring systems: octahydro-spiro[cyclopentane-1,2'(3'H)-quinoxalin], 1',4'-dihydro-spiro[cyclopentane-1,2'(3'H)-quinoxalin], 1',2',4,5-tetrahydro-spiro[furan-3(2H),3'-[3H]indol], 1,3-dihydro-spiro[indene-2,2'-piperazine], 2,3-dihydro-spiro[1H-indene-1,4'-piperidin] and 1,2-dihydro-5-spiro[3H-indole-3,4'-piperidin]:

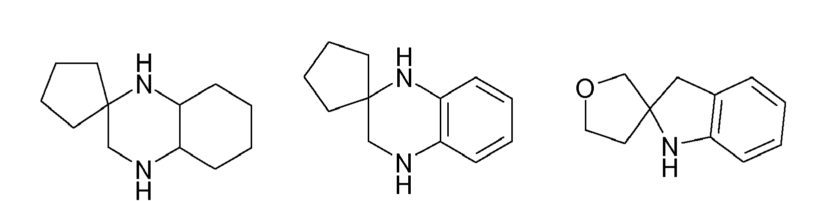

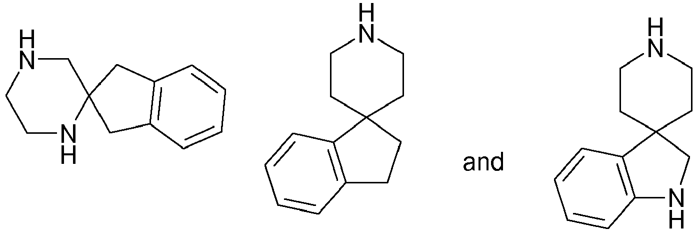

In one embodiment heterocycloalkyls, in which a ring is fused to one ring of a bicyclic spiro system, are derived from 3-dihydro-spiro[indene-2,2'-piperazine], 2,3-dihydro-spiro[1H-indene-1,4'-piperidin] or 1,2-dihydro-5-spiro[3H-indole-3,4'-piperidin]:

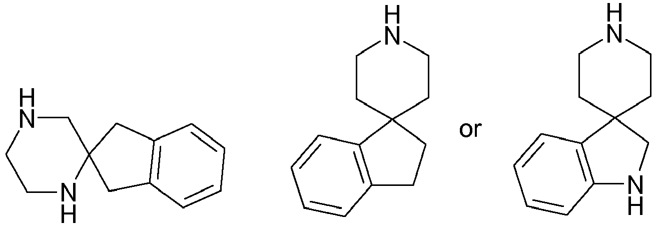

Exemplary non-fused or bridged bicyclic or tricyclic ring heterocycloalkyls are derived from, but not limited to, the following ring systems: 2-aza-bicyclo[2.2.1]heptane, 1-aza-bicyclo[2.2.2]octane, 8-aza-bicyclo[3.2.1]octane, 3-aza-bicyclo[3.2.1]octane, 9-aza-bicyclo[3.3.1]nonane, 2,5-diaza-bicyclo[2.2.1]heptane, 2,5-diaza-bicyclo[2.2.2]octane, 3,8-diaza-bicyclo[3.2.1]octaneand 3,7-diaza-bicyclo[3.3.1]nonane:

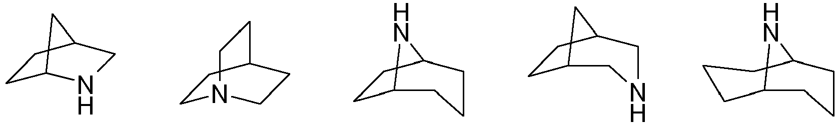

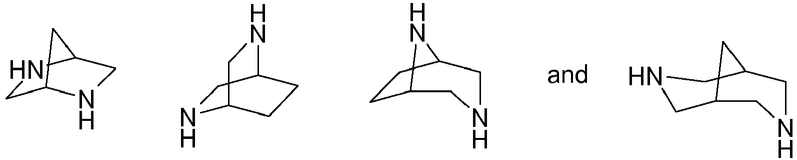

The term "aryl", as used herein, refers to a radical derived from an aromatic hydrocarbon by removal of one hydrogen, such as phenyl or naphthyl (= naphthalenyl).

The term "heteroaryl" or "hetaryl" as used herein, refers to a radical derived from an aromatic mono- or bicyclic ring system, in which 1, 2, 3, 4 or 5 carbon atoms are replaced by heteroatoms. The ring heteroatoms are generally chosen from N, O and S, wherein N includes ring nitrogen atoms which carry a hydrogen atom or a substituent as well as ring nitrogen atoms which do not carry a hydrogen atom or a substituent. Ring heteroatoms can be located in any position, provided that the heterocyclic system is stable and suitable as a subgroup for the desired purpose of the compound of the formula I such as use as a drug substance. Heteroaryl radicals are derived from 5-membered or 6-membered monocyclic rings or 8-membered, 9-membered or 10-membered bicyclic rings, in another embodiment 5-membered or 6-membered monocyclic rings or 9-membered or 10-membered bicyclic rings, in another embodiment 5-membered or 6-membered monocyclic rings.

Exemplary heteroaryl systems are derived from, but not limited to, the following ring systems: pyrrole, furan, thiophene, imidazole, pyrazole, oxazole (= [1,3]oxazole), isoxazole (= [1,2]oxazole), thiazole (= [1,3]thiazole), isothiazole (= [1,2]thiazole), [1,2,3]triazole, [1,2,4]triazole, [1,2,4]oxadiazole, [1,3,4]oxadiazole, [1,2,4]thiadiazole, [1,3,4]thiadiazole, tetrazole, pyridine, pyridazine, pyrimidine, pyrazine, [1,2,3]triazine, [1,2,4]triazine, [1,3,5]triazine, indole, isoindole, benzofuran, benzothiophene [1,3]benzoxazole, [1,3]benzothiazole, benzoimidazole,indazole, quinoline, isoquinoline, cinnoline, quinazoline, quinoxaline, phthalazine, different naphthyridines, e.g. [1,8]naphthyridine, different thienopyridines, e.g. thieno[2,3-b]pyridine and purine:

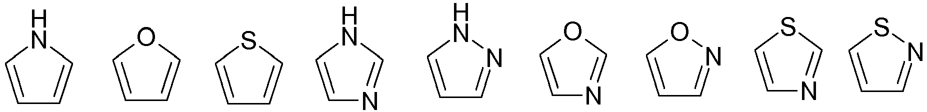

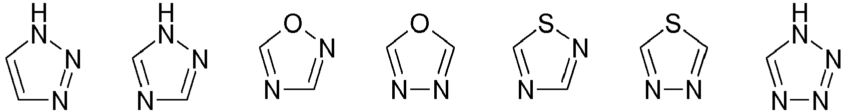

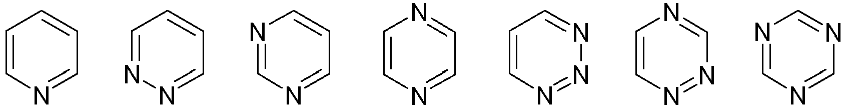

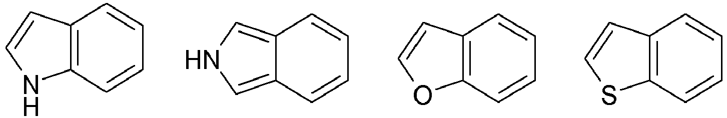

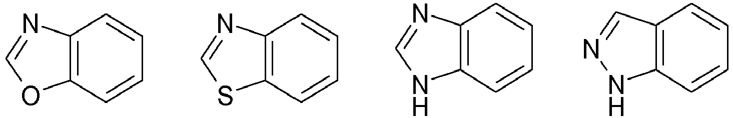

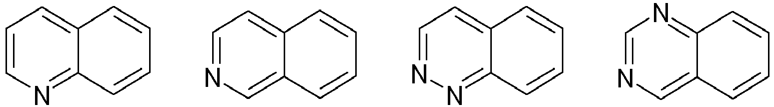

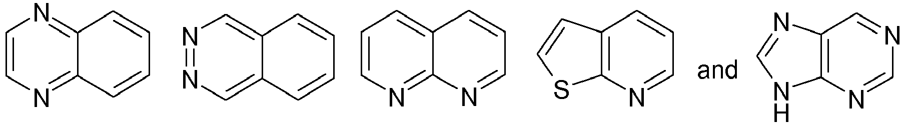

Groups like phenyl, naphthyl (= naphthalenyl) and residues of aromatic heterocycles which are optionally substituted by one or more substituents, can be unsubstituted or substituted, for example by 1, 2, 3, 4 or 5, or by 1, 2, 3 or 4, or by 1, 2 or 3, or by 1 or 2, or by 1, identical or different substituents which can be located in any positions. Aromatic nitrogen heterocycles which in the parent ring system carry a hydrogen atom on a ring nitrogen atom in a 5-membered ring, such as a pyrrole, imidazole, indole or benzoimidazole ring, for example, can be substituted on ring carbon atoms and/or on such ring nitrogen atoms. In one embodiment of the invention, substituents on such ring nitrogen atoms are chosen from (C₁-C₄)-alkyl groups, i.e. such ring nitrogen atoms in aromatic heterocycles carry a hydrogen atom or a (C₁-C₄)-alkyl substituent. When it is stated with respect to ring nitrogen atoms in aromatic heterocycles and any other heterocycles that they can carry a hydrogen atom or a substituent, such ring nitrogen atoms either carry a hydrogen atom or a substituent or they do not carry a hydrogen atom or substituent. Ring nitrogen atoms which carry a hydrogen atom or a substituent, occur in a nitrogen-containing aromatic 5-membered ring as is present in pyrrole, imidazole, indole or benzoimidazole, for example, and in a non-aromatic ring including a saturated ring. Ring nitrogen atoms which do not carry a hydrogen atom or a substituent unless they are present in positively charged form, including any further ring nitrogen atoms in addition to ring nitrogen atoms which carry a hydrogen atom or a substituent, occur in an aromatic ring as is present in thiazole, imidazole, pyridine or benzoimidazole, for example, and in a non-aromatic ring in which they are part of a double bond, and they occur as ring nitrogen atoms via which a ring is bonded. Suitable ring nitrogen atoms in aromatic heterocycles in the compounds of the formula I, such as the ring nitrogen atom in a pyridine ring or a quinoline ring, can in general also be present as N-oxide or as quaternary salt, for example as N-(C₁-C₄)-alkyl salt such as N-methyl salt, wherein in one embodiment of the invention the counter anion in such quaternary salt is a physiologically acceptable anion which is derived from an acid that forms a physiologically acceptable salt.

In monosubstituted phenyl groups, the substituent can be located in the 2-position, the 3-position or the 4-position. In disubstituted phenyl groups, the substituents can be located in 2,3-position, 2,4-position, 2,5-position, 2,6-position, 3,4-position or 3,5-position. In trisubstituted phenyl groups, the substituents can be located in 2,3,4-position, 2,3,5-position, 2,3,6-position, 2,4,5-position, 2,4,6-position or 3,4,5-position. Naphthyl can be 1-naphthyl (= naphthalen-1-yl) or 2-naphthyl (= naphthalen-2-yl). In monosubstituted 1-naphthyl groups, the substituent can be located in the 2-, 3-, 4-, 5-, 6-, 7- or 8-position. In monosubstituted 2-naphthyl groups, the substituent can be located in the 1-, 3-, 4-, 5-, 6-, 7- or 8-position. In disubstituted naphthyl groups, the substituents can likewise be located in any positions both in the ring via which the naphthyl group is bonded and/or in the other ring.

Ring heteroatoms can be located in any positions, provided that the heterocyclic system is known in the art and is stable and suitable as a subgroup for the desired purpose of the compound of the formula I such as use as a drug substance. In one embodiment of the invention, two ring oxygen atoms cannot be present in adjacent ring positions of any heterocycle, in another embodiment two ring heteroatoms chosen from oxygen and sulfur cannot be present in adjacent ring positions of any heterocycle. Substituents on heterocyclic groups can be located in any positions. For example, in a pyridin-2-yl group substituents can be located in the 3-position and/or 4-position and/or 5-position and/or 6-position, in a pyridin-3-yl group substituent can be located in the 2-position and/or 4-position and/or 5-position and/or 6-position, in a pyridin-4-yl group substituents can be located in the 2-position and/or 3-position and/or 5-position and/or 6-position.

Halogen is fluorine, chlorine, bromine or iodine. In one embodiment of the invention, any halogen in a compound of the formula I is independently of any other halogen chosen from fluorine, chlorine and bromine, in another embodiment from fluorine and chlorine, and in yet another embodiment it is fluorine.

When an oxo group is bonded to a carbon atom, it replaces two hydrogen atoms on a carbon atom of the parent system. Thus, if a CH₂ group in a chain or a ring is substituted by oxo, i.e. by a doubly bonded oxygen atom, it becomes a CO group. Evidently, an oxo group cannot occur as a substituent on a carbon atom in an aromatic ring such as in a phenyl group, for example. When a ring sulfur atom in a heterocyclic group can carry one or two oxo groups, it is a non-oxidized sulfur atom S in case it does not carry any oxo group, or it is an S(O) group (= sulfoxide group, S-oxide group) in case it carries one oxo group, or it is an S(O)₂ group (= sulfone group, S,S-dioxide group) in case it carries two oxo groups.

The present invention includes all stereoisomeric forms of the compounds of the formula I and their salts and solvates. With respect to each chiral center, independently of any other chiral center, the compounds of the formula I can be present in S configuration or substantially S configuration, or in R configuration or substantially R configuration, or as a mixture of the S isomer and the R isomer in any ratio. The invention includes all possible enantiomers and diastereomers and mixtures of two or more stereoisomers, for example mixtures of enantiomers and/or diastereomers, in all ratios. Thus, compounds according to the invention which can exist as enantiomers can be present in enantiomerically pure form, both as levorotatory and as dextrorotatory antipodes, and in the form of mixtures of the two enantiomers in all ratios including racemates. In the case of a E/Z isomerism, or cis/trans isomerism, for example on double bonds or rings such as cycloalkyl rings, the invention includes both the E form and Z form, or the cis form and the trans form, as well as mixtures of these forms in all ratios. In one embodiment of the invention, a compound which can occur in two or more stereoisomeric forms is a pure, or substantially pure, individual stereoisomer. The preparation of individual stereoisomers can be carried out, for example, by separation of a mixture of isomers by customary methods, for example by chromatography or crystallization, by the use of stereochemically uniform starting materials in the synthesis, or by stereoselective synthesis. Optionally, a derivatization can be carried out before a separation of stereoisomers. The separation of a mixture of stereoisomers can be carried out at the stage of the compound of the formula I or at the stage of a starting material or an intermediate during the synthesis. The present invention also includes all tautomeric forms of the compounds of the formula I and their salts and solvates.

In case the compounds of the formula I contain one or more acidic and/or basic groups, i.e. salt-forming groups, the invention also includes their corresponding physiologically or toxicologically acceptable salts, i.e. non-toxic salts, in particular their pharmaceutically acceptable salts.

The present invention furthermore includes all solvates of compounds of the formula I, for example hydrates or adducts with alcohols such as (C₁-C₄)-alkanols. Also disclosed are active metabolites of the compounds of the formula I, and also prodrugs and derivatives of the compounds of the formula I which in vitro may not necessarily exhibit pharmacological activity but which in vivo are converted into pharmacologically active compounds, for example esters or amides of carboxylic acid groups.

The compounds of the present invention, PKC inhibitors, can be widely combined with other pharmacologically active compounds, e.g., with all antihypertensives and nephroprotectives, mentioned in the Rote Liste 2011, antidiabetics mentioned in the Rote Liste 2011, chapter 12; all weight-reducing agents/appetite suppressants mentioned in the Rote Liste 2011, chapter 1; all diuretics mentioned in the Rote Liste 2011, chapter 36; all lipid-lowering agents mentioned in the Rote Liste 2011, chapter 58. They can be combined with the inventive compound of the formula I, especially for a synergistic improvement in action. The active ingredient combination can be administered either by separate administration of the active ingredients to the patient or in the form of combination products in which a plurality of active ingredients are present in one pharmaceutical preparation. When the active ingredients are administered by separate administration of the active ingredients, this can be done simultaneously or successively. Most of the active ingredients mentioned hereinafter are disclosed in the USP Dictionary of USAN and International Drug Names, US Pharmacopeia, Rockville 2006.

Antidiabetics include insulin and insulin derivatives, for example Lantus^{®} (see www.lantus.com) or HMR 1964 or Levemir® (insulin detemir), Humalog^{(R)} (Insulin Lispro), insulin degludec, insulin aspart, polyethylene glycosidized (PEGylated) Insulin Lispro as described in WO2009152128, Humulin^{(R)}, VIAject™, SuliXen^{(R)}, VIAject™ or those as described in WO2005005477 (Novo Nordisk), fast-acting insulins (see US 6,221,633), inhalable insulins, for example Exubera^{®}, Nasulin™, or oral insulins, for example IN-105 (Nobex) or Oral-lyn™ (Generex Biotechnology), or Technosphere^{(R)} insulin (MannKind) or Cobalamin™ oral insulin or ORMD-0801 or insulins or insulin precursors as described in WO2007128815, WO2007128817, WO2008034881, WO2008049711, WO2008145721, WO2009034117, WO2009060071, WO2009133099 or insulins which can be administered transdermally; additionally included are also those insulin derivatives which are bonded to albumin by a bifunctional linker, as described, for example, in WO2009121884;

GLP-1 derivatives and GLP-1 agonists, for example exenatide or specific formulations thereof, as described, for example, in WO2008061355, WO2009080024, WO2009080032, liraglutide, taspoglutide (R-1583), albiglutide, lixisenatide or those which have been disclosed in WO 98/08871, WO2005027978, WO2006037811, WO2006037810 by Novo Nordisk A/S, in WO 01/04156 by Zealand or in WO 00/34331 by Beaufour-Ipsen, pramlintide acetate (Symlin; Amylin Pharmaceuticals), inhalable GLP-1 (MKC-253 from MannKind) AVE-0010, BIM-51077 (R-1583, ITM-077), PC-DAC:exendin-4 (an exendin-4 analog which is bonded covalently to recombinant human albumin), biotinylated exendin (WO2009107900), a specific formulation of exendin-4 as described in US2009238879, CVX-73, CVX-98 and CVx-96 (GLP-1 analogs which are bonded covalently to a monoclonal antibody which has specific binding sites for the GLP-1 peptide), CNTO-736 (a GLP-1 analog which is bonded to a domain which includes the Fc portion of an antibody), PGC-GLP-1 (GLP-1 bonded to a nanocarrier), agonists or modulators, as described, for example, in D. Chen et al., Proc. Natl. Acad. Sci. USA 104 (2007) 943, those as described in WO2006124529, WO2007124461, WO2008062457, WO2008082274, WO2008101017, WO2008081418, WO2008112939, WO2008112941, WO2008113601, WO2008116294, WO2008116648, WO2008119238, WO2008148839, US2008299096, WO2008152403, WO2009030738, WO2009030771, WO2009030774, WO2009035540, WO2009058734, WO2009111700, WO2009125424, WO2009129696, WO2009149148, peptides, for example obinepitide (TM-30338), orally active GLP-1 analogs (e.g. NN9924 from Novo Nordisk), amylin receptor agonists, as described, for example, in WO2007104789, WO2009034119, analogs of the human GLP-1, as described in WO2007120899, WO2008022015, WO2008056726, chimeric pegylated peptides containing both GLP-1 and glucagon residues, as described, for example, in WO2008101017, WO2009155257, WO2009155258, glycosylated GLP-1 derivatives as described in WO2009153960, and orally active hypoglycemic ingredients.

Antidiabetics also include gastrin analogs, for example TT-223.

Antidiabetics additionally include poly- or monoclonal antibodies directed, for example, against interleukin 1 beta (IL-1β), for example XOMA-052.

Antidiabetics additionally include peptides which can bind to the human pro-islet peptide (HIP) receptor, as described, for example, in WO2009049222.

Antidiabetics also include agonists of the glucose-dependent insulinotropic polypeptide (GIP) receptor, as described, for example, in WO2006121860.

Antidiabetics also include the glucose-dependent insulinotropic polypeptide (GIP), and also analogous compounds, as described, for example, in WO2008021560, WO2010016935, WO2010016936, WO2010016938, WO2010016940, WO2010016944.

Additionally included are analogs and theivatives of human pancreatic polypeptide, as described, for example, in WO2009007714.

Antidiabetics additionally include encapsulated insulin-producing porcine cells, for example DiabeCell(R).

Antidiabetics also include analogs and theivatives of fibroblast growth factor 21 (FGF-21), as described, for example, in WO2009149171, WO2010006214.

The orally active hypoglycemic ingredients preferably include sulfonylureas,
biguanidines,
meglitinides,
oxadiazolidinediones,
thiazolidinediones,
PPAR and RXR modulators,
glucosidase inhibitors,
inhibitors of glycogen phosphorylase,
glucagon receptor antagonists,
glucokinase activators,
inhibitors of fructose 1,6-bisphosphatase,
modulators of glucose transporter 4 (GLUT4),
inhibitors of glutamine:fructose-6-phosphate amidotransferase (GFAT),
GLP-1 agonists,
potassium channel openers, for example pinacidil, cromakalim, diazoxide, diazoxide choline salt, or those as described in R. D. Carr et al., Diabetes 52, 2003, 2513.2518, in J. B. Hansen et al., Current Medicinal Chemistry 11, 2004, 1595-1615, in T. M. Tagmose et al., J. Med. Chem. 47, 2004, 3202-3211 or in M. J. Coghlan et al., J. Med. Chem. 44, 2001, 1627-1653, or those which have been disclosed in WO 97/26265 and WO 99/03861 by Novo Nordisk A/S,
active ingredients which act on the ATP-dependent potassium channel of the beta cells,
inhibitors of dipeptidyl peptidase-IV (DPP-IV),
insulin sensitizers,
inhibitors of liver enzymes involved in stimulating gluconeogenesis and/or glycogenolysis,
modulators of glucose uptake, of glucose transport and of glucose reabsorption,
modulators of sodium-dependent glucose transporter 1 or 2 (SGLT1, SGLT2),
inhibitors of 11 -beta-hydroxysteroid dehydrogenase-1 (11β-HSD1),
inhibitors of protein tyrosine phosphatase-1B (PTP-1 B),
nicotinic acid receptor agonists,
inhibitors of hormone-sensitive or endothelial lipases,
inhibitors of acetyl-CoA carboxylase (ACC1 and/or ACC2) or
inhibitors of GSK-3 beta.

Also included are compounds which modify the lipid metabolism, such as active antihyperlipidemic ingredients and active antilipidemic ingredients,
HMG-CoA reductase inhibitors,
farnesoid X receptor (FXR) modulators,
fibrates,
cholesterol reabsorption inhibitors,
CETP inhibitors,
bile acid absorption inhibitors,
MTP inhibitors,
estrogen receptor gamma agonists (ERR γ agonists),
sigma-1 receptor antagonists,
antagonists of the somatostatin 5 receptor (SST5 receptor);
compounds which reduce food intake, and
compounds which increase thermogenesis.

In one embodiment of the invention, the compound of the formula I is administered in combination with insulin.

In another embodiment of the invention, the compound of the formula I is administered in combination with an insulin sensitizer, for example PN-2034 or ISIS-113715.

In one embodiment, the compound of the formula I is administered in combination with an active ingredient which acts on the ATP-dependent potassium channel of the beta cells, for example sulfonylureas, for example tolbutamide, glibenclamide, glipizide, gliclazide or glimepiride, or those formulations as described, for example, in EP2103302.

In one embodiment, the compound of the formula I is administered in combination with a tablet which comprises both glimepiride, which is released rapidly, and metformin, which is released over a longer period (as described, for example, in US2007264331, WO2008050987, WO2008062273).

In one embodiment, the compound of the formula I is administered in combination with a biguanide, for example metformin or one of its salts.

In a further embodiment, the compound of the formula I is administered in combination with a guanidine, for example benzylguanidine or one of its salts, or those guanidines as described in WO2009087395.

In another embodiment, the compound of the formula I is administered in combination with a meglitinide, for example repaglinide, nateglinide or mitiglinide.

In a further embodiment, the compound of the formula I is administered with a combination of mitiglinide with a glitazone, e.g. pioglitazone hydrochloride.

In a further embodiment, the compound of the formula I is administered with a combination of mitiglinide with an alpha-glucosidase inhibitor.

In a further embodiment, the compound of the formula I is administered in combination with antidiabetic compounds, as described in WO2007095462, WO2007101060, WO2007105650.

In a further embodiment, the compound of the formula I is administered in combination with antihypoglycemic compounds, as described in WO2007137008, WO2008020607.

In one embodiment, the compound of the formula I is administered in combination with a thiazolidinedione, for example troglitazone, ciglitazone, pioglitazone, rosiglitazone or the compounds disclosed in WO 97/41097 by Dr. Reddy's Research Foundation, especially 5-[[4-[(3,4-dihydro-3-methyl-4-oxo-2-quinazolinylmethoxy]phenyl]methyl]-2,4-thiazolidinedione.

In one embodiment of the invention, the compound of the formula I is administered in combination with a PPAR gamma agonist, for example rosiglitazone, pioglitazone, JTT-501, GI 262570, R-483, CS-011 (rivoglitazone), DRL-17564, DRF-2593 (balaglitazone), INT-131, T-2384, or those as described in WO2005086904, WO2007060992, WO2007100027, WO2007103252, WO2007122970, WO2007138485, WO2008006319, WO2008006969, WO2008010238, WO2008017398, WO2008028188, WO2008066356, WO2008084303, WO2008089461-WO2008089464, WO2008093639, WO2008096769, WO2008096820, WO2008096829, US2008194617, WO2008099944, WO2008108602, WO2008109334, WO2008110062, WO2008126731, WO2008126732, WO2008137105, WO2009005672, WO2009038681, WO2009046606, WO2009080821, WO2009083526, WO2009102226, WO2009128558, WO2009139340.

In one embodiment of the invention, the compound of the formula I is administered in combination with Competact™, a solid combination of pioglitazone hydrochloride with metformin hydrochloride.

In one embodiment of the invention, the compound of the formula I is administered in combination with Tandemact™, a solid combination of pioglitazone with glimepiride.

In a further embodiment of the invention, the compound of the formula I is administered in combination with a solid combination of pioglitazone hydrochloride with an angiotensin II agonist, for example TAK-536.

In one embodiment of the invention, the compound of the formula I is administered in combination with a PPAR alpha agonist or mixed PPAR alpha/PPAR delta agonist, for example GW9578, GW-590735, K-111, LY-674, KRP-101, DRF-10945, LY-518674, CP-900691, BMS-687453, BMS-711939, or those as described in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043, WO2008006043, WO2008006044, WO2008012470, WO2008035359, WO2008087365, WO2008087366, WO2008087367, WO2008117982, JP2009023975, WO2009033561, WO2009047240, WO2009072581, WO2009080248, WO2009080242, WO2009149819, WO2009149820, WO2009147121, WO2009153496, WO2010008299, WO2010014771.

In one embodiment of the invention, the compound of the formula I is administered in combination with a mixed PPAR alpha/gamma agonist, for example naveglitazar, aleglitazar, LY-510929, ONO-5129, E-3030, AVE 8042, AVE 8134, AVE 0847, AVE 0897, CKD-501 (lobeglitazone sulfate), MBX-213, KY-201, BMS-759509, or as described in WO 00/64888, WO 00/64876, WO03/020269, WO2004024726, WO2007099553, US2007276041, WO2007085135, WO2007085136, WO2007141423, WO2008016175, WO2008053331, WO2008109697, WO2008109700, WO2008108735, WO2009026657, WO2009026658, WO2009149819, WO2009149820 or in J.P.Berger et al., TRENDS in Pharmacological Sciences 28(5), 244-251, 2005.

In one embodiment of the invention, the compound of the formula I is administered in combination with a PPAR delta agonist, for example GW-501516, or as described in WO2006059744, WO2006084176, WO2006029699, WO2007039172-WO2007039178, WO2007071766, WO2007101864, US2007244094, WO2007119887, WO2007141423, US2008004281, WO2008016175, WO2008066356, WO2008071311, WO2008084962, US2008176861, WO2009012650, US2009137671, WO2009080223, WO2009149819, WO2009149820, WO2010000353.

In one embodiment of the invention, the compound of the formula I is administered in combination with a pan-SPPARM (selective PPAR modulator alpha, gamma, delta), for example GFT-505, indeglitazar, or those as described in WO2008035359, WO2009072581.

In one embodiment, the compound of the formula I is administered in combination with metaglidasen or with MBX-2044 or other partial PPAR gamma agonists/antagonists.

In one embodiment, the compound of the formula I is administered in combination with an α-glucosidase inhibitor, for example miglitol or acarbose, or those as described, for example, in WO2007114532, WO2007140230, US2007287674, US2008103201, WO2008065796, WO2008082017, US2009076129.

In one embodiment, the compound of the formula I is administered in combination with an inhibitor of glycogen phosphorylase, for example PSN-357 or FR-258900, or those as described in WO2003084922, WO2004007455, WO2005073229-31, WO2005067932, WO2008062739, WO2008099000, WO2008113760, WO2009016118, WO2009016119, WO2009030715, WO2009045830, WO2009045831, WO2009127723.

In another embodiment, the compound of the formula I is administered in combination with an inhibitor of the interaction of liver glycogen phosphorylase with the protein PPP1 R3 (GL subunit of glycogen-associated protein phosphatase 1 (PP1)), as described, for example, in WO2009030715.

In one embodiment, the compound of the formula I is administered in combination with glucagon receptor antagonists, for example A-770077 or NNC-25-2504 or as described in WO2004100875, WO2005065680, WO2006086488, WO2007047177, WO2007106181, WO2007111864, WO2007120270, WO2007120284, WO2007123581, WO2007136577, WO2008042223, WO2008098244, WO2009057784, WO2009058662, WO2009058734, WO2009110520, WO2009120530, WO2009140342, WO2010019828.

In a further embodiment, the compound of the formula I is administered in combination with an antisense compound, e.g. ISIS-325568, which inhibits the production of the glucagon receptor.

In one embodiment, the compound of the formula I is administered in combination with activators of glucokinase, for example LY-2121260 (WO2004063179), PSN-105, PSN-110, GKA-50, or those as described, for example, in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942, WO2008005914, WO2008005964, WO2008043701, WO2008044777, WO2008047821, US2008096877, WO2008050117, WO2008050101, WO2008059625, US2008146625, WO2008078674, WO2008079787, WO2008084043, WO2008084044, WO2008084872, WO2008089892, WO2008091770, WO2008075073, WO2008084043, WO2008084044, WO2008084872, WO2008084873, WO2008089892, WO2008091770, JP2008189659, WO2008104994, WO2008111473, WO2008116107, WO2008118718, WO2008120754, US2008280875, WO2008136428, WO2008136444, WO2008149382, WO2008154563, WO2008156174, WO2008156757, US2009030046, WO2009018065, WO2009023718, WO2009039944, WO2009042435, WO2009046784, WO2009046802, WO2009047798, WO2009063821, WO2009081782, WO2009082152, WO2009083553, WO2009091014, US2009181981, WO2009092432, WO2009099080, WO2009106203, WO2009106209, WO2009109270, WO2009125873, WO2009127544, WO2009127546, WO2009128481, WO2009133687, WO2009140624, WO2010013161, WO2010015849, WO2010018800.

In one embodiment, the compound of the formula I is administered in combination with an inhibitor of gluconeogenesis, as described, for example, in FR-225654, WO2008053446.

In one embodiment, the compound of the formula I is administered in combination with inhibitors of fructose 1,6-bisphosphatase (FBPase), for example MB-07729, CS-917 (MB-06322) or MB-07803, or those as described in WO2006023515, WO2006104030, WO2007014619, WO2007137962, WO2008019309, WO2008037628, WO2009012039, EP2058308, WO2009068467, WO2009068468.

In one embodiment, the compound of the formula I is administered in combination with modulators of glucose transporter 4 (GLUT4), for example KST-48 (D.-O. Lee et al.: Arzneim.-Forsch. Drug Res. 54 (12), 835 (2004)).

In one embodiment, the compound of the formula I is administered in combination with inhibitors of glutamine:fructose-6-phosphate amidotransferase (GFAT), as described, for example, in WO2004101528.

In one embodiment, the compound of the formula I is administered in combination with inhibitors of dipeptidyl peptidase-IV (DPP-IV), for example vildagliptin (LAF-237), sitagliptin (MK-0431), sitagliptin phosphate, saxagliptin (BMS-477118), GSK-823093, PSN-9301, SYR-322, SYR-619, TA-6666, TS-021, GRC-8200 (melogliptin), GW-825964X, KRP-104, DP-893, ABT-341, ABT-279 or another salt thereof, S-40010, S-40755, PF-00734200, BI-1356, PHX-1149, DSP-7238, alogliptin benzoate, linagliptin, melogliptin, carmegliptin, or those compounds as described in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603, WO2007142253, WO2007148185, WO2008017670, US2008051452, WO2008027273, WO2008028662, WO2008029217, JP2008031064, JP2008063256, WO2008033851, WO2008040974, WO2008040995, WO2008060488, WO2008064107, WO2008066070, WO2008077597, JP2008156318, WO2008087560, WO2008089636, WO2008093960, WO2008096841, WO2008101953, WO2008118848, WO2008119005, WO2008119208, WO2008120813, WO2008121506, WO2008130151, WO2008131149, WO2009003681, WO2009014676, WO2009025784, WO2009027276, WO2009037719, WO2009068531, WO2009070314, WO2009065298, WO2009082134, WO2009082881, WO2009084497, WO2009093269, WO2009099171, WO2009099172, WO2009111239, WO2009113423, WO2009116067, US2009247532, WO2010000469, WO2010015664.

In one embodiment, the compound of the formula I is administered in combination with Janumet™, a solid combination of sitagliptin phosphate with metformin hydrochloride.

In one embodiment, the compound of the formula I is administered in combination with Eucreas^{(R)}, a solid combination of vildagliptin with metformin hydrochloride.

In a further embodiment, the compound of the formula I is administered in combination with a solid combination of alogliptin benzoate with pioglitazone.

In one embodiment, the compound of the formula I is administered in combination with a solid combination of a salt of sitagliptin with metformin hydrochloride.

In one embodiment, the compound of the formula I is administered in combination with a combination of a DPP-IV inhibitor with omega-3 fatty acids or omega-3 fatty acid esters, as described, for example, in WO2007128801.

In one embodiment, the compound of the formula I is administered in combination with a combination of a DPP-IV inhibitor with metformin hydrochloride, as described, for example, in WO2009121945.

In one embodiment, the compound of the formula I is administered in combination with a combination of a DPP-IV inhibitor with a GPR-119 agonist, as described, for example, in WO2009123992.

In one embodiment, the compound of the formula I is administered in combination with a combination of a DPP-IV inhibitor with miglitol, as described, for example, in WO2009139362.

In one embodiment, the compound of the formula I is administered in combination with a solid combination of a salt of sitagliptin with metformin hydrochloride.

In one embodiment, the compound of the formula I is administered in combination with a solid combination of alopliptin benzoate with pioglitazone hydrochloride.

In one embodiment, the compound of the formula I is administered in combination with a substance which enhances insulin secretion, for example KCP-265 (WO2003097064), or those as described in WO2007026761, WO2008045484, US2008194617, WO2009109259, WO2009109341.

In one embodiment, the compound of the formula I is administered in combination with agonists of the glucose-dependent insulinotropic receptor (GDIR), for example APD-668.

In one embodiment of the invention, the compound of the formula I is administered in combination with an ATP citrate lyase inhibitor, for example SB-204990.

In one embodiment, the compound of the formula I is administered in combination with modulators of the sodium-dependent glucose transporter 1 and/or 2 (SGLT1, SGLT2), for example KGA-2727, T-1095, SGL-0010, AVE 2268, SAR 7226, SGL-5083, SGL-5085, SGL-5094, ISIS-388626, sergliflozin, dapagliflozin or remogliflozin etabonate, canagliflozin, or as described, for example, in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116, WO2007143316, WO2007147478, WO2008001864, WO2008002824, WO2008013277, WO2008013280, WO2008013321, WO2008013322, WO2008016132, WO2008020011, JP2008031161, WO2008034859, WO2008042688, WO2008044762, WO2008046497, WO2008049923, WO2008055870, WO2008055940, WO2008069327, WO2008070609, WO2008071288, WO2008072726, WO2008083200, WO2008090209, WO2008090210, WO2008101586, WO2008101939, WO2008116179, WO2008116195, US2008242596, US2008287529, WO2009026537, WO2009049731, WO2009076550, WO2009084531, WO2009096503, WO2009100936, WO2009121939, WO2009124638, WO2009128421, WO2009135673, WO2010009197, WO2010018435, WO2010018438 or by A. L. Handlon in Expert Opin. Ther. Patents (2005) 15(11), 1531-1540.

In a further embodiment of the invention, the compound of the formula I is administered in combination with a solid combination of an SGLT inhibitor with a DPP-IV inhibitor, as described in WO2009091082.

In one embodiment, the compound of the formula I is administered in combination with a stimulator of glucose transport, as described, for example, in WO2008136392, WO2008136393.

In one embodiment, the compound of the formula I is administered in combination with inhibitors of 11-beta-hydroxysteroid dehydrogenase 1 (11 β-HSD1), for example BVT-2733, JNJ-25918646, INCB-13739, INCB-20817, DIO-92 ((-)-ketoconazole) or those as described, for example, in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427, WO2007139992, WO2007144394, WO2007145834. WO2007145835, WO2007146761, WO2008000950, WO2008000951, WO2008003611, WO2008005910, WO2008006702, WO2008006703, WO2008011453, WO2008012532, WO2008024497, WO2008024892, WO2008032164, WO2008034032, WO2008043544, WO2008044656, WO2008046758, WO2008052638, WO2008053194, WO2008071169, WO2008074384, WO2008076336, WO2008076862, WO2008078725, WO2008087654, WO2008088540, WO2008099145, WO2008101885, WO2008101886, WO2008101907, WO2008101914, WO2008106128, WO2008110196, WO2008119017, WO2008120655, WO2008127924, WO2008130951, WO2008134221, WO2008142859, WO2008142986, WO2008157752, WO2009001817, WO2009010416, WO2009017664, WO2009020140, WO2009023180, WO2009023181, WO2009023664, WO2009026422, WO2009038064, WO2009045753, WO2009056881, WO2009059666, WO2009061498, WO2009063061, WO2009070497, WO2009074789, WO2009075835, WO2009088997, WO2009090239, WO2009094169, WO2009098501, WO2009100872, WO2009102428, WO2009102460, WO2009102761, WO2009106817, WO2009108332, WO2009112691, WO2009112845, WO2009114173, WO2009117109, US2009264401, WO2009118473, WO2009131669, WO2009132986, WO2009134384, WO2009134387, WO2009134392, WO2009134400, WO2009135581, WO2009138386, WO2010006940, WO2010010157, WO2010010174, WO2010011917.

In one embodiment, the compound of the formula I is administered in combination with inhibitors of protein tyrosine phosphatase-1 B (PTP-1 B), as described, for example, in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058, US2008004325, WO2008033455, WO2008033931, WO2008033932, WO2008033934, WO2008089581, WO2008148744, WO2009032321, WO2009109999, WO2009109998.

In a further embodiment, the compound of the formula I is administered in combination with stimulators of tyrosine kinase B (Trk-B), as described, for example, in WO2010014613.

In a further embodiment, the compound of the formula I is administered in combination with beta 3 agonists (also called beta-3 adrenoceptor agonists), as described, for example, in Physiol. Behav. 2004 Sep 15;82(2-3):489-96, J Clin Invest (1998) 101: 2387-93, Curr. Pharma. Des.2001 Sep;7(14):1433-49., Bioorganic & Medicinal Chemistry Letters volume 14, number 13, July 5, 2004, pages 3525-3529 (BMS-201620).

In one embodiment of the invention, the compound of the formula I is administered in combination with an agonist of GPR109A (HM74A receptor agonists; NAR agonists (nicotinic acid receptor agonists)), for example nicotinic acid or extended release niacin in conjunction with MK-0524A (laropiprant) or MK-0524, or those compounds as described in WO2004041274, WO2006045565, WO2006045564, WO2006069242, WO2006085108, WO2006085112, WO2006085113, WO2006124490, WO2006113150, WO2007002557, WO2007017261, WO2007017262, WO2007017265, WO2007015744, WO2007027532, WO2007092364, WO2007120575, WO2007134986, WO2007150025, WO2007150026, WO2008016968, WO2008051403, WO2008086949, WO2008091338, WO2008097535, WO2008099448, US2008234277, WO2008127591.

In another embodiment of the invention, the compound of the formula I is administered in combination with a solid combination of niacin with simvastatin.

In another embodiment of the invention, the compound of the formula I is administered in combination with nicotinic acid or "extended release niacin" in conjunction with MK-0524A (laropiprant).

In a further embodiment of the invention, the compound of the formula I is administered in combination with nicotinic acid or "extended release niacin" in conjunction with MK-0524A (laropiprant) and with simvastatin.

In one embodiment of the invention, the compound of the formula I is administered in combination with nicotinic acid or another nicotinic acid receptor agonist and a prostaglandin DP receptor antagonist, for example those as described in WO2008039882.

In another embodiment of the invention, the compound of the formula I is administered in combination with a solid combination of niacin with meloxicam, as described, for example, in WO2009149056.

In another embodiment of the invention, the compound of the formula I is administered in combination with an agonist of GPR116, as described, for example, in WO2006067531, WO2006067532.

In one embodiment, the compound of the formula I is administered in combination with modulators of GPR40, as described, for example, in WO2007013689, WO2007033002, WO2007106469, US2007265332, WO2007123225, WO2007131619, WO2007131620, WO2007131621, US2007265332, WO2007131622, WO2007136572, WO2008001931 WO2008030520, WO2008030618, WO2008054674, WO2008054675, WO2008066097, US2008176912, WO2008130514, WO2009038204, WO2009039942, WO2009039943, WO2009048527, WO2009054479, WO2009058237, WO2009111056, WO2010012650.

In one embodiment, the compound of the formula I is administered in combination with modulators of GPR119 (G-protein-coupled glucose-dependent insulinotropic receptor), for example PSN-119-1, PSN-821, PSN-119-2, MBX-2982 or those as described, for example, in WO2004065380, WO2005061489 (PSN-632408), WO2006083491, WO2007003960-62 and WO2007003964, WO2007035355, WO2007116229, WO2007116230, WO2008005569, WO2008005576, WO2008008887, WO2008008895, WO2008025798, WO2008025799, WO2008025800, WO2008070692, WO2008076243, WO200807692, WO2008081204, WO2008081205, WO2008081206, WO2008081207, WO2008081208, WO2008083238, WO2008085316, WO2008109702, WO2008130581, WO2008130584, WO2008130615, WO2008137435, WO2008137436, WO2009012275, WO2009012277, WO2009014910, WO2009034388, WO2009038974, WO2009050522, WO2009050523, WO2009055331, WO2009105715, WO2009105717, WO2009105722, WO2009106561, WO2009106565, WO2009117421, WO2009125434, WO2009126535, WO2009129036, US2009286812, WO2009143049, WO2009150144, WO2010001166, WO2010004343, WO2010004344, WO2010004345, WO2010004346, WO2010004347, WO2010004348, WO2010008739, WO2010006191, WO2010009183, WO2010009195, WO2010009207, WO2010009208, WO2010014593.

In a further embodiment, the compound of the formula I is administered in combination with modulators of GPR120, as described, for example, in EP1688138, WO2008066131, WO2008066131, WO2008103500, WO2008103501, WO2008139879, WO2009038204, WO2009147990, WO2010008831.

In another embodiment, the compound of the formula I is administered in combination with antagonists of GPR105, as described, for example, in WO2009000087, WO2009070873.

In a further embodiment, the compound of the formula I is administered in combination with agonists of GPR43, for example ESN-282.

In one embodiment, the compound of the formula I is administered in combination with inhibitors of hormone-sensitive lipase (HSL) and/or phospholipases, as described, for example, in WO2005073199, WO2006074957, WO2006087309, WO2006111321, WO2007042178, WO2007119837, WO2008122352, WO2008122357, WO2009009287.

In one embodiment, the compound of the formula I is administered in combination with inhibitors of endothelial lipase, as described, for example, in WO2007110216.

In one embodiment, the compound of the formula I is administered in combination with a phospholipase A2 inhibitor, for example darapladib or A-002, or those as described in WO2008048866, WO20080488867, US2009062369.

In one embodiment, the compound of the formula I is administered in combination with myricitrin, a lipase inhibitor (WO2007119827).

In one embodiment, the compound of the formula I is administered in combination with an inhibitor of glycogen synthase kinase-3 beta (GSK-3 beta), as described, for example, in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110, US2007281949, WO2008002244, WO2008002245, WO2008016123, WO2008023239, WO2008044700, WO2008056266, WO2008057940, WO2008077138, EP1939191, EP1939192, WO2008078196, WO2008094992, WO2008112642, WO2008112651, WO2008113469, WO2008121063, WO2008121064, EP-1992620, EP-1992621, EP1992624, EP-1992625, WO2008130312, WO2009007029, EP2020232, WO2009017452, WO2009035634, WO2009035684 WO2009038385, WO2009095787, WO2009095788, WO2009095789, WO2009095792, WO2009145814, US2009291982, WO2009154697, WO2009156857, WO2009156859, WO2009156860, WO2009156861, WO2009156863, WO2009156864, WO2009156865, WO2010013168, WO2010014794.

In one embodiment, the compound of the formula I is administered in combination with an inhibitor of phosphoenolpyruvate carboxykinase (PEPCK), for example those as described in WO2004074288.

In one embodiment, the compound of the formula I is administered in combination with an inhibitor of phosphoinositide kinase-3 (PI3K), for example those as described in WO2008027584, WO2008070150, WO2008125833, WO2008125835, WO2008125839, WO2009010530, WO2009026345, WO2009071888, WO2009071890, WO2009071895.

In one embodiment, the compound of the formula I is administered in combination with an inhibitor of serum/glucocorticoid-regulated kinase (SGK), as described, for example, in WO2006072354, WO2007093264, WO2008009335, WO2008086854, WO2008138448.

In one embodiment, the compound of the formula I is administered in combination with a modulator of the glucocorticoid receptor, as described, for example, in WO2008057855, WO2008057856, WO2008057857, WO2008057859, WO2008057862, WO2008059867, WO2008059866, WO2008059865, WO2008070507, WO2008124665, WO2008124745, WO2008146871, WO2009015067, WO2009040288, WO2009069736, WO2009149139.

In one embodiment, the compound of the formula I is administered in combination with a modulator of the mineralocorticoid receptor (MR), for example drospirenone, or those as described in WO2008104306, WO2008119918.

In one embodiment, the compound of the formula I is administered in combination with an inhibitor of protein kinase C beta (PKC beta), for example ruboxistaurin, or those as described in WO2008096260, WO2008125945.

In one embodiment, the compound of the formula I is administered in combination with an inhibitor of protein kinase D, for example doxazosin (WO2008088006).

In a further embodiment, the compound of the formula I is administered in combination with an activator/modulator of the AMP-activated protein kinase (AMPK), as described, for example, in WO2007062568, WO2008006432, WO2008016278, WO2008016730, WO2008020607, WO2008083124, WO2008136642, WO2009019445, WO2009019446, WO2009019600, WO2009028891 WO2009065131, WO2009076631, WO2009079921, WO2009100130, WO2009124636, WO2009135580, WO2009152909.

In one embodiment, the compound of the formula I is administered in combination with an inhibitor of ceramide kinase, as described, for example, in WO2007112914, WO2007149865.

In a further embodiment, the compound of the formula I is administered in combination with an inhibitor of MAPK-interacting kinase 1 or 2 (MNK1 or 2), as described, for example, in WO2007104053, WO2007115822, WO2008008547, WO2008075741.

In one embodiment, the compound of the formula I is administered in combination with inhibitors of "I-kappaB kinase" (IKK inhibitors), as described, for example, in WO2001000610, WO2001030774, WO2004022057, WO2004022553, WO2005097129, WO2005113544, US2007244140, WO2008099072, WO2008099073, WO2008099073, WO2008099074, WO2008099075, WO2009056693, WO2009075277, WO2009089042, WO2009120801.

In another embodiment, the compound of the formula I is administered in combination with inhibitors of NF-kappaB (NFKB) activation, for example salsalate.

In a further embodiment, the compound of the formula I is administered in combination with inhibitors of ASK-1 (apoptosis signal-regulating kinase 1), as described, for example, in WO2008016131, WO2009123986.

In one embodiment of the invention, the compound of the formula I is administered in combination with an HMG-CoA reductase inhibitor such as simvastatin, fluvastatin, pravastatin, lovastatin, atorvastatin, cerivastatin, rosuvastatin, pitavastatin, L-659699, BMS-644950, NCX-6560, or those as described in US2007249583, WO2008083551, WO2009054682.

In a further embodiment of the invention, the compound of the formula I is administered in combination with a farnesoid X receptor (FXR) modulator, for example WAY-362450 or those as described in WO2003099821, WO2005056554, WO2007052843, WO2007070796, WO2007092751, JP2007230909, WO2007095174, WO2007140174, WO2007140183, WO2008000643, WO2008002573, WO2008025539, WO2008025540, JP2008214222, JP2008273847, WO2008157270, US2008299118, US2008300235, WO2009005998, WO2009012125, WO2009027264, WO2009062874, US2009131409, US2009137554, US2009163552, WO2009127321, EP2128158.

In another embodiment of the invention, the compound of the formula I is administered in combination with a ligand of the liver X receptor (LXR), as described, for example, in WO2007092965, WO2008041003, WO2008049047, WO2008065754, WO2008073825, US2008242677, WO2009020683, US2009030082, WO2009021868, US2009069373, WO2009024550, WO2009040289, WO2009086123, WO2009086129, WO2009086130, WO2009086138, WO2009107387, US2009247587, WO2009133692, WO2008138438, WO2009144961, WO2009150109.

In one embodiment of the invention, the compound of the formula I is administered in combination with a fibrate, for example fenofibrate, clofibrate, bezafibrate, or those as described in WO2008093655.

In one embodiment of the invention, the compound of the formula I is administered in combination with fibrates, for example the choline salt of fenofibrate (SLV-348; Trilipix™)_{.}

In one embodiment of the invention, the compound of the formula I is administered in combination with fibrates, for example the choline salt of fenofibrate (Trilipix™) and an HMG-CoA reductase inhibitor, for example rosuvastatin.

In a further embodiment of the invention, the compound of the formula I is administered in combination with bezafibrate and diflunisal.

In a further embodiment of the invention, the compound of the formula I is administered in combination with a solid combination of fenofibrate or a salt thereof with simvastatin, rosuvastatin, fluvastatin, lovastatin, cerivastatin, pravastatin, pitavastatin or atorvastatin.

In a further embodiment of the invention, the compound of the formula I is administered in combination with Synordia (R), a solid combination of fenofibrate with metformin.

In another embodiment of the invention, the compound of the formula I is administered in combination with a solid combination of metformin with an MTP inhibitor, as described in WO2009090210.

In one embodiment of the invention, the compound of the formula I is administered in combination with a cholesterol reabsorption inhibitor, for example ezetimibe, tiqueside, pamaqueside, FM-VP4 (sitostanol/campesterol ascorbyl phosphate; Forbes Medi-Tech, WO2005042692, WO2005005453), MD-0727 (Microbia Inc., WO2005021497,

WO2005021495) or with compounds as described in WO2002066464, WO2005000353 (Kotobuki Pharmaceutical Co. Ltd.) or WO2005044256 or WO2005062824 (Merck & Co.) or WO2005061451 and WO2005061452 (AstraZeneca AB) and WO2006017257 (Phenomix) or WO2005033100 (Lipideon Biotechnology AG), or as described in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358, WO2008033431, WO2008033465, WO2008052658, WO2008057336, WO2008085300, WO2008104875, US2008280836, WO2008108486.

In one embodiment of the invention, the compound of the formula I is administered in combination with an NPC1 L1 antagonist, for example those as described in WO2008033464, WO2008033465.

In one embodiment of the invention, the compound of the formula I is administered in combination with Vytorin™, a solid combination of ezetimibe with simvastatin.

In one embodiment of the invention, the compound of the formula I is administered in combination with a solid combination of ezetimibe with atorvastatin.

In one embodiment of the invention, the compound of the formula I is administered in combination with a solid combination of ezetimibe with fenofibrate.

In one embodiment of the invention, the further active ingredient is a diphenylazetidinone derivative, as described, for example, in US 6,992,067 or US 7,205,290.

In a further embodiment of the invention, the further active ingredient is a diphenylazetidinone derivative, as described, for example, in US 6,992,067 or US 7,205,290, combined with a statin, for example simvastatin, fluvastatin, pravastatin, lovastatin, cerivastatin, atorvastatin, pitavastatin or rosuvastatin.

In one embodiment of the invention, the compound of the formula I is administered in combination with a solid combination of lapaquistat, a squalene synthase inhibitor, with atorvastatin.

In a further embodiment of the invention, the compound of the formula I is administered in combination with a conjugate consisting of the HMG-CoA reductase inhibitor atorvastatin with the renin inhibitor aliskiren (WO2009090158).

In one embodiment of the invention, the compound of the formula I is administered in combination with a CETP inhibitor, for example torcetrapib, anacetrapib or JTT-705 (dalcetrapib), or those as described in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906, WO2008006257, WO2008009435, WO2008018529, WO2008058961, WO2008058967, WO2008059513, WO2008070496, WO2008115442, WO2008111604, WO2008129951, WO2008141077, US2009118287, WO2009062371, WO2009071509.

In one embodiment of the invention, the compound of the formula I is administered in combination with bile acid reabsorption inhibitors (inhibitors of the intestinal bile acid transporter (IBAT)) (see, for example, US 6,245,744, US 6,221,897 or WO00/61568), for example HMR 1741, or those as described in DE 10 2005 033099.1 and DE 10 2005 033100.9, DE 10 2006 053635, DE 10 2006 053637, WO2007009655-56, WO2008058628, WO2008058629, WO2008058630, WO2008058631.

In one embodiment, the compound of the formula I is administered in combination with agonists of GPBAR1 (G-protein-coupled bile acid receptor 1; TGR5), for example INT-777 or those as described, for example, in US20060199795, WO2007110237, WO2007127505, WO2008009407, WO2008067219, WO2008067222, FR2908310, WO2008091540, WO2008097976, US2009054304, WO2009026241, WO2009146772, WO2010014739, WO2010014836.

In one embodiment, the compound of the formula I is administered in combination with modulators of histone deacetylase, for example ursodeoxycholic acid, as described in WO2009011420.

In one embodiment, the compound of the formula I is administered in combination with inhibitors/modulators of the TRPM5 channel (TRP cation channel M5), as described, for example, in WO2008097504, WO2009038722.

In one embodiment, the compound of the formula I is administered in combination with inhibitors/modulators of the TRPA1 channel (TRP cation channel A1), as described, for example, in US2009176883, WO2009089083, WO2009144548.

In one embodiment, the compound of the formula I is administered in combination with inhibitors/modulators of the TRPV3 channel (TRP cation channel V3), as described, for example, in WO2009084034, WO2009130560.

In one embodiment of the invention, the compound of the formula I is administered in combination with a polymeric bile acid adsorber, for example cholestyramine, colesevelam hydrochloride.

In one embodiment of the invention, the compound of the formula I is administered in combination with colesevelam hydrochloride and metformin or a sulfonylurea or insulin.

In one embodiment of the invention, the compound of the formula I is administered in combination with tocotrienol and insulin or an insulin derivative.

In one embodiment of the invention, the compound of the formula I is administered in combination with a chewing gum comprising phytosterols (Reductol™).

In one embodiment of the invention, the compound of the formula I is administered in combination with an inhibitor of the microsomal triglyceride transfer protein (MTP inhibitor), for example implitapide, BMS-201038, R-103757, AS-1552133, SLx-4090, AEGR-733, JTT-130, or those as described in WO2005085226, WO2005121091, WO2006010423, WO2006113910, WO2007143164, WO2008049806, WO2008049808, WO2008090198, WO2008100423, WO2009014674.

In a further embodiment of the invention, the compound of the formula I is administered in combination with a combination of a cholesterol absorption inhibitor, for example ezetimibe, and an inhibitor of the triglyceride transfer protein (MTP inhibitor), for example implitapide, as described in WO2008030382 or in WO2008079398.

In one embodiment of the invention, the compound of the formula I is administered in combination with an active antihypertriglyceridemic ingredient, for example those as described in WO2008032980.

In another embodiment of the invention, the compound of the formula I is administered in combination with an antagonist of the somatostatin 5 receptor (SST5 receptor), for example those as described in WO2006094682.

In one embodiment of the invention, the compound of the formula I is administered in combination with an ACAT inhibitor, for example avasimibe, SMP-797 or KY-382, or those as described in WO2008087029, WO2008087030, WO2008095189, WO2009030746, WO2009030747, WO2009030750, WO2009030752, WO2009070130, WO2009081957, WO2009081957.

In a further embodiment of the invention, the compound of the formula I is administered in combination with an inhibitor of liver carnitine palmitoyltransferase-1 (L-CPT1), as described, for example, in WO2007063012, WO2007096251 (ST-3473), WO2008015081, US2008103182, WO2008074692, WO2008145596, WO2009019199, WO2009156479, WO2010008473.

In another embodiment of the invention, the compound of the formula I is administered in combination with an inhibitor of carnitin O-palmitoyltransferase II (CPT2), as described, for example, in US2009270500, US2009270505, WO2009132978, WO2009132979.

In a further embodiment of the invention, the compound of the formula I is administered in combination with a modulator of serine palmitoyltransferase (SPT), as described, for example, in WO2008031032, WO2008046071, WO2008083280, WO2008084300.

In one embodiment of the invention, the compound of the formula I is administered in combination with a squalene synthetase inhibitor, for example BMS-188494, TAK-475 (lapaquistat acetate), or as described in WO2005077907, JP2007022943, WO2008003424, WO2008132846, WO2008133288, WO2009136396.

In one embodiment of the invention, the compound of the formula I is administered in combination with ISIS-301012 (mipomersen), an antisense oligonucleotide which is capable of regulating the apolipoprotein B gene.

In one embodiment of the invention, the compound of the formula I is administered in combination with apolipoprotein (ApoB) SNALP, a therapeutic product which comprises an siRNA (directed against the ApoB gene).

In one embodiment of the invention, the compound of the formula I is administered in combination with a stimulator of the ApoA-1 gene, as described, for example, in WO2008092231.

In one embodiment of the invention, the compound of the formula I is administered in combination with a modulator of the synthesis of apolipoprotein C-III, for example ISIS-APOCIIIRx.

In one embodiment of the invention, the compound of the formula I is administered in combination with an LDL receptor inducer (see US 6,342,512), for example HMR1171, HMR1586, or those as described in WO2005097738, WO2008020607.

In another embodiment of the invention, the compound of the formula I is administered in combination with an HDL cholesterol-elevating agent, for example those as described in WO2008040651, WO2008099278, WO2009071099, WO2009086096, US2009247550.

In one embodiment of the invention, the compound of the formula I is administered in combination with an ABCA1 expression enhancer, as described, for example, in WO2006072393, WO2008062830, WO2009100326.

In one embodiment of the invention, the compound of the formula I is administered in combination with a lipoprotein lipase modulator, for example ibrolipim (NO-1886).

In one embodiment of the invention, the compound of the formula I is administered in combination with a lipoprotein(a) antagonist, for example gemcabene (CI-1027).

In one embodiment of the invention, the compound of the formula I is administered in combination with a lipase inhibitor, for example orlistat or cetilistat (ATL-962).

In one embodiment of the invention, the compound of the formula I is administered in combination with an adenosine A1 receptor agonist (adenosine A1 R), for example CVT-3619 or those as described, for example, in EP1258247, EP1375508, WO2008028590, WO2008077050, WO2009050199, WO2009080197, WO2009100827, WO2009112155.

In one embodiment of the invention, the compound of the formula I is administered in combination with an adenosine A2B receptor agonist (adenosine A2B R), for example ATL-801.

In another embodiment of the invention, the compound of the formula I is administered in combination with a modulator of adenosine A2A and/or adenosine A3 receptors, as described, for example, in WO2007111954, WO2007121918, WO2007121921, WO2007121923, WO2008070661, WO2009010871.

In a further embodiment of the invention, the compound of the formula I is administered in combination with a ligand of the adenosine A1/A2B receptors, as described, for example, in WO2008064788, WO2008064789, WO 2009080198, WO2009100827, WO2009143992.

In one embodiment of the invention, the compound of the formula I is administered in combination with an adenosine A2B receptor antagonist (adenosine A2B R), as described in US2007270433, WO2008027585, WO2008080461, WO2009037463, WO2009037467, WO2009037468, WO2009118759.

In one embodiment, the compound of the formula I is administered in combination with inhibitors of acetyl-CoA carboxylase (ACC1 and/or ACC2), for example those as described in WO199946262, WO200372197, WO2003072197, WO2005044814, WO2005108370, JP2006131559, WO2007011809, WO2007011811, WO2007013691, WO2007095601-603, WO2007119833, WO2008065508, WO2008069500, WO2008070609, WO2008072850, WO2008079610, WO2008088688, WO2008088689, WO2008088692, US2008171761, WO2008090944, JP2008179621, US2008200461, WO2008102749, WO2008103382, WO2008121592, WO2009082346, US2009253725, JP2009196966, WO2009144554, WO2009144555, WO2010003624, WO2010002010.

In another embodiment, the compound of the formula I is administered in combination with modulators of microsomal acyl-CoA:glycerol-3-phosphate acyltransferase 3 (GPAT3, described in WO2007100789) or with modulators of microsomal acyl-CoA:glycerol-3-phosphate acyltransferase 4 (GPAT4, described in WO2007100833) or with modulators of mitochondrial glycerol-3-phosphate O-acyltransferase, described in WO2010005922.

In a further embodiment, the compound of the formula I is administered in combination with modulators of xanthine oxidoreductase (XOR).

In another embodiment, the compound of the formula I is administered in combination with inhibitors of soluble epoxide hydrolase (sEH), as described, for example, in WO2008051873, WO2008051875, WO2008073623, WO2008094869, WO2008112022, WO2009011872, WO2009049154, WO2009049157, WO2009049165, WO2009073772, WO2009097476, WO2009111207, WO2009129508, WO2009151800.

In a further embodiment, the compound of the formula I is administered in combination with CART modulators (see "Cocaine-amphetamine-regulated transcript influences energy metabolism, anxiety and gastric emptying in mice" Asakawa, A. et al.: Hormone and Metabolic Research (2001), 33(9), 554-558);
NPY antagonists, for example 4-[(4-aminoquinazolin-2-ylamino)methyl]-cyclohexylmethylnaphthalene-1-sulfonamide hydrochloride (CGP 71683A) or velneperit or those as described in WO2009110510;
NPY-5 receptor antagonists/receptor modulators, such as L-152804 or the compound "NPY-5-BY" from Banyu, or as described, for example, in WO2006001318, WO2007103295, WO2007125952, WO2008026563, WO2008026564, WO2008052769, WO2008092887, WO2008092888, WO2008092891, WO2008129007, WO2008134228, WO2009054434, WO2009095377, WO2009131096;
NPY-4 receptor antagonists, as described, for example, in WO2007038942;
NPY-2 receptor antagonists/modulators, as described, for example, in WO2007038943, WO2009006185, US2009099199, US2009099243, US2009099244, WO2009079593, WO2009079597;
peptide YY 3-36 (PYY3-36) or analogous compounds, for example CJC-1682 (PYY3-36 conjugated with human serum albumin via Cys34) or CJC-1643 (derivative of PYY3-36, which is conjugated in vivo to serum albumin), or those as described in WO2005080424, WO2006095166, WO2008003947, WO2009080608;
NPY-2 receptor agonists, as described, for example, in WO2009080608;
derivatives of the peptide obestatin, as described by WO2006096847;
CB1 R (cannabinoid receptor 1) antagonists/inverse agonists, for example rimonabant, surinabant (SR147778), SLV-319 (ibipinabant), AVE-1625, taranabant (MK-0364) or salts thereof, otenabant (CP-945,598), rosonabant, V-24343 or those compounds as described in, for example, EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249 WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006018662, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459 WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863 WO2007119001, WO2007120454, WO2007121687, WO2007123949, US2007259934, WO2007131219, WO2007133820, WO2007136571, WO2007136607, WO2007136571, US7297710, WO2007138050, WO2007139464, WO2007140385, WO2007140439, WO2007146761, WO2007148061, WO2007148062, US2007293509, WO2008004698, WO2008017381, US2008021031, WO2008024284, WO2008031734, WO2008032164, WO2008034032, WO2008035356, WO2008036021, WO008036022, WO2008039023, WO2998043544, WO2008044111, WO2008048648, EP1921072-A1, WO2008053341, WO2008056377, WO2008059207, WO2008059335, WO2008062424, WO2008068423, WO2008068424, WO2008070305, WO2008070306, WO2008074816, WO2008074982, WO2008075012, WO2008075013, WO2008075019, WO2008075118, WO2008076754, WO2008081009, WO2008084057, EP1944295, US2008090809, US2008090810, WPO2008092816, WO2008094473, WO2008094476, WO2008099076, WO2008099139, WO2008101995, US2008207704, WO2008107179, WO2008109027, WO2008112674, WO2008115705, WO2008118414, WO2008119999, WO200812000, WO2008121257, WO2008127585, WO2008129157, WO2008130616, WO2008134300, US2008262066, US2008287505, WO2009005645, WO2009005646, WO2009005671, WO2009023292, WO2009023653, WO2009024819, WO2009033125, EP2042175, WO2009053548-WO2009053553, WO2009054923, WO2009054929, WO2009059264, WO2009073138, WO2009074782, WO2009075691, WO2009078498, WO2009087285, WO2009074782, WO2009097590, WO2009097995, WO2009097996, WO2009097998, WO2009097999, WO2009098000, WO2009106708, US2009239909, WO2009118473, US2009264436, US2009264476, WO2009130234, WO2009131814, WO2009131815, US2009286758, WO2009141532, WO2009141533, WO2009153569, WO2010003760, WO2010012437, WO2010019762;
cannabinoid receptor 1/cannabinoid receptor 2 (CB1,/CB2) modulating compounds, for example delta-9-tetrahydrocannabivarin, or those as described, for example, in WO2007001939, WO2007044215, WO2007047737, WO2007095513, WO2007096764, WO2007112399, WO2007112402, WO2008122618, WO2009007697, WO2009012227, WO2009087564, WO2009093018, WO2009095752, WO2009120660, WO2010012964;
cannabinoid receptor 2 (CB2) modulating compounds, for example those as described, for example, in WO2008063625, WO2008157500, WO2009004171, WO2009032754, WO2009055357, WO2009061652, WO2009063495, WO2009067613, WO2009114566;
modulators of FAAH (fatty acid amide hydrolase), as described, for example, in WO2007140005, WO2008019357, WO2008021625, WO2008023720, WO2008030532, WO2008129129, WO2008145839, WO2008145843, WO2008147553, WO2008153752, WO2009011904, WO2009048101, WO2009084970, WO2009105220, WO2009109504, WO2009109743, WO2009117444, WO2009127944, WO92009138416 WO2009151991, WO2009152025, WO2009154785, WO2010005572, WO2010017079;
inhibitors of fatty acid synthase (FAS), as described, for example, in WO2008057585, WO2008059214, WO2008075064, WO2008075070, WO2008075077, WO2009079860;
inhibitors of LCE (long chain fatty acid elongase)/long chain fatty acid CoA ligase, as described, for example, in WO2008120653, WO2009038021, WO2009044788, WO2009081789, WO2009099086;
vanilloid-1 receptor modulators (modulators of TRPV1), as described, for example, in WO2007091948, WO2007129188, WO2007133637, WO2008007780, WO2008010061, WO2008007211, WO2008010061, WO2008015335, WO2008018827, WO2008024433, WO2008024438, WO2008032204, WO2008050199, WO2008059339, WO2008059370, WO2008066664, WO2008075150, WO2008090382, WO2008090434, WO2008093024, WO2008107543, WO2008107544, WO2008110863, WO2008125295, WO2008125296, WO2008125337, WO2008125342, WO2008132600, WO2008133973, WO2009010529, WO2009010824, WO2009016241, WO2009023539, WO2009038812, WO2009050348, WO2009055629, WO2009055749, WO2009064449, WO2009081222, WO2009089057, WO2009109710WO2009112677, WO2009112678, WO2009112679, WO2009121036, WO2009124551, WO2009136625, WO2010002209;
modulators, ligands, antagonists or inverse agonists of the opioid receptors, for example GSK-982 or those as described, for example, in WO2007047397, WO2008021849, WO2008021851, WO2008032156, WO2008059335, WO2008125348, WO2008125349, WO2008142454, WO2009030962, WO2009103552, WO2009115257;
modulators of the "orphan opioid (ORL-1) receptor", as described, for example, in US2008249122, WO2008089201;
agonists of the prostaglandin receptor, for example bimatoprost or those compounds as described in WO2007111806;
MC4 receptor agonists (melanocortin-4 receptor agonists, MC4R agonists, for example N-[2-(3a-benzyl-2-methyl-3-oxo-2,3,3a,4,6,7-hexahydropyrazolo[4,3-c]pyridin-5-yl)-1-(4-chlorophenyl)-2-oxoethyl]-1-amino-1,2,3,4-tetrahydronaphthalene-2-carboxamide; (WO 01/91752)) or LB53280, LB53279, LB53278 or THIQ, MB243, RY764, CHIR-785, PT-141, MK-0493, or those as described in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2004089307, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763, WO2007141343, WO2008007930, WO2008017852, WO2008039418, WO2008087186, WO2008087187, WO2008087189, WO2008087186-WO2008087190, WO2008090357, WO2008142319, WO2009015867, WO2009061411, US2009076029, US2009131465, WO2009071101, US2009305960, WO2009144432, WO2009151383, WO2010015972;
MC4 receptor modulators (melanocortin-4 receptor modulators), as described, for example, in WO2009010299, WO2009074157;
orexin receptor 1 antagonists (OX1 R antagonists), orexin receptor 2 antagonists (OX2R antagonists) or mixed OX1 R/OX2R antagonists (e.g. 1-(2-methylbenzoxazol-6-yl)-3-[1,5]naphthyridin-4-ylurea hydrochloride (SB-334867-A), or those as described, for example, in WO200196302, WO200185693, WO2004085403, WO2005075458, WO2006067224, WO2007085718, WO2007088276, WO2007116374, WO2007122591, WO2007126934, WO2007126935, WO2008008517, WO2008008518, WO2008008551, WO2008020405, WO2008026149, WO2008038251, US2008132490, WO2008065626, WO2008078291, WO2008087611, WO2008081399, WO2008108991, WO2008107335,
US2008249125, WO2008147518, WO2008150364, WO2009003993, WO2009003997, WO2009011775, WO2009016087, WO2009020642, WO2009058238, US2009186920, US2009203736, WO2009092642, WO2009100994, WO2009104155, WO2009124956, WO2009133522, WO2009156951, WO2010017260);
histamine H3 receptor antagonists/inverse agonists (e.g. 3-cyclohexyl-1-(4,4-dimethyl-1,4,6,7-tetrahydroimidazo[4,5-c]pyridin-5-yl)propan-1-one oxalic acid salt (WO 00/63208), or those as described in WO200064884, WO2005082893, WO2005123716, US2005171181 (e.g. PF-00389027), WO2006107661, WO2007003804, WO2007016496, WO2007020213, WO2007049798, WO2007055418, WO2007057329, WO2007062999, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111, WO2007137955, US2007281923, WO2007137968, WO2007138431, WO2007146122, WO2008005338, WO2008012010, WO2008015125, WO2008045371, EP1757594, WO2008068173, WO2008068174, US20080171753, WO2008072703, WO2008072724, US2008188484, US2008188486, US2008188487, WO2008109333, WO2008109336, WO2008126886, WO2008154126, WO2008151957, US2008318952, WO2009003003, WO2009013195, WO2009036132, WO2009039431, WO2009045313, WO2009058300, WO2009063953, WO2009067401, WO2009067405, WO2009067406, US2009163464, WO2009100120, WO2009105206, WO2009121812, WO2009126782, WO2010011653, WO2010011657);
histamine H1/histamine H3 modulators, for example betahistine or its dihydrochloride;
modulators of the histamine H3 transporter or of the histamine H3/serotonin transporter, as described, for example, in WO2008002816, WO2008002817, WO2008002818, WO2008002820;
modulators of vesicular monoamine transporter 2 (VMAT2), as described, for example, in WO2009126305;
histamine H4 modulators, as described, for example, in WO2007117399, US2009156613;
CRF antagonists (e.g. [2-methyl-9-(2,4,6-trimethylphenyl)-9H-1,3,9-triazafluoren-4-yl]dipropylamine (WO 00/66585) or those CRF1 antagonists as described in WO2007105113, WO2007133756, WO2008036541, WO2008036579, WO2008083070, WO2010015628, WO2010015655);
CRF BP antagonists (e.g. urocortin);
urocortin agonists;
modulators of the beta-3 adrenoceptor, for example 1-(4-chloro-3-methanesulfonylmethylphenyl)-2-[2-(2,3-dimethyl-1 H-indol-6-yloxy)ethylamino]ethanol hydrochloride (WO 01/83451) or solabegron (GW-427353) or N-5984 (KRP-204), or those as described in JP2006111553, WO2002038543, WO2002038544, WO2007048840-843, WO2008015558, EP1947103, WO2008132162;
MSH (melanocyte-stimulating hormone) agonists;
MCH (melanine-concentrating hormone) receptor antagonists (for example NBI-845, A-761, A-665798, A-798, ATC-0175, T-226296, T-71 (AMG-071, AMG-076), GW-856464, NGD-4715, ATC-0453, ATC-0759, GW-803430, or those compounds as described in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916, WO2007141200, WO2007142217, US2007299062, WO2007146758, WO2007146759, WO2008001160, WO2008016811, WO2008020799, WO2008022979, WO2008038692, WO2008041090, WO2008044632, WO2008047544, WO2008061109, WO2008065021, WO2008068265, WO2008071646, WO2008076562, JP2008088120, WO2008086404, WO2008086409, US2008269110, WO2008140239, WO2009021740, US2009011994, US2009082359, WO2009041567, WO2009076387, WO2009089482, WO2009103478, WO2009119726, WO2009120655, WO2009123194, WO2009137270, WO2009146365, WO2009154132);
CCK-A (CCK-1) modulators (for example {2-[4-(4-chloro-2,5-dimethoxyphenyl)-5-(2-cyclohexylethyl)-thiazol-2-ylcarbamoyl]-5,7-dimethylindol-1-yl}acetic acid trifluoroacetic acid salt (WO 99/15525) or SR-146131 (WO 0244150) or SSR-125180) or those as described in WO2005116034, WO2007120655, WO2007120688, WO2007120718, WO2008091631;
serotonin reuptake inhibitors (e.g. dexfenfluramine), or those as described in WO2007148341, WO2008034142, WO2008081477, WO2008120761, WO2008141081, WO2008141082, WO2008145135, WO2008150848, WO2009043834, WO2009077858;
mixed serotonin/dopamine reuptake inhibitors (e.g. bupropion), or those as described in WO2008063673, or solid combinations of bupropion with naltrexone or bupropion with zonisamide;
mixed reuptake inhibitors, for example DOV-21947 or those as described in WO2009016214, WO2009016215, WO2009077584, WO2009098208,
WO2009098209, WO2009106769, WO2009109517, WO2009109518, WO2009109519, WO2009109608, WO2009145357, WO2009149258;
mixed serotoninergic and noradrenergic compounds (e.g. WO 00/71549);
5-HT receptor agonists, for example 1-(3-ethylbenzofuran-7-yl)piperazine oxalic acid salt (WO 01/09111);
mixed dopamine/norepinephrine/acetylcholine reuptake inhibitors (e.g. tesofensine), or those as described, for example, in WO2006085118, WO2008150480;
dopamine antagonists, as described, for example, in WO2008079838, WO2008079839, WO2008079847, WO2008079848;
norepinephrine reuptake inhibitors, as described, for example, in US2008076724, WO2009062318;
5-HT1A receptor modulators, as described, for example, in WO2009006227, WO2009137679, WO2009137732;
5-HT2A receptor antagonists, as described, for example, in WO2007138343;
5-HT2C receptor agonists (for example lorcaserine hydrochloride (APD-356) or BVT-933, or those as described in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213, WO2008007661, WO2008007664, WO2008009125, WO2008010073, WO2008108445, WO2009063991, WO2009063992, WO2009063993, WO2009079765);
5-HT6 receptor modulators, for example E-6837, BVT-74316, PF-3246799 or PRX-07034, or those as described, for example, in WO2005058858, WO2007054257, WO2007107373, WO2007108569, WO2007108742-744, WO2008003703, WO2008027073, WO2008034815, WO2008054288, EP1947085, WO2008084491, WO2008084492, WO2008092665, WO2008092666, WO2008101247, WO2008110598, WO2008116831, WO2008116833, WO2008117169, WO2008136017, WO2008147812, EP2036888, WO2009013010, WO2009034581, WO2009053997, WO2009056632, WO2009073118, WO2009115515, WO2009135925, WO2009135927, WO2010000456, WO2010012806, EP2145887;
agonists of estrogen receptor gamma (ERRγ agonists), as described, for example, in WO2007131005, WO2008052709;
agonists of estrogen receptor alpha (ERRα / ERR1 agonists), as described, for example, in WO2008109727;
agonists of estrogen receptor beta (ERRβ agonists), as described, for example, in WO2009055734, WO2009100335, WO2009127686;
sigma-1 receptor antagonists, as described, for example, in WO2007098953, WO2007098961, WO2008015266, WO2008055932, WO2008055933, WO2009071657;
muscarin 3 receptor (M3R) antagonists, as described, for example, in WO2007110782, WO2008041184;
bombesin receptor agonists (BRS-3 agonists), as described, for example, in WO2008051404, WO2008051405, WO2008051406, WO2008073311;
galanin receptor antagonists;
growth hormone (e.g. human growth hormone or AOD-9604);
growth hormone releasing compounds (tert-butyl 6-benzyloxy-1-(2-diisopropylaminoethylcarbamoyl)-3,4-dihydro-1H-isoquinoline-2-carboxylate (WO 01/85695));
growth hormone secretagogue receptor antagonists (ghrelin antagonists), for example A-778193, or those as described in WO2005030734, WO2007127457, WO2008008286, WO2009056707;
growth hormone secretagogue receptor modulators (ghrelin modulators), for example JMV-2959, JMV-3002, JMV-2810, JMV-2951, or those as described in WO2006012577 (e.g. YIL-781 or YIL-870), WO2007079239, WO2008092681, WO2008145749, WO2008148853, WO2008148854, WO2008148856, WO2009047558, WO2009071283, WO2009115503;
TRH agonists (see, for example, EP 0 462 884);
decoupling protein 2 or 3 modulators (as described, for example, in WO2009128583);
chemical decouplers (e.g. WO2008059023, WO2008059024, WO2008059025, WO2008059026);
leptin receptor agonists (see, for example, Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
leptin receptor modulators, as described, for example, in WO2009019427, WO2009071658, WO2009071668, WO2009071677, WO2009071678, WO2009147211, WO2009147216, WO2009147219, WO2009147221;
DA agonists (bromocriptin, bromocriptin mesylate, doprexin) or those as described in US2009143390;
lipase/amylase inhibitors (e.g. WO 00/40569, WO2008107184, WO2009049428, WO2009125819);
inhibitors of diacylglycerol O-acyltransferases (DGATs), for example BAY-74-4113, or as described, for example, in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311, WO2007141502, WO2007141517, WO2007141538, WO2007141545, WO2007144571, WO2008011130, WO2008011131, WO2008039007, WO2008048991, WO2008067257, WO2008099221, WO2008129319, WO2008141976, WO2008148840, WO2008148849, WO2008148851, WO2008148868, WO2009011285, WO2009016462, WO2009024821, US2009076275, WO2009040410, WO2009071483, WO2009081195, WO2009119534, WO2009126624, WO2009126861, WO2010007046, WO2010017040;
inhibitors of monoacylglycerol acyltransferase (2-acylglycerol O-acyltransferase; MGAT), as described, for example, in WO2008038768;
inhibitors of fatty acid synthase (FAS), for example C75, or those as described in WO2004005277, WO2008006113;
inhibitors of stearoyl-CoA delta9 desaturase (SCD1), as described, for example, in WO2007009236, WO2007044085, WO2007046867, WO2007046868, WO20070501124, WO2007056846, WO2007071023, WO2007130075, WO2007134457, WO2007136746, WO2007143597, WO2007143823, WO2007143824, WO2008003753, WO2008017161, WO2008024390, WO2008029266, WO2008036715, WO2008043087, WO2008044767, WO2008046226, WO2008056687, WO2008062276, WO2008064474, WO2008074824, WO2008074832, WO2008074833, WO2008074834, WO2008074835, WO2008089580, WO2008096746, WO2008104524, WO2008116898, US2008249100, WO2008120744, WO2008120759, WO2008123469, WO2008127349, WO2008128335, WO2008135141, WO2008139845, WO2008141455, US20080255130, US2008255161, WO2008141455, WO2009010560, WO2009016216, WO2009012573, WO2009024287, JP2009019013, WO2009037542, WO2009056556, WO2009060053, WO2009060054, WO2009070533, WO2009073973, WO2009103739, WO2009117659, WO2009117676, US2009253693, US2009253738, WO2009124259, WO2009126123, WO2009126527, WO2009129625, WO2009137201, WO2009150196, WO2009156484, WO2010006962, WO2010007482;
inhibitors of fatty acid desaturase 1 (delta5 desaturase), as described, for example, in WO2008089310;
inhibitors of monoglyceride lipase (MGL), as described in WO2008145842;
hypoglycemic/hypertriglyceridemic indoline compounds, as described in WO2008039087, WO2009051119;
inhibitors of "adipocyte fatty acid-binding protein aP2", for example BMS-309403 or those as described in WO2009028248;
activators of adiponectin secretion, as described, for example, in WO2006082978, WO2008105533, WO2008136173;
promoters of adiponectin production, as described, for example, in WO2007125946, WO2008038712;
modified adiponectins, as described, for example, in WO2008121009;
oxyntomodulin or analogs thereof (for example, TKS-1225);
oleoyl-estrone
or agonists or partial agonists of the thyroid hormone receptor (thyroid hormone receptor agonists), for example: KB-2115 (eprotirome), QRX-431 (sobetirome) or DITPA, or those as described in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864, WO2008001959, WO2008106213, JP2009155261;
or agonists of the thyroid hormone receptor beta (TR-beta), for example MB-07811 or MB-07344, or those as described in WO2008062469.

In one embodiment of the invention, the compound of the formula I is administered in combination with a combination of eprotirome with ezetimibe.

In one embodiment of the invention, the compound of the formula I is administered in combination with an inhibitor of site-1 protease (S1 P), for example PF-429242.

In a further embodiment of the invention, the compound of the formula I is administered in combination with a modulator of the "trace amine associated receptor 1" (TAAR1), as described, for example, in US2008146523, WO2008092785.

In one embodiment of the invention, the compound of the formula I is administered in combination with an inhibitor of growth factor receptor bound protein 2 (GRB2), as described, for example, in WO2008067270.

In a further embodiment of the invention, the compound of the formula I is administered in combination with an RNAi (siRNA) therapeutic agent directed against PCSK9 (proprotein convertase subtilisin/kexin type 9).

In one embodiment, the compound of the formula I is administered in combination with Omacor® or Lovaza™ (omega-3 fatty acid ester; highly concentrated ethyl ester of eicosapentaenoic acid and of docosahexaenoic acid).

In one embodiment, the compound of the formula I is administered in combination with lycopene.

In one embodiment of the invention, the compound of the formula I is administered in combination with an antioxidant, for example OPC-14117, AGI-1067 (succinobucol), probucol, tocopherol, ascorbic acid, β-carotene or selenium, or those as described in WO2009135918.

In one embodiment of the invention, the compound of the formula I is administered in combination with a vitamin, for example vitamin B6 or vitamin B12.

In one embodiment, the compound of the formula I is administered in combination with more than one of the aforementioned compounds, for example in combination with a sulfonylurea and metformin, a sulfonylurea and acarbose, repaglinide and metformin (PrandiMet (TM)), insulin and a sulfonylurea, insulin and metformin, insulin and troglitazone, insulin and lovastatin, etc.

In a further embodiment, the compound of the formula I is administered in combination with an activator of soluble guanylate cyclase (sGC), as described, for example, in WO2009032249.

In another embodiment, the compound of the formula I is administered in combination with an inhibitor of carboanhydrase type 2 (carbonic anhydrase type 2), for example those as described in WO2007065948, WO2009050252.

In another embodiment, the compound of the formula I is administered in combination with topiramat or a derivative thereof, as described in WO2008027557, US2009304789.

In a further embodiment, the compound of the formula I is administered in combination with a solid combination of topiramat with phentermin (Qnexa™).

In a further embodiment, the compound of the formula I is administered in combination with an antisense compound, e.g. ISIS-377131, which inhibits the production of the glucocorticoid receptor.

In another embodiment, the compound of the formula I is administered in combination with an aldosterone synthase inhibitor and an antagonist of the glucocorticoid receptor, a cortisol synthesis inhibitor and/or an antagonist of the corticotropin releasing factor, as described, for example, in EP1886695, WO2008119744.

In one embodiment, the compound of the formula I is administered in combination with an agonist of the RUP3 receptor, as described, for example, in WO2007035355, WO2008005576.

In another embodiment, the compound of the formula I is administered in combination with an activator of the gene which codes for ataxia telangiectasia mutated (ATM) protein kinase, for example chloroquine.

In one embodiment, the compound of the formula I is administered in combination with a tau protein kinase 1 inhibitor (TPK1 inhibitor), as described, for example, in WO2007119463, WO2009035159, WO2009035162.

In one embodiment, the compound of the formula I is administered in combination with a "c-Jun N-terminal kinase" inhibitor (JNK inhibitor), for example B1-78D3 or those as described, for example, in WO2007125405, WO2008028860, WO2008118626.

In one embodiment, the compound of the formula I is administered in combination with an endothelin A receptor antagonist, for example avosentan (SPP-301).

In one embodiment, the compound of the formula I is administered in combination with inhibitors of neutral endopeptidase (NEP inhibitors), as described, for example, in WO2009138122, WO2009135526.

In one embodiment, the compound of the formula I is administered in combination with modulators of the glucocorticoid receptor (GR), for example KB-3305 or those compounds as described, for example, in WO2005090336, WO2006071609, WO2006135826, WO2007105766, WO2008120661, WO2009040288, WO2009058944, WO2009108525, WO2009111214.

In one embodiment, the further active ingredient is varenicline tartrate, a partial agonist of the alpha 4-beta 2 nicotinic acetylcholine receptor.

In one embodiment, the further active ingredient is an agonist of the alpha 7-nicotinic acetylcholine receptor, as described, for example, in WO2009018551, WO2009071519, WO2009071576, WO2009071577.

In one embodiment, the further active ingredient is trodusquemine.

In one embodiment, the further active ingredient is a modulator of the enzyme SIRT1 and/or SIRT3 (an NAD⁺-dependent protein deacetylase); this active ingredient may, for example, be resveratrol in suitable formulations, or those compounds as specified in WO2007019416 (e.g. SRT-1720), WO2008073451, WO2008156866, WO2008156869, WO2009026701, WO2009049018, WO2009058348, WO2009061453, WO2009134973, WO2009146358, WO2010003048.

In one embodiment of the invention, the further active ingredient is DM-71 (N-acetyl-L-cysteine with bethanechol).

In one embodiment, the compound of the formula I is administered in combination with antihypercholesterolemic compounds, as described, for example, in WO2004000803, WO2006000804, WO2004000805, WO2004087655, WO2005113496,

WO2007059871, WO2007107587, WO2007111994, WO2008052658, WO2008106600, WO2008113796, US2008280836, WO2009113952, US2009312302

In a further embodiment, the compound of the formula I is administered in combination with inhibitors of SREBP (sterol regulatory element-binding protein), for example fatostatin, or those as described, for example, in WO2008097835.

In another embodiment, the compound of the formula I is administered in combination with a cyclic peptide agonist of the VPAC2 receptor, as described, for example, in WO2007101146, WO2007133828.

In a further embodiment, the compound of the formula I is administered in combination with an agonist of the endothelin receptor, as described, for example, in WO2007112069.

In a further embodiment, the compound of the formula I is administered in combination with AKP-020 (bis(ethylmaltolato)oxovanadium(IV)).

In another embodiment, the compound of the formula I is administered in combination with tissue-selective androgen receptor modulators (SARM), as described, for example, in WO2007099200, WO2007137874.

In a further embodiment, the compound of the formula I is administered in combination with an AGE (advanced glycation endproduct) inhibitor, as described, for example, in JP2008024673.

In one embodiment of the invention, the further active ingredient is leptin; see, for example, "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

In another embodiment of the invention, the further active ingredient is metreleptin (recombinant methionyl-leptin) combined with pramlintide.

In a further embodiment of the invention, the further active ingredient is the tetrapeptide ISF-402.

In one embodiment, the further active ingredient is dexamphetamine or amphetamine.

In one embodiment, the further active ingredient is fenfluramine or dexfenfluramine. In another embodiment, the further active ingredient is sibutramine or those derivatives as described in WO2008034142.

In one embodiment, the further active ingredient is mazindol or phentermin.

In a further embodiment, the further active ingredient is geniposidic acid (WO2007100104) or derivatives thereof (JP2008106008).

In another embodiment, the further active ingredient is a neuropeptide FF2 agonist, as described, for example, in WO2009038012.

In one embodiment, the further active ingredient is a nasal calcium channel blocker, for example diltiazem, or those as described in US 7,138,107.

In one embodiment, the further active ingredient is an inhibitor of sodium-calcium ion exchange, for example those as described in WO2008028958, WO2008085711.

In a further embodiment, the further active ingredient is a blocker of calcium channels, for example of CaV3.2 or CaV2.2, as described in WO2008033431, WO2008033447, WO2008033356, WO2008033460, WO2008033464, WO2008033465, WO2008033468, WO2008073461.

In one embodiment, the further active ingredient is a modulator of a calcium channel, for example those as described in WO2008073934, WO2008073936, WO2009107660.

In one embodiment, the further active ingredient is an inhibitor of the calcium metabolism, for example those as described in US2009124680.

In one embodiment, the further active ingredient is a blocker of the "T-type calcium channel", as described, for example, in WO2008033431, WO2008110008, US2008280900, WO2008141446, US2009270338, WO2009146540, US2009325979, WO2009146539.

In one embodiment, the further active ingredient is an inhibitor of KCNQ potassium channel 2 or 3, for example those as described in US2008027049, US2008027090.

In one embodiment, the further active ingredient is a modulator of KCNN potassium channel 1, 2 or 3 (modulators of the SK1, SK2 and/or SK3 channel), for example those as described in US2009036475.

In one embodiment, the further active ingredient is an inhibitor of the potassium Kv1.3 ion channel, for example those as described in WO2008040057, WO2008040058, WO2008046065, WO2009043117.

In one embodiment, the further active ingredient is a potassium channel modulator, for example those as described in WO2008135447, WO2008135448, WO2008135591, WO2009099820.

In a further embodiment, the further active ingredient is a hyperpolarization-activated cyclic nucleotide-gated (HCN) potassium-sodium channel inhibitor, for example those as described in US2009069296.

In another embodiment, the further active ingredient is an inhibitor of the sodium-potassium-2 chloride (NKCCI) cotransporter, for example those as described in WO2009130735.

In another embodiment, the further active ingredient is a voltage-gated sodium channel inhibitor, for example those as described in WO2009049180, WO2009049181.

In another embodiment, the further active ingredient is a modulator of the MCP-1 receptor (monocyte chemoattractant protein-1 (MCP-1)), for example those as described in WO2008014360, WO2008014381.

In one embodiment, the further active ingredient is a modulator of somatostatin receptor 3 (SSTR3), for example those as described in WO2009011836.

In one embodiment, the further active ingredient is a modulator of somatostatin receptor 5 (SSTR5), for example those as described in WO2008019967, US2008064697, US2008249101, WO2008000692, US2008293756, WO2008148710.

In one embodiment, the further active ingredient is a modulator of somatostatin receptor 2 (SSTR2), for example those as described in WO2008051272.

In one embodiment, the further active ingredient is a compound which is capable of reducing the amount of retinol-binding protein 4 (RBP4), for example those as described in WO2009051244, WO2009145286.

In one embodiment, the further active ingredient is an erythropoietin-mimetic peptide which acts as an erythropoietin (EPO) receptor agonist. Such molecules are described, for example, in WO2008042800.

In a further embodiment, the further active ingredient is an anorectic/a hypoglycemic compound, for example those as described in WO2008035305, WO2008035306, WO2008035686.

In one embodiment, the further active ingredient is an inductor of lipoic acid synthetase, for example those as described in WO2008036966, WO2008036967.

In one embodiment, the further active ingredient is a stimulator of endothelial nitric oxide synthase (eNOS), for example those as described in WO2008058641, WO2008074413.

In one embodiment, the further active ingredient is a modulator of carbohydrate and/or lipid metabolism, for example those as described in WO2008059023, WO2008059024, WO2008059025, WO2008059026.

In a further embodiment, the further active ingredient is an angiotensin II receptor antagonist, for example those as described in WO2008062905, WO2008067378, WO2008062905.

In one embodiment, the further active ingredient is an agonist of the sphingosine 1-phosphate receptor (S1 P), for example those as described in WO2008064315, WO2008074820, WO2008074821, WO2008135522, WO2009019167, WO2009043013, WO2009080663, WO2009085847, WO2009151529, WO2009151621, WO2009151626, WO2009154737.

In one embodiment, the further active ingredient is an agent which retards gastric emptying, for example 4-hydroxyisoleucine (WO2008044770).

In one embodiment, the further active ingredient is a trytophan-5-hydroxylase inhibitor-1 (TPH1 inhibitor), which modulates gastrointestinal motility, as described, for example, in WO2009014972.

In one embodiment, the further active ingredient is a muscle-relaxing substance, as described, for example, in WO2008090200.

In a further embodiment, the further active ingredient is an inhibitor of monoamine oxidase B (MAO-B), for example those as described in WO2008092091, WO2009066152.

In a further embodiment, the further active ingredient is an inhibitor of monoamine oxidase A (MAO-A), for example those as described in WO2009030968.

In another embodiment, the further active ingredient is an inhibitor of the binding of cholesterol and/or triglycerides to the SCP-2 protein (sterol carrier protein-2), for example those as described in US2008194658.

In a further embodiment, the further active ingredient is a compound which binds to the β-subunit of the trimeric GTP-binding protein, for example those as described in WO2008126920.

In one embodiment, the further active ingredient is a urate anion exchanger inhibitor 1, as described, for example, in WO2009070740.

In one embodiment, the further active ingredient is a modulator of the ATP transporter, as described, for example, in WO2009108657.

In another embodiment, the further active ingredient is lisofylline, which prevents autoimmune damage to insulin-producing cells.

In yet another embodiment, the further active ingredient is an extract from Bidens pilosa with the ingredient cytopiloyne as described in EP1955701.

In one embodiment, the further active ingredient is an inhibitor of glucosylceramide synthase, as described, for example, in WO2008150486.

In a further embodiment of the invention, the further active ingredient is a glycosidase inhibitor, as described, for example, in WO2009117829, WO2009155753.

In another embodiment, the further active ingredient is an ingredient from the plant *Hoodia Gordonii,* as described in US2009042813, EP2044852.

In one embodiment, the further active ingredient is an antidiabetic, for example D-tagatose.

In one embodiment, the further active ingredient is a zinc complex of curcumin, as described in WO2009079902.

In one embodiment, the further active ingredient is an inhibitor of the "cAMP response element binding protein" (CREB), as described in WO2009143391.

In another embodiment, the further active ingredient is an antagonist of the bradykinin B1 receptor, as described in WO2009124746.

In a further embodiment, the further active ingredient is a compound which is capable of modulating diabetic peripheral neuropathy (DPN). Such modulators are, for example, FK-1706 or SB-509, or those as described in WO1989005304, WO2009092129, WO2010002956.

In one embodiment, the further active ingredient is a compound which is capable of modulating diabetic nephropathy. Such compounds are described, for example, in WO2009089545, WO2009153261.

In one embodiment, the further active ingredient is an inhibitor (e.g. an anti-CD38 antibody) of CD38, as described in US2009196825.

In one embodiment, the further active ingredient is an inhibitor of human fibroblast growth factor receptor 4 (FGFR4), as described, for example, in WO2009046141.

In a further embodiment of the invention, the further active ingredient is a compound which protects the beta cell, for example 14-alpha-lipolyl-andrographolide (AL-1).

In yet another embodiment of the invention, the further active ingredient is the INGAP (islet neogenesis associated protein) peptide, a peptide which reestablishes insulin production in patients with diabetes mellitus.

In one embodiment of the invention, the further active ingredient is a modulator of the CFTR (cystic fibrosis transmembrane conductance regulator), as described, for example, in US2009246137, US2009264433, US2009264441, US2009264471, US2009264481, US2009264486, WO2010019239.

In one embodiment of the invention, the further active ingredient is a compound which stimulates/modulates insulin release, for example those as described in WO2009109258, WO2009132739, US2009281057, WO2009157418.

In one embodiment of the invention, the further active ingredient is an extract from *Hippophae rhamnoides,* as described, for example, in WO2009125071.

In one embodiment of the invention, the further active ingredient is an from *Huanglian* and *Ku Ding Cha,* as described, for example, in WO2009133458.

In another embodiment, the further active ingredient is a root extract from *Cipadessa baccifera,* as described in US2009238900.

In one embodiment of the invention, the further active ingredients are borapetoside A and/or borapetoside C, which can be isolated from the plant SDH-V, a species of *Tinospora crispa,* as described, for example, in US2010016213.

In one embodiment, the compound of the formula I is administered in combination with bulking agents, preferably insoluble bulking agents (see, for example, Carob/Caromax^{®} (Zunft H J; et al., Carob pulp preparation for treatment of hypercholesterolemia, ADVANCES IN THERAPY (2001 Sep-Oct), 18(5), 230-6). Caromax is a carob-containing product from Nutrinova, Nutrition Specialties & Food Ingredients GmbH, Industriepark Höchst, 65926 Frankfurt/Main)). Combination with Caromax^{®} is possible in one preparation or by separate administration of compounds of the formula I and Caromax^{®}. Caromax^{®} can in this connection also be administered in the form of food products such as, for example, in bakery products or muesli bars.

It will be appreciated that every suitable combination of the compounds of the invention with one or more of the aforementioned compounds and optionally one or more other pharmacologically active substances is considered to be covered within the scope of protection conferred by the present invention.

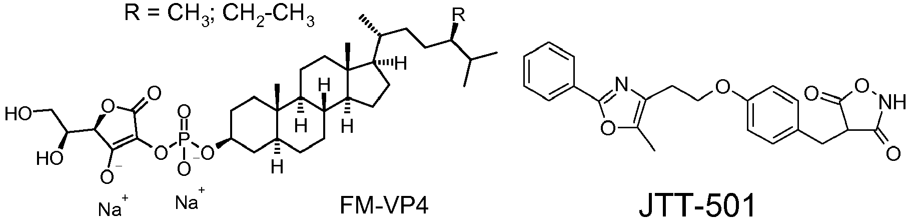

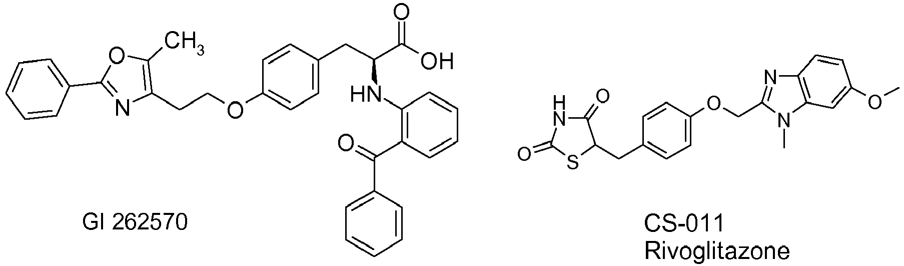

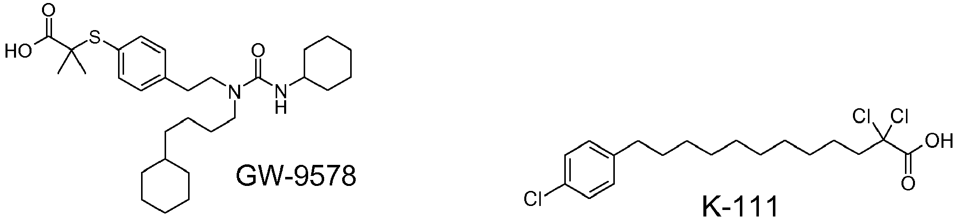

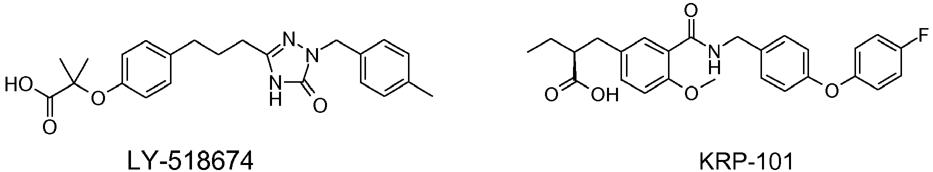

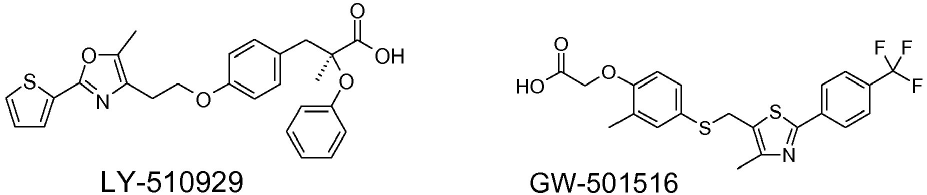

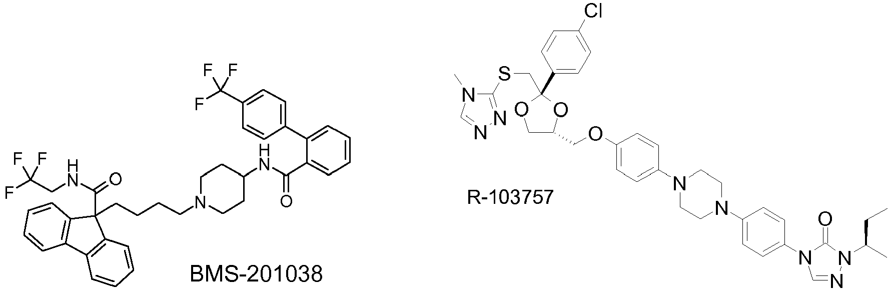

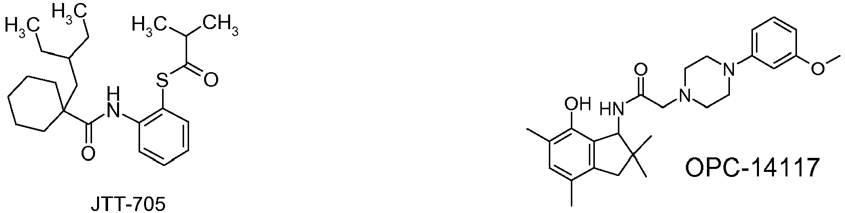

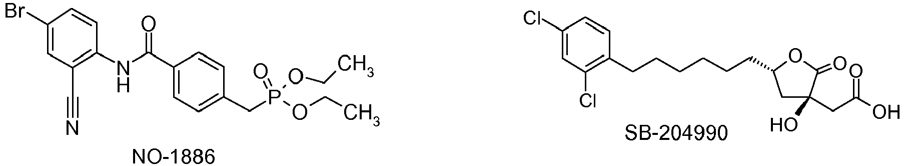

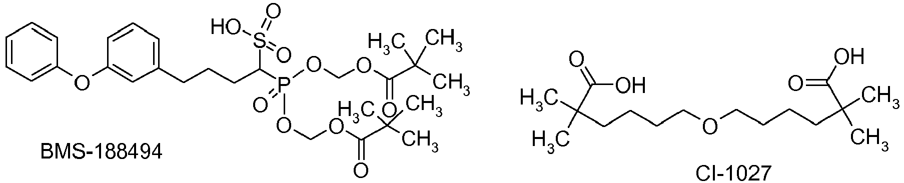

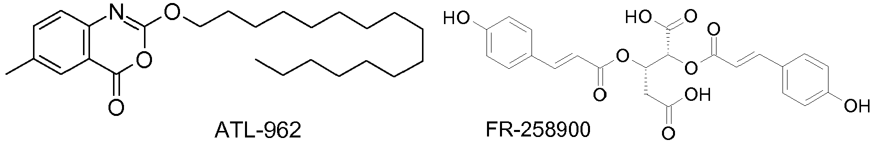

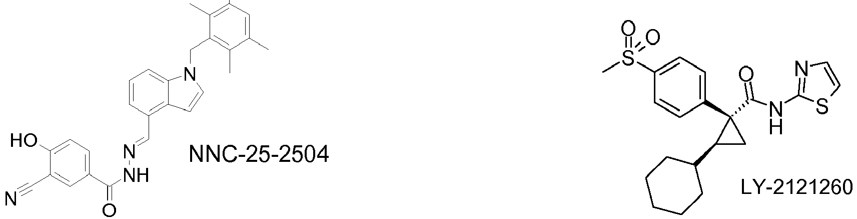

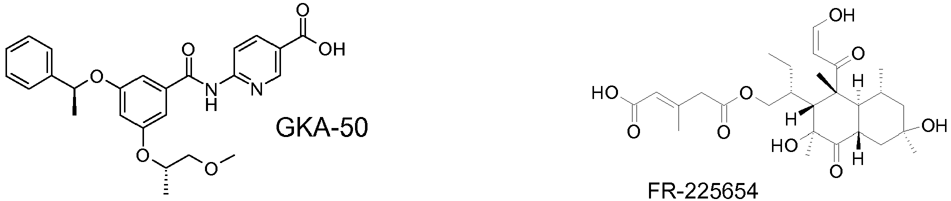

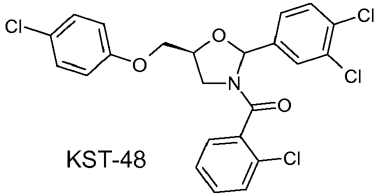

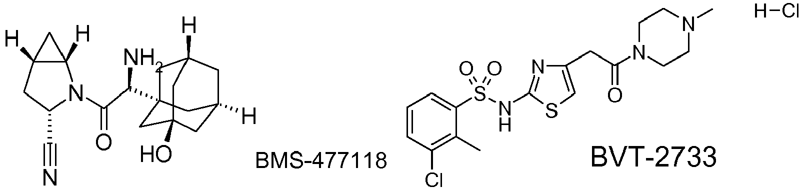

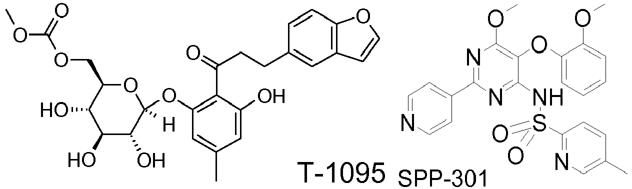

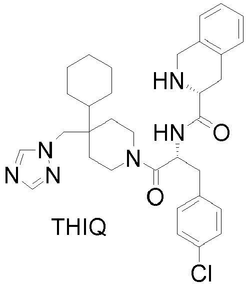

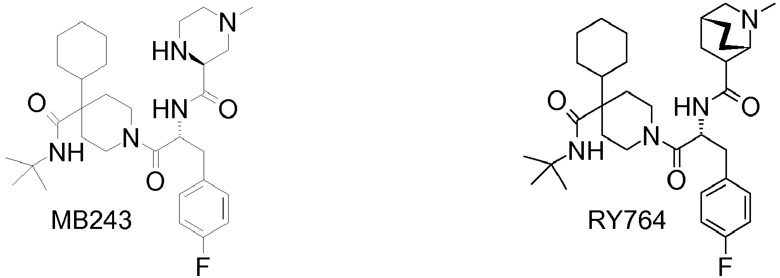

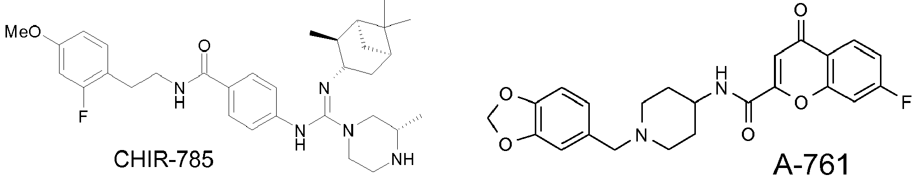

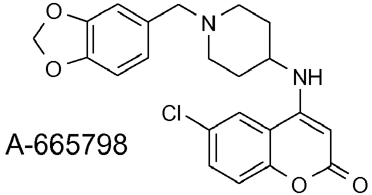

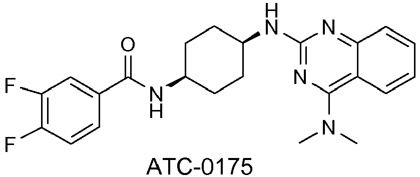

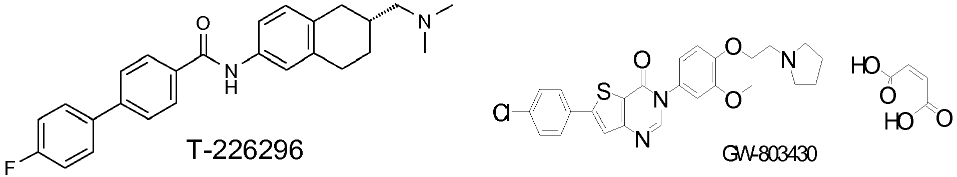

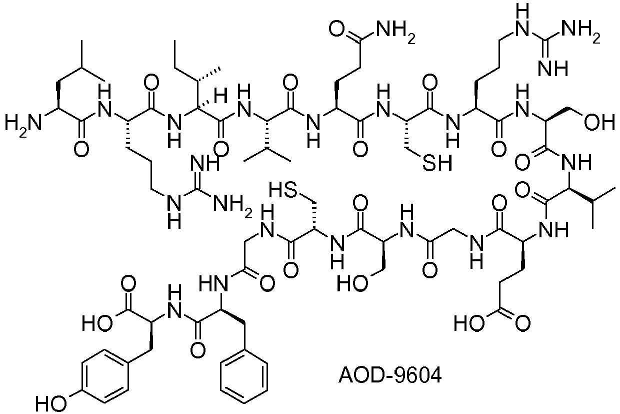

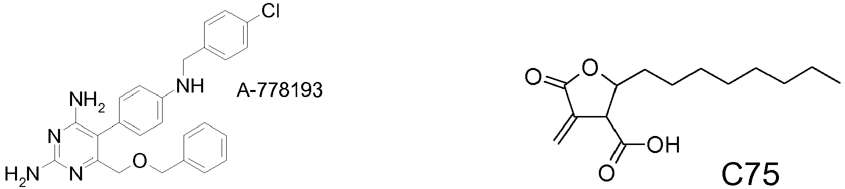

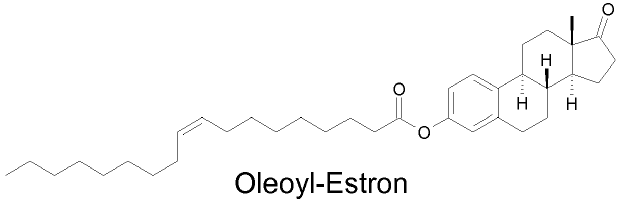

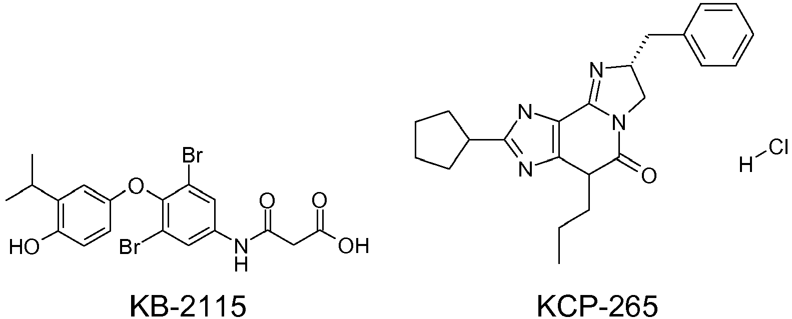

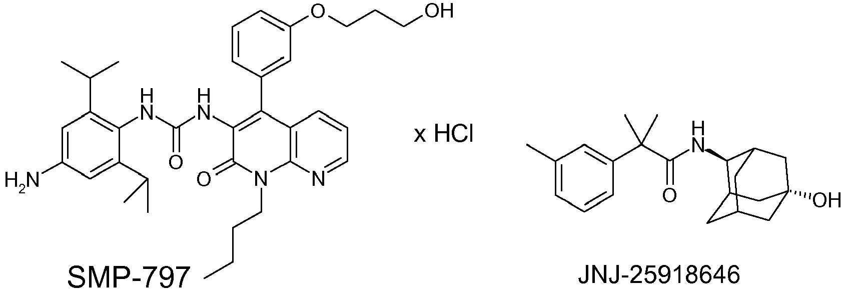

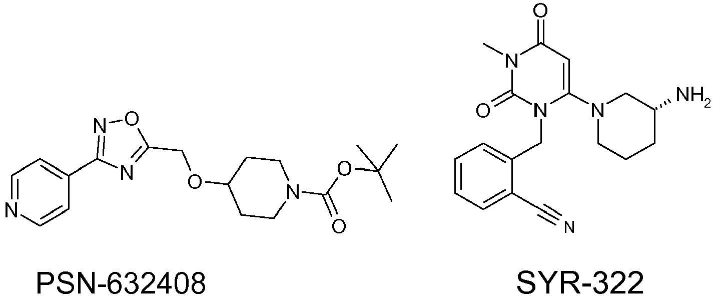

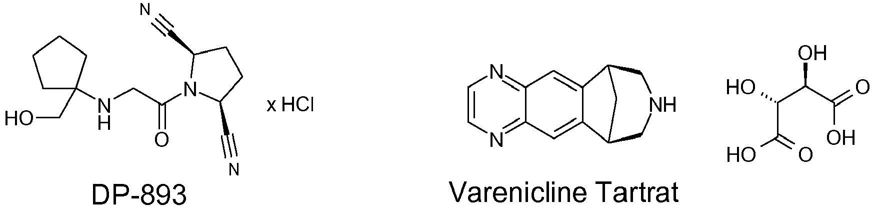

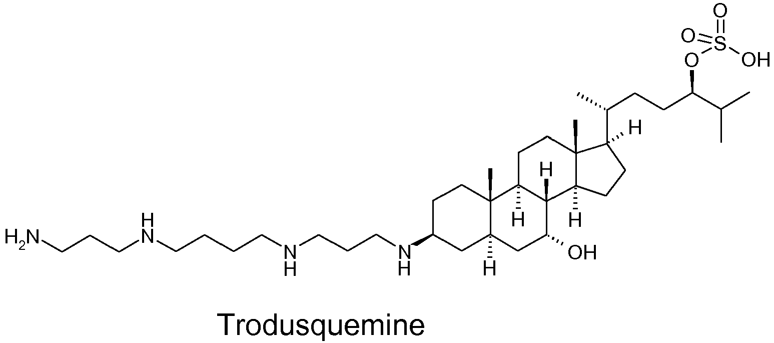

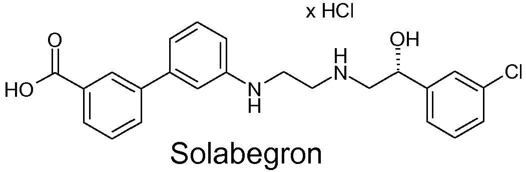

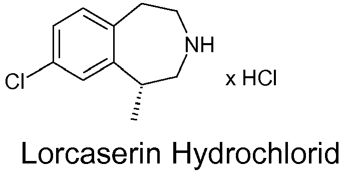

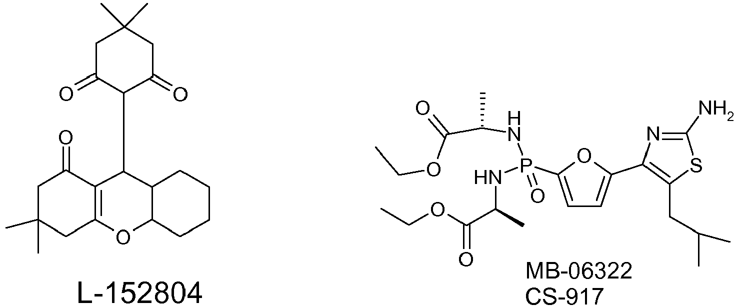

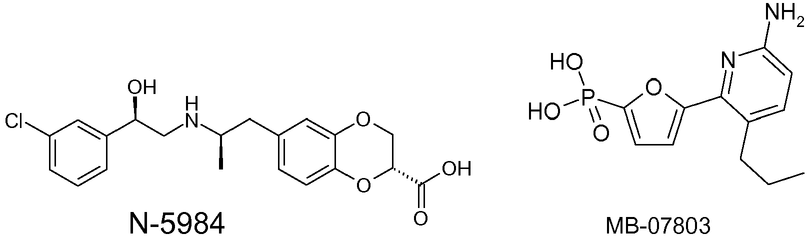

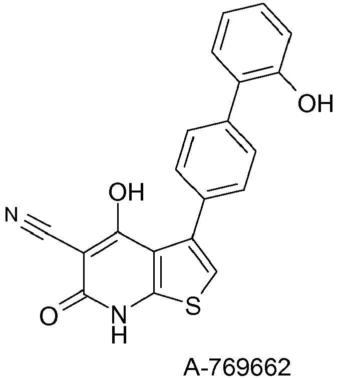

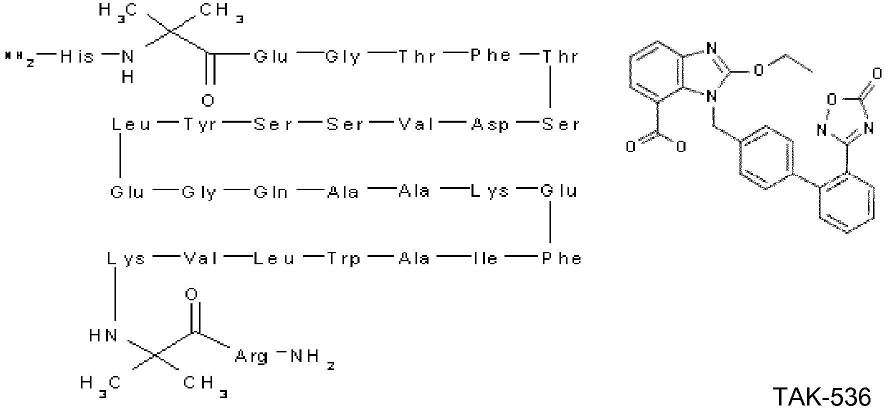

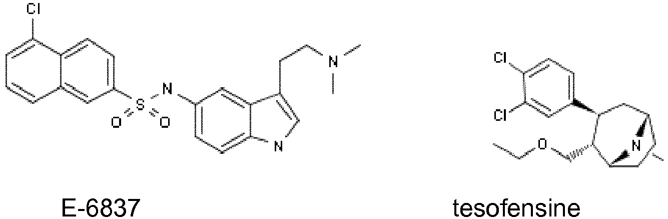

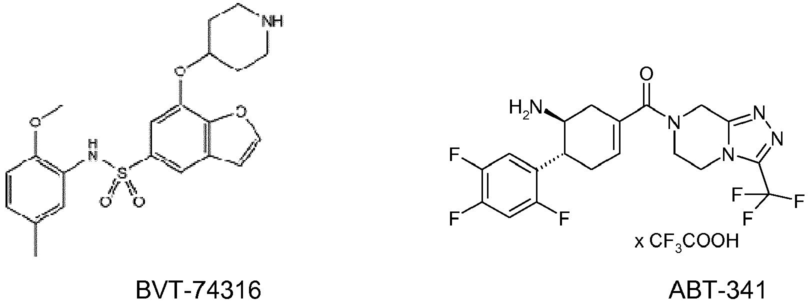

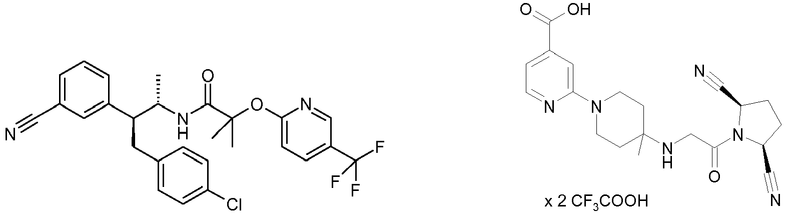

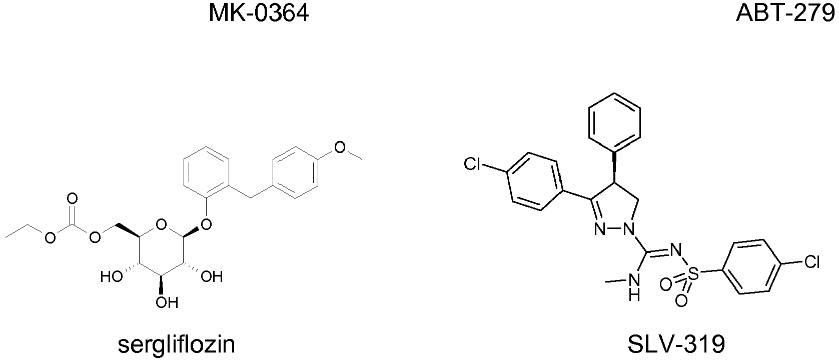

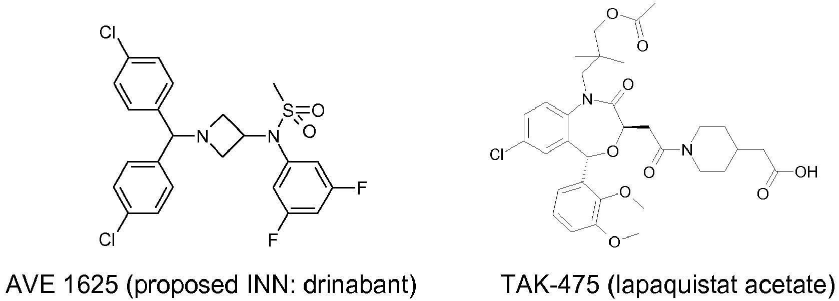

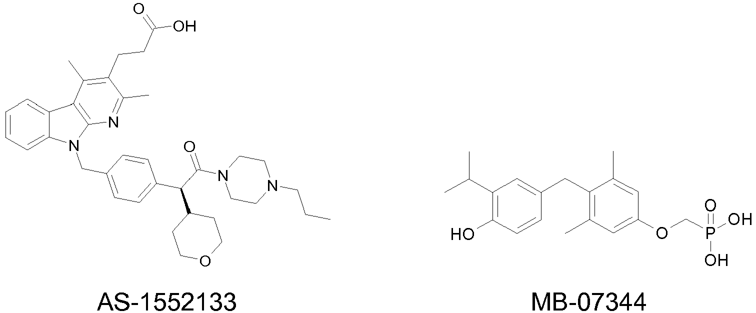

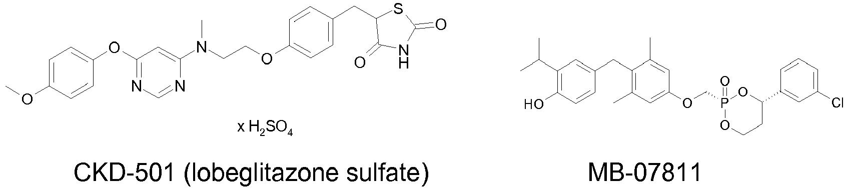

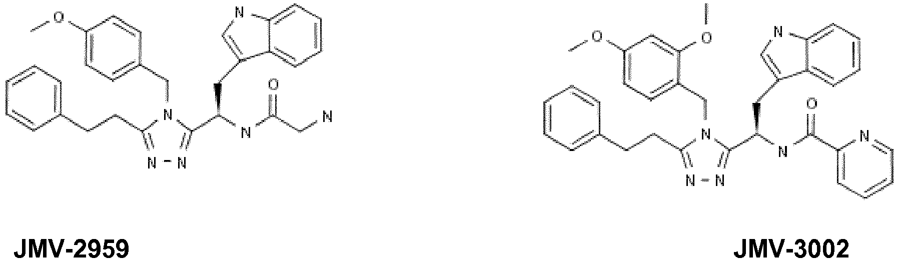

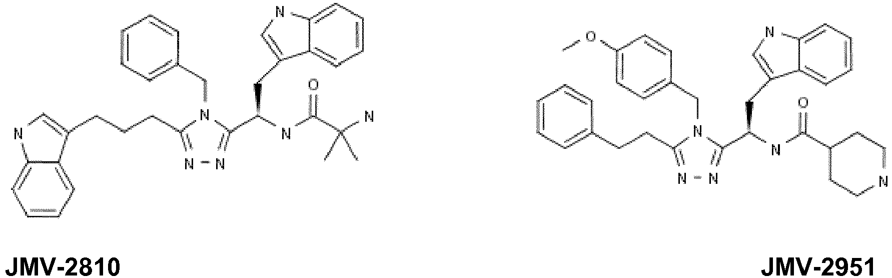

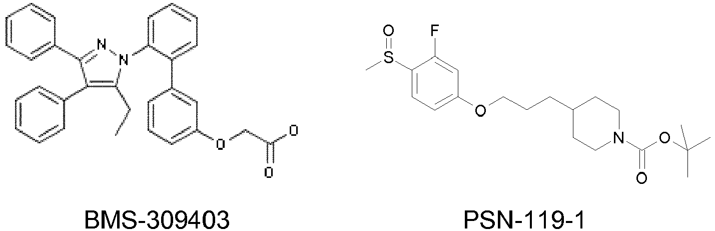

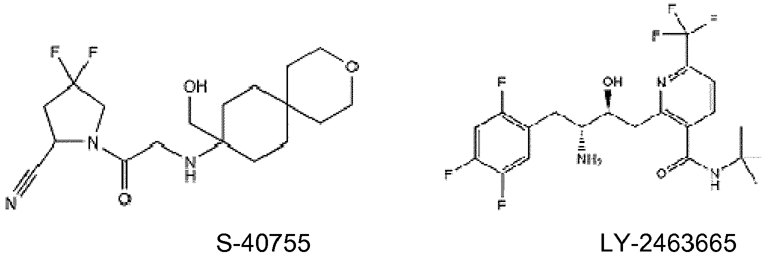

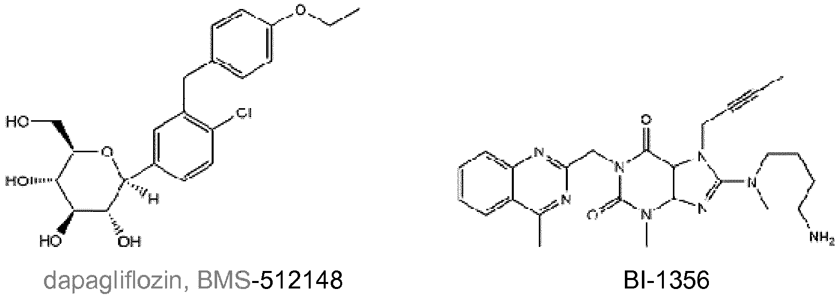

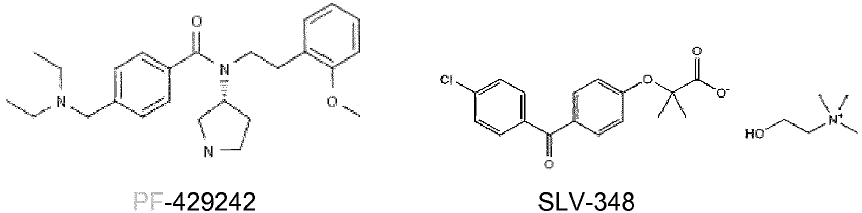

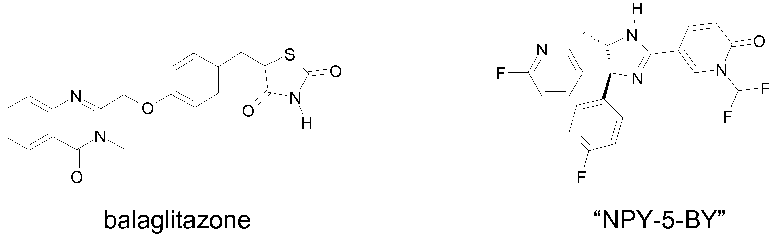

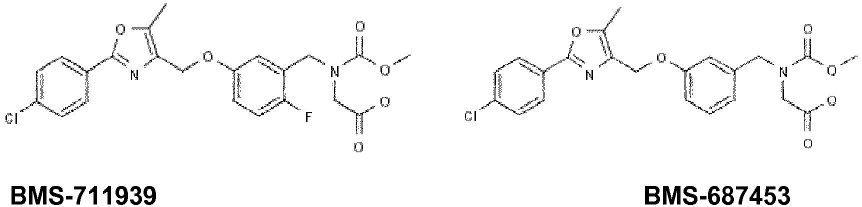

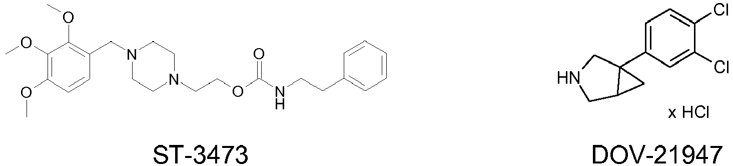

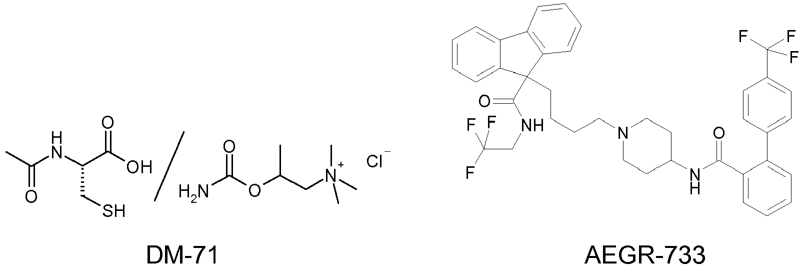

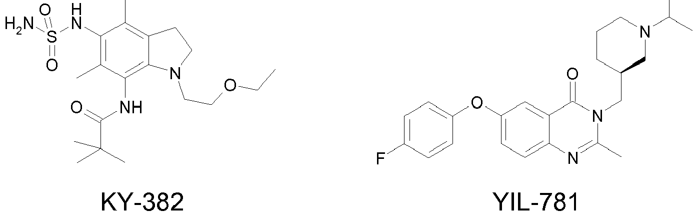

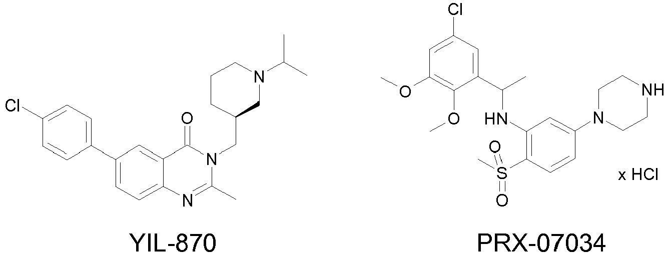

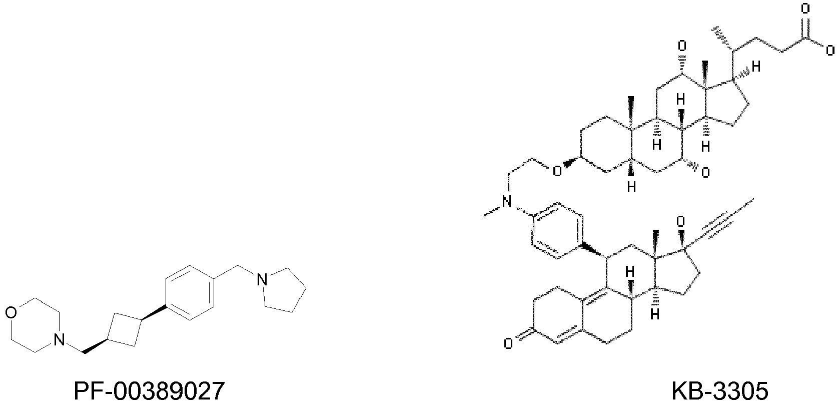

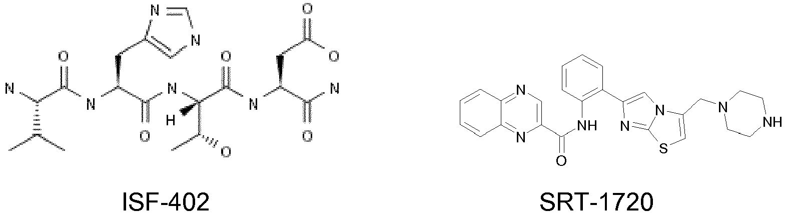

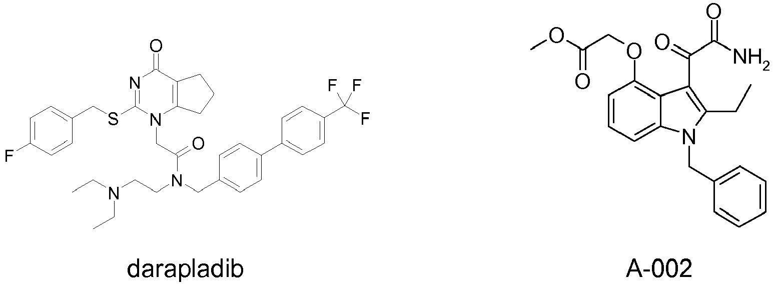

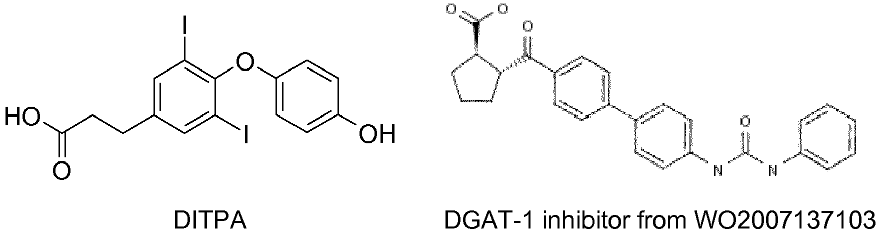

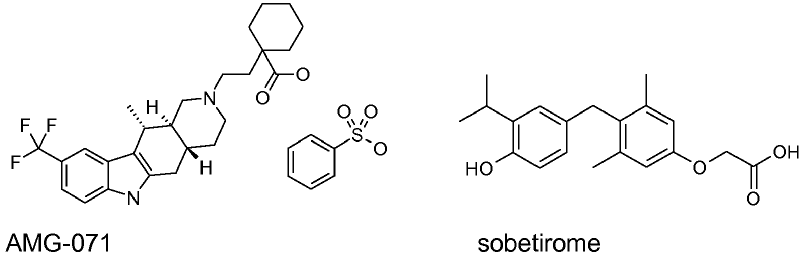

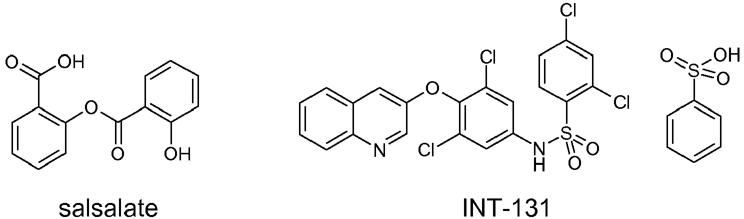

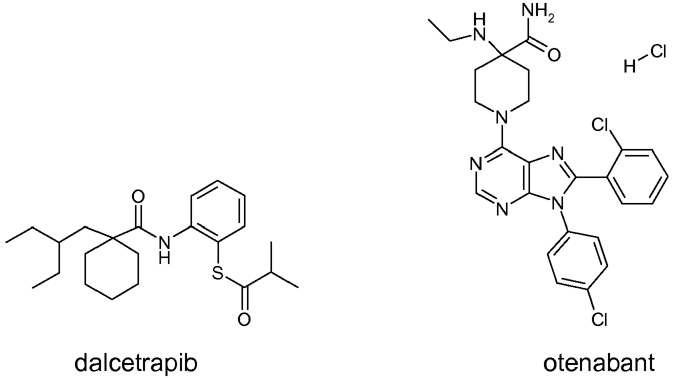

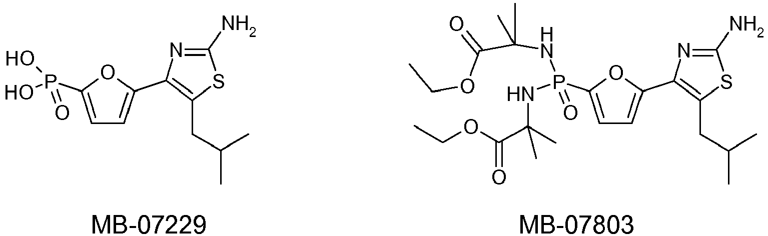

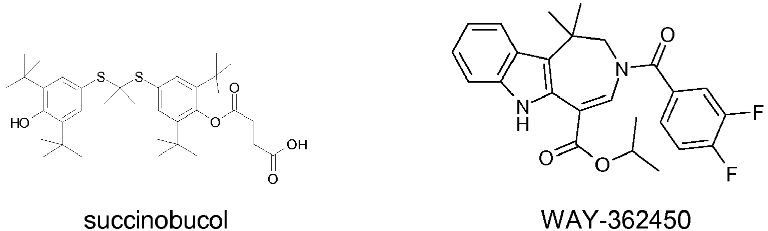

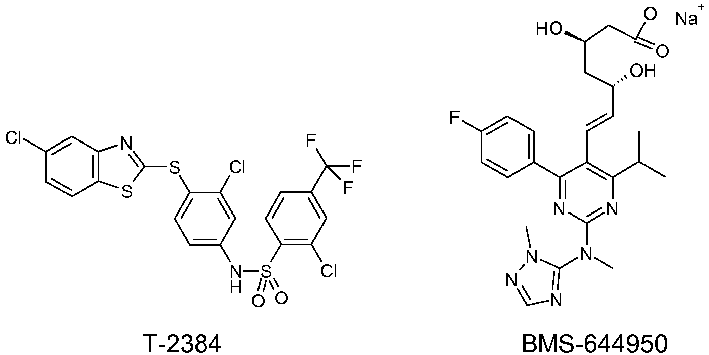

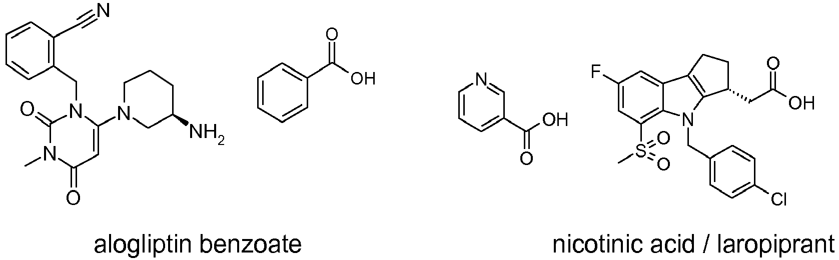

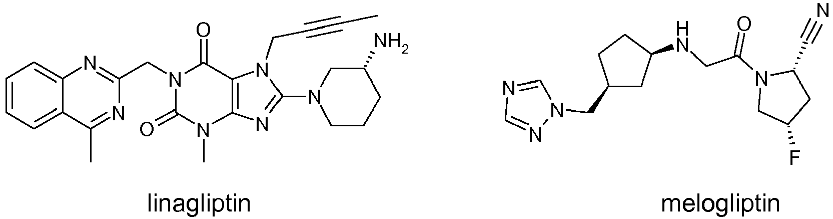

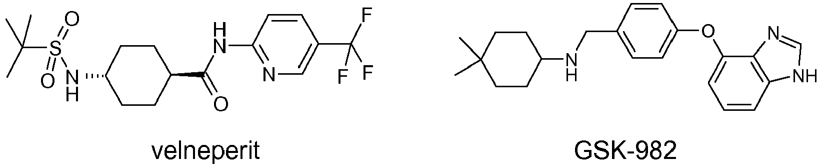

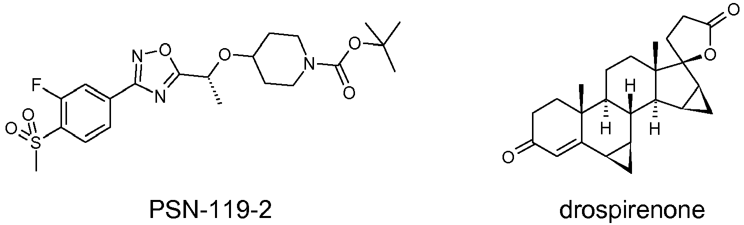

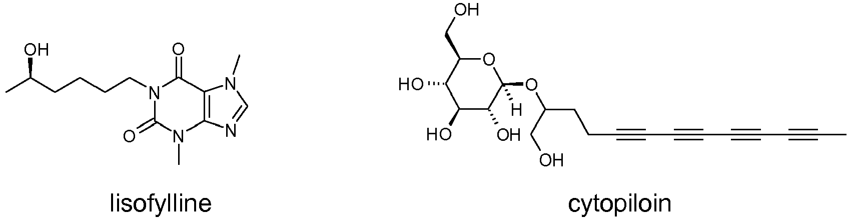

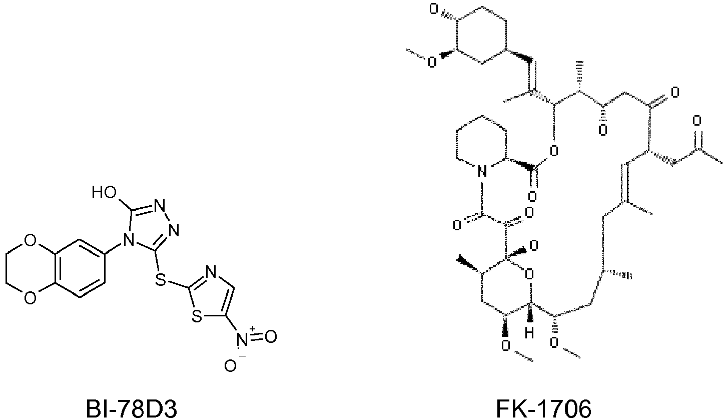

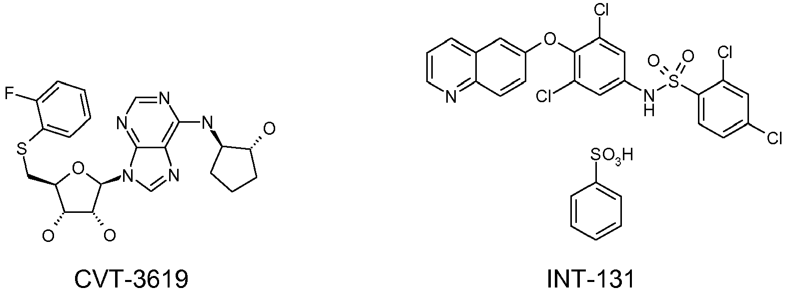

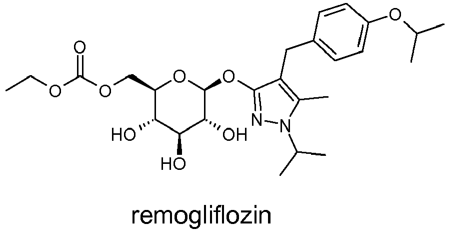

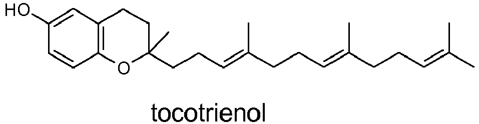

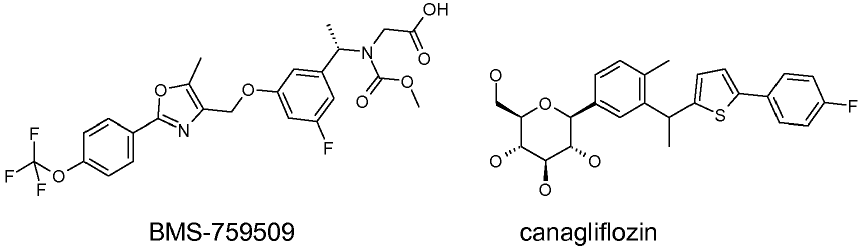

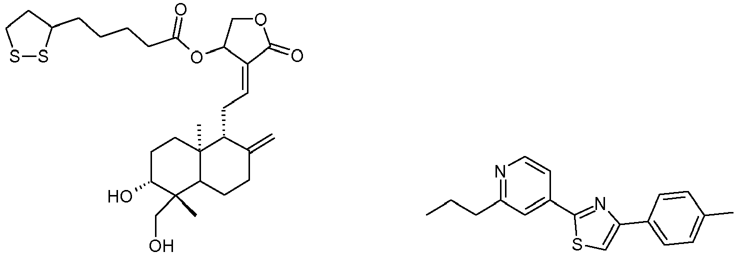

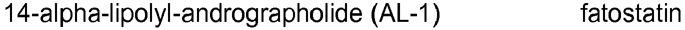

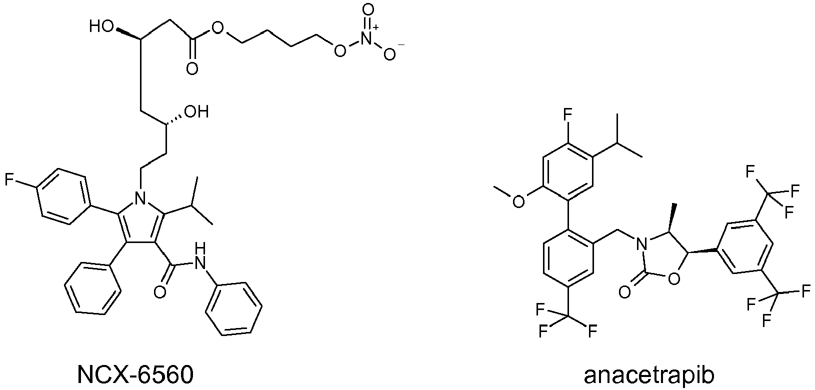

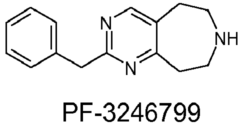

Also suitable are the following active ingredients for combination preparations:
all antiepileptics specified in the Rote Liste 2011, chapter 15;
all antihypertensives specified in the Rote Liste 2011, chapter 17;
all hypotonics specified in the Rote Liste 2011, chapter 19;
all anticoagulants specified in the Rote Liste 2011, chapter 20;
all arteriosclerosis drugs specified in the Rote Liste 2011, chapter 25;
all beta receptors, calcium channel blockers and inhibitors of the renin angiotensin system specified in the Rote Liste 2011, chapter 27;
all diuretics and perfusion-promoting drugs specified in the Rote Liste 2011, chapter 36 and 37;
all withdrawal drugs/drugs for the treatment of addictive disorders specified in the Rote Liste 2011, chapter 39;
all coronary drugs and gastrointestinal drugs specified in the Rote Liste 2011, chapter 55 and 60;
all migraine drugs, neuropathy preparations and Parkinson's drugs specified in the Rote Liste 2011, chapter 61, 66 and 70.

It will be appreciated that every suitable combination of the compounds of the invention with one or more of the aforementioned compounds and optionally one or more other pharmacologically active substances is considered to be covered within the scope of protection conferred by the present invention.

PKC-inhibitors can be administered to animals, in particular to mammals including humans, as pharmaceuticals by themselves, in mixtures with one another, or in the form of pharmaceutical compositions. The administration can be carried out orally, for example in the form of tablets, film-coated tablets, sugar-coated tablets, granules, hard and soft gelatin capsules, solutions including aqueous, alcoholic and oily solutions, juices, drops, syrups, emulsions or suspensions, rectally, for example in the form of suppositories, or parenterally, for example in the form of solutions for subcutaneous, intramuscular or intravenous injection or infusion, in particular aqueous solutions.

Suitable pharmaceutical compounds for oral administration may be in the form of separate units, for example capsules, cachets, lozenges or tablets, each of which contains a defined amount of the compound of formula I; as powders or granules; as solution or suspension in an aqueous or nonaqueous liquid; or as an oil-in-water or water-in-oil emulsion. These compositions may, as already mentioned, be prepared by any suitable pharmaceutical method which includes a step in which the active ingredient and the carrier (which may consist of one or more additional ingredients) are brought into contact. The compositions are generally produced by uniform and homogeneous mixing of the active ingredient with a liquid and/or finely divided solid carrier, after which the product is shaped if necessary. Thus, for example, a tablet can be produced by compressing or molding a powder or granules of the compound, where appropriate with one or more additional ingredients. Compressed tablets can be produced by tableting the compound in free-flowing form such as, for example, a powder or granules, where appropriate mixed with a binder, glidant, inert diluent and/or one (or more) surfactant(s)/dispersant(s) in a suitable machine. Molded tablets can be produced by molding the compound, which is in powder form and has been moistened with an inert liquid diluent, in a suitable machine.

Pharmaceutical compositions which are suitable for peroral (sublingual) administration comprise lozenges which contain a compound of formula I with a flavoring, typically sucrose, and gum arabic or tragacanth, and pastilles which comprise the compound in an inert base such as gelatin and glycerol or sucrose and gum arabic.

Coated formulations and coated slow-release formulations, especially acid- and gastric juice-resistant formulations, also belong within the framework of the invention. Suitable coatings resistant to gastric juice comprise cellulose acetate phthalate, polyvinyl acetate phthalate, hydroxypropylmethylcellulose phthalate and anionic polymers of methacrylic acid and methyl methacrylate.

Pharmaceutical compositions suitable for rectal administration are preferably in the form of single-dose suppositories. These can be produced by mixing a compound of formula I with one or more conventional solid carriers, for example cocoa butter, and shaping resulting mixture.

Pharmaceutical compositions suitable for parenteral administration comprise preferably sterile aqueous preparations of a compound of formula I, which are preferably isotonic with the blood of the intended recipient. These preparations are preferably administered intravenously, although administration may also take place by subcutaneous, intramuscular or intradermal injection. These preparations can preferably be produced by mixing the compound with water and making the resulting solution sterile and isotonic with blood. Injectable compositions of the invention generally contain 0.1 to 5% by weight of the active compound.

Other suitable administration forms are, for example, percutaneous or topical administration, for example in the form of ointments, creams, tinctures, sprays, powders or transdermal therapeutic systems, or inhalative administration, for example in the form of nasal sprays or aerosol mixtures, or forms such as microcapsules, implants or rods.

Pharmaceutical compositions suitable for topical use on the skin are preferably in the form of ointment, cream, lotion, paste, spray, aerosol or oil. The carriers used may be petrolatum, lanolin, polyethylene glycols, alcohols and combinations of two or more of these substances. The active ingredient is generally present in a concentration of 0.1 to 15% by weight of the composition, for example 0.5 to 2%.

Transdermal administration is also possible. Pharmaceutical compositions suitable for transdermal uses may be in the form of single patches which are suitable for long-term close contact with the patient's epidermis. Such patches suitably contain the active ingredient in an aqueous solution which is buffered where appropriate, dissolved and/or dispersed in an adhesive or dispersed in a polymer. A suitable active ingredient concentration is about 1% to 35%, preferably about 3% to 15%. A particular option is for the active ingredient to be released by electrotransport or iontophoresis as described, for example, in Pharmaceutical Research, 2(6): 318 (1986).

PKC inhibitors can additionally be used in systems for local drug delivery, for example in coated stents for preventing or reducing in-stent restenosis or by applying them locally by means of a catheter. The appropriate administration form depends, among others, on the disease to be treated and on its severity.

The dosing of PKC inhibitors to achieve the desireable therapeutic effect depends on a number of factors, for example the specific compound chosen, the intended use, the mode of administration and the clinical condition of the patient. The daily dose is generally in the range from 0.3 mg to 100 mg (typically from 3 mg to 50 mg) per day and per kilogram of body weight, for example 3-10 mg/kg/day. An intravenous dose may be, for example, in the range from 0.3 mg to 1.0 mg/kg, which can suitably be administered as infusion of 10 ng to 100 ng per kilogram and per minute. Suitable infusion solutions for these purposes may contain, for example, 0.1 ng to 100 mg, typically 1 ng to 100 mg, per milliliter. Single doses may contain, for example, 1 mg to 10 g of the active ingredient. Thus, ampoules for injections may contain, for example, from 1 mg to 100 mg, and orally administrable single-dose formulations, for example tablets or capsules, may contain, for example, from 1.0 to 1000 mg, typically from 10 to 600 mg. For treatment of the abovementioned conditions, the compounds of the formula I themselves may be used as the compound, but they are preferably present with a compatible carrier in the form of a pharmaceutical composition. The carrier must, of course, be acceptable in the sense that it is compatible with the other ingredients of the composition and is not harmful for the patient's health. The carrier may be a solid or a liquid or both and is preferably formulated with the compound as a single dose, for example as a tablet, which may contain 0.05% to 95% by weight of the active ingredient. Other pharmaceutically active substances may likewise be present, including other compounds of formula I. The pharmaceutical compositions of the invention can be produced by one of the known pharmaceutical methods, which essentially consist of mixing the ingredients with pharmacologically acceptable carriers and/or excipients.

The present invention provides novel and potent PKC inhibitors. The compounds of the present invention are selective to PKC over other kinases and are isozyme-selective. Selective PKC inhibitors are useful in preventing and treating diseases associated with diabetes and diabetic complications (e.g. diabetic cardiomyopathy, diabetic nephropathy, diabetic micro- and macrovascular complications, diabetic neuropathy and diabetic retinopathy, preferably diabetic nephropathy, diabetic neuropathy and diabetic retinopathy), cardiovascular diseases, diseases associated with hypertension- and non-hypertension-related and ischemic and non-ischemic end-organ damage (e.g. mycardial infarction, coronary heart disease, atherosclerosis, cardiac and renal hypertrophy, stroke), diseases associated with inflammation and fibrosis, central nervous system disorders (e.g. neuropathic pain), dermatological diseases, autoimmune diseases (e.g. psoriasis, type 1 diabetes) and cancer (e.g. hematological tumours, glioma, gastric and intestinal cancer, skin cancer and lung cancer).

Another subject of the present invention are processes for the preparation of the compounds of the formula I and their salts and solvates, by which the compounds are obtainable and which are outlined in the following.

In one process, a compound of the formula II is reacted with a compound of the formula III to give a compound of the formula Ia, which can be a compound of formula I already or which can be converted into a compound of formula I, 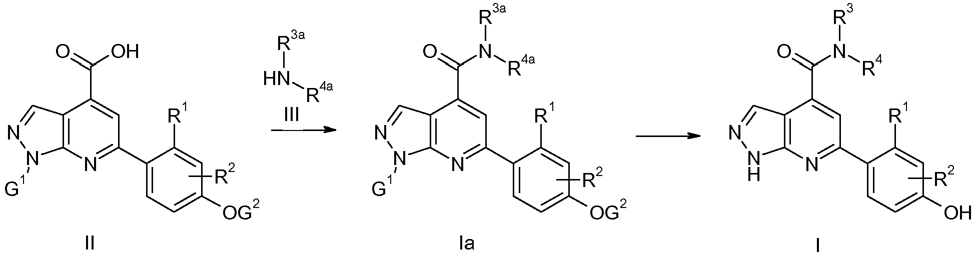 wherein the groups R¹, R², R³ and R⁴ in the compounds of the formulae II and Ia are defined as in the compounds of the formula I. The groups R^{3a} and R^{4a} in the compounds of formulae Ia and III are, independently of each other, either defined as the groups R³ and R⁴ in formula I, or they are precursors of the groups R³ and R⁴ in formu-Ia I, they can for example contain functional groups in protected form or functional groups which can be converted to obtain the final groups R³ and R⁴. The group G¹ in the compounds of formulae Ia and II is defined as a hydrogen or a protectting group for a pyrazole nitrogen, such as, for example, a 2-tetrahydropyranyl-group, a benzyl group, a 4-methoxybenzyl or a 2,5-dimethoxybenzyl group. The group G² in the compounds of formulae Ia and II is defined as a hydrogen or a protecting group for a phenolic hydroxyl group, such as, for example, a 2-tetrahydropyranyl-group, a benzyl group, a 4-methoxybenzyl or a 2,5-dimethoxybenzyl group.

The compounds of the formula II may also be present in another tautomeric form, especially for compounds, in which G¹ is hydrogen, for example in the form of the respective 2H-pyrazolo[3,4-b]pyridine derivatives in which the mobile hydrogen atom, which in formula II is bonded to the ring nitrogen atom in the 1-position of the pyrazolo[3,4-b]pyridine ring system, is bonded to to the ring nitrogen atom in the 2-position of the pyrazolo[3,4-b]pyridine ring system. As far as applicable, it applies to all compounds occurring in the preparation of the compounds of the formula I that they can be present in any other tautomeric form than the one represented in their formulae.

The reaction of the compounds of the formulae II and III to form an amide of formula Ia is generally performed in the presence of activating agents, such as CDI, DCC, EDC, HOAt, HOBt, HATU, TOTU, TBTU, BEP or combinations thereof, and optionally an additional base, such as TEA, DIPEA or N-methylmorpholin in an appropriate inert solvent, for example a hydrocarbon or a chlorinated hydrocarbon such as benzene, toluene, chlorobenzene, dichloromethane, dichloroethane, chloroform, or an ether such as tetrahydrofurane, 1,4-dioxane, dibutylether, diisopropylether, methyl-tert-butylether, dimethoxyethane, or an ester such as ethyl acetate or ethyl butanoate or an amide such as N,N-dimethylformamide or N,N-dimethylacetamide or N-methyl-pyridone or a in mixture of solvents. The reaction temperature in this case is generally from -30°C to 200°C, preferably from -20°C to 80°, more preferably from 0°C to 20 °C. The reaction time is generally from 15 min to 6 days, preferably from 15 min to 16 h, depending on the composition of the mixture and the chosen temperature range. The acids of formula II can be subjected to the reaction in form of their salts, for example their sodium salts. They can also be transformed into an activated derivative prior to the coupling with the amine, for example into an acid chloride or an acid anhydride by standard transformations. The amines of formula III can be subjected to the reaction in form of their salts, for example as hydrochloride or triflate salts, in which case usually an additional equivalent of the base is added to the reaction. As far as applicable and unless otherwise indicated, it applies to all acidic or basic compounds occurring in the preparation of the compounds of the formula I that they can be present in form of their salts.

A compound of the formula Ia can already be a compound of formula I, if G¹ and G² are both H and if R^{3a} is R³ and R^{4a} is R⁴. If a compound of formula Ia is not already a compound of formula I, it can be transformed into a compound of formula I in one step or in several steps depending of the meaning of the groups G¹, G², R^{3a} and R^{4a}. If the groups R^{3a} and/or R^{4a} contain or if the groups G¹ and/or G² consist of protecting groups that can be cleaved by hydrogenation, e.g. a benzyl group or a 4-methoxybenzyl group, one step in the transformation of a compound of formula Ia to a compound of formula I can be a catalytic hydrogenation or a transfer hydrogenation. If the groups R^{3a} and/or R^{4a} contain or if the groups G¹ and/or G² consist of protecting groups that can be cleaved by treatment with acid, e.g. a 2-tetrahydropyranyl group, a 4-methoxybenzyl group, a 2,4-dimethoxybenzyl group or a tert-butoxycarbonyl group, one step in the transformation of a compound of formula Ia to a compound of formula I can be an acidic deprotection. All deprotection reactions used in the above-described transformation of compounds of the formula Ia, in which the groups R^{3a} and/or R^{4a} contain or in which the groups G¹ and/or G² consist of protecting groups, to compounds of formula I are per se well known to the skilled person and can be carried out under standard conditions according to, or analogously to, procedures described in the literature, for example in P. J. Kocienski, Protecting Groups, Georg Thieme Verlag, Stuttgart, 1994 or T. W. Greene, P. G. M. Wuts, Protective Groups in Organic Synthesis, John Wiley, New York, 1999).

A transformation of a compound of formula Ia, in which the groups R^{3a} and/or R^{4a} are precursors to the groups R³ and/or R⁴ into a compound of formula I can also include a functionalization or modification of contained functional groups according to standard procedures, for example by esterification, amidation, hydrolysis, etherification, alkylation, acylation, sulfonylation, reduction, oxidation, conversion into salts, and others. For example, a hydroxy group, which may be obtained from a protected hydroxy group by deprotection, can be esterified to give a carboxylic acid ester or a sulfonic acid ester, or etherified. Etherifications of hydroxy groups can favorably be performed by alkylation with the respective halogen compound, for example a bromide or iodide, in the presence of a base, for example an alkaline metal carbonate such potassium carbonate or cesium carbonate in an inert solvent, for example an amide like DMF or NMP or a ketone like acetone or butan-2-one, or with the respective alcohol under the conditions of the Mitsunobu reaction referred to above. A hydroxy group can be converted into a halide by treatment with a halogenating agent. A halogen atom can be replaced with a variety of groups in a substitution reaction which may also be a transition-metal catalyzed reaction. A nitro group can be reduced to an amino group, for example by catalytic hydrogenation. An amino group, which may be obtained from a protected amino group by deprotection, can be modified under standard conditions for alkylation, for example by reaction with a halogen compound or by reductive amination of a carbonyl compound, or for acylation or sulfonylation, for example by reaction with a reactive carboxylic acid derivative, like an acid chloride or anhydride or a sulfonic acid chloride, or with a carboxylic acid in the presence of activating agents, such as CDI, DCC, EDC, HOAt, HOBt, HATU, TOTU, TBTU, BEP or combinations thereof, or for carbamoylation, for example by reaction with an isocyanate. A carboxylic ester group can be hydrolyzed under acidic or basic conditions to give a carboxylic acid. A carboxylic acid group can be activated or converted into a reactive derivative as mentioned afore and reacted with an alcohol or an amine or ammonia to give an ester or amide. A primary amide can be dehydrated to give a nitrile. A sulfur atom, for example in an alkyl-S- group or in a heterocyclic ring, can be oxidized with a peroxide like hydrogen peroxide or a peracid to give a sulfoxide moiety S(O) or a sulfone moiety S(O)₂. A carboxylic acid group, carboxylic acid ester group and a ketone group can be reduced to an alcohol, for example by means of a complex hydride such as lithium aluminium hydride, lithium borohydride or sodium borohydride. All reactions used in the above-described syntheses of the compounds of the formula I are per se well known to the skilled person and can be carried out under standard conditions according to, or analogously to, procedures described in the literature, for example in Houben-Weyl, Methoden der Organischen Chemie (Methods of Organic Chemistry), Thieme-Verlag, Stuttgart, or Organic Reactions, John Wiley & Sons, New York.

The compounds of the formula II can be obtained by reacting an aminopyrazole compound of the formula IV with a benzaldehyde of formula V and a pyruvic acid derivative of formula VI to give a compound of formula IIa, which can be a compound of formula II already or which can be converted into a compound of formula II, 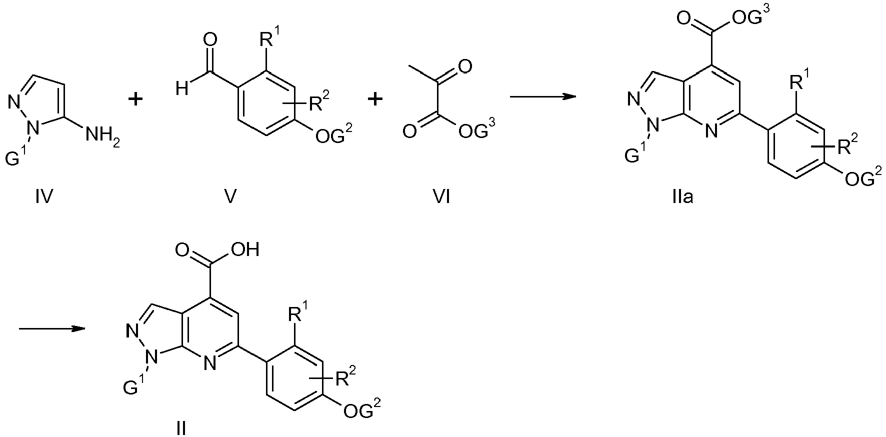 wherein the groups R¹ and R² in the compounds of the formulae II, IIa and V are defined as in the compounds of the formula I. The group G¹ in the compounds of formulae II, IIa and IV is defined as a hydrogen or a protecting group for a pyrazole nitrogen, such as, for example, a 2-tetrahydropyranyl-group, a benzyl group, a 4-methoxybenzyl or a 2,5-dimethoxybenzyl group. The group G² in the compounds of formulae II, IIa and V is defined as a hydrogen or a protecting group for a phenolic hydroxyl group, such as, for example, a 2-tetrahydropyranyl-group, a benzyl group, a 4-methoxybenzyl or a 2,5-dimethoxybenzyl group. The group G³ in the compounds of formulae IIa and VI is hydrogen, or a protecting group for a carboxylic acid, such as, for example methyl, ethyl, propyl, tert-butyl or benzyl, preferably methyl or ethyl.

The reaction of an aminopyrazole compound of the formula IV with a benzaldehyde of formula V and a pyruvic acid derivative of formula VI is generally performed in the presence of acids, for example acetic acid or hydrochloric acid, in an appropriate solvent, for example in an alcohol as ethanol, methanol or iso-propanol or an ether such as tetrahydrofurane, 1,4-dioxane, dibutylether, diisopropylether, methyl-tert-butylether, dimethoxyethane or in water, or in mixtures thereof. The reaction temperature in this case is generally from -30°C to 200°C, preferably from 0°C to 150°, more preferably from 0°C to 80 °C. The reaction time is generally from 30 min to 48 h, preferably from 30 min to 6 h, depending on the composition of the mixture and the chosen temperature range. Depending on the solvents and conditions applied the preferred regionisomer of formula IIa can be easily isolated by precipitation from the reaction mixture.

A compound of the formula IIa can already be a compound of formula II, if G¹ and G² are identical in the compounds of formula IIa and II and if G³ in the compound of formula IIa is H. If a compound of formula IIa is not already a compound of formula II, it can be transformed into a compound of formula II in one step or in several steps depending of the meaning of the groups G¹, G² and G³. For example, a compound of formula IIa, in which G1 is hydrogen, can be converted into a compound of formula IIa, in which G1 is a protecting group for a pyrazole nitrogen, by a suitable protection reaction, e.g. by the reaction with 3,4-dihydro-2H-pyrane under acidic catalysis. Alternatively, or as an additional step, a compound of formula IIa, in which G² is hydrogen, can be converted to a compound of formula II, in which G² is a protecting group for a phenolic hydroxyl group, for example a benzyl group, by a suitable protection reaction, e.g. by the reaction with a benzyl halide such as benzyl bromide or benzyl chloride in the presence of a base such as sodium carbonate. Alternatively or as an additional step, a compound of formula IIa, in which G³ is not hydrogen, but a protecting group for a carboxylic acid, the compound of formula IIa can be reacted to a compound of formula II by a suitable deprotection reaction, e.g. a basic hydrolysis, if G3 is a methyl, ethyl or propyl residue. As it is known to the skilled person the employed protection groups should be chosen in a manner to be compatible with the desired reaction conditions for all subsequent steps. All protection and deprotection reactions used in the above-described transformation of compounds of the formula IIa to compounds of formula II are well known to the skilled person and can be carried out under standard conditions according to, or analogously to, procedures described in the literature, for example in P. J. Kocienski, Protecting Groups, Georg Thieme Verlag, Stuttgart, 1994 or T. W. Greene, P. G. M. Wuts, Protective Groups in Organic Synthesis, John Wiley, New York, 1999).

Alternatively, the compounds of the formula II can be obtained by reacting an aminopyrazole of formula IV with a compound of formula VII-I (a 2-oxo succinic acid derivative or a tautomer and/or salt thereof) or with a compound of formula VII-II (an acetylene dicarboxylic acid derivative) to a compound of formula VIII, the obtained compound of formula VIII is then converted into a compound of formula IXa, which is then reacted with a compound of formula X to to give a compound of formula IIa, which can be a compound of formula II already or which can be converted into a compound of formula II, 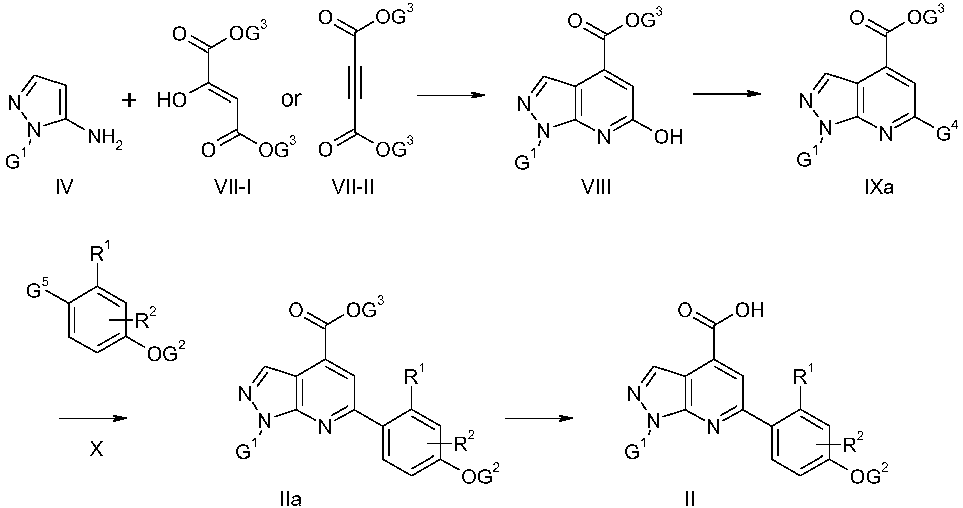 wherein the groups R¹ and R² in the compounds of the formulae II, IIa and X are defined as in the compounds of the formula I. The group G¹ in the compounds of formulae II, IIa, IV, VIII and IXa is defined as a hydrogen or a protecting group for a pyrrazole nitrogen, such as, for example, a 2-tetrahydropyranyl-group, a benzyl group, a 4-methoxybenzyl or a 2,5-dimethoxybenzyl group. The group G² in the compounds of formulae II, IIa and X is defined as a hydrogen or a protecting group for a phenolic hydroxyl group, such as, for example, a 2-tetrahydropyranyl-group, a benzyl group, a 4-methoxybenzyl or a 2,5-dimethoxybenzyl group. The group G³ in the compounds of formulae IIa, VII-I, VII-II, VIII and IXa is hydrogen or a protecting group for a carboxylic acid, such as, for example methyl, ethyl, propyl, tert-butyl or benzyl, preferably methyl or ethyl. The group G⁴ in the compounds of formula IXa is a leaving group, that can be replaced in a Suzuki-type reaction, such as a halide, e.g. a bromide or a chloride or as a sulfonate, e.g. a trifluoromethanesulfonate or methanesulfonate. The group G⁵ in the compounds of formula X is a boronic acid or a boronic ester or cyclic boronic ester.

The reaction of an aminopyrazole of formula IV with a compound of of formula VII-I (a 2-oxo succinic acid derivative or a tautomer and/or salt thereof, e.g. a dialkyl oxalacetate sodium salt) or with a compound of formula VII-II (an acetylene dicarboxylic acid derivative) to a compound of formula VIII is generally carried out in the presence of an acid, such as aqueous hydrochloric acid or acetic acid or trifluoro acetic acid at temperatures from about 0 °C to about 200 °C, for example at temperatures from about 20 °C to about 120 °C. The reaction can be carried out in neat conditions or in a suitable inert solvent. The reaction time is generally from 30 min to 48 h, preferably from 30 min to 16 h, depending on the composition of the mixture and the chosen temperature range. Depending on the solvents and conditions applied the preferred regioisomer of formula VIII can be easily isolated by precipitation from the reaction mixture.

The conversion of a compound of formula VIII to a compound of formula IXa, in which G⁴ is a halide, for example a bromide or a chloride, is generally carried out by reaction with a phosphorhalide like phosphorous trichloride or phosphorous tribromide or a phosphorous oxyhalide like phosphorous oxychloride or phosphorous oxybromide. The reaction can be carried out in neat conditions or in a suitable inert solvent, for example a hydrocarbon, such as benzene, toluene or xylene, or a chlorinated hydrocarbon, such as chlorobenzene, or a mixture of solvents, at temperatures from about 20 °C to about 200 °C, for example at temperatures from about 80 °C to about 120 °C, favorably with addition of a base, for example a tertiary amine, such as triethylamine, ethyldiisopropylamine, N-methylmorpholine or 1,8-diazabicyclo[5.4.0]unde-7-ene. The reaction time is generally from 30 min to 48 h, preferably from 30 min to 4 h, depending on the composition of the mixture and the chosen temperature range. The conversion of a compound of formula VIII to a compound of formula IXa, in which G⁴ is an alkylsulfonate, e.g. trifluoromethylsulfonate or methylsulfonate, is generally carried out by reaction with an alkanesulfonyl halide, such as methanesulfonyl chloride, or an alkane sulfonic anhydride, such as trifluoromethane sulfonic anhydride. The reaction can be carried out in neat conditions or in a suitable inert solvent, for example a hydrocarbon, such as benzene, toluene or xylene, or a chlorinated hydrocarbon, such as chlorobenzene, or an ether, such as THF, dioxane or DME, or a mixture of solvents, at temperatures from about 0 °C to about 200 °C, for example at temperatures from about 20 °C to about 120 °C, favorably with addition of a base, for example a tertiary amine, such as triethylamine, ethyldiisopropylamine, N-methylmorpholine or 1,8-diazabicyclo[5.4.0]unde-7-ene. The reaction time is generally from 30 min to 48 h, preferably from 30 min to 4 h, depending on the composition of the mixture and the chosen temperature range.

The reaction of the compound of the formula IXa with compounds of the formula X to a compound of formula IIa is a Suzuki-type reaction and is generally carried out in the presence of catalytic palladium compound, for example a palladium(II) salt such as palladium(II) acetate or palladium(II) chloride, which can be employed in the presence of a phosphine such as tricyclohexylphosphine or triphenylphosphine, or a palladium complex such as tetrakis(triphenylphosphine)palladium(0), palladium(0) bis(tri-tert-butylphosphin) or bis(triphenylphosphine)palladium(II) chloride, and favourably in the presence of a base, for example an alkaline metal carbonate or phosphate such as sodium carbonate or tripotassium phosphate, in an inert solvent, for example a hydrocarbon, such as benzene, toluene or xylene, or an ether, such as THF, dioxane or DME, or water, or a mixture of solvents, at temperatures from about 20 °C to about 200 °C, for example at temperatures from about 80 °C to about 120 °C. The reaction time is generally from 30 min to 48 h, preferably from 30 min to 16 h, depending on the composition of the mixture and the chosen temperature range.

A compound of the formula IIa can already be a compound of formula II, if G¹ and G² are identical in the compounds of formula IIa and II and if G³ in the compound of formula IIa is H. If a compound of formula IIa is not already a compound of formula II, it can be transformed into a compound of formula II in one step or in several steps depending of the meaning of the groups G¹, G² and G³ as it was described above.

Alternatively, the compounds of the formula II can be obtained by reacting a ketone of formula XI with an oxalic acid derivative of formula XII in a Claisen-type condensation to a compound of formula XIII, which is then reacted with an amino pyrazole compound of formula IV to to give a compound of formula IIa, which can be a compound of formula II already or which can be converted into a compound of formula II, 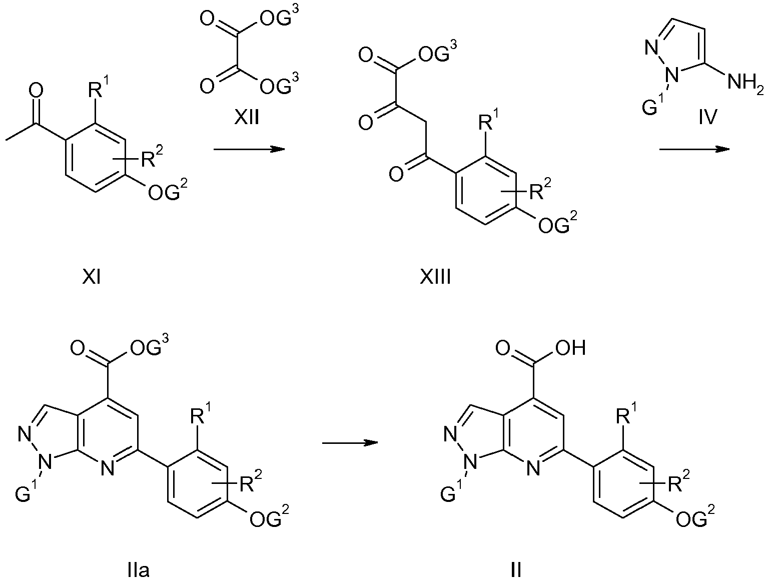 wherein the groups R¹ and R² in the compounds of the formulae II, IIa, XI and XIII are defined as in the compounds of the formula I. The group G¹ in the compounds of formulae II, IIa and IV is defined as a hydrogen or a protecting group for a pyrazole nitrogen, such as, for example, a 2-tetrahydropyranyl-group, a benzyl group, a 4-methoxybenzyl or a 2,5-dimethoxybenzyl group. The group G² in the compounds of formulae II, IIa, XI and XIII is defined as a hydrogen or a protecting group for a phenolic hydroxyl group, such as, for example, a 2-tetrahydropyranyl-group, a benzyl group, a 4-methoxybenzyl or a 2,5-dimethoxybenzyl group. The group G³ in the compounds of formulae IIa, VI and XII is hydrogen or a protecting group for a carboxylic acid, such as, for example, methyl, ethyl, propyl, tert-butyl or benzyl, preferably methyl or ethyl.

The Claisen-type condensation of a a ketone of formula XI with an oxalic acid derivative of formula XII is generally carried out in the presence of a base, for example an inorganic base such as an alkaline metal hydride, like sodium hydride, or an alkoxide or amide such as sodium methoxide, sodium ethoxide, potassium methoxide, potassium tert-butoxide, sodium amide or lithium diisopropylamide, and in an inert solvent, for example a hydrocarbon or chlorinated hydrocarbon such as benzene, toluene, xylene and chlorobenzene, an ether such as THF, dioxane, dibutyl ether, diisopropyl ether or DME, an alcohol such as methanol, ethanol, isopropanol or tert-butanol, or a mixture of solvents at temperatures from about 0 °C to about 200 °C, for example at temperatures from about 20 °C to about 120 °C. The reaction time is generally from 30 min to 48 h, preferably from 30 min to 8 h, depending on the composition of the mixture and the chosen temperature range. The compounds of formula XIII can be obtained in form of a tautomer and/or salt, e.g. as sodium 1-methoxycarbonyl-3-oxo-3-aryl-propen-1-olate for compounds of formula XIII, in which G³ is methyl.

The reaction of a compound of formula XIII with an amino pyrazole compound of formula IV to to give a compound of the formula IIa is generally carried out in the presence of an acid, for example acetic acid or hydrochloric acid or trifluoro acetic acid. The reation can be carried out neat or in suitable solvents at temperatures from about 0 °C to about 200 °C, for example at temperatures from about 20 °C to about 150 °C, preferably from about 80°C to 120°C. The reaction time is generally from 30 min to 48 h, preferably from 30 min to 16 h, depending on the composition of the mixture and the chosen temperature range. Depending on the solvents and conditions applied the preferred regioisomer of formula IIa can be easily isolated by precipitation from the reaction mixture.

A compound of the formula IIa can already be a compound of formula II, if G¹ and G² are identical in the compounds of formula IIa and II and if G³ in the compound of formula IIa is H. If a compound of formula IIa is not already a compound of formula II, it can be transformed into a compound of formula II in one step or in several steps depending of the meaning of the groups G¹, G² and G³ as it was described above.

In another process for the preparation of compounds of the formula I, a compound of the formula IXa, in which G³ is not hydrogen, is converted into a compound of formula IX, the latter compound is then reacted with a compound of the formula III to give a compound of formula XIV, which is then reacted with a compound of formula X to give a compound of the formula Ia, which can be a compound of formula I already or which can be converted into a compound of formula I, 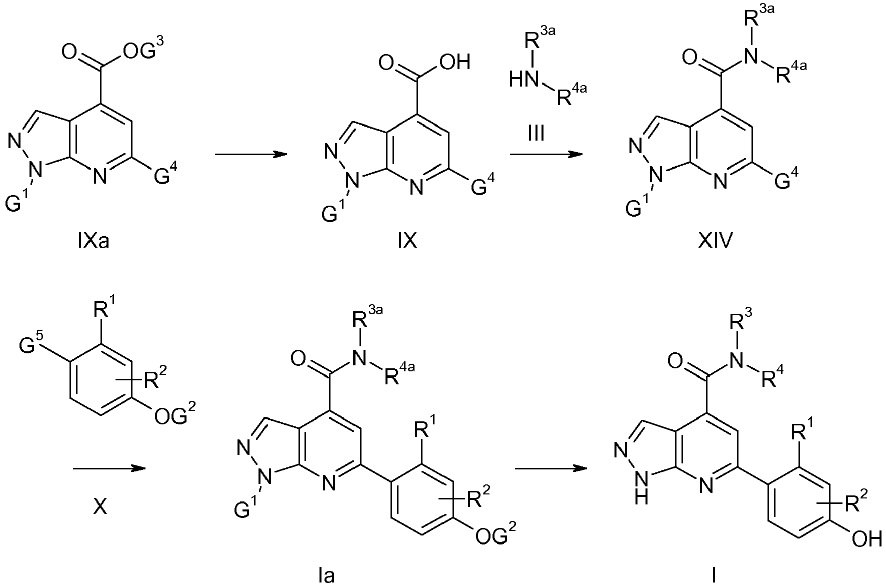 wherein the groups R¹ and R² in the compounds of the formulae Ia and X are defined as in the compounds of the formula I. The groups R^{3a} and R^{4a} in the compounds of formulae Ia, III and XIV are, independently of each other, either defined as the groups R³ and R⁴ in formula I, or they are precursors of the groups R³ and R⁴ in formula I, they can for example contain functional groups in protected form or functional groups which can be converted to obtain the final groups R³ and R⁴. The group G¹ in the compounds of formulae Ia, IX, IXa and XIV is defined as a hydrogen or a protecting group for a pyrazole nitrogen, such as, for example, a 2-tetrahydropyranyl-group, a benzyl group, a 4-methoxybenzyl or a 2,5-dimethoxybenzyl group. The group G² in the compounds of formulae Ia and X is defined as a hydrogen or a protecting group for a phenolic hydroxyl group, such as, for example, a 2-tetrahydropyranyl-group, a benzyl group, a 4-methoxybenzyl or a 2,5-dimethoxybenzyl group. The group G³ in the compounds of formula IXa is a protecting group for a carboxylic acid, such as, for example methyl, ethyl, propyl, tert-butyl or benzyl, preferably methyl or ethyl. The group G⁴ in the compounds of formulae IX, IXa and XIV is a leaving group, that can be replaced in a Suzuki-type reaction, such as a halide, e.g. bromide or chloride or as a sulfonate, e.g. a trifluoromethanesulfonate or a methanesulfonate. The group G⁵ in the compounds of formula X is a boronic acid or a boronic ester or cyclic boronic ester.

The conversion of a compound of formula IXa, in which G³ is not hydrogen, but a protecting group for a carboxylic acid, to a compound of formula IX is a suitable deprotection reaction, such as a basic hydrolysis, if G³ is a methyl, ethyl or propyl residue, or as an acidic deprotection, if G³ is a tert-butyl group, or as a hydrogenation, if G³ is a benzyl group. As it is known to the skilled person the employed protection groups should chosen in a manner to be compatible with the desired reaction conditions for all subsequent steps. All protection and deprotection reactions used in the above-described transformation of compounds of the formula IX to compounds of formula IX are well known to the skilled person and can be carried out under standard conditions according to, or analogously to, procedures described in the literature, for example in P. J. Kocienski, Protecting Groups, Georg Thieme Verlag, Stuttgart, 1994 or T. W. Greene, P. G. M. Wuts, Protective Groups in Organic Synthesis, John Wiley, New York, 1999).

The reaction of the compounds of the formulae IX and III to form an amide of formula Ia is generally performed in the presence of activating agents, such as CDI, DCC, EDC, HOAt, HOBt, HATU, TOTU, TBTU BEP or combinations thereof, and optionally an additional base, such as TEA, DIPEA or N-methylmorpholin in an appropriate inert solvent, for example a hydrocarbon or a chlorinated hydrocarbon such as benzene, toluene, chlorobenzene, dichloromethane, dichloroethane, chloroform, or an ether such as tetrahydrofurane, 1,4-dioxane, dibutylether, diisopropylether, methyl-tert-butylether, dimethoxyethane, or an ester such as ethyl acetate or ethyl butanoate or an amide such as N,N-dimethylformamide or N,N-dimethylacetamide or N-methylpyridone or a in mixture of solvents. The reaction temperature in this case is generally from -30°C to 200°C, preferably from -20°C to 80°, more preferably from 0°C to 20 °C. The reaction time is generally from 15 min to 6 days, preferably from 15 min to 16 h, depending on the composition of the mixture and the chosen temperature range. The acids of formula IX can be subjected to the reaction in form of their salts, for example their sodium salts. They can also be transformed into an activated derivative prior to the coupling with the amine, for example into an acid chloride or an acid anhydride by standard transformations. The amines of formula III can be subjected to the reaction in form of their salts, for example as hydrochloride or triflate salts, in which case usually an additional equivalent of the base is added to the reaction.

The reaction of the compound of the formula XIV with a compound of the formula X to a compound of formula Ia is a Suzuki-type reaction and is generally carried out in the presence of catalytic palladium compound, for example a palladium(II) salt such as palladium(II) acetate or palladium(II) chloride, which can be employed in the presence of a phosphine such as tricyclohexylphosphine or triphenylphosphine, or a palladium complex such as tetrakis(triphenylphosphine)palladium(0), palladium(0) bis(tri-tert-butylphosphin) or bis(triphenylphosphine)palladium(II) chloride, and favourably in the presence of a base, for example an alkaline metal carbonate or phosphate such as sodium carbonate or tripotassium phosphate, in an inert solvent, for example a hydrocarbon, such as benzene, toluene or xylene, or an ether, such as THF, dioxane or DME, or water, or a mixture of solvents, at temperatures from about 20 °C to about 200 °C, for example at temperatures from about 80 °C to about 120 °C. The reaction time is generally from 30 min to 48 h, preferably from 30 min to 16 h, depending on the composition of the mixture and the chosen temperature range.

A compound of the formula Ia can already be a compound of formula I, if G¹ and G² are both H and if R^{3a} is R³ and R^{4a} is R⁴. If a compound of formula Ia is not already a compound of formula I, it can be transformed into a compound of formula I in one step or in several steps depending of the meaning of the groups G¹, G², R^{3a} and R^{4a}, as it was described above.

All reactions used in the above-described syntheses of the compounds of the formula I are per se well known to the skilled person and can be carried out under standard conditions according to, or analogously to, procedures described in the literature, for example in Houben-Weyl, Methoden der Organischen Chemie (Methods of Organic Chemistry), Thieme-Verlag, Stuttgart, or Organic Reactions, John Wiley & Sons, New York. If desired, the obtained compounds of the formula I, as well as any intermediate compounds, can be purified by customary purification procedures, for example by recrystallization or chromatography. As already mentioned, all starting compounds and intermediates employed into the above-described syntheses which contain an acidic or basic group, can also be employed in the form of salts, and all intermediates and final target compounds can also be obtained in the form of salts. As likewise mentioned above, depending on the circumstances of the specific case, in order to avoid an unwanted course of a reaction or side reactions during the synthesis of a compound it can generally be necessary or advantageous to temporarily block functional groups by introducing protective groups and deprotect them at a later stage of the synthesis, or to introduce functional groups in the form of precursor groups which later are converted into the desired functional groups. As examples of protecting groups amino-protecting groups may be mentioned which can be acyl groups or alkyloxycarbonyl groups, for example a tert-butyloxycarbonyl group (= Boc) which can be removed by treatment with trifluoroacetic acid (= TFA), a benzyloxycarbonyl group which can be removed by catalytic hydrogenation, or a fluoren-9-ylmethoxycarbonyl group which can be removed by treatment with piperidine, and protecting groups of carboxylic acid groups which can be protected as ester groups, such as tert-butyl esters which can be deprotected by treatment with trifluoroacetic acid, or benzyl esters which can be deprotected by catalytic hydrogenation. As an example of a precursor group the nitro group may be mentioned, which can be converted into an amino group by reduction, for example by catalytic hydrogenation. Such synthesis strategies, and protective groups and precursor groups which are suitable in a specific case, are known to the skilled person.

Another subject of the present invention are the novel starting compounds and intermediates occurring in the synthesis of the compounds of the formula I, including the compounds of the formulae Ia, II, IIa, III, IV, V, VI, VII-I, VII-II, VIII, IX, IXa, X, XI, XII, XIII and XIV, wherein R¹, R², R³, R⁴, R^{3a}, R^{4a}, G¹, G², G³, G⁴ and G⁵ are defined as above, in any of their stereoisomeric forms or a mixture of stereoisomeric forms in any ratio, and their salts, and solvates of any of them, and their use as intermediates. The invention also includes all tautomeric forms of the said intermediates and starting compounds. All explanations given above and embodiments specified above with respect to the compounds of the formula I apply correspondingly to the said intermediates and starting compounds. Another subject of the invention are in particular the novel specific starting compounds and intermediates disclosed herein. Independently thereof whether they are disclosed as a free compound and/or as a specific salt, they are a subject of the invention both in the form of the free compounds and in the form of their salts, and if a specific salt is disclosed, additionally in the form of this specific salt, and in the form of solvates of any of them.

The following examples illustrate the invention.

When example compounds containing a basic group were purified by preparative high pressure liquid chromatography (HPLC) on reversed phase (RP) column material and, as customary, the eluent was a gradient mixture of water and acetonitrile containing trifluoroacetic acid (TFA), they were in part obtained in the form of their acid addition salt with trifluoroacetic acid, depending on the details of the workup such as evaporation or lyophilization conditions. In the names of the example compounds and their structural formulae any such contained trifluoroacetic acid is not specified. When example compounds containing a basic group were obtained after an acidic deprotection step, they were in part obtained in the form of their acid addition salt with hydrochloric acid, depending on the details of the workup such as washing steps with bases or evaporation or lyophilization conditions. In the names of the example compounds and their structural formulae any such contained hydrochloric acid may or may not be specified.

The prepared compounds were in general characterized by spectroscopic data and chromatographic data, in particular mass spectra (MS) and HPLC retention times (Rₜ; in min) which were obtained by combined analytical HPLC/MS characterization (LC/MS), and/or nuclear magnetic resonance (NMR) spectra. In the NMR characterization, the chemical shift δ (in ppm), the number of hydrogen atoms and the multiplicity (s = singlet, d = doublet, dd = double doublet, t = triplet, dt = double triplet, q = quartet, m = multiplet; b = broad) of the peaks is given. In the MS characterization, in general the mass number (m/z) of the peak of the molecular ion M, e.g. M⁺, or of a related ion such as the ion M+1, e.g. [M+1]⁺, i.e. the protonated molecular ion [M+H]⁺, which was formed depending on the ionization method used, is given. Generally, the ionization method was electrospray ionization (ESI). The LC/MS conditions used were as follows.

### Method LC1:

Column: BEH C18 2.1 x 50 mm; 1.7pm; flow: 0.9 ml; solvent A: water + 0.1% formic acid; solvent B: acetonitrile + 0.08 % formic acid; gradient from 95% A + 5% B to 5% A + 95% B in 1.1min; then 5% A + 95 % B for 0.6 min; MS ionisation method: ESI⁺.

### Method LC2:

Column: Waters Xbridge C18 4.6 mm x 50 mm, 2.5 µM; flow: 1.3 ml/min; solvent A: water + 0.05% TFA; solvent B: methanol + 0.05% TFA; gradient from 98% A + 2% B for 1 min, then from 98% A + 2% B to 5% A + 95% B in 4 min, then 5% A + 95% B for 1.25min; MS ionisation method: ESI⁺.

### Method LC3:

Column: Waters Xbridge C18 4.6 mm x 50 mm, 2.5 µM; flow: 1.3 ml/min; solvent A: water + 0.1% formic acid; solvent B: acetonitrile + 0.1% formic acid; gradient from 97% A + 3% B to 40% A + 60% B in 3.5min, then to 2% A + 98% B in 0.5min; then 2% A + 98% B for1.0 min; MS ionisation method: ESI⁺.

### Method LC4:

Column: Phenomenex, 10 x 2 mm, 1.7 µm; flow: 1.1 ml/min; solvent A: water + 0.05% trifluoroacetic acid; solvent B: acetonitrile; gradient: from 93% A + 7% B to 5% A + 95% B in 1.2 min, then 5% A + 95% B for 0.2 min; MS-Ionisationmethod: ESI⁺.

### Method LC5:

Column: BEH C18 2.1 x 50 mm; 1.7µm, flow: 0.9 ml, solvent A water + 0.05% formic acid; solvent B: acetonitrlie + 0.035% formic acid; gradient from 95% A + 5% B to 5% A + 95% B in 1.1min; then 5% A + 95 % B for 0.6 min; MS ionisation method: ESI⁺.

### Method LC6:

Column: YMC-Pack Jsphere H80 33 x 2.1 mm, 4 µm; flow: 1.3 ml/min; solvent A: water + 0.05% trifluoroacetic acid; solvent B: acetonitrile + 0.05% trifluoroacetic acid; gradient from 95% A + 5% B to 5% A + 95% B in 2.5 min, then to 95% A +5% B in 0.7 min; MS-Ionisationmethod: ESI⁺

### Method LC7:

Column: YMC-Pack Jsphere H80 33 x 2.1 mm, 4 µm; flow: 1.3 ml/min; solvent A: water + 0.05% trifluoroacetic acid; solvent B: acetonitrile + 0.05% trifluoroacetic acid; gradient from 95% A + 5% B to 5% A + 95% B in 2.5 min, MS-Ionisationmethod: ESI⁺

### Method LC8:

Column Waters Xbridge C18 4.6 mm x 50 mm, 2.5 µM; flow: 1.3 ml/min; solvent A: water + 0.1% formic acid; solvent B: acetonitrile + 0.1% formic acid; gradient from 97% A + 3% B to 40% A + 60% B in 3.5min, then to 2% A + 98% B in 0.5min; then 2% A + 98% B for1.0 min; then to 97% A + 3% B in 0.2 min, then 97% A + 3% B for 1.3 min; MS ionisation method: ESI⁺.

### Method LC9:

Instrument: Waters UPLC, column: BEH C18, 2.1 x 50 mm; 1.7µm, flow: 0.9 ml, solvent A water + 0.05% formic acid; solvent B: acetonitrlie + 0.035% formic acid; gradient from 98% A + 2% B to 5% A + 95% B in 2 min, then 5% A + 95% B for 0.6 min; MS-Ionisationmethod: ESI⁺.

### Method LC10:

Column: YMC Jsphere H80 33 x 2.1 mm, 4 µm; flow: 1 ml/min; solvent A: water + 0.05% trifluoroacetic acid; solvent B: methanol + 0.05% trifluoroacetic acid; gradient 98% A + 2% B for 1 min, then from 98% A + 2% B to 5% A + 95% B in 4.0 min, then 5% A + 95% B for 1.25min; MS ionisation method: ESI⁺.

### Method LC11:

Column: Waters Xbridge C18 4.6 mm x 50 mm, 2.5 µM; flow: 1.3 ml/min; solvent A: water + 0.05% trifluoroacetic acid; solvent B: acetonitrile + 0.05% trifluoroacetic acid; gradient 95% A + 5% B for 0.3 min, then from 95% A + 5% B to 5% A + 95% B in 3.2 min, then 5% A + 95% B for 0.5 min; MS ionisation method: ESI⁺.

### Method LC12:

Column: Waters Xbridge C18 4.6 mm x 50 mm, 2.5 µM; flow: 1.3 ml/min; solvent A: water + 0.1% formic acid; solvent B: acetonitrile + 0.08% formic acid; gradient from 97% A + 3% B to 40% A + 60% B in 3.5min, then to 2% A + 98% B in 0.5min; then 2% A + 98% B for1.0 min; MS ionisation method: ESI⁺.

### Method LC13:

Column: YMC Jsphere ODS H80 33 x 2 mm, 4 µm; flow: 1.0 ml/min; solvent A: water + 0.05% formic acid; solvent B: acetonitrile; gradient from 90% A + 10% B to 5% A + 95% B in 2.5min, then 5% A + 95% B for 0.85 min; then to 90% A + 10% B in 0.05min; MS ionisation method: ESI+.

### Method LC14:

Ccolumn: YMC Jsphere ODS H80 20 x 2 mm, 1 4 µm; flow: 1.0 ml/min; solvent A: water + 0.05% formic acid; solvent B: acetonitrile; gradient from 96% A + 4% B to 5% A + 95% B in 2.0min, then 5% A + 95% B for 0.40 min; MS ionisation method: ESI+.

### Method LC15:

Column: Phenomenex, 10 x 2 mm, 1.7 µm; flow: 1.1 ml/min; solvent A: water + 0.05% trifluoroacetic acid; solvent B: acetonitrile; gradient: 80% A + 20% B for 0.8 min, then from 80% A + 20% B to 5% A + 95% B in 0.6 min, then to 80% A + 20% B in 0.05 min; MS-Ionisationmethod: ESI⁺.

### List of abbreviations

- BEP: 2-bromo-1-ethyl-pyridinium tetrafluoroborate
- BOC: tert.butoxy carbonyl
- CDI: N,N'-carbonyldiimidazole
- DCC: 1,3-dicyclohexylcarbodiimide
- DCM: dichloromethane
- DEA: diethylamine
- DMF: N,N-dimethylformamide
- EDC: 1-(3-dimethylaminopropyl)-3-ethylcarbodiimide hydrochloride
- EtOAc: ethyl acetate
- EtOH: ethanol
- Exp. No.: Example number
- h: hour/s
- HOAt: 1-hydroxy-aza-benzotriazole
- HOBt: 1-hydroxy-benzotirazole
- HATU: O-(7-azabenzotriazol-1-yl)-N,N,N',N'-tetramethyl-uronium hexafluorophosphate
- HPLC: high pressure liquid chromatography
- LC: liquid chromatography
- MeOH: methanol
- Min: minutes
- MS: mass spectroscopy
- Rt: retention time
- r.t.: room temperature
- sep.: separation
- TBTU: [(benzotriazol-1-yloxy)-dimethylamino-methylene]-dimethyl-ammonium tetrafluoroborate
- TEA: triethylamine
- TFA: trifluoroacetic acid
- THF: tetrahydrofurane

- TOTU: O-(cyano(ethoxycarbonyl)methyleneamino)-N,N,N',N'-tetramethyluronium tetrafluoroborate

### Example 1: 6-(4-Hydroxy-phenyl)-1H-pyrazolo[3,4-b]pyridine-4-carboxylic acid (2,2,6,6-tetramethyl-piperidin-4-yl)-amide

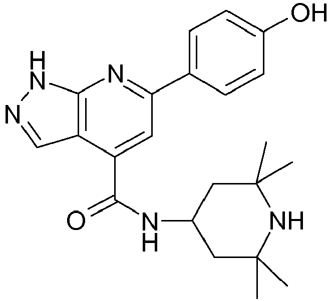

### (a) 6-(4-Hydroxy-phenyl)-1H-pyrazolo[3,4-b]pyridine-4-carboxylic acid methyl ester

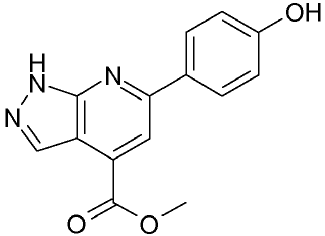

A mixture of 3-amino-pyrazole (2.5 g) was dissolved in acetic acid/methanol (v/v = 1/1, 100 mL) and pyruvic acid methyl ester (3.75 g) and 4-hydroxybenzaldehyde (3.02 mL) were added. The solution was allowed to stir at 75°C for 15 h, cooled to r.t., and the precipitate formed was isolated by filtration. The liquid was evaporated to dryness, water was added and the mixture was lyophilized. The resulting residue was purified by chromatography to give 6-(4-Hydroxy-phenyl)-1H-pyrazolo[3,4-b]pyridine-4-carboxylic acid methyl ester (1.62 g, 20%) 1 H-NMR (500MHz, d6-DMSO): 4.00 (s, 3H), 6.95 (d, 2H), 8.05 (d, 2H), 8.10 (s, 1 H), 8.35 (s, 1 H), 10.00 (s, 1 H), 13.90 (s, 1 H).
LC/MS (Method LC6): Rₜ = 1.29 min; m/z = 270.03 [M+H]⁺.

### (b) 6-(4-Hydroxy-phenyl)-1H-pyrazolo[3,4-b]pyridine-4-carboxylic acid

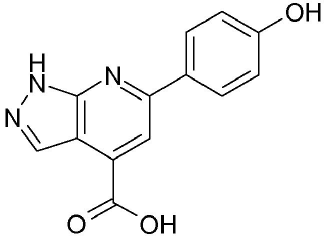

0.68 g of 6-(4-Hydroxy-phenyl)-1H-pyrazolo[3,4-b]pyridine-4-carboxylic acid methyl ester were dissolved in 16 mL of a mixture of 2N aqueous LiOH:MeOH (1:1). After the reaction was complete, methanol was removed in vacuo, water was added and the solution acidified to pH 1 by addition of hydrochloric acid. The resulting precipitate was filtered off to give 0.38 g (59%) of (4-Hydroxy-phenyl)-1H-pyrazolo[3,4-b]pyridine-4-carboxylic acid.
LC/MS (Method LC6): Rₜ = 1.06 min; m/z = 256.22 [M+H]⁺.

### (c) 6-(4-Hydroxy-phenyl)-1H-pyrazolo[3,4-b]pyridine-4-carboxylic acid (2,2,6,6-tetramethyl-piperidin-4-yl)-amide

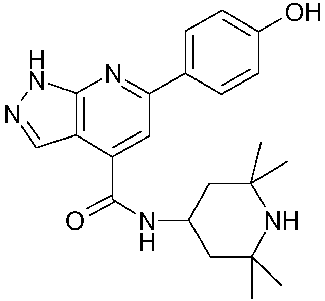

500 mg of 6-(4-Hydroxy-phenyl)-1H-pyrazolo[3,4-b]pyridine-4-carboxylic acid was dissolved in 1 mL of dry DMF. 38 mg of EDC, 26 mg of HOBT and 0.2 mL of N,N-diisopropylethylamine and 37 mg 4-amino-2,2,6,6,-tetramethylpiperidine were added. If necessary, additional piperidine and EDC was added. The reaction mixture was purified by HPLC to give 44 mg of the desired product as trifluoroacetate salt. LC/MS (Method LC7): Rₜ = 0.97 min; m/z = 394.28 [M+H]⁺.

### Example 2: 6-(4-Hydroxy-phenyl)-1H-pyrazolo[3,4-b]pyridine-4-carboxylic acid piperidin-4-ylamide

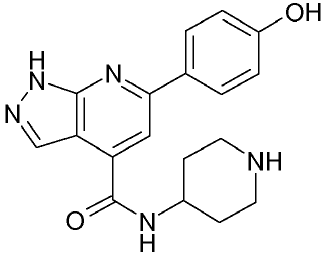

4-{[6-(4-Hydroxy-phenyl)-1H-pyrazolo[3,4-b]pyridine-4-carbonyl]-amino}-piperidine-1-carboxylic acid tert-butyl ester, obtained by reacting 100 mg of 6-(4-Hydroxy-phenyl)-1H-pyrazolo[3,4-b]pyridine-4-carboxylic acid (Example 1, step (b)) and 157 mg of 4-amino-1-boc-piperidine under similar conditions as described for Example 1, step (c), was dissolved in 15 mL of isopropanol and 2 mL of isopropanolic HCl were added.After completion of the reaction, water was added and the mixture was lyophilized. Water was added and the mixture was lyophilized again to give 31 mg of 6-(4-Hydroxyphenyl)-1H-pyrazolo[3,4-b]pyridine-4-carboxylic acid piperidin-4-ylamide as hydrochloride salt.
LC/MS (Method LC7): Rₜ = 0.81 min; m/z = 338.27 [M+H]⁺.

### Example 3: (3-Amino-piperidin-1-yl)-[6-(4-hydroxy-phenyl)-1H-pyrazolo[3,4-b]pyridin-4-yl]-methanone

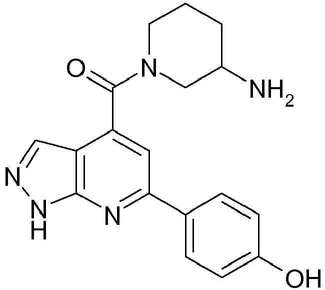

### (a) 6-(4-Hydroxy-phenyl)-1-(tetrahydro-pyran-2-yl)-1H-pyrazolo[3,4-b]pyridine-4-carboxylic acid methyl ester

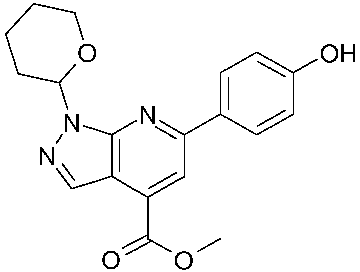

A mixture of crude 6-(4-hydroxy-phenyl)-1H-pyrazolo[3,4-b]pyridine-4-carboxylic acid methyl ester (44 g, obtained from reacting 25 g 3-aminopyrazole, 36.75 g of 4-hydroxybenzaldehyde and 30.2 mL of pyruvic acid methyl ester in 250 mL of acetic acid and methanol (v/v: 1:1) under similar conditions as described for Example 1, Step (a)), 3,4-dihydro-2H-pyran (32 mL) and p-toluenesulfonic acid monohydrate (9.2 g) in THF (1 I) was stirred at r.t. for 70 h. The formed precipitate was filtered off and additional 2 eq of 3,4-dihydro-2H-pyran were added and stirring was continued for another 24 h. Water was added and the mixture was extracted with ethyl acetate, the combined organic phases were dried over magnesium sulfate and concentrated. The residue was purified by silica gel chromatography (ethyl acetate:heptane) to give 3.47g of the desired product.
1 H-NMR (500MHz, d6-DMSO): 1.60 (m, 2H), 1.75 (m, 1 H), 1.95 (m, 1 H), 2.10 (m, 1 H), 2.55 (m, 1 H), 3.70 (m, 1 H), 3.95 (m, 1 H), 4.05 (s, 3H), 6.15 (dd,1 H), 6.95 (d, 2H), 8.15 (d, 2H), 8.20 (s, 1 H), 8.40 (s, 1 H), 10.00 (s, 1 H).
LC/MS (Method LC4): Rt = 0.87 min; m/z = 354.10 [M+H]⁺.

### (b) 6-(4-Hydroxy-phenyl)-1-(tetrahydro-pyran-2-yl)-1H-pyrazolo[3,4-b]pyridine-4-carboxylic acid

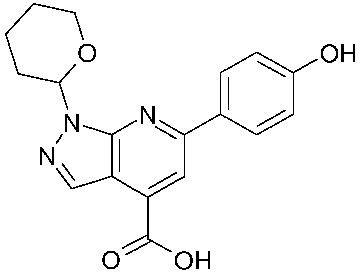

A solution of 6-(4-Hydroxy-phenyl)-1-(tetrahydro-pyran-2-yl)-1H-pyrazolo[3,4-b]pyri-dine-4-carboxylic acid methyl ester (3.44 g) in isopropanol (40 mL) and sodium hydroxide solution (1 N, 40 mL) was stirred at r.t. After completion of the reaction the pH was adjusted to 4 with acetic acid and the mixture was extracted with ethyl acetate. The organic layer wasdried over magnesium sulfate and evaporated to dryness to give 3.06 g (93%) of the desired product.
1 H-NMR (500MHz, d6-DMSO): 1.60 (m, 2H), 1.75 (m, 1 H), 1.95 (m, 1 H), 2.10 (m, 1 H), 2.55 (m, 1 H), 3.75 (m, 1 H), 3.95 (m, 1 H), 6.15 (dd,1 H), 6.95 (d, 2H), 8.10 (d, 2H), 8.15 (s, 1 H), 8.35 (s, 1 H), 10.00 (s, 1 H), 14.00 (s, br, 1 H).
LC/MS (Method LC4): Rₜ = 0.81 min; m/z = 340.00 [M+H]⁺.

Alternatively, 6-(4-Hydroxy-phenyl)-1-(tetrahydro-pyran-2-yl)-1H-pyrazolo[3,4-b]pyridine-4-carboxylic acid was prepared by the following reaction sequence:

### (a) 6-Hydroxy-1H-pyrazolo[3,4-b]pyridine-4-carboxylic acid ethyl ester

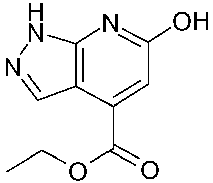

5-Aminopyrazole (25 g) and diethyl oxalacetate sodium salt (74.4 g) were dissolved under cooling in 1 N hydrochloric acid. 68.75 mL of glacial acetic acid were added and the mixture was stirred at 80°C for 14 h. The mixture was cooled to room temperature and the formed precipite was filtered off and titurated in ethyl acetate to give 16.06 g (26%) of the desired product.
LC/MS (Method LC1): Rₜ = 0.86 min; m/z = 208.07 [M+H]⁺.

### (b) 6-Bromo-1-(tetrahydro-pyran-2-yl)-1H-pyrazolo[3,4-b]pyridine-4-carboxylic acid ethyl ester

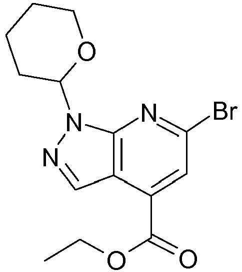

6-Hydroxy-1H-pyrazolo[3,4-b]pyridine-4-carboxylic acid ethyl ester (20 g) and phosphorus oxybromide (30.45 g) were dissolved in toluene and stirred at 110°C. Upon completion of the reaction, the mixture was cooled to room temperature and poured under cooling to a solution of 28.4 g of potassium acetate in water (400 mL). The mixture was extracted with ethyl acetate, the combined organic layers were dried over magnesium sulfate and evaporated to dryness. The obtained mterial was dissolved in 150 mL of THF and 8.87 mL of 3,4-dihydro-2H-pyran and 5.09 g of p-toluenesulfonic acid were added. Upon complete conversion, water was added and the mixture was extracted with ethyl acetate. The combined organic layers were dried over magnesium sulfate, evaporated to dryness and the product was purified by silica gel chromatography (ethyl acetate/heptane) to give 18.5 g of the desired product.
LC/MS (Method LC1): Rₜ = 1.27min; m/z = 353.96 [M+H]⁺.

### (c) 6-(4-Hydroxy-phenyl)-1-(tetrahydro-pyran-2-yl)-1H-pyrazolo[3,4-b]pyridine-4-carboxylic acid

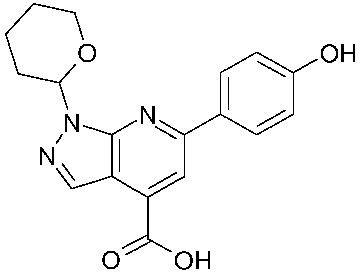

6-Bromo-1-(tetrahydro-pyran-2-yl)-1H-pyrazolo[3,4-b]pyridine-4-carboxylic acid ethyl ester (2.00 g), 4-Hydroxyphenyl boronic acid (935 mg) and cesium carbonate (4.60 g) were dissolved in 40 mL of a degased mixture of THF and water (v/v 1:1). Dichloro-[1,1'-bis(diphenylphosphino)ferrocene]palladium(II) (148 mg) was added and the mixture was heated to 80° for 3h. The pH was adjusted to 11 by addition of aqeous sodium hydroxide (5N) and heating was continued until the reaction was complete. The mixture was allowed to cool to room temperature, filtered and the solution was carefully acidified under cooling by addition of 2N hydrochloric acid. The formed precipitate was filtered off and dried in vacuo to give 1.96 g (100%) of the desired product.
1 H-NMR (500MHz, d6-DMSO): 1.60 (m, 2H), 1.75 (m, 1 H), 1.95 (m, 1 H), 2.10 (m, 1 H), 2.55 (m, 1 H), 3.75 (m, 1 H), 3.95 (m, 1 H), 6.15 (dd,1 H), 6.95 (d, 2H), 8.10 (d, 2H), 8.15 (s, 1 H), 8.35 (s, 1 H), 10.00 (s, 1 H), 14.00 (s, br, 1 H).
LC/MS (Method LC4): Rₜ = 0.81 min; m/z = 340.00 [M+H]⁺.

### (d) {1-[6-(4-Hydroxy-phenyl)-1-(tetrahydro-pyran-2-yl)-1H-pyrazolo[3,4-b]pyridine-4-carbonyl]-piperidin-3-yl}-carbamic acid tert-butyl ester

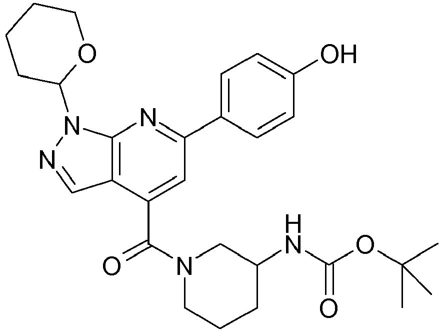

To a suspension of 6-(4-hydroxy-phenyl-1-(tetrahydro-pyran-2-yl)-1H-pyrazolo[3,4-b]-pyridine-4-carboxylic acid (150 mg) in dry dichloromethane (3 mL), N,Ndiisopropylethylamine (70 µL), 2-bromo-1-ethylpyridinium tetrafluoroborate (221 mg) and Piperidin-3-yl-carbamic acid tert-butyl ester (87 mg) were added. The solution was stirred for 30 min. Upon complete conversion, the solution was evaporated in vacuo and 3 mL of 30% sodium methoxide in methanol were added. The mixture was stirred for 30 min, the pH was adjusted to 5 by addition of 2 M hydrochloric acid. Ethyl acetate was added and the organic layer was extracted with saturated sodium bicarbonate, 20% aqueous ammonium chloride solution and multiple times with water. The organic layer was dried over sodium sulfate and evaporated to dryness to give the title compound (132 mg, 58% yield).
LC/MS (Method LC1): Rₜ = 1.29 min; m/z = 522.32 [M+H]⁺.

### (e) (3-Amino-piperidin-1-yl)-[6-(4-hydroxy-phenyl)-1H-pyrazolo[3,4-b]pyridin-4-yl]-methanone

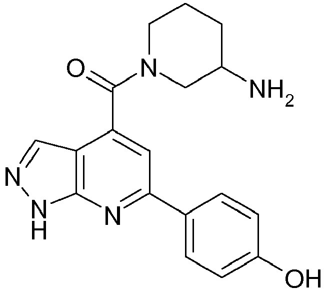

130 mg of {1-[6-(4-Hydroxy-phenyl)-1-(tetrahydro-pyran-2-yl)-1H-pyrazolo[3,4-b]pyridine-4-carbonyl]-piperidin-3-yl}-carbamic acid tert-butyl ester were dissolved in 4.6 mL of 4M hydrochloric acid in dioxane. After complete conversion, dioxane was removed in vacuo, water was added and the solution was extracted twice with dichloromethane /isopropanol (10/1). The aqueous layer was lyophilized, taken up in water three times and lyophilized again to give the title compound as hydrochloride salt (96mg, 100% yield).
LC/MS (Method LC1): Rt = 0.77 min; m/z = 338.17 [M+H]⁺.

The following examples have been prepared in a similar fashion as the synthesis for example 3 described immediately above employing the respective amine precursors:

| Exp. No. | Name | Structure | LC/MS method | m/z [M+H]⁺ | Rt [min] |
|---|---|---|---|---|---|
| 4 | [6-(4-Hydroxy-phenyl)-1H-pyrazolo[3,4-b]pyridin-4-yl]-piperazin-1-yl-methanone | 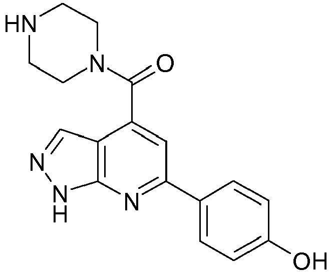 | LC7 | 324.28 | 0.77 |
| 5 | 6-(4-Hydroxy-phenyl)-1H-pyrazolo[3,4-b]pyridine-4-carboxylic acid pyrrolidin-3-ylamide | 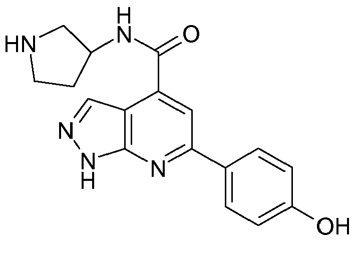 | LC1 | 324.15 | 0.74 |
| 6 | 6-(4-Hydroxy-phenyl)-1H-pyrazolo[3,4-b]pyridine-4-carboxylic acid ((3S,4S)-4-methoxy-pyrrolidin-3-yl)-amide | 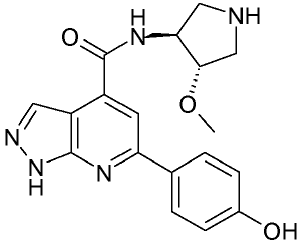 | LC1 | 354.18 | 0.78 |
| 7 | [1,4]Diazepan-1-yl-[6-(4-hydroxy-phenyl)-1H-pyrazolo[3,4-b]pyridin-4-yl]-methanone | 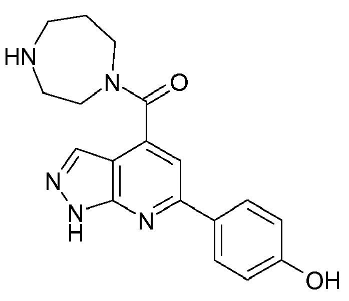 | LC1 | 338.2 | 0.66 |
| 8 | 6-(4-Hydroxy-phenyl)-1H-pyrazolo[3,4-b]pyridine-4-carboxylic acid (cis-4-amino-cyclohexyl)-amide | 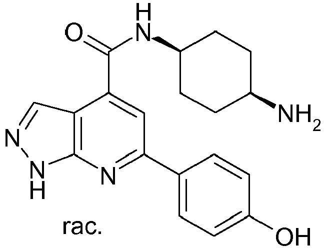 | LC1 | 352.21 | 0.84 |
| 9 | 6-(4-Hydroxy-phenyl)-1H-pyrazolo[3,4-b]pyridine-4-carboxylic acid (trans-4-amino-cyclohexyl)-amide | 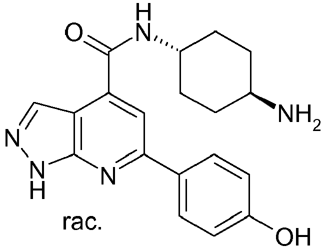 | LC4 | 352.1 | 0.51 |
| 10 | (2,2-Dimethyl-piperazin-1-yl)-[6-(4-hydroxy-phenyl)-1H-pyrazolo[3,4-b]pyridin-4-yl]-methanone | 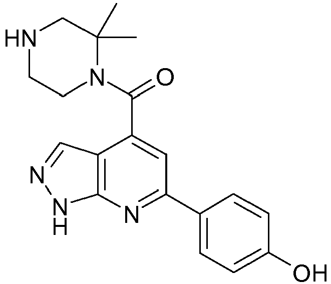 | LC4 | 352.1 | 0.50 |
| 11 | 6-(4-Hydroxy-phenyl)-1H-pyrazolo[3,4-b]pyridine-4-carboxylic acid (trans-4-amino-cyclohexylmethyl)-amide | 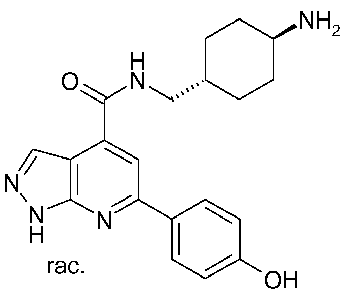 | LC1 | 366.22 | 0.81 |
| 12 | 6-(4-Hydroxy-phenyl)-1H-pyrazolo[3,4-b]pyridine-4-carboxylic acid (cis-4-amino-cyclohexylmethyl)-amide | 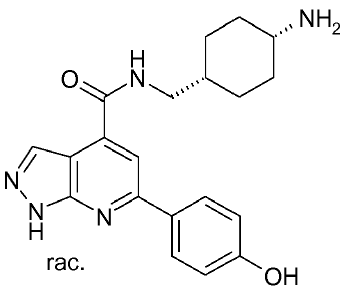 | LC1 | 366.25 | 0.86 |
| 13 | 6-(4-Hydroxy-phenyl)-1H-pyrazolo[3,4-b]pyridine-4-carboxylic acid (azetidin-3-ylmethyl)-amide | 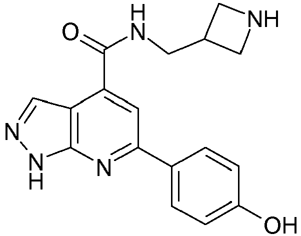 | LC4 | 324.10 | 0.44 |
| 14 | 6-(4-Hydroxy-phenyl)-1H-pyrazolo[3,4-b]pyridine-4-carboxylic acid (3-amino-cyclobutyl)-amide | 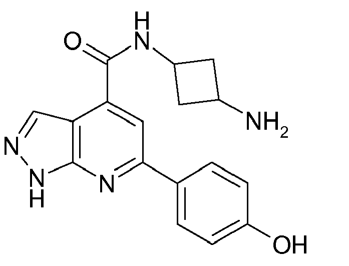 | LC4 | 324.10 | 0.45 |

### Example 15: [6-(2-Fluoro-4-hydroxy-phenyl)-1H-pyrazolo[3,4-b]pyridin-4-yl]-(5-isopropyl-2,2-dimethyl-piperazin-1-yl)-methanone

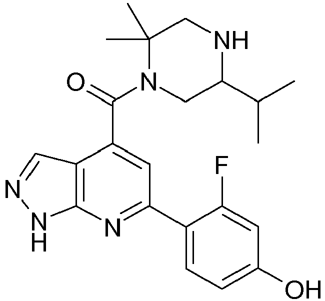

### (a) 6-Chloro-1H-pyrazolo[3,4-b]pyridine-4-carboxylic acid ethyl ester

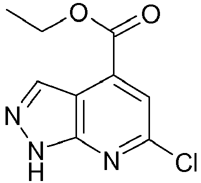

To a suspension of 4.17 g 6-hydroxy-1H-pyrazolo[3,4-b]pyridine-4-carboxylic acid ethyl ester (prepared as described in example 3, step (a) in the second sequence) in 60 ml of toluene was added 3.31 ml of 1,8-diazabicyclo[5.4.0]undec-7-ene. After stirring for 10 min at r.t. a solution of 1.88 ml of phosphorous oxychloride in 30 ml of toluene was slowly added at r.t.. Afterwards, the reaction was heated to 110°C for 5 h. After cooling the reaction mixture was slowly and under vigorous stirring added to 100 ml of a sat. aqueous potassium acetate solution. To the resulting mixture 100 ml of brine were added. The layers were separated and the aqueous layer was extracted with ethyl acetate. The combined organic layers were dried over sodium sulfate, filtered and concentrated in vacuo. 3.41 g (75 %) of the title compound were obtained, which were used without further purification in the next step.
LC/MS (Method LC4): Rt = 0.74 min; m/z = 226.05 [M+H]⁺.

### (b) 6-Chloro-1-(tetrahydro-pyran-2-yl)-1H-pyrazolo[3,4-b]pyridine-4-carboxylic acid ethyl ester

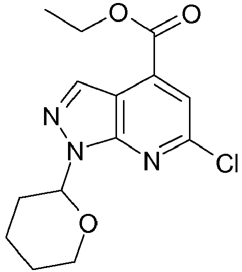

3.38 g of 6-chloro-1H-pyrazolo[3,4-b]pyridine-4-carboxylic acid ethyl ester were dissolved in 110 ml of tetrahydrofurane, and 2.03 ml of 3,4-dihydro-2H-pyran were added, followed by the addition of 0.85 g of p-toluenesulfonic acid monohydrate. The reaction was stirred at r.t. overnight, then it was poured unto 250 ml water and extracted with ethyl acetate twice. The combined organic layers were dried over sodium sulfate, filtered and concentrated in vacuo. After flash chromatography (silica, heptane/ethyl acetate gradient) 3.18 g (69 %) of the title compound were obtained. LC/MS (Method LC4): Rt = 1.03 min; m/z = 225.95 [M+H-THP]⁺.

### (c) 6-Chloro-1-(tetrahydro-pyran-2-yl)-1H-pyrazolo[3,4-b]pyridine-4-carboxylic acid

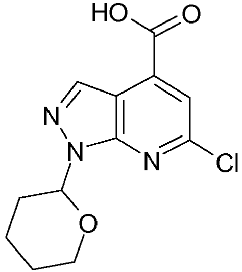

3.18 g 6-chloro-1-(tetrahydro-pyran-2-yl)-1H-pyrazolo[3,4-b]pyridine-4-carboxylic acid ethyl ester were dissolved in 40 ml of iso-propanol and 40 ml of 1 M aqueous sodium hydroxide solution were added. After 2 h at r.t. the reaction was poured unto water and brought to pH 5 by the addition of acetic acid. After removal of iso-propanol in vacuo a solid precipitated. It was collected by filtration and washed with water until neutral. The aqueous mother liquor was extracted with ethyl acetate twice. The combined organic layers were dried over sodium sulfate, filtered and concentrated in vacuo. The resulting residue was combined with the solid isolated before by filtration and after freeze-drying 2.18 g (75 %) of the title compound were obtained.
LC/MS (Method LC4): Rt = 0.77 min; m/z = 198.05 [M+H-THP]⁺.

### (d) 6-(4-Benzyloxy-2-fluoro-phenyl)-1-(tetrahydro-pyran-2-yl)-1H-pyrazolo[3,4-b]pyridine-4-carboxylic acid

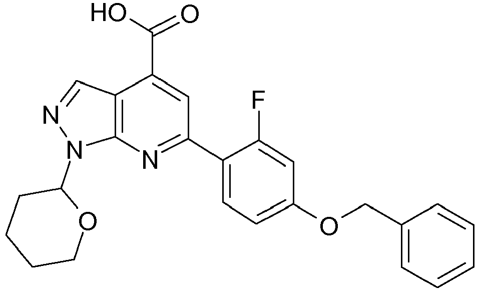

600 mg of 6-chloro-1-(tetrahydro-pyran-2-yl)-1H-pyrazolo[3,4-b]pyridine-4-carboxylic acid and 629 mg of 4-benzyloxy-2-fluoro-phenylboronic acid were dissolved in 8 ml of 1,2-dimethoxyethane. 883 mg of potassium carbonate and 8 ml of water were added. After purging the reaction with Argon, 33 mg of palladium(0) bis(tri-tert-butylphosphine) were added. In two portions the reaction was heated in a microwave reactor to 80°C for 15 min. The combined reaction mixtures were extracted with ethyl acetate twice. The combined organic layers were dried using sodium sulfate, filtered and concentrated in vacuo. After flash chromatography (silica, heptane/ethyl acetate gradient) 220 mg (23 %) of the title compound were obtained.
LC/MS (Method LC4): Rt = 1.14 min; m/z = 448.10 [M+H]⁺.

### (e) 4-[6-(4-Benzyloxy-2-fluoro-phenyl)-1-(tetrahydro-pyran-2-yl)-1H-pyrazolo[3,4-b]pyridine-4-carbonyl]-2-isopropyl-5,5-dimethyl-piperazine-1-carboxylic acid tert-butyl ester

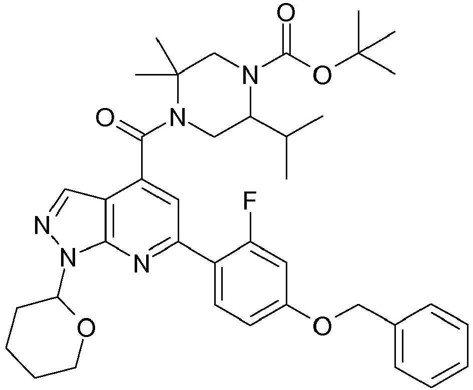

To a solution of 200 mg of 6-(4-benzyloxy-2-fluoro-phenyl)-1-(tetrahydro-pyran-2-yl)-1H-pyrazolo[3,4-b]pyridine-4-carboxylic acid in 3 ml of dichloromethane 0.31 ml of Hünig's base were added. Then 147 mg of 2-bromo-1-ethylpyridinium tetrafluoroborate and 138 mg of 2-isopropyl-5,5-dimethyl-piperazine-1-carboxylic acid tert-butyl ester were added. After stirring at r.t. overnight, additional 70 mg of 2-bromo-1-ethylpyridinium tetrafluoroborate and 0.1 ml of Hünigs's base were added. After additional 16 h at r.t. the reaction was taken up in ethyl acetate and water. The layers were separated and the organic layer was extracted with ethyl acetate twice. The combined organic layers were dried using sodium sulfate, filtered and concentrated in vacuo. After flash chromatography (silica, heptane/ethyl acetate gradient) 105 mg (34 %) of the title compound were obtained.
LC/MS (Method LC4): Rt = 1.38 min; m/z = 686.20 [M+H]⁺.

### (f) 4-[6-(2-Fluoro-4-hydroxy-phenyl)-1-(tetrahydro-pyran-2-yl)-1H-pyrazolo[3,4-b]pyridine-4-carbonyl]-2-isopropyl-5,5-dimethyl-piperazine-1-carboxylic acid tert-butyl ester

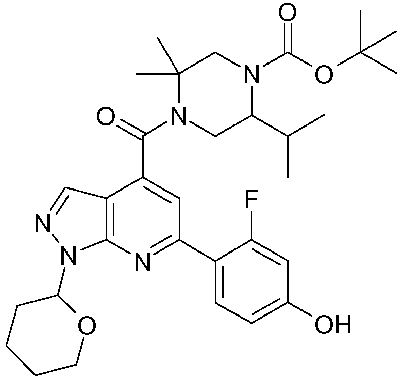

To a solution of 100 mg of 4-[6-(4-benzyloxy-2-fluoro-phenyl)-1-(tetrahydro-pyran-2-yl)-1H-pyrazolo[3,4-b]pyridine-4-carbonyl]-2-isopropyl-5,5-dimethyl-piperazine-1-carboxylic acid tert-butyl ester in 5 ml of dichloromethane 30 mg palladium on charcoal (10 %) were added and the reaction was hydrogenated at ambient pressure for 16 h. The catalyst was filtered off, and the solvent was removed in vacuo to obtain 67 mg (77%) of the title compound.
LC/MS (Method LC15): Rt = 0.83 min; m/z = 596.20 [M+H]⁺.

### (g) [6-(2-Fluoro-4-hydroxy-phenyl)-1H-pyrazolo[3,4-b]pyridin-4-yl]-(5-isopropyl-2,2-dimethyl-piperazin-1-yl)-methanone

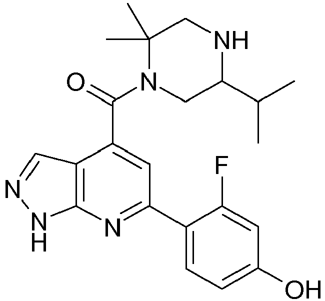

To a solution of 67 mg of 4-[6-(2-fluoro-4-hydroxy-phenyl)-1-(tetrahydro-pyran-2-yl)-1H-pyrazolo[3,4-b]pyridine-4-carbonyl]-2-isopropyl-5,5-dimethyl-piperazine-1-carboxylic acid tert-butyl ester in 1.3 ml of tetrahydrofurane 3 ml of hydrochloric acid (1 M in 1,4-dioxane) was added. After 16 h a r.t. the volatiles were removed in vacuo and the resulting residue was purified by HPLC to obtain 18 mg (36 %) of the title compound.
LC/MS (Method LC4): Rt = 0.57 min; m/z = 412.10 [M+H]+.

### Example 16: ((R)-3-Amino-pyrrolidin-1-yl)-[6-(4-hydroxy-phenyl)-1H-pyrazolo[3,4-b]pyridin-4-yl]-methanone

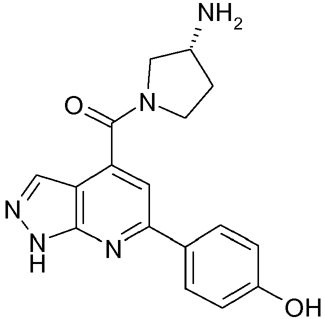

### (a) 2-(4-Methoxy-benzyl)-2H-pyrazol-3-ylamine

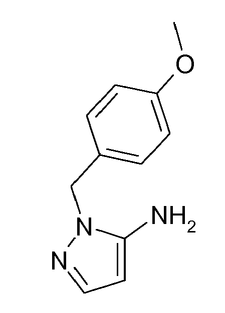

In a modification of a protocol previously described by R. N. Misra et al (Bioorg. Med. Chem. Lett. 2003, 13, 1133-1136) to 2.4 I of tetrahydrofuran at 0 °C 250 g (4.7 mol) of acrylonitrile were added slowly. 237 g (1 eq, 4.7 mol) hydrazine hydrate were added dropwise keeping the temperature around 10°C. The reaction mixture was stirred for 2 hours at this temperature. Then 667 g (1.04 eq, 4.9 mol) of anisaldehyde were added and the reaction mixture was stirred over night. The solvent was removed under reduced pressure and the residue was dried in vacuo. The crude product, as obtained above, was added at room temperature to a suspension of 300 g (5.5 mol) of sodium methoxide and 200 g powdered molecular sieve in 2 I of anhydrous n-butanol. The reaction mixture was heated to 120°C for four hours (exothermic reaction starts at 80°C and temperatures rises by itself to 110°C). For workup the reaction was cooled to room temperature, and water and ethyl acetate were added. The reaction mixture was filtered over a small pad of celite and the celite was washed with ethyl acetate. The phases were separated and the aqueous phase was extracted three times with ethylacetate. The organic phase was evaporated to yield the crude product. The residue was taken up in 2 I ethyl acetate and washed once with water. The product was precipitated by adding 1 I 5 - 6 N HCl in isopropanol and the solution was cooled to 5 °C. The solid precipitate was collected by filtration and dried in vacuo to give 565 g (50 %) 2-(4-methoxy-benzyl)-2H-pyrazol-3-yl amine hydrochlorid as a colourless powder.
1 H-NMR (400MHz, d6-DMSO): 3.74 (s, 3H), 5.34 (s, 2H), 5.71 (d, 1 H, 3.0 Hz), 6.93 (d, 2H, 8.5 Hz), 7.31 (d, 2H, 8.5 Hz), 7.93 (d, 1 H, 3.0 Hz).
LC/MS (Method LC14): Rt = 0.57 min; m/z = 204.1 [M+H]⁺.

### (b) 6-Hydroxy-1-(4-methoxy-benzyl)-1H-pyrazolo[3,4-b]pyridine-4-carboxylic acid methyl ester

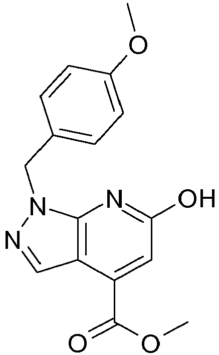

560 g (2.36 mol) 2-(4-Methoxy-benzyl)-2H-pyrazol-3-y lamine hydrochloride, as obtained above, were dissolved in 2 I of dichloromethane and washed twice with 1 I NaOH (2 N), then with saturated aqueous sodium hydrogencarbonate solution and brine. The organic layer was concentrated in vacuo to yield free 2-(4-methoxy-benzyl)-2H-pyrazol-3-ylamine. The free 2-(4-methoxy-benzyl)-2H-pyrazol-3-ylamine was dissolved in 2.9 I of acetic acid and stirred vigorously at 50 °C. To this solution was added 472 g (3.3 mol, 1.4 eq) dimethylacetylenedicarboxylate slowly. After 1 h the solution was warmed to 100 °C and stirred at this temperature for 12 h. For workup the solvent was removed in vacuo and the crude product was crystallized from ethanol to yield regioisomerically pure product (230 g, 31%) as a colourless solid.
1 H-NMR (400MHz, d6-DMSO): 3.70 (s, 3H), 3.94 (s, 3H), 5.46 (s, 2H), 6.87 (d, 2H, 8.4 Hz), 6.97 (br, s, 1 H), 7.16 (d, 2H, 8.4 Hz), 8.13 (br, s, 1 H), 12 (br, s, 1 H).
LC/MS (Method LC14): Rt = 1.18 min; m/z = 314.14 [M+H]⁺.

### (c) 1-(4-Methoxy-benzyl)-6-trifluoromethanesulfonyloxy-1H-pyrazolo[3,4-b]pyridine-4-carboxylic acid methyl ester

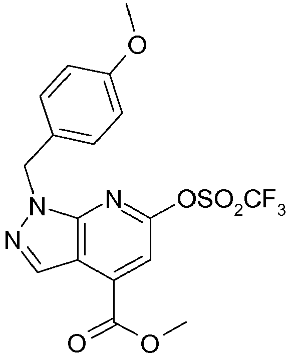

5 g of 6-Hydroxy-1-(4-methoxy-benzyl)-1H-pyrazolo[3,4-b]pyridine-4-carboxylic acid methyl ester were dissolved in dry dichloromethane and 3.4 ml of triethylamine were added. At 0°C 3 mL of trifluoromethanesulfonic anhydride were added slowly and the mixture was allowed to stir for ten minutes. Saturated sodium bicarbonate solution was added and the phases were quickly separated, the organic layer was extracted with 1 N hydrochloric acid, dried over sodium sulfate and evaporated. The reaction was performed 4 times to yield a total of 21.61 g (76%) of the desired product.
1 H-NMR (400MHz, d6-DMSO): 3.69 (s, 3H), 4.02 (s, 3H), 5.60 (s, 2H), 6.88 (d, 2H), 7.25 (d, 2H), 7.80 (s, 1 H), 8.55 (s, 1 H)

### (d) 6-(4-Hydroxy-phenyl)-1-(4-methoxy-benzyl)-1H-pyrazolo[3,4-b]pyridine-4-carboxylic acid

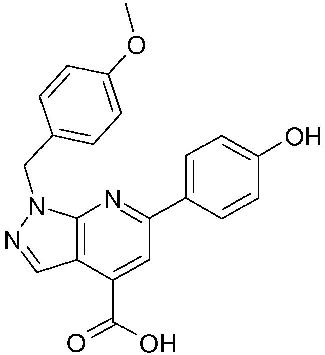

1-(4-Methoxy-benzyl)-6-trifluoromethanesulfonyloxy-1H-pyrazolo[3,4-b]pyridine-4-carboxylic acid methyl ester (10.0 g, 22.43 mmol), 4-hydroxyphenyl boronic acid (3.8 g, 13.4 mmol) and cesium carbonate (18.3 g, 56.2 mmol) were dissolved in 240 mL of a degased mixture of THF and water (v/v 1:1). Dichloro [1,1'-bis(diphenylphosphino)-ferrocene]palladium(II) (550 mg, 0.674 mmol) was added and the mixture was heated to 70° for 4 h. LC/MS indicated completion of the reaction. Then 22 mL of 5N KOH were added and the mixture was heated at 70°C until the ester was completely hydrolysed. The mixture was allowed to cool to room temperature, filtered and the solution was acidified under cooling by addition of 1 N hydrochloric acid until precipitation was observed. The formed precipitate was filtered off and dried in vacuo to give the crude product, which was recrystalised from acetonitrile and water to yield 7.2 g (81 %) of the title compound.
1 H-NMR (400MHz, d6-DMSO): 3.7 (s, 3H), 5.68 (s, 2H), 6.87 (d, 2H), 6.94 (d, 2H), 7.39 (d, 2H), 8.12 (m, 3H), 8.31 (s, 1 H), 9.91 (s, 1 H), 13.9 (bs, 1 H).
LC/MS (Method LC6): Rₜ = 1.61 min; m/z = 376.11 [M+H]⁺.

### (e) 6-(4-Hydroxy-phenyl)-1H-pyrazolo[3,4-b]pyridine-4-carboxylic acid

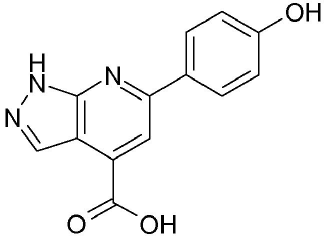

6-(4-Hydroxy-phenyl)-1-(4-methoxy-benzyl)-1H-pyrazolo[3,4-b]pyridine-4-carboxylic acid (7.2 g, 19.2 moml) was dissolved in 80 mL trifluoroacetic acid and after short stirring at r. t. the mixture was heated in a microwave reactor at 120°C for 20 min. The completion of the reaction was monitored via LC/MS and after cooling, water was added and a precipitate was formed. The solid was filtered off and then washed with 5N NaOH. Then the aqueous layer was treated with 5N NaOH until pH 4 and the product precipitated as solid. The solid was collected and recrystallised from acetonitrile and water.
1 H-NMR (500MHz, d6-DMSO): 6.91 (d, 2H), 8.05 (d, 2H), 8.10(s, 1H), 8.32 (s, 1 H), 9.91 (s, 1H), 13.9 (bs, 1 H).
LC/MS (Method LC6): Rₜ = 1.07min; m/z = 256.13 [M+H]⁺.

### (f) {(R)-1-[6-(4-Hydroxy-phenyl)-1H-pyrazolo[3,4-b]pyridine-4-carbonyl]-pyrrolidin-3-yl}-carbamic acid tert-butyl ester

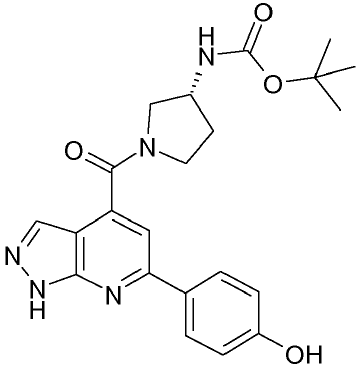

To a solution of (R)-pyrrolidin-3-yl-carbamic acid tert-butyl ester (31 mg, 0.165 mmol) in 1 mL of DMF, N-methyl morpholine was added (45 mg, 0.45 mmol) followed by 6-(4-hydroxy-phenyl)-1H-pyrazolo[3,4-b]pyridine-4-carboxylic acid (38.2 mg, 0.15 mmol) dissolved in 0.5 mL of DMF. Then a solution of TOTU (54 mg, 0.165 mmol) in 0.5 mL DMFwas added and the mixture stirred at r.t. over the weekend. The mixture was filtered and purified by prep. HPLC.
LC/MS (Method LC13): Rt = 1.43 min; m/z = 424.7 [M+H]⁺.

### (g) ((R)-3-Amino-pyrrolidin-1-yl)-[6-(4-hydroxy-phenyl)-1H-pyrazolo[3,4-b]pyridin-4-yl]-methanone

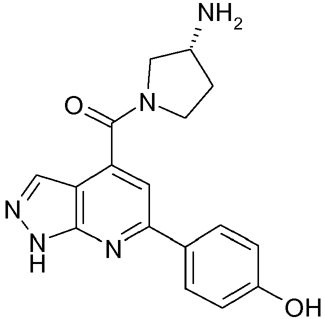

{(R)-1-[6-(4-Hydroxy-phenyl)-1H-pyrazolo[3,4-b]pyridine-4-carbonyl]-pyrrolidin-3-yl}-carbamic acid tert-butyl ester was dissolved in 1 mL of 4M HCl in dioxane and left stirring at r.t. overnight. Then 2 mL of water were added and the mixture was freezed dried to give the desired product ((R)-3-Amino-pyrrolidin-1-yl)-[6-(4-hydroxy-phenyl)-1H-pyrazolo[3,4-b]pyridin-4-yl]-methanone as the hydrochloride salt (28.5 mg, 0.07 mmol, 53%).
1 H-NMR (500MHz, d6-DMSO): 1,98-2,12 (m, 1 H), 2,14-2,34 (m, 1 H), 3,39-3,48 (m, 2H), 3,6-4,2 (bm, 3H), 6,92 (d, 2H), 7,70+7.71 (two singlets, 1 H), 8.0-8.14 (m, 3H), 8.20 (bs, 1 H), 8.50 (bs, 2H).
LC/MS (Method LC6): Rt = 1,82min; m/z = 324.22 [M+H]⁺.

The follwing examples have been prepared in a similar manner as example 16 (steps (f) to (g)) employing 6-(4-hydroxy-phenyl)-1H-pyrazolo[3,4-b]pyridine-4-carboxylic acid and the respective amine precursors:

| Exp. No. | Name | Structure | LC/MS method | m/z [M+H]⁺ | Rt [min] |
|---|---|---|---|---|---|
| 17 | ((S)-3-Amino-pyrrolidin-1-yl)-[6-(4-hydroxy-phenyl)-1H-pyrazolo[3,4-b]pyridin-4-yl]-methanone | 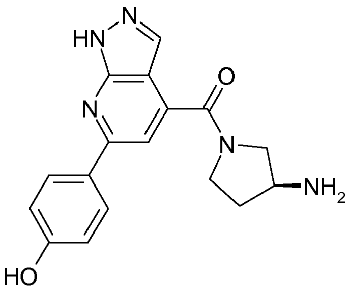 | LC10 | 324.22 | 1.82 |
| 18 | 6-(4-Hydroxyphenyl)-1H-pyrazolo[3,4-b]pyridine-4-carboxylic acid-(R)-(1-Azabicyclo[2.2.2]oct-3-yl)amide | 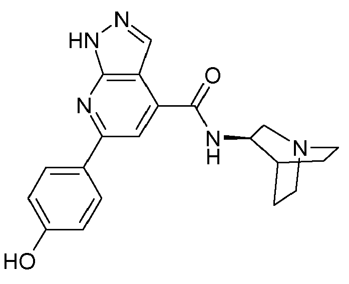 | LC10 | 364.26 | 2.04 |
| 19 | 6-(4-Hydroxy-phenyl)-1H-pyrazolo[3,4-b]pyridine-4-carboxylic acid (R)-piperidin-3-ylamide | 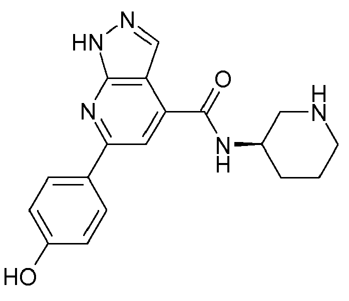 | LC10 | 338.24 | 2.02 |
| 20 | (2,7-Diazaspiro[3.5]non-2-yl)-[6-(4-hydroxyphenyl)-1H-pyrazolo[3,4-b]pyridin-4-yl]-methanone | 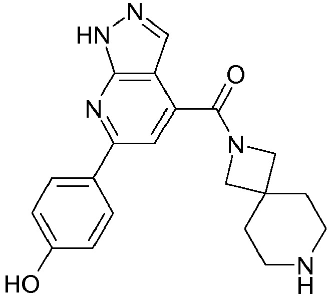 | LC10 | 405.43 | 1.93 |
| 21 | (2,7-Diazaspiro[3.5]non-7-yl)-[6-(4-hydroxyphenyl)-1H-pyrazolo[3,4-b]pyridin-4-yl]-methanone | 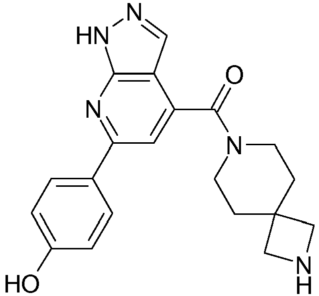 | LC10 | 364.38 | 1.89 |
| 22 | (2,8-Diazaspiro[4.5]dec-2-yl)-[6-(4-hydroxyphenyl)-1H-pyrazolo[3,4-b]pyridin-4-yl]-methanone | 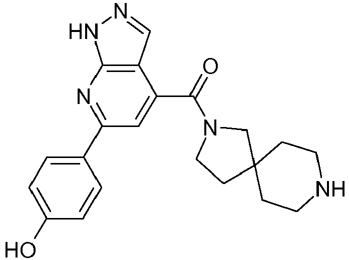 | LC10 | 378.41 | 1.97 |
| 23 | (2,7-Diazaspiro[4.5]dec-2-yl)-[6-(4-hydroxy-phenyl)-1H-pyrazolo[3,4-b]pyridin-4-yl]-methanone | 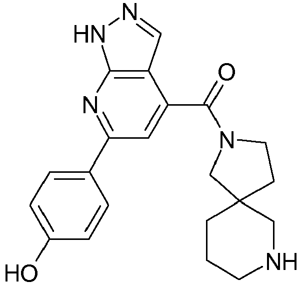 | LC10 | 378.41 | 1.97 |
| 24 | (2,7-Diazaspiro[4.5]dec-7-yl)-[6-(4-hydroxyphenyl)-1H-pyrazolo[3,4-b]pyridin-4-yl]-methanone | 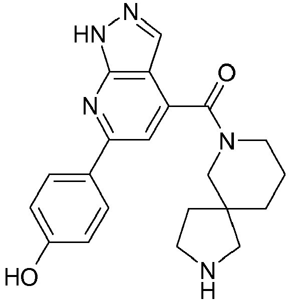 | LC10 | 378.41 | 2.04 |
| 25 | (2,7-Diazaspiro[4.4]non-2-yl)-[6-(4-hydroxyphenyl)-1H-pyrazolo[3,4-b]pyridin-4-yl]-methanone | 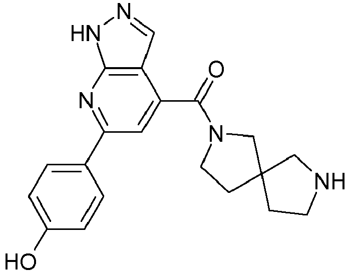 | LC10 | 405.43 | 1.93 |
| 26 | [6-(4-Hydroxy-phenyl)-1H-pyrazolo[3,4-b]pyridin-4-yl]-(1-oxa-4,9-diazaspiro[5.5]undec-9-yl)-methanone | 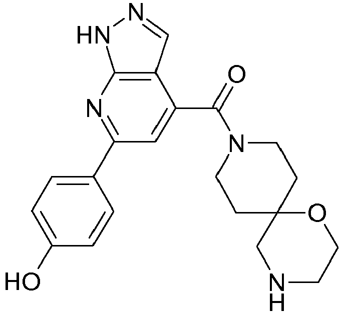 | LC10 | 394.41 | 2.04 |
| 27 | [6-(4-Hydroxy-phenyl)-1H-pyrazolo[3,4-b]pyridin-4-yl]-(1-oxa-4,8-diaza-spiro[5.5]undec-4-yl)-methanone | 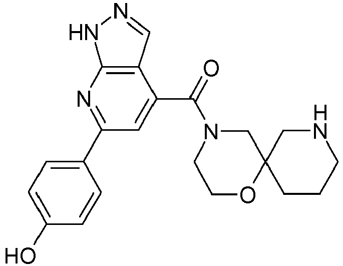 | LC10 | 394.42 | 1.99 |
| 28 | [6-(4-Hydroxyphenyl)-1H-pyrazolo[3,4-b]pyridin-4-yl]-(1-oxa-4,8-diazaspiro[5.5]undec-8-yl)-methanone | 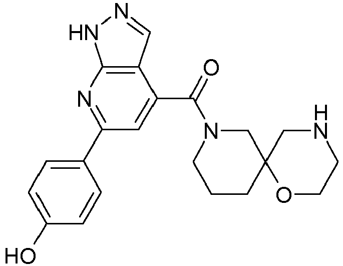 | LC10 | 394.41 | 2.02 |
| 29 | 6-(4-Hydroxyphenyl)-1H-pyrazolo[3,4-b]pyridine-4-carboxylic acid (trans-4-methoxypyrrolidin-3-yl)-amide | 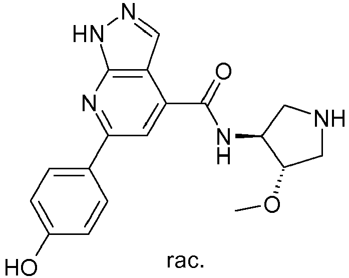 | LC10 | 354.16 | 2.04 |
| 30 | 6-(4-Hydroxyphenyl)-1H-pyrazolo[3,4-b]pyridine-4-carboxylic acid (trans-3-amino-cyclobutyl)-amide | 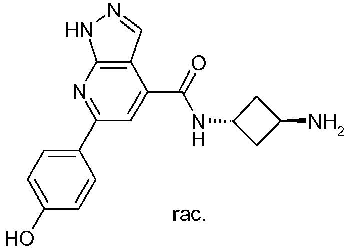 | LC10 | 324.22 | 1.95 |
| 31 | [6-(4-Hydroxy-phenyl)-1H-pyrazolo[3,4-b]pyridin-4-yl]-(6-oxa-2,9-diaza-spiro[4.5]dec-2-yl)-methanone | 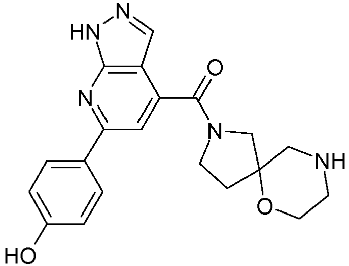 | LC10 | 380.25 | 1.93 |
| 32 | 6-(4-Hydroxyphenyl)-1H-pyrazolo[3,4-b]pyridine-4-carboxylic acid (5-aza-spiro[3.5]non-8-yl)-amide | 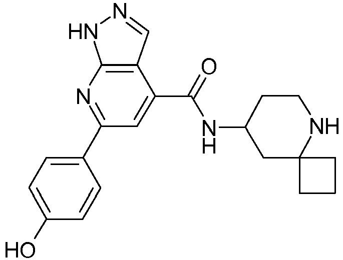 | LC10 | 378.26 | 2.15 |
| 33 | 6-(4-Hydroxyphenyl)-1H-pyrazolo[3,4-b]pyridine-4-carboxylic acid (3-amino-cyclohexyl)-amide | 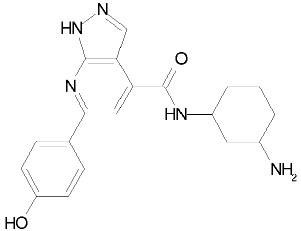 | LC10 | 352.25 | 2.09 |
| 34 | (Hexahydropyrrolo[3,4-b]pyrrol-1-yl)-[6-(4-hydroxy-phenyl)-1H-pyrazolo[3,4-b]pyridin-4-yl]-methanone | 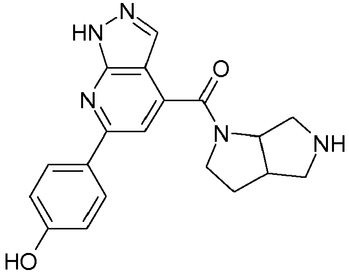 | LC10 | 350.23 | 1.93 |
| 35 | 6-(4-Hydroxy-phenyl)-1H-pyrazolo[3,4-b]pyridine-4-carboxylic acid azetidin-3-ylamide | 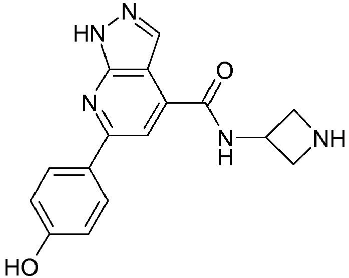 | LC10 | 310.31 | 1.93 |
| 36 | (3-Amino-azetidin-1-yl)-[6-(4-hydroxy-phenyl)-1H-pyrazolo[3,4-b]pyridin-4-yl]-methanone | 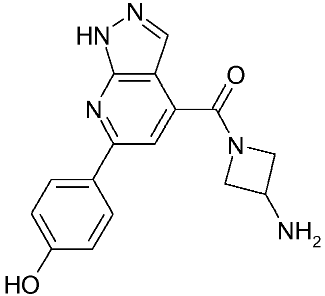 | LC10 | 310.20 | 1.84 |
| 37 | [6-(4-Hydroxy-phenyl)-1H-pyrazolo[3,4-b]pyridin-4-yl]-(3-methyl-piperazin-1-yl)-methanone | 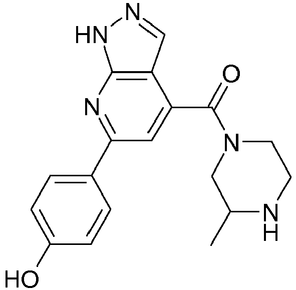 | LC11 | 338.18 | 1.95 |
| 38 | (4-Amino-piperidin-1-yl)-[6-(4-hydroxyphenyl)-1H-pyrazolo[3,4-b]pyridin-4-yl]-methanone | 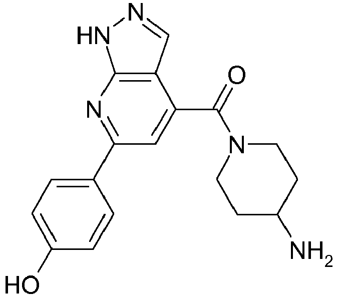 | LC11 | 338.18 | 1.70 |
| 39 | [6-(4-Hydroxyphenyl)-1H-pyrazolo[3,4-b]pyridin-4-yl]-(3-methylaminopyrrolidin-1-yl)-methanone | 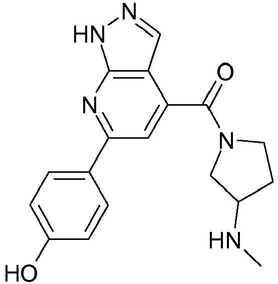 | LC11 | 338.17 | 1.7 |
| 40 | 6-(4-Hydroxyphenyl)-1H-pyrazolo[3,4-b]pyridine-4-carboxylic acid-(S)-(1-Azabicyclo[2.2.2]oct-3-yl)amide | 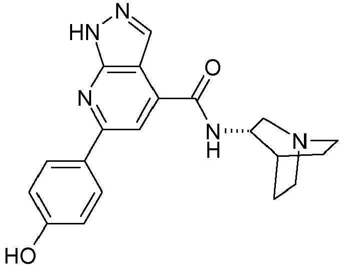 | LC10 | 364.26 | 2.02 |
| 41 | 6-(4-Hydroxyphenyl)-1H-pyrazolo[3,4-b]pyridine-4-carboxylic acid (1-methyl-piperidin-3-yl)-amide | 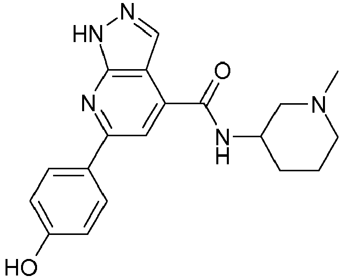 | LC10 | 352.25 | 2 |
| 42 | 6-(4-Hydroxyphenyl)-1H-pyrazolo[3,4-b]pyridine-4-carboxylic acid methyl-piperidin-4-yl-amide | 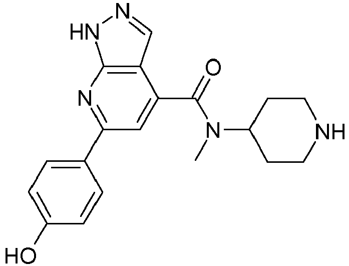 | LC10 | 352.25 | 1.87 |
| 43 | (2,8-Diazaspiro[4.5]dec-8-yl)-[6-(4-hydroxyphenyl)-1H-pyrazolo[3,4-b]pyridin-4-yl]-methanone | 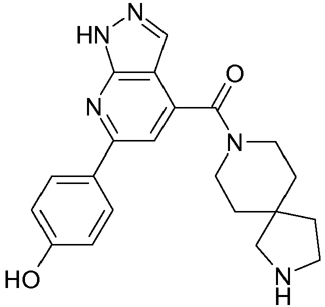 | LC10 | 419.45 | 1.93 |
| 44 | (3,9-Diazaspiro[5.5]undec-3-yl)-[6-(4-hydroxyphenyl)-1H-pyrazolo[3,4-b]pyridin-4-yl]-methanone | 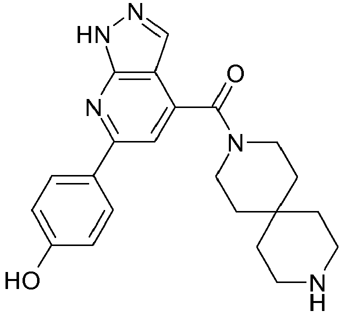 | LC10 | 392.43 | 1.95 |
| 45 | (3-Amino-pyrrolidin-1-yl)-[6-(4-hydroxy-phenyl)-1H-pyrazolo[3,4-b]pyridin-4-yl]-methanone | 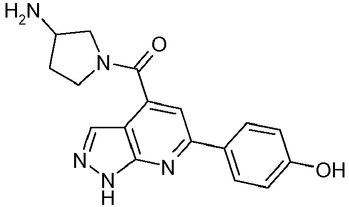 | LC11 | 324.08 | 1.82 |
| 46 | (Hexahydro-pyrrolo[3,4-c]pyrrol-2-yl)-[6-(4-hydroxy-phenyl)-1H-pyrazolo[3,4-b]pyridin-4-yl]-methanone | 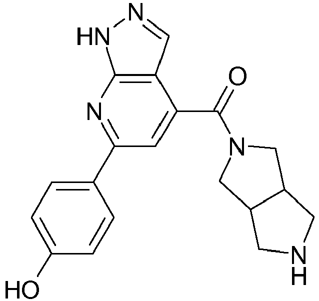 | LC12 | 391.25 | 1.97 |
| 47 | 2,5-Diazabicyclo[2.2.1]hept-2-yl-[6-(4-hydroxyphenyl)-1H-pyrazolo[3,4-b]pyridin-4-yl]-methanone | 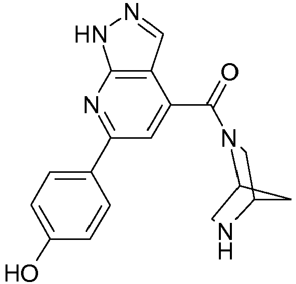 | LC11 | 336.17 | 1.7 |
| 48 | 6-(4-Hydroxyphenyl)-1H-pyrazolo[3,4-b]pyridine-4-carboxylic acid (2,3-dihydro-spiro[1H-indene-1,4'-piperidin]-3-yl)-amide | 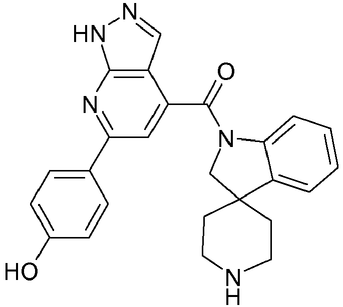 | LC10 | 467.48 | 2.39 |
| 49 | 6-(4-Hydroxyphenyl)-1H-pyrazolo[3,4-b]pyridine-4-carboxylic acid (1,2-dihydrospiro[3H-indole-3,4'-piperidin]-1'-yl)-amide | 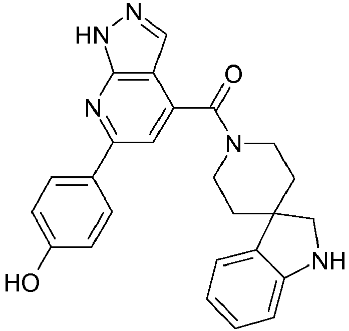 | LC10 | 426.43 | 2.22 |
| 50 | 6-(4-hydroxy-phenyl)-1H-pyrazolo[3,4-b]pyridine-4-carboxylic acid (2,3-dihydrospiro[1H-indene-1,4'-piperidin]-3-yl)-amide | 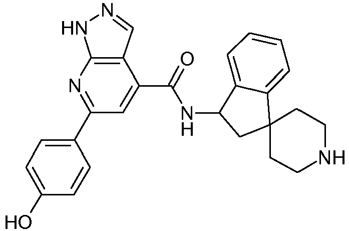 | LC10 | 440.46 | 2.40 |
| 51 | 6-(4-Hydroxy-phenyl)-1H-pyrazolo[3,4-b]pyridine-4-carboxylic acid (trans-2-amino-cyclopropyl)-amide | 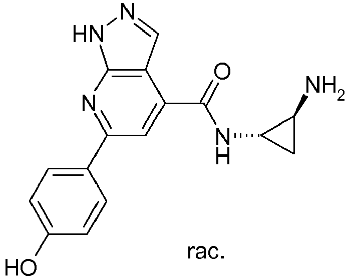 | LC10 | 310.20 | 1.93 |
| 52 | [6-(4-Hydroxyphenyl)-1H-pyrazolo[3,4-b]pyridin-4-yl]-(4-methyl-piperazin-1-yl)-methanone | 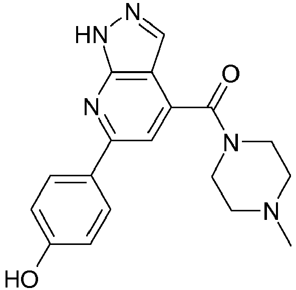 | LC11 | 338.08 | 1.89 |
| 53 | 1-{4-[6-(4-Hydroxy-phenyl)-1H-pyrazolo[3,4-b]pyridine-4-carbonyl]-piperazin-1-yl}-ethanone | 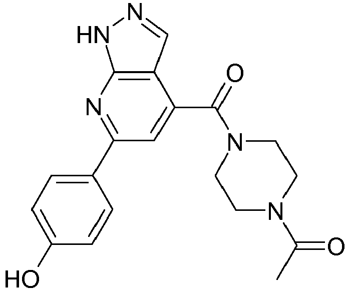 | LC11 | 366.07 | 2 |
| 54 | [6-(4-Hydroxyphenyl)-1H-pyrazolo[3,4-b]pyridin-4-yl]-((S)-2-pyrrolidin-1-ylmethyl-pyrrolidin-1-yl)-methanone | 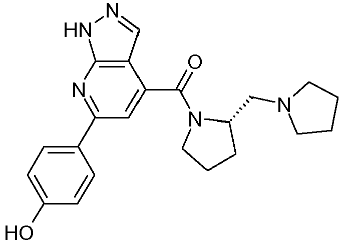 | LC12 | 392.3 | 2.39 |
| 55 | ((S)-3-Dimethylaminopyrrolidin-1-yl)-[6-(4-hydroxy-phenyl)-1H-pyrazolo[3,4-b]pyridin-4-yl]-methanone | 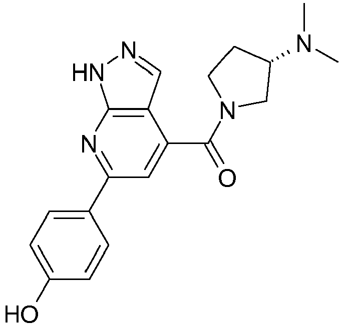 | LC11 | 352.14 | 1.87 |
| 56 | [6-(4-Hydroxyphenyl)-1H-pyrazolo[3,4-b]pyridin-4-yl]-(4-isopropylpiperazin-1-yl)-methanone | 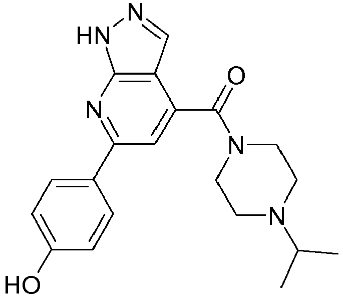 | LC10 | 366.40 | 1.99 |
| 57 | (4-Cyclopropane-carbonyl-piperazin-1-yl)-[6-(4-hydroxyphenyl)-1H-pyrazolo[3,4-b]pyridin-4-yl]-methanone | 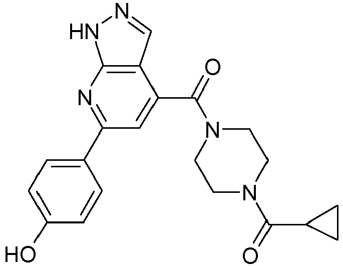 | LC10 | 392.25 | 2.35 |
| 58 | (4-Cycloheptyl-piperazin-1-yl)-[6-(4-hydroxy-phenyl)-1H-pyrazolo[3,4-b]pyridin-4-yl]-methanone | 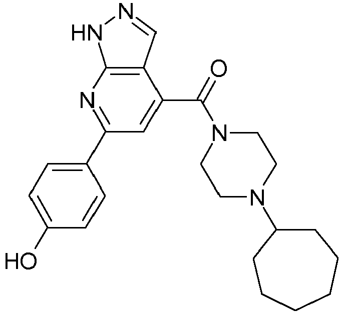 | LC10 | 420.31 | 2.34 |
| 59 | 1-{4-[6-(4-Hydroxy-phenyl)-1H-pyrazolo[3,4-b]pyridine-4-carbonyl]-[1,4]diazepan-1-yl}-ethanone | 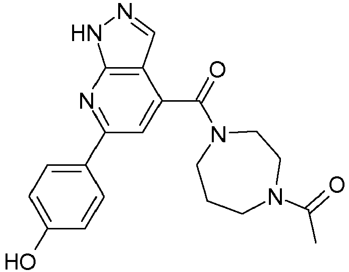 | LC10 | 380.25 | 2.18 |
| 60 | 6-(4-Hydroxy-phenyl)-1H-pyrazolo[3,4-b]pyridine-4-carboxylic acid (1-ethyl-piperidin-3-yl)-amide | 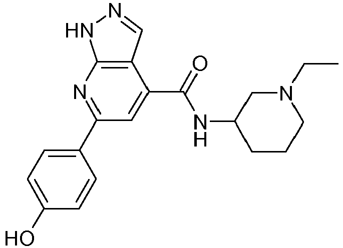 | LC10 | 366.27 | 2.05 |
| 61 | [6-(4-Hydroxyphenyl)-1H-pyrazolo[3,4-b]pyridin-4-yl]-[4-(tetrahydro-furan-2-carbonyl)-piperazin-1-yl]-methanone | 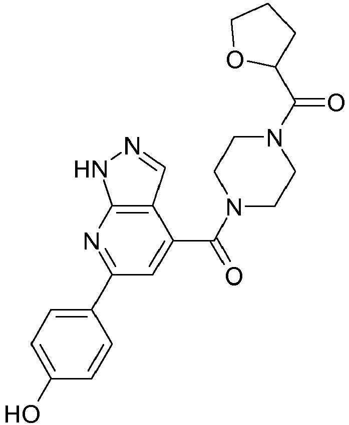 | LC11 | 422.18 | 2.05 |
| 62 | 6-(4-Hydroxyphenyl)-1H-pyrazolo[3,4-b]pyridine-4-carboxylic acid (1-methyl-azetid in-3-yl)-amide | 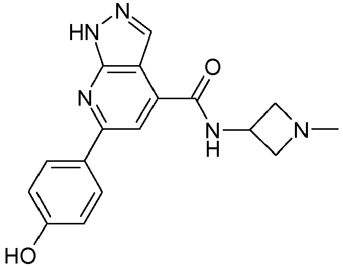 | LC11 | 324.16 | 1.79 |
| 63 | 6-(4-Hydroxy-phenyl)-1H-pyrazolo[3,4-b]pyridine-4-carboxylic acid (5-oxo-pyrrolidin-2-ylmethyl)-amide | 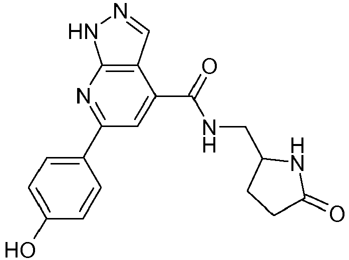 | LC11 | 352.15 | 2.01 |
| 64 | (4-Ethyl-[1,4]diazepan-1-yl)-[6-(4-hydroxyphenyl)-1H-pyrazolo[3,4-b]pyridin-4-yl]-methanone | 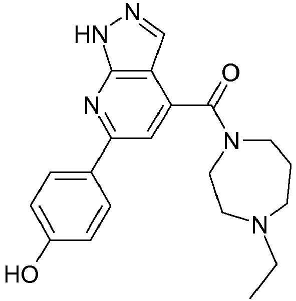 | LC11 | 366.19 | 1.82 |
| 65 | [6-(4-Hydroxyphenyl)-1H-pyrazolo[3,4-b]pyridin-4-yl]-[3-(4-methyl-piperidin-1-yl)-pyrrolidin-1-yl]-methanone | 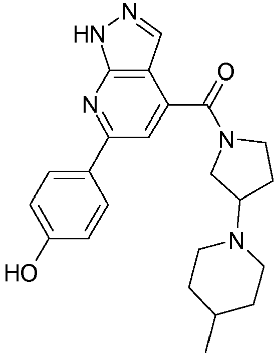 | LC11 | 406.21 | 1.99 |
| 66 | 6-(4-Hydroxy-phenyl)-1H-pyrazolo[3,4-b]pyridine-4-carboxylic acid (1-methyl-piperidin-4-yl)-amide | 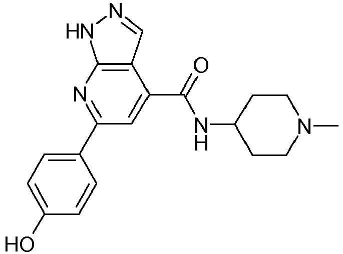 | LC11 | 352.18 | 1.81 |
| 67 | 6-(4-Hydroxy-phenyl)-1H-pyrazolo[3,4-b]pyridine-4-carboxylic acid (1-cyclohexyl-piperidin-4-yl)-amide | 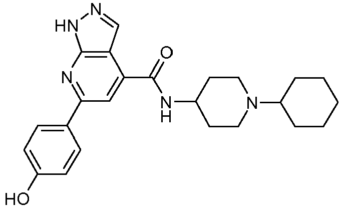 | LC10 | 420.31 | 2.25 |
| 68 | 6-(4-Hydroxy-phenyl)-1H-pyrazolo[3,4-b]pyridine-4-carboxylic acid (1-acetyl-piperidin-4-yl)-amide | 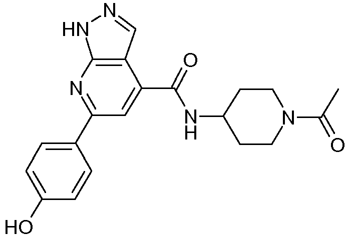 | LC10 | 380.25 | 2.27 |
| 69 | 6-(4-Hydroxy-phenyl)-1H-pyrazolo[3,4-b]pyridine-4-carboxylic acid ((R)-6-oxo-piperidin-3-yl)-amide | 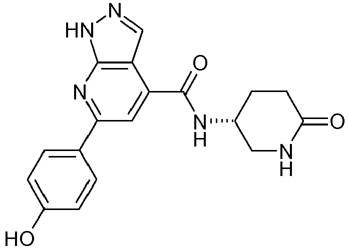 | LC11 | 352.17 | 1.94 |
| 70 | (5-Ethyl-2,5-diaza-bicyclo[2.2.1]hept-2-yl)-[6-(4-hydroxyphenyl)-1H-pyrazolo[3,4-b]pyridin-4-yl]-methanone | 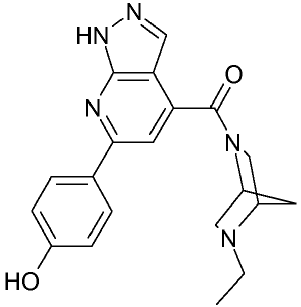 | LC11 | 364.19 | 1.76 |
| 71 | [6-(4-Hydroxyphenyl)-1H-pyrazolo[3,4-b]pyridin-4-yl]-[4-(2-methoxy-ethyl)-piperazin-1-yl]-methanone | 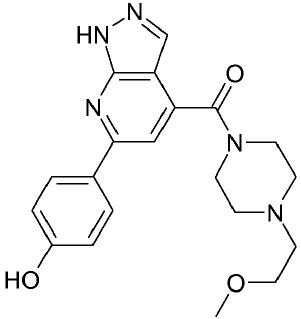 | LC10 | 382.27 | 1.97 |
| 72 | 6-(4-Hydroxy-phenyl)-1H-pyrazolo[3,4-b]pyridine-4-carboxylic acid (1-aza-bicyclo[2.2.2]oct-3-yl)-amide | 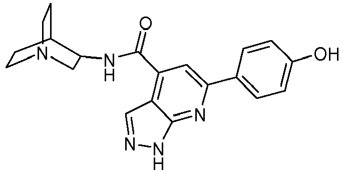 | LC10 | 364.39 | 2.05 |
| 73 | 6-(4-Hydroxy-phenyl)-1H-pyrazolo[3,4-b]pyridine-4-carboxylic acid methyl-(1-methyl-piperidin-4-yl)-amide | 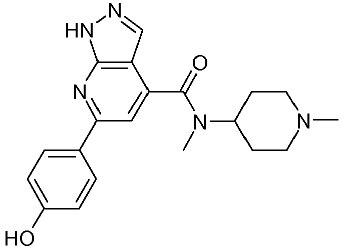 | LC10 | 366.40 | 1.9 |
| 74 | [6-(4-Hydroxy-phenyl)-1H-pyrazolo[3,4-b]pyridin-4-yl]-[4-(pyridine-3-carbonyl)-piperazin-1-yl]-methanone | 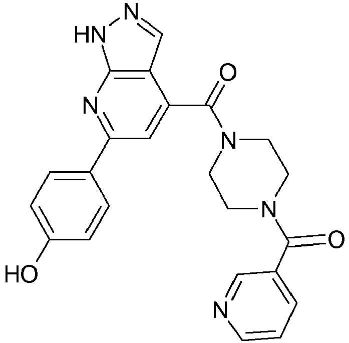 | LC10 | 429.26 | 2.05 |
| 75 | 6-(4-Hydroxy-phenyl)-1H-pyrazolo[3,4-b]pyridine-4-carboxylic acid (1-pyridin-4-ylmethyl-piperidin-4-yl)-amide | 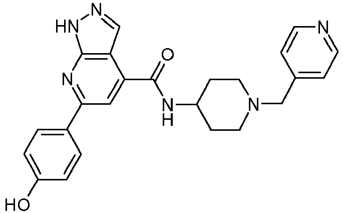 | LC11 | 429.23 | 1.87 |
| 76 | 4-[6-(4-Hydroxy-phenyl)-1H-pyrazolo[3,4-b]pyridine-4-carbonyl]-1-methyl-piperazin-2-one | 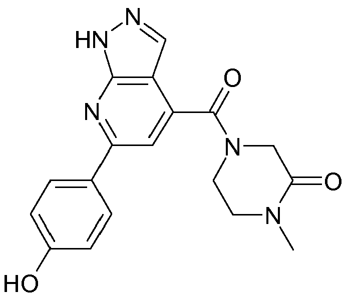 | LC11 | 352.14 | 1.89 |
| 77 | 6-(4-Hydroxyphenyl)-1H-pyrazolo[3,4-carboxylic acid ((R)-1-benzyl-piperidin-3-yl)-amide | 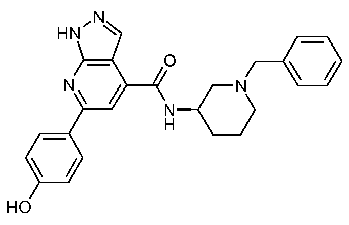 | LC11 | 428.19 | 2.24 |
| 78 | 6-(4-Hydroxyphenyl)-1H-pyrazolo[3,4-b]pyridine-4-carboxylic acid (1-pyridin-3-ylmethyl-piperidin-4-yl)-amide | 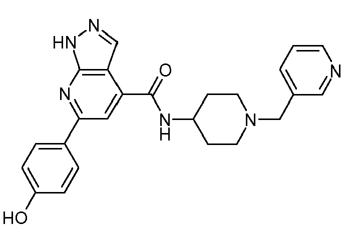 | LC11 | 429.23 | 2.07 |

### Synthesis of intermediates

### 2-Isopropyl-5,5-dimethyl-piperazine-1-carboxylic acid tert-butyl ester

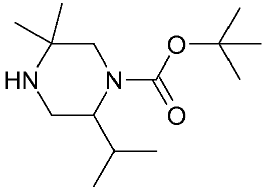

### (a) 6-Isopropyl-3,3-dimethyl-piperazin-2-one

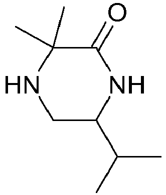

To a solution of 8.54 g of 3-methyl-butane-1,2-diamine-dihydrochloride in 70 ml of methanol were added 18 ml of sodium methanolate (5.4 M in methanol). After stirring for 0.5 h at r.t., the precipitated sodium chloride was removed by filtration and the solution concentrated to a third of its volume. Again, sodium chloride was removed by filtration and the mother liquor was concentrated in vacuo. The residue was dissolved in 12 ml of water and 3.7 ml of acetone cyanohydrin were added dropwise. The reaction was heated to 90°C for 5 h, then to reflux for additional 12 h. The volatiles were removed in vacuo, and the residue was co-distilled with toluene twice. 6.50 g (78%) of the title compound were obtained, which could be used in the next step without further purification.
LC/MS (Method LC4): Rt = 0.06 min; m/z = 171.20 [M+H]⁺.

### (b) 4-Benzyl-6-isopropyl-3,3-dimethyl-piperazin-2-one

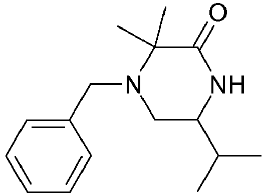

To a solution of 6.50 g of 6-isopropyl-3,3-dimethyl-piperazin-2-one in 50 ml of DMF were added 5.0 ml of benzyl bromide and 14.3 ml of Hünig's base. The reaction was stirred at r.t. overnight. The reaction was then concentrated in vacuo, and the resulting residue was taken up in ethyl acetate and water. The phases were separated, and the aqueous phase was extracted with ethyl acetate twice. The combined organic layers were dried over Na₂SO₄, filtered and concentrated to obtain 7.94 g (80 %) of the title compound.
LC/MS (Method LC4): Rt = 0.45 min; m/z = 261.20 [M+H]⁺.

### (c) 1-Benzyl-5-isopropyl-2,2-dimethyl-piperazine

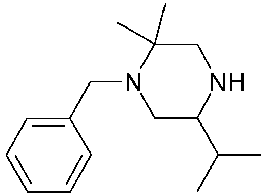

Under Argon atmosphere to a solution of 7.93 g of 4-benzyl-6-isopropyl-3,3-dimethyl-piperazin-2-one in 125 ml THF 25 ml of a 2.4 M solution of lithium aluminium hydride in THF were slowly added. The reaction was heated to 55°C, and 3.8 ml of chlorotrimethylsilane were added dropwise. The reaction was kept at 55-60°C for 1 h and then cooled to 0°C. 12 ml of water were added dropwise, and THF was added. The mixture was filtered by suction over celite and the filtrate was concentrated in vacuo to a volume of 10 ml. This was extracted with methyl tert-butyl ether twice. The combined organic layers were dried using sodium sulfate, filtered and concentrated to obtain 7.00 g (93%) of the title compound.
LC/MS (Method LC4): Rt = 0.29 min; m/z = 247.20 [M+H]⁺.

### (d) 4-Benzyl-2-isopropyl-5,5-dimethyl-piperazine-1-carboxylic acid tert-butyl ester

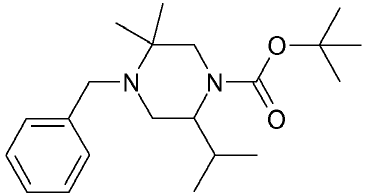

4.0 g of 1-benzyl-5-isopropyl-2,2-dimethyl-piperazine were dissolved in 60 ml of DCM and 3.54 g of di-tert-butyl dicarbonate were added. After stirring at r.t. for 16 h the reaction was concentrated in vacuo to yield 6.2 g (100%) of the title compound, which was used without further purification.
LC/MS (Method LC4): Rt = 0.75min; m/z = 347.20 [M+H]⁺.

### (e) 2-Isopropyl-5,5-dimethyl-piperazine-1-carboxylic acid tert-butyl ester

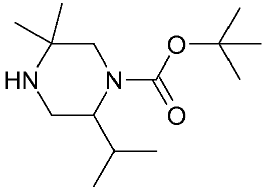

To 6.20 g of 4-benzyl-2-isopropyl-5,5-dimethyl-piperazine-1-carboxylic acid tert-butyl ester in 100 ml of methanol was added 6.20 g palladium on charcoal (10%) and the mixture was hydrogenated at r.t. and ambient pressure for 16 h, and then at 40°C for additional 4 h. The catalyst was removed by filtration over celite and the filtrate was concentrated in vacuo to yield 3.49 g (76%) of the title compound, which was used without further purification.
LC/MS (Method LC4): Rt = 0.82min; m/z = 257.10 [M+H]⁺.

### Determination of PKC βII inhibition

PKC βII inhibition was determined according to the following protocol:
Active human full-length recombinant PKC βII and the peptide substrate, Fluorescein-RFARKGSLRQKNV, were purchased from Invitrogen GmbH, Darmstadt, Germany. Adenosine-5'-triphosphate (ATP), bovine serum albumine (BSA), dimethylsulphoxide (DMSO), 4-(2-hydroxyethyl)piperazine-1-ethanesulfonic acid (Hepes), Triton X-100, 1,2-Dioleoyl-sn-glycerol (DAG), L-α-Phosphatidyl-L-serine (PS), calcium chloride (CaCl₂), and Pluronic F-68 were purchased from Sigma-Aldrich, Munich, Germany. Magnesium chloride, 1 M sodium hydroxide solution, 1 M hydrochloric acid solution and EDTA were obtained from Merck Biosciences, Darmstadt, Germany.

Test compounds were diluted to three times the test concentration in buffer 1 (30 mM Hepes-NaOH, pH 7.4, 0.01 % Pluronic F-68 and 3 % (v/v) DMSO). The PKC βII enzyme was diluted to a concentration of 30 ng/ml in buffer 2 (30 mM Hepes-NaOH, pH 7.4, 15 mM MgCl₂, 150 µM CaCl₂, 150 µg/ml PS, 60 µg/ml DAG, and 0.045 % (w/v) Triton X-100). The peptide substrate and ATP were diluted to concentrations of 3 µM and 120 µM, respectively, in buffer 2. Two µl of the compound solution were mixed with 2 µl of the diluted enzyme in a 384-well small volume microtiter plate (Greiner, Bio-One, Frickenhausen, Germany), and the kinase reaction was initiated by addition of 2 µl of the solution containing peptide substrate and ATP. After 60 min incubation at 32 °C, the reaction was stopped by addition of 20 µl of a solution containing 130 mM Hepes-NaOH, pH 7.4, 0.0195 % (v/v) Brij-35, 6.5 mM EDTA, 0.13 % chip coating reagent 3 (Caliper Lifescience Inc, Hopkinton, MA) and 6.5 % (v/v) DMSO. Phosphorylation of the substrate peptide was then detected on a Caliper 3000 instrument essentially as described by Pommereau et al (J. Biomol. Screening 2004, 9(5), 409-416). Separation conditions were as follows: Pressure -0.8 psi, upstream voltage -3000 V, downstream voltage -800 V. Positive controls (buffer 1 instead of compound) and negative controls (buffer 1 instead of compound and buffer 2 instead of kinase solution) were run in parallel on each plate. Fractional turnover (R) was determined as the height of the peak of the phosphorylated peptide product divided by the sum of the unphosphorylated substrate and phosphorylated product peak heights. Percent-inhibition values for the test compounds were determined using the formula $% Inhibition = 100 * ( 1 - \left({R}_{compound}-{R}_{negative control}\right) / \left({R}_{positive control}-{R}_{negative control}\right) .$

In the following table % inhibition values observed with example compounds at a final concentration of 1.14 µM (±0.15 µM) are listed:

| Exp. No. | % Inhibition |
|---|---|
| 1 | 18 |
| 2 | 27 |
| 3 | 95 |
| 4 | 88 |
| 5 | 85 |
| 6 | 87 |
| 7 | 84 |
| 8 | 46 |
| 9 | 82 |
| 10 | 96 |
| 11 | 87 |
| 12 | 46 |
| 13 | 39 |
| 14 | 69 |
| 15 | 94 |
| 16 | 66 |
| 17 | 64 |
| 18 | 72 |
| 19 | 83 |
| 20 | 77 |
| 21 | 59 |
| 22 | 42 |
| 23 | 52 |
| 24 | 29 |
| 25 | 58 |
| 26 | 36 |
| 27 | 42 |
| 28 | 15 |
| 29 | 87 |
| 30 | 68 |
| 31 | 53 |
| 32 | 69 |
| 33 | 87 |
| 34 | 45 |
| 35 | 82 |
| 36 | 54 |
| 37 | 91 |
| 38 | 60 |
| 39 | 62 |
| 40 | 71 |
| 41 | 73 |
| 42 | 39 |
| 43 | 52 |
| 44 | 44 |
| 45 | 50 |
| 46 | 39 |
| 47 | 81 |
| 48 | 39 |
| 49 | 28 |
| 50 | 97 |
| 51 | 55 |
| 52 | 38 |
| 53 | 46 |
| 54 | 20 |
| 55 | 47 |
| 56 | 23 |
| 57 | 28 |
| 58 | 40 |
| 59 | 48 |
| 60 | 54 |
| 61 | 22 |
| 62 | 73 |
| 63 | 48 |
| 64 | 45 |
| 65 | 34 |
| 66 | 59 |
| 67 | 64 |
| 68 | 40 |
| 69 | 59 |
| 70 | 46 |
| 71 | 27 |
| 72 | 78 |
| 73 | 31 |
| 74 | 35 |
| 75 | 45 |
| 76 | 30 |
| 77 | 57 |
| 78 | 84 |

## Claims

1. A compound of the formula I 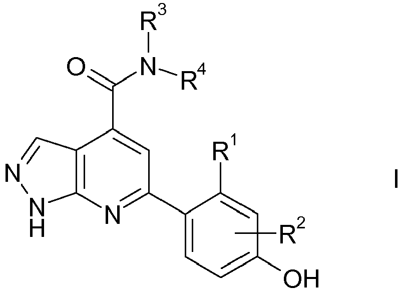 wherein
R¹ is H, halogen or (C₁-C₄)-alkyl;
R² is H, halogen or (C₁-C₄)-alkyl;
R³ is H or (C₁-C₄)-alkyl;
R⁴ is
a) (C₀-C₆)-alkyl which is mono-substituted by
i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
ia) F,
ib) (C₁-C₄)-alkyl, which is unsubstituted or one to fivefold substituted by F,
ic) O-(C₁-C₄)-alkyl, which is unsubstituted or one to fivefold substituted by F,
id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
ie) (C₁-C₄)-alkylene-phenyl, which is unsubstituted or one to fivefold substituted by F,
if) (C₃-C₈)- cycloalkyl,
ig) oxo (=O), and
ih) (CO)-(C₁-C₄)-alkyl,
and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
iia) F,
iib) (C₁-C₄)-alkyl, wherein (C₁-C₄)-alkyl is unsubstituted or one to fivefold substituted by F,
iic) O-(C₁-C₄)-alkyl, which is unsubstituted or one to fivefold substituted by F,
iid) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F, and
iie) (C₁-C₄)-alkylene-phenyl, which is unsubstituted or one to fivefold substituted by F;
or
iii) NH₂, NH(C₁-C₆)-alkyl, or N((C₁-C₆)-alkyl)₂,
and wherein (C₁-C₆)-alkyl can be further mono-substituted by phenyl, phenylene-(C₁-C₄)-alkyl or phenylene-O-(C₁-C₄)-alkyl;
b) a bicyclic (C₆-C₁₁)-cycloalkyl group, which is mono-substituted by (C₀-C₂)-alkylene-NH₂, (C₀-C₂)-alkylene-NH-(C₁-C₄)-alkyl, (C₀-C₂)-alkylene-N((C₁-C₄)-alkyl)₂;
c) a fused bicyclic (C₆-C₁₀)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F;
d) a spiro bicylic (C₇-C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
e) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
f) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 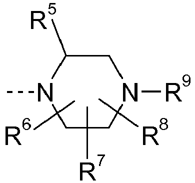 wherein
R⁵ is H or (C₀-C₆)-alkyl, which is unsubstituted or one to fivefold substituted by F, or mono-substituted by a substituent selected from the group consisting of
i) O-(C₁-C₄)-alkyl, which is unsubstituted or one to fivefold substituted by F,
ii) (C₃-C₆)-cycloalkyl,
iii) phenyl, which is unsubstituted or substituted by one to two identical or different substituents selected from the group consisting o fhalogen, CF₃, O-(C₁-C₄)-alkyl, OCF₃, and (C₁-C₄)-alkyl,
iv) a 5- to 6-membered monocyclic heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl,
v) benzo[1,3]dioxole, and
vi) CO-O-(C₁-C₄)-alkyl or CO- NH-(C₁-C₆)-alkyl;
R⁶ is H or (C₀-C₆)-alkyl, which is unsubstituted or one to fivefold substituted by F or mono-substituted by a substituent selected from the group consisting of
i) CO-O-(C₁-C₄)-alkyl;
ii) (C₃-C₆)-cycloalkyl,
iii) phenyl, which is unsubstituted or substituted by one to two identical or different substituents selected from the group consisting of halogen, CF₃, O-(C₁-C₄)-alkyl, OCF₃, and (C₁-C₄)-alkyl,
iv) O-(C₁-C₄)-alkyl, which is unsubstituted or one to fivefold substituted by F, and
v) a 5- to 6-membered monocyclic heteroaromatic ring comprising one heteroatom selected from the group consisting of nitrogen, oxygen, and sulphur, which is unsubstituted or substituted by one to two identical or different substituents selected from the group consisting of halogen, CF₃, O-(C₁-C₄)-alkyl, OCF₃, and (C₁-C₄)-alkyl;
R⁷ is H, (C₁-C₆)-alkyl, which is unsubstituted or one to fivefold substituted by F, or phenyl;
R⁸ is H, (C₁-C₆)-alkyl or oxo (=O);
R⁹ is H, (C₁-C₆)-alkyl, (C₃-C₆)-cycloalkyl or CO-R¹⁰, where (C₁-C₆)-alkyl is unsubstituted or one to fivefold substituted by F, or mono-substituted by a substituent selected from the group consisting of O-(C₁-C₄)-alkyl, SO₂-(C₁-C₄)-alkyl, phenyl, a 5- to 6-membered monocyclic heterocyclic ring comprising one heteroatom selected from the group consisting of nitrogen and oxygen, a 5- to 6-membered monocyclic heteroaromatic ring comprising one heteroatom selected from the group consisting of nitrogen and oxygen; wherein
R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen, wherein phenyl can be further mono-substituted by (C₁-C₄)-alkyl or O-(C₁-C₄)-alkyl;
b) a four to seven membered monocyclic heterocycloalkyl group containing a nitrogen atom, which is attached via said nitrogen and which is mono-substituted by (C₀-C₆)-alkylene-NH₂, (C₀-C₆)-alkylene-NH-(C₁-C₄)-alkyl, (C₀-C₆)-alkylene-NH-phenyl, (C₀-C₆)-alkylene-N((C₁-C₄)-alkyl)₂, (C₀-C₆)-alkylene-N((C₁-C₄)-alkyl)(phenyl), (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, which is unsubstituted or one to fivefold substituted by F, CO-O-(C₁-C₄)-alkyl or phenyl, and wherein said azetidinyl, pyrroldinyl and piperidyl group can be further mono-substituted by (C₁-C₆)-alkyl, which is unsubstituted or one to fivefold substituted by F;
c) a 1,4-diazepanyl, which is unsubstituted or mono-substituted by a substituent selected from the group consisting of
i) (C₁-C₆)-alkyl, wherein (C₁-C₆)-alkyl is unsubstituted or one to fivefold substituted by F,
ii) CO-(C₁-C₆)-alkyl, wherein (C₁-C₆)-alkyl is unsubstituted or one to fivefold substituted by F,
ii) CO-phenyl, and
iv) CO-pyridyl;
d) a fused bicyclic (C₆-C₁₀)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further heteroatom selected from the group consisting of nitrogen, oxygen and sulphur, wherein said heterocycloalkyl group is unsubstituted or mono- or di-substituted by F or (C₁-C₄)-alkyl, which is unsubstituted or one to fivefold substituted by F;
f) a bridged bicyclic (C₇-C₉)-heterocycloalkyl group containing one nitrogen atom, which is attached via said nitrogen atom, which is mono-substituted by (C₀-C₂)-alkylene-NH₂, (C₀-C₂)-alkylene-NH-(C₁-C₄)-alkyl, (C₀-C₂)-alkylene-N((C₁-C₄)-alkyl)₂
g) a bridged bicyclic (C₇-C₉)-heterocycloalkyl group containing two nitrogen atoms, which is attached via a nitrogen atom; and which is unsubstitued or substituted by one to four identical or different substituents selected from the group consisting of F, OH, and (C₁-C₄)-alkyl, which is unsubstituted or one to fivefold substituted by F; or
h) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
in any of its stereoisomeric forms, or a mixture of stereoisomeric forms in any ratio, or a physiologically acceptable salt thereof, or a physiologically acceptable solvate of any of them.

2. A compound of the formula I as claimed in claim 1,
wherein
R¹ is H, halogen or (C₁-C₄)-alkyl;
R² is H or halogen;
R³ is H or (C₁-C₄)-alkyl;
R⁴ is
a) (C₀-C₄)-alkyl which is mono-substituted by
i) a 3- to 8-membered monocyclic heterocycle comprising a ring nitrogen atom and optionally one further ring heteroatom selected from the group consisting of nitrogen and oxygen, which is unsubstituted or substituted by one to five identical or different substituents selected from the group consisting of
ia) F,
ib) (C₁-C₄)-alkyl, which is unsubstituted or one to fivefold substituted by F,
ic) O-(C₁-C₄)-alkyl, which is unsubstituted or one to fivefold substituted by F,
id) phenyl, which is unsubstituted or substituted by one to two identical or different substituents selected from the group consisting of halogen and (C₁-C₄)-alkyl, wherein (C₁-C₄)-alkyl is unsubstituted or one to fivefold substituted by F,
ie) (C₁-C₄)-alkylene-phenyl, which is unsubstituted or one to fivefold substituted by F,
if) (C₃-C₈)- cycloalkyl,
ig) oxo (=O), and
ih) (CO)-(C₁-C₄)-alkyl,
and wherein (C₀-C₆)-alkyl can be further mono-substituted by phenyl or pyridyl, wherein phenyl or pyridyl is unsubstituted or substituted by one to two identical or different substituents selected from the group consisting of halogen and (C₁-C₄)-alkyl, wherein (C₁-C₄)-alkyl is unsubstituted or one to fivefold substituted by F;
ii) (C₃-C₈)-cycloalkyl which is substituted by one to two identical or different substituents selected from the group consisting of NH₂, NH((C₁-C₄)-alkyl) and N((C₁-C₄)-alkyl)₂, and wherein (C₃-C₈)-cycloalkyl can be further substituted by one to three identical or different substituents selected from the group consisting of
iia) F,
iib) (C₁-C₄)-alkyl, wherein (C₁-C₄)-alkyl is unsubstituted or one to fivefold substituted by F;
b) a spiro bicylic (C₇-C₁₁)-heterocycloalkyl group containing one nitrogen atom, which is attached via a carbon atom and which is unsubstituted or substituted by one or two identical or different substituents selected from the group consisting of Fand (C₁-C₄)-alkyl, which is unsubstituted or one to fivefold substituted by F;
c) a brigded bicyclic (C₇-C₉)-heterocycloalkyl group containing one nitrogen atom, which is attached via a carbon atom and which is unsubstituted or substituted by one or two identical or different substituents selected from the group consisting of F and (C₁-C₄)-alkyl, which is unsubstituted or one to fivefold substituted by F; or
d) a tricyclic (C₁₁-C₁₅)-heterocycloalkyl group containing one nitrogen atom, which is attached via a carbon atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F;
or
R³ and R⁴ together with the N-atom carrying them denote
a) a 1,4-piperazinyl of the formula 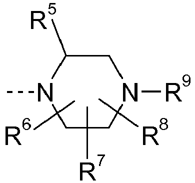 wherein
R⁵ is H or (C₁-C₆)-alkyl, which is unsubstituted or one to fivefold substituted by F, or mono-substituted by a substituent selected from the group consisting of
i) O-(C₁-C₄)-alkyl,
ii) (C₃-C₆)-cycloalkyl,
iii) phenyl,
iv) a 5- to 6-membered monocyclic heteroaromatic ring comprising one heteroatom selected from the group consisting of nitrogen, oxygen, and sulphur;
R⁶ is H or (C₁-C₆)-alkyl, which is unsubstituted or one to fivefold substituted by F;
R⁷ is H, (C₁-C₆)-alkyl;
R⁸ is H, (C₁-C₆)-alkyl or oxo (=O);
R⁹ is H, (C₁-C₆)-alkyl, (C₃-C₆)-cycloalkyl or CO-R¹⁰, where (C₁-C₆)-alkyl is unsubstituted or one to fivefold substituted by F, or mono-substituted O-(C₁-C₄)-alkyl ; wherein
R¹⁰ is (C₁-C₄)-alkyl, (C₃-C₆)-cycloalkyl, NH₂, phenyl, a 5- to 6-membered monocyclic heterocyclic ring comprising one heteroatom selected from the group consisting of nitrogen and oxygen, or a 5- to 6-membered monocyclic heteroaromatic ring comprising one heteroatom selected from the group consisting of nitrogen and oxygen;
b) a four to seven membered monocyclic heterocycloalkyl group containing one nitrogen atom, which is attached via said nitrogen and which is mono-substituted by (C₀-C₆)-alkylene-NH₂, (C₀-C₆)-alkylene-NH-(C₁-C₄)-alkyl, (C₀-C₆)-alkylene-N((C₁-C₄)-alkyl)₂, (C₀-C₂)-alkylene-azetidinyl, (C₀-C₂)-alkylene-pyrrolidinyl, or (C₀-C₂)-alkylene-piperidyl; wherein said heterocycloalkyl group can be further mono-substituted by (C₁-C₆)-alkyl, and wherein said azetidinyl, pyrroldinyl and piperidyl group can be further mono-substituted by (C₁-C₆)-alkyl,;
c) a 1,4-diazepanyl, which is unsubstituted or mono-substituted by a substituent selected from the group consisting of
i) (C₁-C₆)-alkyl, wherein (C₁-C₆)-alkyl is unsubstituted or one to fivefold substituted by F,
ii) CO-(C₁-C₆)-alkyl, wherein (C₁-C₆)-alkyl is unsubstituted or one to fivefold substituted by F;
d) a fused bicyclic (C₆-C₁₀)-heterocycloalkyl group containing two nitrogen atoms, which is attached via a nitrogen atom, wherein said heterocycloalkyl group is unsubstituted or substituted by one to two identical or different substituents selected from the group consisting of F, (C₀-C₂)-alkylene-phenyl, oxo (=O), and (C₁-C₄)-alkyl, wherein (C₁-C₄)-alkyl is unsubstituted or one to fivefold substituted by F;
e) a spiro bicylic (C₇-C₁₁)-heterocycloalkyl group containing two nitrogen atoms, which is attached via a nitrogen atom and which can contain one further oxygen atom, wherein said heterocycloalkyl group is unsubstituted or mono- or di-substituted by F or (C₁-C₄)-alkyl, which is unsubstituted or one to fivefold substituted by F;
or
f) a tricyclic (C₁₁-C₁₅)-heterocycloalkyl group containing two nitrogen atoms, which is attached via a nitrogen atom and which consists of a spiro bicyclic ring with an additional fused phenyl ring, and which is unsubstituted or substituted by one or two identical or different substituents selected from the group consisting of F and (C₁-C₄)-alkyl, which is unsubstituted or one to fivefold substituted by F.

3. A compound of the formula I as claimed in claim 1 or 2,
wherein
R¹ is H or halogen;
R² is H;
R³ is H or (C₁-C₄)-alkyl;
R⁴ is
a) (C₀-C₄)-alkyl which is mono-substituted by
i) a 3- to 8-membered monocyclic heterocycle comprising a ring nitrogen, which is unsubstituted or substituted by one to four identical or different substituents selected from the group consisting of (C₁-C₄)-alkyl, O-(C₁-C₄)-alkyl, (C₁-C₄)-alkylene-phenyl, (C₁-C₄)-alkylene-pyridyl, (C₃-C₈)-cycloalkyl, oxo (=O), and (CO)-(C₁-C₄)-alkyl;
ii) (C₃-C₈)-cycloalkyl which is monosubstituted by NH₂;
b) a spiro bicylic (C₉)-heterocycloalkyl group containing one nitrogen atom, which is attached via a carbon atom;
c) a brigded bicyclic (C₈)-heterocycloalkyl group containing one nitrogen atom, which is attached via a carbon atom;
d) a tricyclic (C₁₄)-heterocycloalkyl group containing one nitrogen atom, which is attached via a carbon atom and which consists of a spiro bicyclic ring with an additional fused phenyl ring;
or
R³ and R⁴ together with the N-atom carrying them denote
a) a 1,4-piperazinyl of the formula 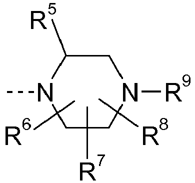 wherein
R⁵ is H or (C₁-C₆)-alkyl;
R⁶ is H or (C₁-C₆)-alkyl;
R⁷ is H or (C₁-C₆)-alkyl;
R⁸ is H or oxo (=O);
R⁹ is H, (C₁-C₆)-alkyl, (C₃-C₆)-cycloalkyl or CO-R¹⁰, where (C₁-C₆)-alkyl is unsubstituted or mono-substituted by O-(C₁-C₄)-alkyl;
R¹⁰ is (C₁-C₄)-alkyl, (C₃-C₆)-cycloalkyl, a 5- to 6-membered aromatic or aliphatic heterocyclic ring comprising one heteroatom selected from nitrogen or oxygen;
b) 1-azetidinyl, which is mono-substituted by NH₂;
c) 1-pyrrolidinyl, which is mono-substituted by NH₂, NH-(C₁-C₄)-alkyl, NH(C₁-C₄)-alkyl)₂, (C₀-C₂)-alkylene-pyrrolidinyl or (C₀-C₂)-piperidinyl which is unsubstituted or monosubstituted by (C₁-C₄)-alkyl;
d) 1-piperidyl, which is mono-substituted by NH₂;
e) 1,4-diazepanyl of the formula 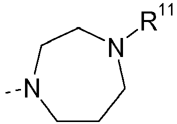 wherein
R¹¹ is H, (C₁-C₆)-alkyl or CO-(C₁-C₄)-alkyl;
f) a fused bicyclic (C₈-C₁₀)-heterocycloalkyl group containing two nitrogen atoms, which is attached via a nitrogen;
g) a spiro bicylic (C₈-C₁₁)-heterocylcoalkyl group containing two nitrogen atoms, which is attached via a nitrogen atom and which can contain one further oxygen atom;
h) a tricyclic (C₁₄)-heterocycloalkyl group containing two nitrogen atoms, which is attached via a nitrogen atom and which consists of a spiro bicyclic ring with an additional fused phenyl ring.

4. A compound of the formula I as claimed in any of claims 1 to 3,
wherein
R¹ is H or F;
R² is H;
R³ is H or CH₃;
R⁴ is
a) (C₀-C₁)-alkyl which is mono-substituted by
i) azetidyl, pyrrolidinyl or piperidyl, which are unsubstituted or substituted by one to four identical or different substituents selected from the group consisting of CH₃, C₂H₅, O-CH₃, methylene-phenyl, methylene-pyridyl, cyclohexyl, oxo (=O), and (CO)-CH₃;
ii) (C₃-C₆)-cycloalkyl which is monosubstituted by NH₂;
b) a spiro bicyclic ring 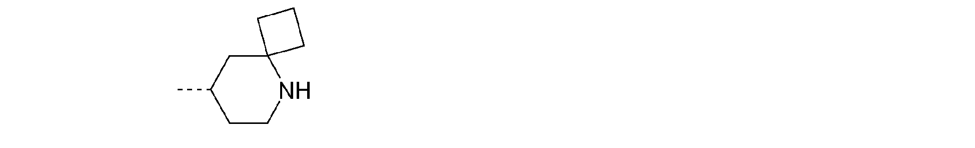
c) a bridged bicyclic ring selected from the group consisting of 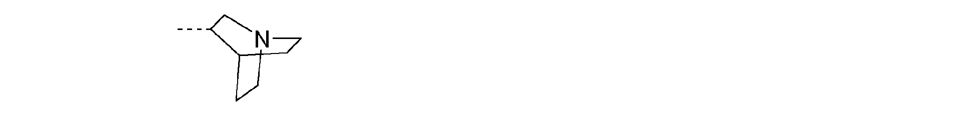
d) a spiro bicyclic ring with a fused ring selected from the group consisting of 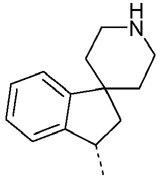 or
R³ and R⁴ together with the N-atom carrying them denote
a) a 1,4-piperazinyl of the formula 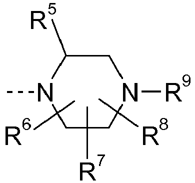 wherein
R⁵ is H or CH₃;
R⁶ is H or CH₃;
R⁷ is H or (C₁-C₄)-alkyl;
R⁸ is H or oxo (=O);
R⁹ is H, (C₁-C₃)-alkyl, cycloheptyl or CO-R¹⁰, where (C₁-C₃)-alkyl is unsubstituted or mono-substituted by O-CH₃;
R¹⁰ is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 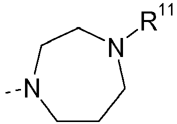 wherein
R¹¹ is H, ethyl or CO-CH₃;
f) a fused bicyclic ring selected from the group consisting of 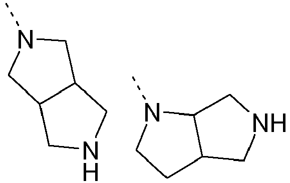
g) a spiro bicyclic ring selected from the group consisting of 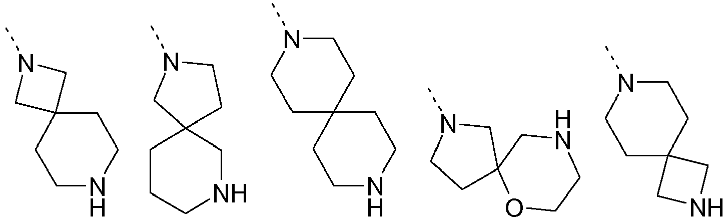 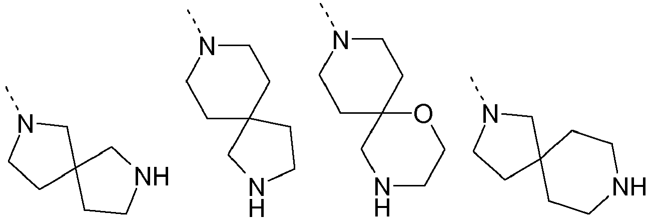 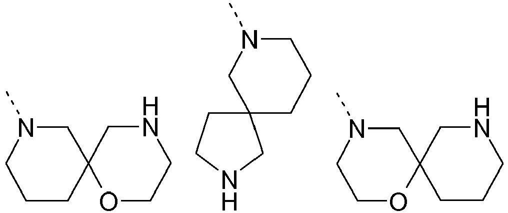
h) a bridged bicyclic ring selected from the group consisting of 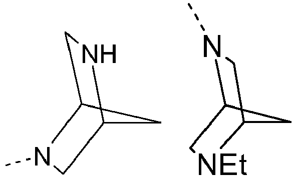
i) a spiro bicyclic ring with a fused ring selected from the group consisting of

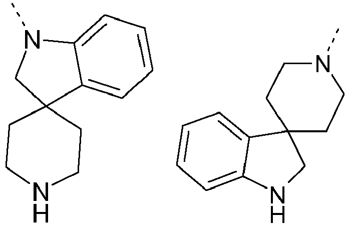

5. A compound of the formula I as claimed in any of claims 1 to 4,
wherein
R¹ is H or F;
R² is H;
R³ is H or CH₃;
R⁴ is
a) methylene which is mono-substituted by
i) azetidyl or pyrrolidinyl, which are attached by a carbon atom and which are unsubstituted or substituted by oxo (=O);
ii) cyclohexyl which is monosubstituted by NH₂;
b) azetidyl which is attached by a carbon atom and which is unsubstituted or substituted by CH_{3;}
c) pyrrolidinyl, which is attached by a carbon atom and which is unsubstituted or substituted by O-CH₃;
d) piperidyl, which is attached by a carbon atom and which is unsubstituted or substituted by one to four identical or different substituents selected from the group consisting of CH₃, C₂H₅, methylene-phenyl, methylene-pyridyl, cyclohexyl, oxo (=O), and (CO)-CH₃;
e) (C₃-C₆)-cycloalkyl which is monosubstituted by NH₂;
f) a spiro bicyclic ring 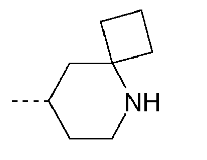
g) a bridged bicyclic ring selected from the group consisting of 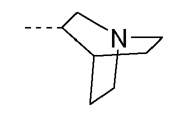
h) a spiro bicyclic ring with a fused ring selected from the group consisting of 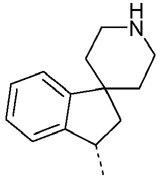 or
R³ and R⁴ together with the N-atom carrying them denote
a) a 1,4-piperazinyl of the formula 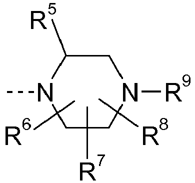 wherein
R⁵ is H or CH₃;
R⁶ is H or CH₃;
R⁷ is H or (C₁-C₄)-alkyl;
R⁸ is H or oxo (=O);
R⁹ is H, (C₁-C₃)-alkyl, cycloheptyl or CO-R¹⁰, where (C₁-C₃)-alkyl is unsubstituted or mono-substituted by O-CH₃;
R¹⁰ is CH₃, cyclopropyl, 2-furyl or 3-pyridyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 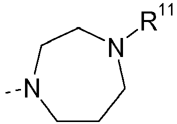 wherein
R¹¹ is H, ethyl or CO-CH₃;
f) a fused bicyclic ring selected from the group consisting of 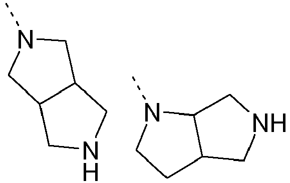
g) a spiro bicyclic ring selected from the group consisting of 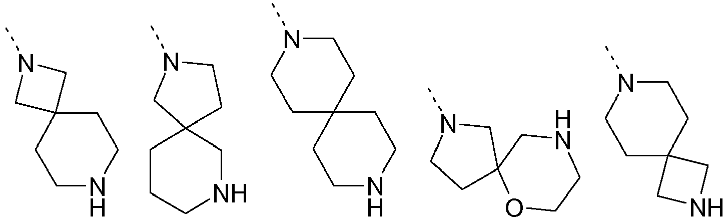 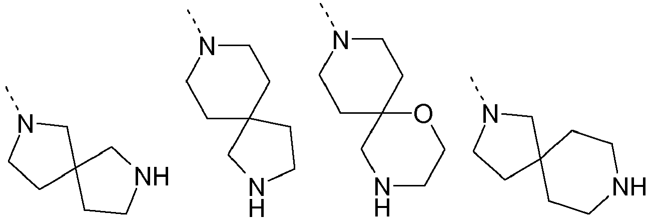 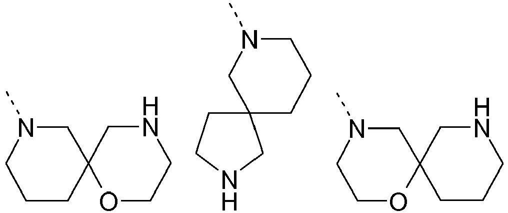
h) a bridged bicyclic ring selected from the group consisting of 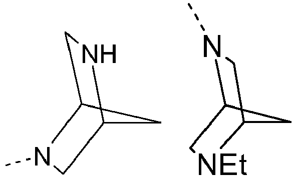
i) a spiro bicyclic ring with a fused ring selected from the group consisting of

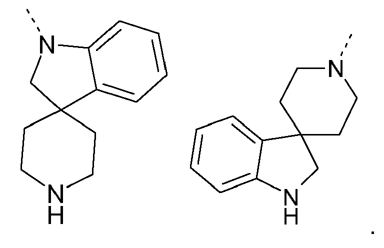

6. A compound of the formula I as claimed in any of claims 1 to 5,
wherein
R¹ is H or F;
R² is H;
R³ is H or CH₃;
R⁴ is
a) methylene which is mono-substituted by
i) 3-azetidyl;
ii) cyclohexyl which is monosubstituted by NH₂;
b) azetidyl which is attached by a carbon atom;
c) pyrrolidinyl, which is attached by a carbon atom;
d) piperidyl, which is attached by a carbon atom and which is unsubstituted or substituted by one substituent selected from the group consisting of CH₃, C₂H₅, methylene-phenyl, methylene-pyridyl, and cyclohexyl;
e) cyclohexyl which is monosubstituted by NH_{2;}
f) a spiro bicyclic ring 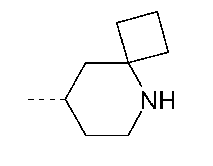 or
R³ and R⁴ together with the N-atom carrying them denote
a) a 1,4-piperazinyl of the formula 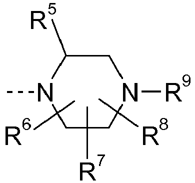 wherein
R⁵ is H or CH₃;
R⁶ is H or CH₃;
R⁷ is H or (C₁-C₄)-alkyl;
R⁸ is H;
R⁹ is H or (C₁-C₃)-alkyl;
b) 1-azetidinyl, which is mono-substituted by NH₂;
c) 1-pyrrolidinyl, which is mono-substituted by NH₂, NH(CH₃), NH(CH₃)₂, methylene-1-pyrrolidinyl or 1-piperidinyl-4-methyl;
d) 1-piperidyl, which is mono-substituted by NH₂;
e) a 1,4-diazepanyl of the formula 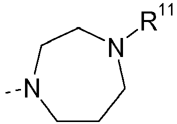 wherein
R¹¹ is H or ethyl;
f) a fused bicyclic ring selected from the group consisting of 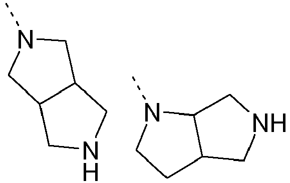
g) a spiro bicyclic ring selected from the group consisting of

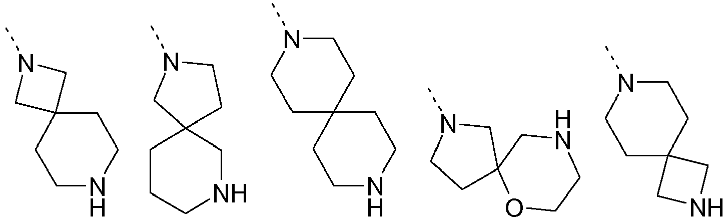

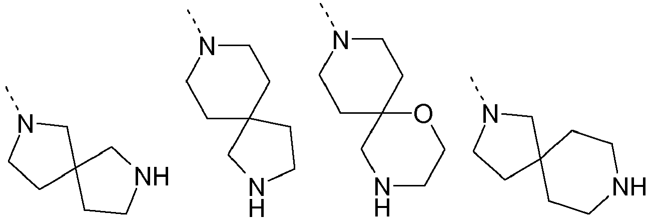

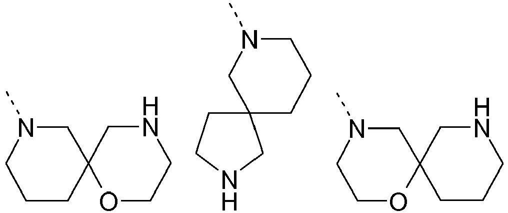

7. A compound of the formula I as claimed in any of claims 1 to 6,
wherein
R¹ is H;
R² is H;
R³ is H;
R⁴ is
a) methylene which is mono-substituted by
i) 3-azetidyl;
ii) 4-amino-cyclohexyl;
b) 3-azetidyl;
c) 3-pyrrolidinyl;
d) 3-piperidyl or 4-piperidyl, which are unsubstituted or substituted at the nitrogen atom by one substituent selected from the group consisting of CH₃, C₂H₅, methylene-phenyl, methylene-pyridyl, and cyclohexyl;
e) 3-amino-cyclohexyl or 4-amino-cylohexyl;
f) a spiro bicyclic ring 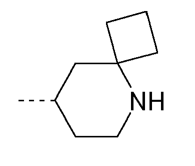 or
R³ and R⁴ together with the N-atom carrying them denote
a) a 1,4-piperazinyl of the formula 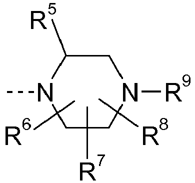 wherein
R⁵ is H or CH₃;
R⁶ is H or CH₃;
R⁷ is H or iso-propyl;
R⁸ is H;
R⁹ is H or (C₁-C₃)-alkyl;
b) 3-amino-1-azetidinyl;
c) 1-pyrrolidinyl, which is mono-substituted in 3-position by NH₂, NH(CH₃), NH(CH₃)₂, or 1-piperidinyl-4-methyl;
d) 4-amino-1-piperidyl;
e) a 1,4-diazepanyl of the formula 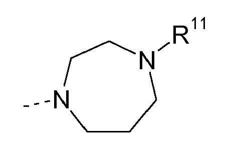 wherein
R¹¹ is H or ethyl;
f) a fused bicyclic ring selected from the group consisting of 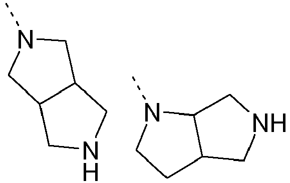
g) a spiro bicyclic ring selected from the group consisting of

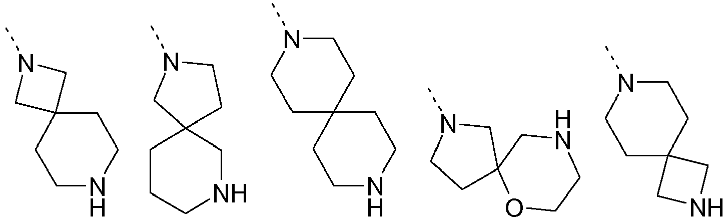

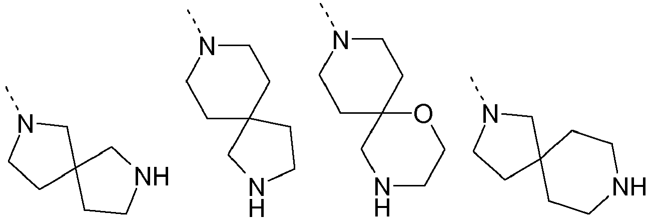

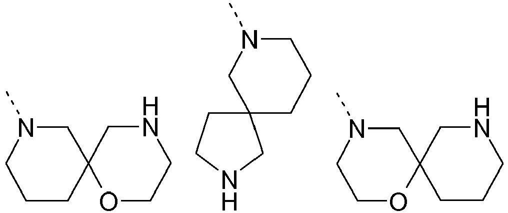

8. A compound as claimed in claim 1 selected from the group consisting of
| | |
|---|---|
| 1 | 6-(4-Hydroxy-phenyl)-1H-pyrazolo[3,4-b]pyridine-4-carboxylic acid (2,2,6,6-tetramethyl-piperidin-4-yl)-amide |
| 2 | 6-(4-Hydroxy-phenyl)-1H-pyrazolo[3,4-b]pyridine-4-carboxylic acid piperidin-4-ylamide |
| 3 | (3-Amino-piperidin-1-yl)-[6-(4-hydroxy-phenyl)-1H-pyrazolo[3,4-b]pyridin-4-yl]-methanone |
| 4 | [6-(4-Hydroxy-phenyl)-1H-pyrazolo[3,4-b]pyridin-4-yl]-piperazin-1-yl-methanone |
| 5 | 6-(4-Hydroxy-phenyl)-1H-pyrazolo[3,4-b]pyridine-4-carboxylic acid pyrrolidin-3-ylamide |
| 6 | 6-(4-Hydroxy-phenyl)-1H-pyrazolo[3,4-b]pyridine-4-carboxylic acid ((3S,4S)-4-methoxy-pyrrolidin-3-yl)-amide |
| 7 | [1,4]Diazepan-1-yl-[6-(4-hydroxy-phenyl)-1H-pyrazolo[3,4-b]pyridin-4-yl]-methanone |
| 8 | 6-(4-Hydroxy-phenyl)-1H-pyrazolo[3,4-b]pyridine-4-carboxylic acid (cis-4-amino-cyclohexyl)-amide |
| 9 | 6-(4-Hydroxy-phenyl)-1H-pyrazolo[3,4-b]pyridine-4-carboxylic acid (trans-4-amino-cyclohexyl)-amide |
| 10 | (2,2-Dimethyl-piperazin-1-yl)-[6-(4-hydroxy-phenyl)-1H-pyrazolo[3,4-b]pyridin-4-yl]-methanone |
| 11 | 6-(4-Hydroxy-phenyl)-1H-pyrazolo[3,4-b]pyridine-4-carboxylic acid (trans-4-amino-cyclohexylmethyl)-amide |
| 12 | 6-(4-Hydroxy-phenyl)-1H-pyrazolo[3,4-b]pyridine-4-carboxylic acid (cis-4-amino-cyclohexylmethyl)-amide |
| 13 | 6-(4-Hydroxy-phenyl)-1H-pyrazolo[3,4-b]pyridine-4-carboxylic acid (azetidin-3-ylmethyl)-amide |
| 14 | 6-(4-Hydroxy-phenyl)-1H-pyrazolo[3,4-b]pyridine-4-carboxylic acid (3-amino-cyclobutyl)-amide |
| 15 | [6-(4-Hydroxy-phenyl)-1H-pyrazolo[3,4-b]pyridin-4-yl]-(5-isopropyl-2,2-dimethyl-piperazin-1-yl)-methanone |
| 16 | ((R)-3-Amino-pyrrolidin-1-yl)-[6-(4-hydroxy-phenyl)-1H-pyrazolo[3,4-b]pyridin-4-yl]-methanone |
| 17 | ((S)-3-Amino-pyrrolidin-1-yl)-[6-(4-hydroxy-phenyl)-1H-pyrazolo[3,4-b]pyridin-4-yl]-methanone |
| 18 | 6-(4-Hydroxy-phenyl)-1H-pyrazolo[3,4-b]pyridine-4-carboxylic acid-(R)-(1-Aza-bicyclo[2.2.2]oct-3-yl)amide |
| 19 | 6-(4-Hydroxy-phenyl)-1H-pyrazolo[3,4-b]pyridine-4-carboxylic acid (R)-piperidin-3-ylamide |
| 20 | (2,7-Diaza-spiro[3.5]non-2-yl)-[6-(4-hydroxy-phenyl)-1H-pyrazolo[3,4-b]pyridin-4-yl]-methanone |
| 21 | (2,7-Diaza-spiro[3.5]non-7-yl)-[6-(4-hydroxy-phenyl)-1H-pyrazolo[3,4-b]pyridin-4-yl]-methanone |
| 22 | (2,8-Diaza-spiro[4.5]dec-2-yl)-[6-(4-hydroxy-phenyl)-1H-pyrazolo[3,4-b]pyridin-4-yl]-methanone |
| 23 | (2,7-Diaza-spiro[4.5]dec-2-yl)-[6-(4-hydroxy-phenyl)-1H-pyrazolo[3,4-b]pyridin-4-yl]-methanone |
| 24 | (2,7-Diaza-spiro[4.5]dec-7-yl)-[6-(4-hydroxy-phenyl)-1H-pyrazolo[3,4-b]pyridin-4-yl]-methanone |
| 25 | (2,7-Diaza-spiro[4.4]non-2-yl)-[6-(4-hydroxy-phenyl)-1H-pyrazolo[3,4-b]pyridin-4-yl]-methanone |
| 26 | [6-(4-Hydroxy-phenyl)-1H-pyrazolo[3,4-b]pyridin-4-yl]-(1-oxa-4,9-diaza-spiro[5.5]undec-9-yl)-methanone |
| 27 | [6-(4-Hydroxy-phenyl)-1H-pyrazolo[3,4-b]pyridin-4-yl]-(1-oxa-4,8-diaza-spiro[5.5]undec-4-yl)-methanone |
| 28 | [6-(4-Hydroxy-phenyl)-1H-pyrazolo[3,4-b]pyridin-4-yl]-(1-oxa-4,8-diaza-spiro[5.5]undec-8-yl)-methanone |
| 29 | 6-(4-Hydroxy-phenyl)-1H-pyrazolo[3,4-b]pyridine-4-carboxylic acid (trans-4-methoxy-pyrrolidin-3-yl)-amide |
| 30 | 6-(4-Hydroxy-phenyl)-1H-pyrazolo[3,4-b]pyridine-4-carboxylic acid (trans-3-amino-cyclobutyl)-amide |
| 31 | [6-(4-Hydroxy-phenyl)-1H-pyrazolo[3,4-b]pyridin-4-yl]-(6-oxa-2,9-diaza-spiro[4.5]dec-2-yl)-methanone |
| 32 | 6-(4-Hydroxy-phenyl)-1H-pyrazolo[3,4-b]pyridine-4-carboxylic acid (5-aza-spiro[3.5]non-8-yl)-amide |
| 33 | 6-(4-Hydroxy-phenyl)-1H-pyrazolo[3,4-b]pyridine-4-carboxylic acid (3-amino-cyclohexyl)-amide |
| 34 | (Hexahydro-pyrrolo[3,4-b]pyrrol-1-yl)-[6-(4-hydroxy-phenyl)-1H-pyrazolo[3,4-b]pyridin-4-yl]-methanone |
| 35 | 6-(4-Hydroxy-phenyl)-1H-pyrazolo[3,4-b]pyridine-4-carboxylic acid azetidin-3-ylamide |
| 36 | (3-Amino-azetidin-1-yl)-[6-(4-hydroxy-phenyl)-1H-pyrazolo[3,4-b]pyridin-4-yl]-methanone |
| 37 | [6-(4-Hydroxy-phenyl)-1H-pyrazolo[3,4-b]pyridin-4-yl]-(3-methyl-piperazin-1-yl)-methanone |
| 38 | (4-Amino-piperidin-1-yl)-[6-(4-hydroxy-phenyl)-1H-pyrazolo[3,4-b]pyridin-4-yl]-methanone |
| 39 | [6-(4-Hydroxy-phenyl)-1H-pyrazolo[3,4-b]pyridin-4-yl]-(3-methylamino-pyrrolidin-1-yl)-methanone |
| 40 | 6-(4-Hydroxy-phenyl)-1H-pyrazolo[3,4-b]pyridine-4-carboxylic acid-(S)-(1-Aza-bicyclo[2.2.2]oct-3-yl)amide |
| 41 | 6-(4-Hydroxy-phenyl)-1H-pyrazolo[3,4-b]pyridine-4-carboxylic acid (1-methyl-piperidin-3-yl)-amide |
| 42 | 6-(4-Hydroxy-phenyl)-1H-pyrazolo[3,4-b]pyridine-4-carboxylic acid methyl-piperidin-4-yl-amide |
| 43 | (2,8-Diaza-spiro[4.5]dec-8-yl)-[6-(4-hydroxy-phenyl)-1H-pyrazolo[3,4-b]pyridin-4-yl]-methanone |
| 44 | (3,9-Diaza-spiro[5.5]undec-3-yl)-[6-(4-hydroxy-phenyl)-1H-pyrazolo[3,4-b]pyridin-4-yl]-methanone |
| 45 | (3-Amino-pyrrolidin-1-yl)-[6-(4-hydroxy-phenyl)-1H-pyrazolo[3,4-b]pyridin-4-yl]-methanone |
| 46 | (Hexahydro-pyrrolo[3,4-c]pyrrol-2-yl)-[6-(4-hydroxy-phenyl)-1H-pyrazolo[3,4-b]pyridin-4-yl]-methanone |
| 47 | 2,5-Diaza-bicyclo[2.2.1]hept-2-yl-[6-(4-hydroxy-phenyl)-1H-pyrazolo[3,4-b]pyridin-4-yl]-methanone |
| 48 | 6-(4-Hydroxy-phenyl)-1H-pyrazolo[3,4-b]pyridine-4-carboxylic acid (2,3-dihydro-spiro[1H-indene-1,4'-piperidin]-3-yl)-amide |
| 49 | 6-(4-Hydroxy-phenyl)-1H-pyrazolo[3,4-b]pyridine-4-carboxylic acid (1,2-dihydrospiro[3H-indole-3,4'-piperidin]-1'-yl)-amide |
| 50 | 6-(4-hydroxy-phenyl)-1H-pyrazolo[3,4-b]pyridine-4-carboxylic acid (2,3-dihydrospiro[1H-indene-1,4'-piperidin]-3-yl)-amide |
| 51 | 6-(4-Hydroxy-phenyl)-1H-pyrazolo[3,4-b]pyridine-4-carboxylic acid (trans-2-amino-cyclopropyl)-amide |
| 52 | [6-(4-Hydroxy-phenyl)-1H-pyrazolo[3,4-b]pyridin-4-yl]-(4-methyl-piperazin-1-yl)-methanone |
| 53 | 1-{4-[6-(4-Hydroxy-phenyl)-1H-pyrazolo[3,4-b]pyridine-4-carbonyl]-piperazin-1-yl}-ethanone |
| 54 | [6-(4-Hydroxy-phenyl)-1H-pyrazolo[3,4-b]pyridin-4-yl]-((S)-2-pyrrolidin-1-ylmethyl-pyrrolidin-1-yl)-methanone |
| 55 | ((S)-3-Dimethylamino-pyrrolidin-1-yl)-[6-(4-hydroxy-phenyl)-1H-pyrazolo[3,4-b]pyridin-4-yl]-methanone |
| 56 | [6-(4-Hydroxy-phenyl)-1H-pyrazolo[3,4-b]pyridin-4-yl]-(4-isopropyl-piperazin-1-yl)-methanone |
| 57 | (4-Cyclopropanecarbonyl-piperazin-1-yl)-[6-(4-hydroxy-phenyl)-1H-pyrazolo[3,4-b]pyridin-4-yl]-methanone |
| 58 | (4-Cycloheptyl-piperazin-1-yl)-[6-(4-hydroxy-phenyl)-1H-pyrazolo[3,4-b]pyridin-4-yl]-methanone |
| 59 | 1-{4-[6-(4-Hydroxy-phenyl)-1H-pyrazolo[3,4-b]pyridine-4-carbonyl]-[1,4]diazepan-1-yl}-ethanone |
| 60 | 6-(4-Hydroxy-phenyl)-1H-pyrazolo[3,4-b]pyridine-4-carboxylic acid (1-ethyl-piperidin-3-yl)-amide |
| 61 | [6-(4-Hydroxy-phenyl)-1H-pyrazolo[3,4-b]pyridin-4-yl]-[4-(tetrahydrofuran-2-carbonyl)-piperazin-1-yl]-methanone |
| 62 | 6-(4-Hydroxy-phenyl)-1 H-pyrazolo[3,4-b]pyridine-4-carboxylic acid (1-methyl-azetidin-3-yl)-amide |
| 63 | 6-(4-Hydroxy-phenyl)-1H-pyrazolo[3,4-b]pyridine-4-carboxylic acid (5-oxo-pyrrolidin-2-ylmethyl)-amide |
| 64 | (4-Ethyl-[1,4]diazepan-1-yl)-[6-(4-hydroxy-phenyl)-1H-pyrazolo[3,4-b]pyridin-4-yl]-methanone |
| 65 | [6-(4-Hydroxy-phenyl)-1H-pyrazolo[3,4-b]pyridin-4-yl]-[3-(4-methyl-piperidin-1-yl)-pyrrolidin-1-yl]-methanone |
| 66 | 6-(4-Hydroxy-phenyl)-1H-pyrazolo[3,4-b]pyridine-4-carboxylic acid (1-methyl-piperidin-4-yl)-amide |
| 67 | 6-(4-Hydroxy-phenyl)-1H-pyrazolo[3,4-b]pyridine-4-carboxylic acid (1-cyclohexyl-piperidin-4-yl)-amide |
| 68 | 6-(4-Hydroxy-phenyl)-1H-pyrazolo[3,4-b]pyridine-4-carboxylic acid (1-acetyl-piperidin-4-yl)-amide |
| 69 | 6-(4-Hydroxy-phenyl)-1H-pyrazolo[3,4-b]pyridine-4-carboxylic acid ((R)-6-oxo-piperidin-3-yl)-amide |
| 70 | (5-Ethyl-2,5-diaza-bicyclo[2.2.1]hept-2-yl)-[6-(4-hydroxy-phenyl)-1H-pyrazolo[3,4-b]pyridin-4-yl]-methanone |
| 71 | [6-(4-Hydroxy-phenyl)-1H-pyrazolo[3,4-b]pyridin-4-yl]-[4-(2-methoxy-ethyl)-piperazin-1-yl]-methanone |
| 72 | 6-(4-Hydroxy-phenyl)-1H-pyrazolo[3,4-b]pyridine-4-carboxylic acid (1-aza-bicyclo[2.2.2]oct-3-yl)-amide |
| 73 | 6-(4-Hydroxy-phenyl)-1H-pyrazolo[3,4-b]pyridine-4-carboxylic acid methyl-(1-methyl-piperidin-4-yl)-amide |
| 74 | [6-(4-Hydroxy-phenyl)-1H-pyrazolo[3,4-b]pyridin-4-yl]-[4-(pyridine-3-carbonyl)-piperazin-1-yl]-methanone |
| 75 | 6-(4-Hydroxy-phenyl)-1H-pyrazolo[3,4-b]pyridine-4-carboxylic acid (1-pyridin-4-ylmethyl-piperidin-4-yl)-amide |
| 76 | 4-[6-(4-Hydroxy-phenyl)-1H-pyrazolo[3,4-b]pyridine-4-carbonyl]-1-methyl-piperazin-2-one |
| 77 | 6-(4-Hydroxy-phenyl)-1H-pyrazolo[3,4-b]pyridine-4-carboxylic acid ((R)-1-benzyl-piperidin-3-yl)-amide |
| 78 | 6-(4-Hydroxy-phenyl)-1H-pyrazolo[3,4-b]pyridine-4-carboxylic acid (1-pyridin-3-ylmethyl-piperidin-4-yl)-amide |

9. A pharmaceutical composition comprising at least one compound of the formula I as claimed in any of claims 1 to 8 or a physiologically acceptable solvate of any of them, for use as a pharmaceutical.

10. A compound of the formula I as claimed in any of claims 1 to 8 or a physiologically acceptable salt thereof, or a physiologically acceptable solvate of any of them, for use as a pharmaceutical.

11. A compound of the formula I as claimed in any of claims 1 to 8 or a physiologically acceptable salt thereof, or a physiologically acceptable solvate of any of them, for use in the treatment of diseases associated with diabetes and diabetic complications.

12. A compound of the formula I as claimed in any of claims 1 to 8 or a physiologically acceptable salt thereof, or a physiologically acceptable solvate of any of them, for use in the prevention and treatment of nephropathy, neuropathy, retinopathy, ischemia, inflammation, central nervous system disorders, cardiovascular diseases, dermatological diseases, autoimmune diseases and cancer.

13. A compound of the formula I as claimed in any of claims 1 to 8 or a physiologically acceptable salt thereof, or a physiologically acceptable solvate of any of them, for use in the treatment of a disease associated with the PKC receptor.

## Patentansprüche

1. Verbindung der Formel I 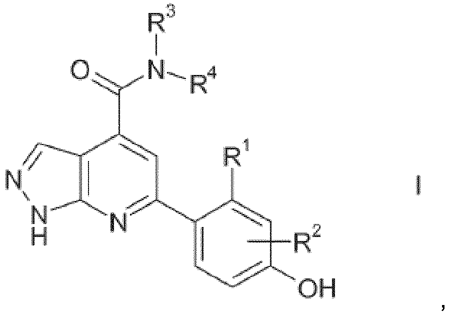 wobei
R¹ für H, Halogen oder (C₁-C₄)-Alkyl steht,
R² für H, Halogen oder (C₁-C₄)-Alkyl steht,
R³ für H oder (C₁-C₄)-Alkyl steht,
R⁴ für
a) (C₀-C₆)-Alkyl, welches monosubstituiert ist durch
i) einen 3- bis 8-gliedrigen monocyclischen Heterocyclus mit einem Ringstickstoffatom und gegebenenfalls einem weiteren Ringheteroatom ausgewählt aus der Gruppe bestehend aus Stickstoff und Sauerstoff, welcher unsubstituiert ist oder substituiert ist durch einen bis fünf gleiche oder verschiedene Substituenten ausgewählt aus der Gruppe bestehend aus
ia) F,
ib) (C₁-C₄)-Alkyl, welches unsubstituiert ist oder ein- bis fünffach substituiert ist durch F,
ic) O-(C₁-C₄)-Alkyl, welches unsubstituiert ist oder ein- bis fünffach substituiert ist durch F,
id) Phenyl, welches unsubstituiert ist oder substituiert ist durch einen bis zwei gleiche oder verschiedene Substituenten ausgewählt aus der Gruppe bestehend aus Halogen und (C₁-C₄)-Alkyl, wobei (C₁-C₄)-Alkyl unsubstituiert ist oder ein- bis fünffach substituiert ist durch F,
ie) (C₁-C₄)-Alkylen-phenyl, welches unsubstituiert ist oder ein- bis fünffach substituiert ist durch F,
if) (C₃-C₈)-Cycloalkyl,
ig) Oxo (=O) und
ih) (CO)-(C₁-C₄)-Alkyl,
und wobei (C₀-C₆)-Alkyl weiter durch Phenyl oder Pyridyl monosubstituiert sein kann, wobei Phenyl bzw. Pyridyl unsubstituiert ist oder substituiert ist durch einen bis zwei gleiche oder verschiedene Substituenten ausgewählt aus der Gruppe bestehend aus Halogen und (C₁-C₄)-Alkyl, wobei (C₁-C₄)-Alkyl unsubstituiert ist oder ein- bis fünffach substituiert ist durch F,
ii) (C₃-C₈)-Cycloalkyl, welches substituiert ist durch einen bis zwei gleiche oder verschiedene Substituenten ausgewählt aus der Gruppe bestehend aus NH₂, NH((C₁-C₄)-Alkyl) und N((C₁-C₄)-Alkyl)₂, und wobei (C₃-C₈)-Cycloalkyl weiter substituiert sein kann durch einen bis drei gleiche oder verschiedene Substituenten ausgewählt aus der Gruppe bestehend aus
iia) F,
iib) (C₁-C₄)-Alkyl, wobei (C₁-C₄)-Alkyl unsubstituiert ist oder ein- bis fünffach substituiert ist durch F,
iic) O-(C₁-C₄)-Alkyl, welches unsubstituiert ist oder ein- bis fünffach substituiert ist durch F,
iid) Phenyl, welches unsubstituiert ist oder substituiert ist durch einen bis zwei gleiche oder verschiedene Substituenten ausgewählt aus der Gruppe bestehend aus Halogen und (C₁-C₄)-Alkyl, wobei (C₁-C₄)-Alkyl unsubstituiert ist oder ein- bis fünffach substituiert ist durch F, und
iie) (C₁-C₄)-Alkylen-phenyl, welches unsubstituiert ist oder ein- bis fünffach substituiert ist durch F,
oder
iii) NH₂, NH(C₁-C₆)-Alkyl oder N((C₁-C₆)-Alkyl)₂,
und wobei (C₁-C₆)-Alkyl weiter monosubstituiert sein kann durch Phenyl, Phenylen-(C₁-C₄)-Alkyl oder Phenylen-O-(C₁-C₄)-alkyl,
b) eine bicyclische (C₆-C₁₁)-Cycloalkylgruppe, welche monosubstituiert ist durch (C₀-C₂)-Alkylen-NH₂, (C₀-C₂)-Alkylen-NH-(C₁-C₄)-alkyl, (C₀-C₂)-Alkylen-N((C₁-C₄)-alkyl)₂,
c) eine kondensierte bicyclische (C₆-C₁₀)-Heterocycloalkylgruppe mit einem Stickstoffatom, welche über ein Kohlenstoffatom gebunden ist und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
d) eine spirobicyclische (C₇-C₁)-Heterocycloalkylgruppe mit einem Stickstoffatom, welche über ein Kohlenstoffatom gebunden ist und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
e) eine verbrückte bicyclische (C₇-C₉)-Heterocycloalkylgruppe mit einem Stickstoffatom, welche über ein Kohlenstoffatom gebunden ist und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F, oder
f) eine tricyclische (C₁₁-C₁₅)-Heterocycloalkylgruppe mit einem Stickstoffatom, welche über ein Kohlenstoffatom gebunden ist und welche aus einem spirobicyclischen Ring mit einem zusätzlichen kondensierten Phenylring besteht und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
steht, oder
R³ und R⁴ zusammen mit dem Stickstoffatom, das sie trägt, für
a) ein 1,4-Piperazinyl der Formel 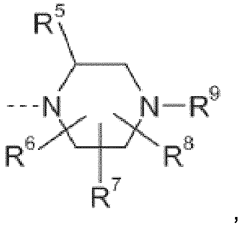 wobei
R⁵ für H oder (C₀-C₆)-Alkyl steht, welches unsubstituiert ist oder ein- bis fünffach substituiert ist durch F, oder monosubstituiert ist durch einen Substituenten ausgewählt aus der Gruppe bestehend aus
i) O-(C₁-C₄)-Alkyl, welches unsubstituiert ist oder ein- bis fünffach substituiert ist durch F,
ii) (C₃-C₆)-Cycloalkyl,
iii) Phenyl, welches unsubstituiert ist oder substituiert ist durch einen bis zwei gleiche oder verschiedene Substituenten ausgewählt aus der Gruppe bestehend aus Halogen, CF₃, O-(C₁-C₄)-Alkyl, OCF₃ und (C₁-C₄)-Alkyl,
iv) einem 5- bis 6-gliedrigen monocyclischen heteroaromatischen Ring mit einem Heteroatom ausgewählt aus der Gruppe bestehend aus Stickstoff, Sauerstoff und Schwefel, welcher unsubstituiert ist oder substituiert ist durch einen bis zwei gleiche oder verschiedene Substituenten ausgewählt aus der Gruppe bestehend aus Halogen, CF₃, O-(C₁-C₄)-Alkyl, OCF₃ und (C₁-C₄)-Alkyl,
v) Benzo[1,3]dioxol und
vi) CO-O-(C₁-C₄)-Alkyl oder CO-NH-(C₁-C₆)-Alkyl,
R⁶ für H oder (C₀-C₆)-Alkyl steht, welches unsubstituiert ist oder ein- bis fünffach substituiert ist durch F oder monosubstituiert ist durch einen Substituenten ausgewählt aus der Gruppe bestehend aus
i) CO-O-(C₁-C₄)-Alkyl,
ii) (C₃-C₆)-Cycloalkyl,
iii) Phenyl, welches unsubstituiert ist oder substituiert ist durch einen bis zwei gleiche oder verschiedene Substituenten ausgewählt aus der Gruppe bestehend aus Halogen, CF₃, O-(C₁-C₄)-Alkyl, OCF₃ und (C₁-C₄)-Alkyl,
iv) O-(C₁-C₄)-Alkyl, welches unsubstituiert ist oder ein- bis fünffach substituiert ist durch F, und
v) einem 5- bis 6-gliedrigen monocyclischen heteroaromatischen Ring mit einem Heteroatom ausgewählt aus der Gruppe bestehend aus Stickstoff, Sauerstoff und Schwefel, welcher unsubstituiert ist oder substituiert ist durch einen bis zwei gleiche oder verschiedene Substituenten ausgewählt aus der Gruppe bestehend aus Halogen, CF₃, O-(C₁-C₄)-Alkyl, OCF₃ und (C₁-C₄)-Alkyl,
R⁷ für H, (C₁-C₆)-Alkyl, welches unsubstituiert ist oder ein- bis fünffach substituiert ist durch F, oder Phenyl steht,
R⁸ für H, (C₁-C₆)-Alkyl oder Oxo (=O) steht,
R⁹ für H, (C₁-C₆)-Alkyl, (C₃-C₆)-Cycloalkyl oder CO-R¹⁰ steht, wobei (C₁-C₆)-Alkyl unsubstituiert ist oder ein- bis fünffach substituiert ist durch F, oder monosubstituiert ist durch einen Substituenten ausgewählt aus der Gruppe bestehend aus O-(C₁-C₄)-Alkyl, SO₂-(C₁-C₄)-Alkyl, Phenyl, einem 5- bis 6-gliedrigen monocyclischen heterocyclischen Ring mit einem Heteroatom ausgewählt aus der Gruppe bestehend aus Stickstoff und Sauerstoff, einem 5- bis 6-gliedrigen monocyclischen heteroaromatischen Ring mit einem Heteroatom ausgewählt aus der Gruppe bestehend aus Stickstoff und Sauerstoff, wobei
R¹⁰ für (C₁-C₄)-Alkyl, (C₃-C₆)-Cycloalkyl, NH₂, Phenyl, einen 5- bis 6-gliedrigen monocyclischen heterocyclischen Ring mit einem Heteroatom ausgewählt aus der Gruppe bestehend aus Stickstoff und Sauerstoff oder einen 5- bis 6-gliedrigen monocyclischen heteroaromatischen Ring mit einem Heteroatom ausgewählt aus der Gruppe bestehend aus Stickstoff und Sauerstoff steht, wobei Phenyl weiter durch (C₁-C₄)-Alkyl oder O-(C₁-C₄)-Alkyl monosubstituiert sein kann,
b) eine vier- bis siebengliedrige monocyclische Heterocycloalkylgruppe mit einem Stickstoffatom, welche über den Stickstoff gebunden ist und welche monosubstituiert ist durch (C₀-C₆)-Alkylen-NH₂, (C₀-C₆)-Alkylen-NH-(C₁-C₄)-alkyl, (C₀-C₆)-Alkylen-NH-phenyl, (C₀-C₆)-Alkylen-N-((C₁-C₄)-alkyl)₂, (C₀-C₆)-Alkylen-N((C₁-C₄)-alkyl)(phenyl), (C₀-C₂)-Alkylen-azetidinyl, (C₀-C₂)-Alkylen-pyrrolidinyl oder (C₀-C₂)-Alkylen-piperidyl, wobei die Heterocycloalkylgruppe weiter monosubstituiert sein kann durch (C₁-C₆)-Alkyl, welches unsubstituiert ist oder ein- bis fünffach substituiert ist durch F, CO-O-(C₁-C₄)-Alkyl oder Phenyl, und wobei die Azetidinyl-, Pyrrolidinyl-bzw. Piperidylgruppe weiter monosubstituiert sein kann durch (C₁-C₆)-Alkyl, welches unsubstituiert ist oder ein- bis fünffach substituiert ist durch F,
c) ein 1,4-Diazepanyl, welches unsubstituiert ist oder monosubstituiert ist durch einen Substituenten ausgewählt aus der Gruppe bestehend aus
i) (C₁-C₆)-Alkyl, wobei (C₁-C₆)-Alkyl unsubstituiert ist oder einbis fünffach substituiert ist durch F,
ii) CO-(C₁-C₆)-Alkyl, wobei (C₁-C₆)-Alkyl unsubstituiert ist oder ein- bis fünffach substituiert ist durch F,
iii) CO-Phenyl und
iv) CO-Pyridyl,
d) eine kondensierte bicyclische (C₆-C₁₀)-Heterocycloalkylgruppe mit zwei Stickstoffatomen, welche über ein Stickstoffatom gebunden ist und welche ein weiteres Heteroatom ausgewählt aus der Gruppe bestehend aus Stickstoff, Sauerstoff und Schwefel enthalten kann, wobei die Heterocycloalkylgruppe unsubstituiert ist oder substituiert ist durch einen bis zwei gleiche oder verschiedene Substituenten ausgewählt aus der Gruppe bestehend aus F, (C₀-C₂)-Alkylen-phenyl, Oxo (=O) und (C₁-C₄)-Alkyl, wobei (C₁-C₄)-Alkyl unsubstituiert ist oder ein- bis fünffach substituiert ist durch F,
e) eine spirobicyclische (C₇-C₁₁)-Heterocycloalkylgruppe mit zwei Stickstoffatomen, welche über ein Stickstoffatom gebunden ist und welche ein weiteres Heteroatom ausgewählt aus der Gruppe bestehend aus Stickstoff, Sauerstoff und Schwefel enthalten kann, wobei die Heterocycloalkylgruppe unsubstituiert ist oder mono- oder disubstituiert ist durch F oder (C₁-C₄)-Alkyl, welches unsubstituiert ist oder ein- bis fünffach substituiert ist durch F,
f) eine verbrückte bicyclische (C₇-C₉)-Heterocycloalkylgruppe mit einem Stickstoffatom, welche über das Stickstoffatom gebunden ist, welche monosubstituiert ist durch (C₀-C₂)-Alkylen-NH₂, (C₀-C₂)-Alkylen-NH-(C₁-C₄)-alkyl, (C₀-C₂)-Alkylen-N((C₁-C₄)-alkyl)₂,
g) eine verbrückte bicyclische (C₇-C₉)-Heterocycloalkylgruppe mit zwei Stickstoffatomen, welche über ein Stickstoffatom gebunden ist und welche unsubstituiert ist oder substituiert ist durch einen bis vier gleiche oder verschiedene Substituenten ausgewählt aus der Gruppe bestehend aus F, OH und (C₁-C₄)-Alkyl, welches unsubstituiert ist oder ein- bis fünffach substituiert ist durch F, oder
h) eine tricyclische (C₁₁-C₁₅)-Heterocycloalkylgruppe mit zwei Stickstoffatomen, welche über ein Stickstoffatom gebunden ist und welche aus einem spirobicyclischen Ring mit einem zusätzlichen kondensierten Phenylring besteht, und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
stehen,
in einer beliebigen ihrer stereoisomeren Formen, oder einer Mischung stereoisomerer Formen in einem beliebigen Verhältnis, oder ein physiologisch akzeptables Salz davon, oder ein physiologisch akzeptables Solvat einer dieser.

2. Verbindung der Formel I nach Anspruch 1,
wobei
R¹ für H, Halogen oder (C₁-C₄)-Alkyl steht,
R² für H oder Halogen steht,
R³ für H oder (C₁-C₄)-Alkyl steht,
R⁴ für
a) (C₀-C₄)-Alkyl, welches monosubstituiert ist durch
i) einen 3- bis 8-gliedrigen monocyclischen Heterocyclus mit einem Ringstickstoffatom und gegebenenfalls einem weiteren Ringheteroatom ausgewählt aus der Gruppe bestehend aus Stickstoff und Sauerstoff, welcher unsubstituiert ist oder substituiert ist durch einen bis fünf gleiche oder verschiedene Substituenten ausgewählt aus der Gruppe bestehend aus
ia) F,
ib) (C₁-C₄)-Alkyl, welches unsubstituiert ist oder ein- bis fünffach substituiert ist durch F,
ic) O-(C₁-C₄)-Alkyl, welches unsubstituiert ist oder ein- bis fünffach substituiert ist durch F,
id) Phenyl, welches unsubstituiert ist oder substituiert ist durch einen bis zwei gleiche oder verschiedene Substituenten ausgewählt aus der Gruppe bestehend aus Halogen und (C₁-C₄)-Alkyl, wobei (C₁-C₄)-Alkyl unsubstituiert ist oder ein- bis fünffach substituiert ist durch F,
ie) (C₁-C₄)-Alkylen-phenyl, welches unsubstituiert ist oder ein- bis fünffach substituiert ist durch F,
if) (C₃-C₈)-Cycloalkyl,
ig) Oxo (=O) und
ih) (CO)-(C₁-C₄)-Alkyl,
und wobei (C₀-C₆)-Alkyl weiter durch Phenyl oder Pyridyl monosubstituiert sein kann, wobei Phenyl bzw. Pyridyl unsubstituiert ist oder substituiert ist durch einen bis zwei gleiche oder verschiedene Substituenten ausgewählt aus der Gruppe bestehend aus Halogen und (C₁-C₄)-Alkyl, wobei (C₁-C₄)-Alkyl unsubstituiert ist oder ein- bis fünffach substituiert ist durch F,
ii) (C₃-C₈)-Cycloalkyl, welches substituiert ist durch einen bis zwei gleiche oder verschiedene Substituenten ausgewählt aus der Gruppe bestehend aus NH₂, NH((C₁-C₄)-Alkyl) und N((C₁-C₄)-Alkyl)₂, und wobei (C₃-C₈)-Cycloalkyl weiter substituiert sein kann durch einen bis drei gleiche oder verschiedene Substituenten ausgewählt aus der Gruppe bestehend aus
iia) F,
iib) (C₁-C₄)-Alkyl, wobei (C₁-C₄)-Alkyl unsubstituiert ist oder ein- bis fünffach substituiert ist durch F,
b) eine spirobicyclische (C₇-C₁₁)-Heterocycloalkylgruppe mit einem Stickstoffatom, welche über ein Kohlenstoffatom gebunden ist und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
c) eine verbrückte bicyclische (C₇-C₉)-Heterocycloalkylgruppe mit einem Stickstoffatom, welche über ein Kohlenstoffatom gebunden ist und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F, oder
d) eine tricyclische (C₁₁-C₁₅)-Heterocycloalkylgruppe mit einem Stickstoffatom, welche über ein Kohlenstoffatom gebunden ist und welche aus einem spirobicyclischen Ring mit einem zusätzlichen kondensierten Phenylring besteht und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
steht, oder
R³ und R⁴ zusammen mit dem Stickstoffatom, das sie trägt, für
a) ein 1,4-Piperazinyl der Formel 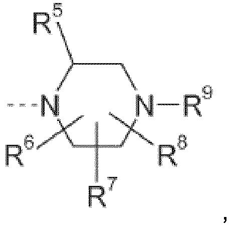 wobei
R⁵ für H oder (C₁-C₆)-Alkyl steht, welches unsubstituiert ist oder ein- bis fünffach substituiert ist durch F, oder monosubstituiert ist durch einen Substituenten ausgewählt aus der Gruppe bestehend aus
i) O-(C₁-C₄)-Alkyl,
ii) (C₃-C₆)-Cycloalkyl,
iii) Phenyl,
iv) einem 5- bis 6-gliedrigen monocyclischen heteroaromatischen Ring mit einem Heteroatom ausgewählt aus der Gruppe bestehend aus Stickstoff, Sauerstoff und Schwefel,
R⁶ für H oder (C₁-C₆)-Alkyl steht, welches unsubstituiert ist oder ein- bis fünffach substituiert ist durch F,
R⁷ für H oder (C₁-C₆)-Alkyl steht,
R⁸ für H, (C₁-C₆)-Alkyl oder Oxo (=O) steht,
R⁹ für H, (C₁-C₆)-Alkyl, (C₃-C₆)-Cycloalkyl oder CO-R¹⁰ steht, wobei (C₁-C₆)-Alkyl unsubstituiert ist oder ein- bis fünffach substituiert ist durch F, oder monosubstituiert ist durch O-(C₁-C₄)-Alkyl, wobei
R¹⁰ für (C₁-C₄)-Alkyl, (C₃-C₆)-Cycloalkyl, NH₂, Phenyl, einen 5- bis 6-gliedrigen monocyclischen heterocyclischen Ring mit einem Heteroatom ausgewählt aus der Gruppe bestehend aus Stickstoff und Sauerstoff oder einen 5- bis 6-gliedrigen monocyclischen heteroaromatischen Ring mit einem Heteroatom ausgewählt aus der Gruppe bestehend aus Stickstoff und Sauerstoff steht,
b) eine vier- bis siebengliedrige monocyclische Heterocycloalkylgruppe mit einem Stickstoffatom, welche über den Stickstoff gebunden ist und welche monosubstituiert ist durch (C₀-C₆)-Alkylen-NH₂, (C₀-C₆)-Alkylen-NH-(C₁-C₄)-alkyl, (C₀-C₆)-Alkylen-N-((C₁-C₄)-alkyl)₂, (C₀-C₂)-Alkylen-azetidinyl, (C₀-C₂)-Alkylen-pyrrolidinyl oder (C₀-C₂)-Alkylenpiperidyl, wobei die Heterocycloalkylgruppe weiter durch (C₁-C₆)-Alkyl monosubstituiert sein kann und wobei die Azetidinyl-, Pyrrolidinyl- bzw. Piperidylgruppe weiter durch (C₁-C₆)-Alkyl monosubstituiert sein kann,
c) ein 1,4-Diazepanyl, welches unsubstituiert ist oder monosubstituiert ist durch einen Substituenten ausgewählt aus der Gruppe bestehend aus
i) (C₁-C₆)-Alkyl, wobei (C₁-C₆)-Alkyl unsubstituiert ist oder einbis fünffach substituiert ist durch F,
ii) CO-(C₁-C₆)-Alkyl, wobei (C₁-C₆)-Alkyl unsubstituiert ist oder ein- bis fünffach substituiert ist durch F,
d) eine kondensierte bicyclische (C₆-C₁₀)-Heterocycloalkylgruppe mit zwei Stickstoffatomen, welche über ein Stickstoffatom gebunden ist, wobei die Heterocycloalkylgruppe unsubstituiert ist oder substituiert ist durch einen bis zwei gleiche oder verschiedene Substituenten ausgewählt aus der Gruppe bestehend aus F, (C₀-C₂)-Alkylenphenyl, Oxo (=O) und (C₁-C₄)-Alkyl, wobei (C₁-C₄)-Alkyl unsubstituiert ist oder ein- bis fünffach substituiert ist durch F,
e) eine spirobicyclische (C₇-C₁₁)-Heterocycloalkylgruppe mit zwei Stickstoffatomen, welche über ein Stickstoffatom gebunden ist und welche ein weiteres Sauerstoffatom enthalten kann, wobei die Heterocycloalkylgruppe unsubstituiert ist oder mono- oder disubstituiert ist durch F oder (C₁-C₄)-Alkyl, welches unsubstituiert ist oder ein- bis fünffach substituiert ist durch F,
oder
f) eine tricyclische (C₁₁-C₁₅)-Heterocycloalkylgruppe mit zwei Stickstoffatomen, welche über ein Stickstoffatom gebunden ist und welche aus einem spirobicyclischen Ring mit einem zusätzlichen kondensierten Phenylring besteht, und welche unsubstituiert ist oder substituiert ist durch einen oder zwei gleiche oder verschiedene Substituenten ausgewählt aus der Gruppe bestehend aus F und (C₁-C₄)-Alkyl, welches unsubstituiert ist oder ein- bis fünffach substituiert ist durch F,
stehen.

3. Verbindung der Formel I nach Anspruch 1 oder 2,
wobei
R¹ für H oder Halogen steht,
R² für H steht,
R³ für H oder (C₁-C₄)-Alkyl steht,
R⁴ für
a) (C₀-C₄)-Alkyl, welches monosubstituiert ist durch
i) einen 3- bis 8-gliedrigen monocyclischen Heterocyclus mit einem Ringstickstoff, welcher unsubstituiert ist oder substituiert ist durch einen bis vier gleiche oder verschiedene Substituenten ausgewählt aus der Gruppe bestehend aus (C₁-C₄)-Alkyl, O-(C₁-C₄)-Alkyl, (C₁-C₄)-Alkylen-phenyl, (C₁-C₄)-Alkylen-pyridyl, (C₃-C₈)-Cycloalkyl, Oxo (=O) und (CO)-(C₁-C₄)-Alkyl,
ii) (C₃-C₈)-Cycloalkyl, welches monosubstituiert ist durch NH₂,
b) eine spirobicyclische (C₉)-Heterocycloalkylgruppe mit einem Stickstoffatom, welche über ein Kohlenstoffatom gebunden ist,
c) eine verbrückte bicyclische (C₈)-Heterocycloalkylgruppe mit einem Stickstoffatom, welche über ein Kohlenstoffatom gebunden ist,
d) eine tricyclische (C₁₄)-Heterocycloalkylgruppe mit einem Stickstoffatom, welches über ein Kohlenstoffatom gebunden ist und welche aus einem spirobicyclischen Ring mit einem zusätzlichen kondensierten Phenylring besteht,
steht, oder
R³ und R⁴ zusammen mit dem Stickstoffatom, das sie trägt, für
a) ein 1,4-Piperazinyl der Formel 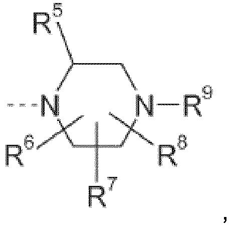 wobei
R⁵ für H oder (C₁-C₆)-Alkyl steht,
R⁶ für H oder (C₁-C₆)-Alkyl steht,
R⁷ für H oder (C₁-C₆)-Alkyl steht,
R⁸ für H oder Oxo (=O) steht,
R⁹ für H, (C₁-C₆)-Alkyl, (C₃-C₆)-Cycloalkyl oder CO-R¹⁰ steht, wobei (C₁-C₆)-Alkyl unsubstituiert ist oder monosubstituiert ist durch O-(C₁-C₄)-Alkyl,
R¹⁰ für (C₁-C₄)-Alkyl, (C₃-C₆)-Cycloalkyl, einen 5- bis 6-gliedrigen aromatischen oder aliphatischen heterocyclischen Ring mit einem Heteroatom ausgewählt aus Stickstoff und Sauerstoff steht,
b) 1-Azetidinyl, welches monosubstituiert ist durch NH₂,
c) 1-Pyrrolidinyl, welches monosubstituiert ist durch NH₂, NH-(C₁-C₄)-Alkyl, NH(C₁-C₄)-Alkyl)₂, (C₀-C₂)-Alkylen-pyrrolidinyl oder (C₀-C₂)-Piperidinyl, welches unsubstituiert ist oder monosubstituiert ist durch (C₁-C₄)-Alkyl,
d) 1-Piperidyl, welches monosubstituiert ist durch NH₂,
e) 1,4-Diazepanyl der Formel 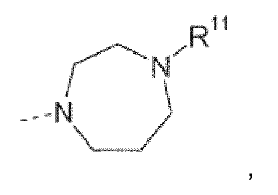 wobei
R¹¹ für H, (C₁-C₆)-Alkyl oder CO-(C₁-C₄)-Alkyl steht,
f) eine kondensierte bicyclische (C₈-C₁₀)-Heterocylcoalkylgruppe mit zwei Stickstoffatomen, welche über ein Stickstoff verbunden ist,
g) eine spirobicyclische (C₈-C₁₁)-Heterocycloalkylgruppe mit zwei Stickstoffatomen, welche über ein Stickstoffatom gebunden ist und welche ein weiteres Sauerstoffatom enthalten kann,
h) eine tricyclische (C₁₄)-Heterocycloalkylgruppe mit zwei Stickstoffatomen, welche über ein Stickstoffatom gebunden ist und welche aus einem spirobicyclischen Ring mit einem zusätzlichen kondensierten Phenylring besteht,
stehen.

4. Verbindung der Formel I nach einem der Ansprüche 1 bis 3,
wobei
R¹ für H oder F steht,
R² für H steht,
R³ für H oder CH₃ steht,
R⁴ für
a) (C₀-C₁)-Alkyl, welches monosubstituiert ist durch
i) Azetidyl, Pyrrolidinyl oder Piperidyl, welche unsubstituiert sind oder substituiert sind durch einen bis vier gleiche oder verschiedene Substituenten ausgewählt aus der Gruppe bestehend aus CH₃, C₂H₅, O-CH₃, Methylen-phenyl, Methylen-pyridyl, Cyclohexyl, Oxo (=O) und (CO)-CH₃,
ii) (C₃-C₆)-Cycloalkyl, welches monosubstituiert ist durch NH₂,
b) einen spirobicyclischen Ring 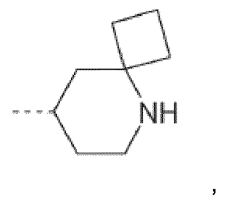
c) einen verbrückten bicyclischen Ring ausgewählt aus der Gruppe bestehend aus 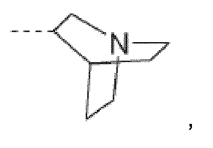
d) einen spirobicyclischen Ring mit einem kondensierten Ring ausgewählt aus der Gruppe bestehend aus 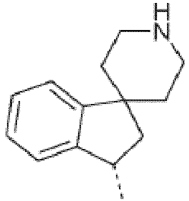 steht oder
R³ und R⁴ zusammen mit dem Stickstoffatom, das sie trägt, für
a) ein 1,4-Piperazinyl der Formel 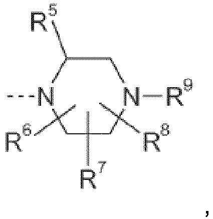 wobei
R⁵ für H oder CH₃ steht,
R⁶ für H oder CH₃ steht,
R⁷ für H oder (C₁-C₄)-Alkyl steht,
R⁸ für H oder Oxo (=O) steht,
R⁹ für H, (C₁-C₃)-Alkyl, Cycloheptyl oder CO-R¹⁰ steht, wobei (C₁-C₃)-Alkyl unsubstituiert ist oder monosubstituiert ist durch O-CH₃,
R¹⁰ für CH₃, Cyclopropyl, 2-Furyl oder 3-Pyridyl steht,
b) 1-Azetidinyl, welches monosubstituiert ist durch NH₂,
c) 1-Pyrrolidinyl, welches monosubstituiert ist durch NH₂, NH(CH₃), NH(CH₃)₂, Methylen-1-pyrrolidinyl oder 1-Piperidinyl-4-methyl,
d) 1-Piperidyl, welches monosubstituiert ist durch NH₂,
e) ein 1,4-Diazepanyl der Formel 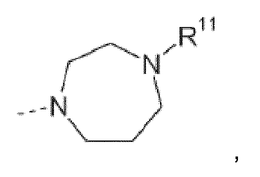 wobei
R¹¹ für H, Ethyl oder CO-CH₃ steht,
f) einen kondensierten bicyclischen Ring ausgewählt aus der Gruppe bestehend aus 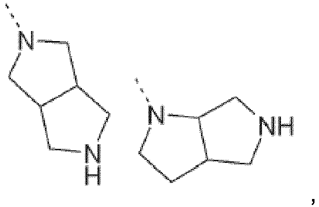
g) einen spirobicyclischen Ring ausgewählt aus der Gruppe bestehend aus 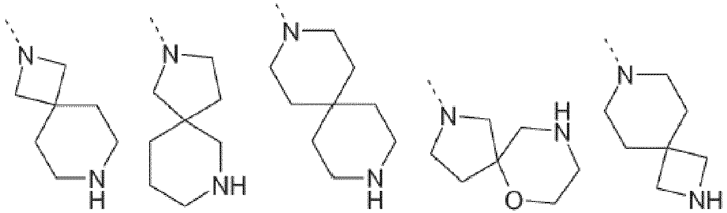 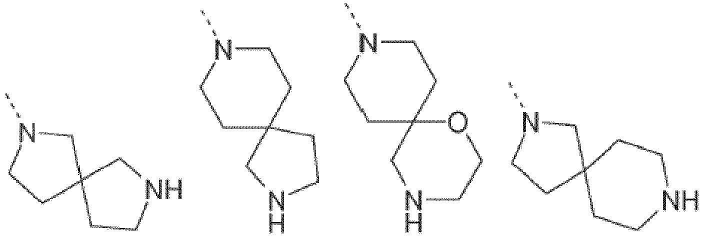 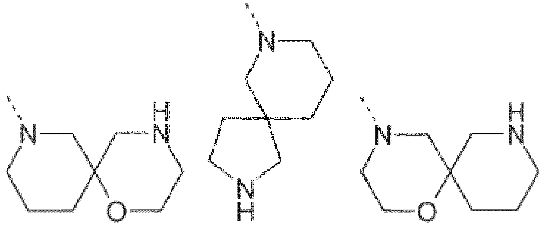
h) einen verbrückten bicyclischen Ring ausgewählt aus der Gruppe bestehend aus 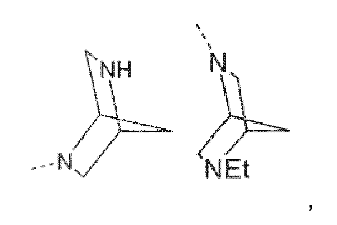
i) einen spirobicyclischen Ring mit einem kondensierten Ring ausgewählt aus der Gruppe bestehend aus 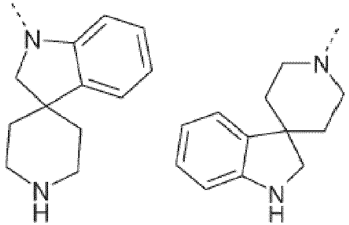 stehen.

5. Verbindung der Formel I nach einem der Ansprüche 1 bis 4,
wobei
R¹ für H oder F steht,
R² für H steht,
R³ für H oder CH₃ steht,
R⁴ für
a) Methylen, welches monosubstituiert ist durch
i) Azetidyl oder Pyrrolidinyl, welche über ein Kohlenstoffatom gebunden sind und welche unsubstituiert sind oder substituiert sind durch Oxo (=O),
ii) Cyclohexyl, welches monosubstituiert ist durch NH₂,
b) Azetidyl, welches über ein Kohlenstoffatom gebunden ist und welches unsubstituiert ist oder substituiert ist durch CH₃,
c) Pyrrolidinyl, welches über ein Kohlenstoffatom gebunden ist und welches unsubstituiert ist oder substituiert ist durch O-CH₃,
d) Piperidyl, welches über ein Kohlenstoffatom gebunden ist und welches unsubstituiert ist oder substituiert ist durch einen bis vier gleiche oder verschiedene Substituenten ausgewählt aus der Gruppe bestehend aus CH₃, C₂H₅, Methylen-phenyl, Methylenpyridyl, Cyclohexyl, Oxo (=O) und (CO)-CH₃,
e) (C₃-C₆)-Cycloalkyl, welches monosubstituiert ist durch NH₂,
f) einen spirobicyclischen Ring 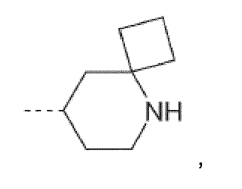
g) einen verbrückten bicyclischen Ring ausgewählt aus der Gruppe bestehend aus 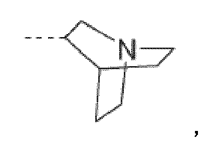
h) einen spirobicyclischen Ring mit einem kondensierten Ring ausgewählt aus der Gruppe bestehend aus 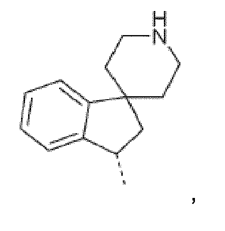 steht oder
R³ und R⁴ zusammen mit dem Stickstoffatom, das sie trägt, für
a) ein 1,4-Piperazinyl der Formel 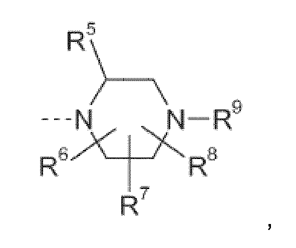 wobei
R⁵ für H oder CH₃ steht,
R⁶ für H oder CH₃ steht,
R⁷ für H oder (C₁-C₄)-Alkyl steht,
R⁸ für H oder Oxo (=O) steht,
R⁹ für H, (C₁-C₃)-Alkyl, Cycloheptyl oder CO-R¹⁰ steht, wobei (C₁-C₃)-Alkyl unsubstituiert ist oder monosubstituiert ist durch O-CH₃,
R¹⁰ für CH₃, Cyclopropyl, 2-Furyl oder 3-Pyridyl steht,
b) 1-Azetidinyl, welches monosubstituiert ist durch NH₂,
c) 1-Pyrrolidinyl, welches monosubstituiert ist durch NH₂, NH(CH₃), NH(CH₃)₂, Methylen-1-pyrrolidinyl oder 1-Piperidinyl-4-methyl,
d) 1-Piperidyl, welches monosubstituiert ist durch NH₂,
e) ein 1,4-Diazepanyl der Formel 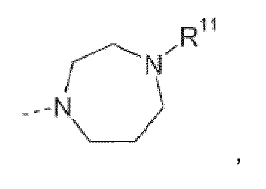 wobei
R¹¹ für H, Ethyl oder CO-CH₃ steht,
f) einen kondensierten bicyclischen Ring ausgewählt aus der Gruppe bestehend aus 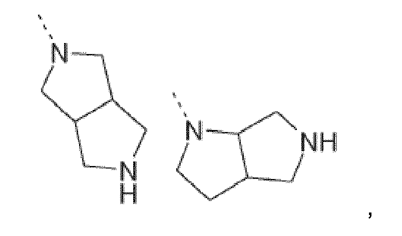
g) einen spirobicyclischen Ring ausgewählt aus der Gruppe bestehend aus 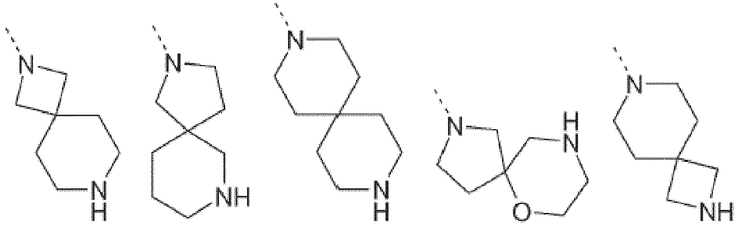 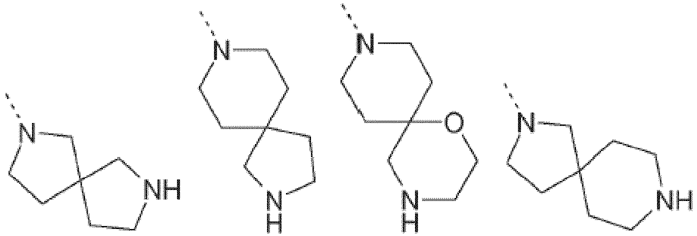 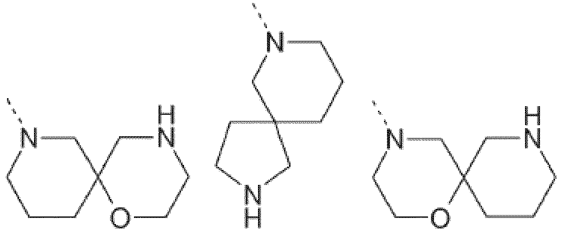
h) einen verbrückten bicyclischen Ring ausgewählt aus der Gruppe bestehend aus 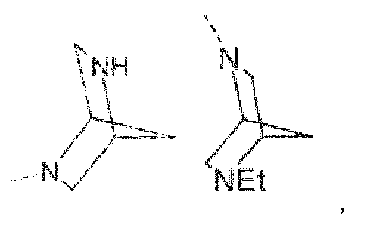
i) einen spirobicyclischen Ring mit einem kondensierten Ring ausgewählt aus der Gruppe bestehend aus 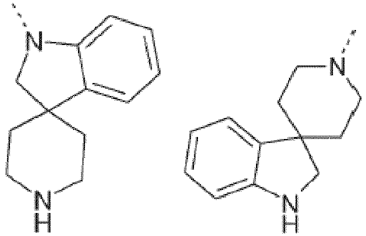 stehen.

6. Verbindung der Formel I nach einem der Ansprüche 1 bis 5,
wobei
R¹ für H oder F steht,
R² für H steht,
R³ für H oder CH₃ steht,
R⁴ für
a) Methylen, welches monosubstituiert ist durch
i) 3-Azetidyl,
ii) Cyclohexyl, welches monosubstituiert ist durch NH₂,
b) Azetidyl, welches über ein Kohlenstoffatom gebunden ist,
c) Pyrrolidinyl, welches über ein Kohlenstoffatom gebunden ist,
d) Piperidyl, welches über ein Kohlenstoffatom gebunden ist und welches unsubstituiert ist oder substituiert ist durch einen Substituenten ausgewählt aus der Gruppe bestehend aus CH₃, C₂H₅, Methylen-phenyl, Methylen-pyridyl und Cyclohexyl,
e) Cyclohexyl, welches monosubstituiert ist durch NH₂,
f) einen spirobicyclischen Ring 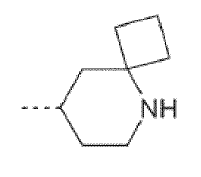 steht oder
R³ und R⁴ zusammen mit dem Stickstoffatom, das sie trägt, für
a) ein 1,4-Piperazinyl der Formel 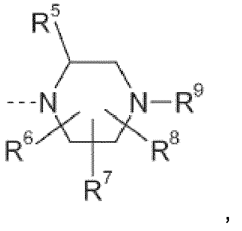 wobei
R⁵ für H oder CH₃ steht,
R⁶ für H oder CH₃ steht,
R⁷ für H oder (C₁-C₄)-Alkyl steht,
R⁸ für H steht,
R⁹ für H oder (C₁-C₃)-Alkyl steht,
b) 1-Azetidinyl, welches monosubstituiert ist durch NH₂,
c) 1-Pyrrolidinyl, welches monosubstituiert ist durch NH₂, NH(CH₃), NH(CH₃)₂, Methylen-1-pyrrolidinyl oder 1-Piperidinyl-4-methyl,
d) 1-Piperidyl, welches monosubstituiert ist durch NH₂,
e) ein 1,4-Diazepanyl der Formel 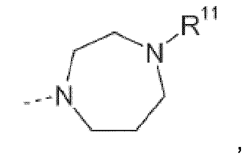 wobei
R¹¹ für H oder Ethyl steht,
f) einen kondensierten bicyclischen Ring ausgewählt aus der Gruppe bestehend aus 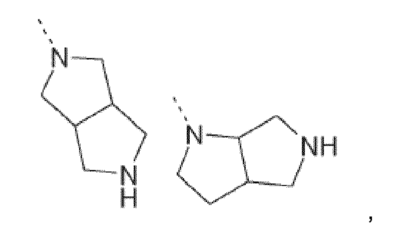
g) einen spirobicyclischen Ring ausgewählt aus der Gruppe bestehend aus 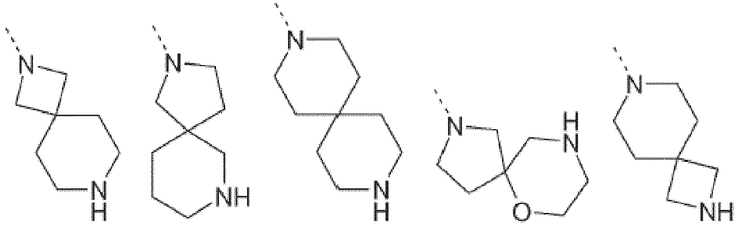 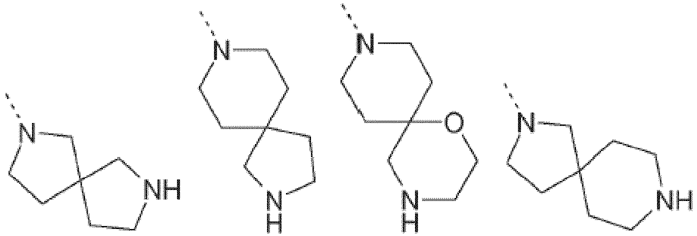 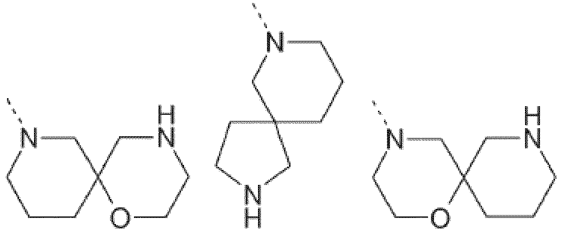 stehen.

7. Verbindung der Formel I nach einem der Ansprüche 1 bis 6,
wobei
R¹ für H steht,
R² für H steht,
R³ für H steht,
R⁴ für
a) Methylen, welches monosubstituiert ist durch
i) 3-Azetidyl,
ii) 4-Aminocyclohexyl,
b) 3-Azetidyl,
c) 3-Pyrrolidinyl,
d) 3-Piperidyl oder 4-Piperidyl, welche unsubstituiert sind oder am Stickstoffatom substituiert sind durch einen Substituenten ausgewählt aus der Gruppe bestehend aus CH₃, C₂H₅, Methylenphenyl, Methylen-pyridyl und Cyclohexyl,
e) 3-Aminocyclohexyl oder 4-Aminocyclohexyl,
f) einen spirobicyclischen Ring 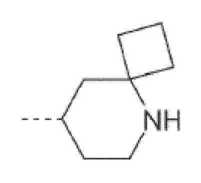 steht oder
R³ und R⁴ zusammen mit dem Stickstoffatom, das sie trägt, für
a) ein 1,4-Piperazinyl der Formel 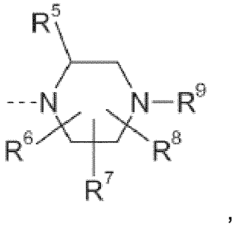 wobei
R⁵ für H oder CH₃ steht,
R⁶ für H oder CH₃ steht,
R⁷ für H oder Isopropyl steht,
R⁸ für H steht,
R⁹ für H oder (C₁-C₃)-Alkyl steht,
b) 3-Amino-1-azetidinyl,
c) 1-Pyrrolidinyl, welches in der 3-Position monosubstituiert ist durch NH₂, NH(CH₃), NH(CH₃)₂ oder 1-Piperidinyl-4-methyl,
d) 4-Amino-1-piperidyl,
e) ein 1,4-Diazepanyl der Formel 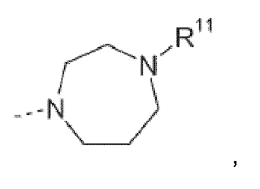 wobei
R¹¹ für H oder Ethyl steht,
f) einen kondensierten bicyclischen Ring ausgewählt aus der Gruppe bestehend aus 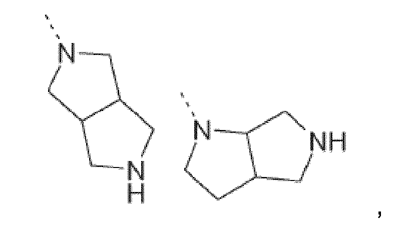
g) einen spirobicyclischen Ring ausgewählt aus der Gruppe bestehend aus 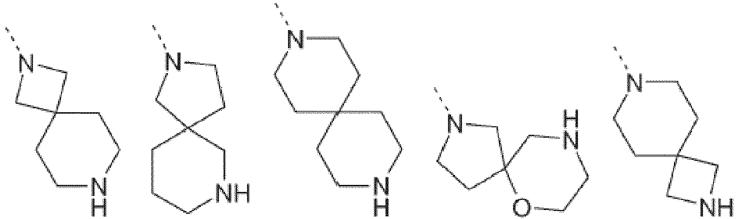 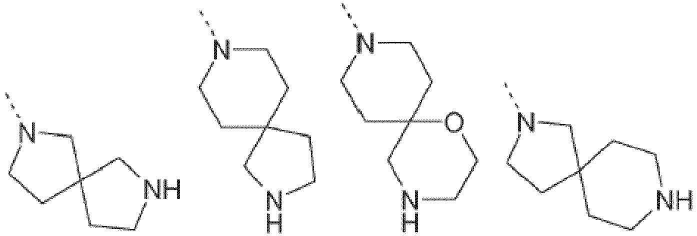 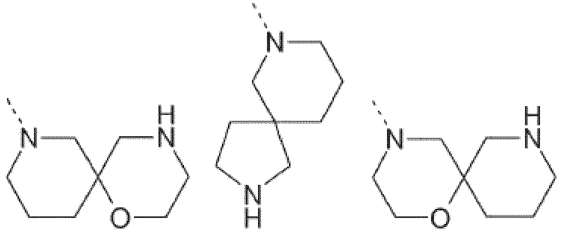 stehen.

8. Verbindung nach Anspruch 1, ausgewählt aus der Gruppe bestehend aus
| | |
|---|---|
| 1 | 6-(4-Hydroxyphenyl)-1H-pyrazolo[3,4-b]pyridin-4-carbonsäure-(2,2,6,6-tetramethylpiperidin-4-yl)amid |
| 2 | 6-(4-Hydroxyphenyl)-1H-pyrazolo[3,4-b]pyridin-4-carbonsäure-piperidin-4-ylamid |
| 3 | (3-Aminopiperidin-1-yl)-[6-(4-hydroxyphenyl)-1H-pyrazolo[3,4-b]pyridin-4-yl]methanon |
| 4 | [6-(4-Hydroxyphenyl)-1H-pyrazolo[3,4-b]pyridin-4-yl]piperazin-1-ylmethanon |
| 5 | 6-(4-Hydroxyphenyl)-1H-pyrazolo[3,4-b]pyridin-4-carbonsäure-pyrrolidin-3-ylamid |
| 6 | 6-(4-Hydroxyphenyl)-1H-pyrazolo[3,4-b]pyridin-4-carbonsäure-((3S,4S)-4-methoxypyrrolidin-3-yl)amid |
| 7 | [1,4]Diazepan-1-yl-[6-(4-hydroxyphenyl)-1H-pyrazolo[3,4-b]pyridin-4-yl]methanon |
| 8 | 6-(4-Hydroxyphenyl)-1H-pyrazolo[3,4-b]pyridin-4-carbonsäure(cis-4-aminocyclohexyl)amid |
| 9 | 6-(4-Hydroxyphenyl)-1H-pyrazolo[3,4-b]pyridin-4-carbonsäure(trans-4-aminocyclohexyl)amid |
| 10 | (2,2-Dimethylpiperazin-1-yl)-[6-(4-hydroxyphenyl)-1H-pyrazolo[3,4-b]pyridin-4-yl]methanon |
| 11 | 6-(4-Hydroxyphenyl)-1H-pyrazolo[3,4-b]pyridin-4-carbonsäure(trans-4-aminocyclohexylmethyl)amid |
| 12 | 6-(4-Hydroxyphenyl)-1H-pyrazolo[3,4-b]pyridin-4-carbonsäure(cis-4-aminocyclohexylmethyl)amid |
| 13 | 6-(4-Hydroxyphenyl)-1H-pyrazolo[3,4-b]pyridin-4-carbonsäure(azetidin-3-ylmethyl)amid |
| 14 | 6-(4-Hydroxyphenyl)-1H-pyrazolo[3,4-b]pyridin-4-carbonsäure-(3-aminocyclobutyl)amid |
| 15 | [6-(4-Hydroxyphenyl)-1H-pyrazolo[3,4-b]pyridin-4-yl]-(5-isopropyl-2,2-dimethylpiperazin-1-yl)methanon |
| 16 | ((R)-3-Aminopyrrolidin-1-yl)-[6-(4-hydroxyphenyl)-1H-pyrazolo[3,4-b]pyridin-4-yl]methanon |
| 17 | ((S)-3-Aminopyrrolidin-1-yl)-[6-(4-hydroxyphenyl)-1H-pyrazolo[3,4-b]pyridin-4-yl]methanon |
| 18 | 6-(4-Hydroxyphenyl)-1H-pyrazolo[3,4-b]pyridin-4-carbonsäure-(R)-(1-azabicyclo[2.2.2]oct-3-yl)amid |
| 19 | 6-(4-Hydroxyphenyl)-1H-pyrazolo[3,4-b]pyridin-4-carbonsäure-(R)-piperidin-3-ylamid |
| 20 | (2,7-Diazaspiro[3.5]non-2-yl)-[6-(4-hydroxyphenyl)-1H-pyrazolo[3,4-b]pyridin-4-yl]methanon |
| 21 | (2,7-Diazaspiro[3.5]non-7-yl)-[6-(4-hydroxyphenyl)-1H-pyrazolo[3,4-b]pyridin-4-yl]methanon |
| 22 | (2,8-Diazaspiro[4.5]dec-2-yl)-[6-(4-hydroxyphenyl)-1H-pyrazolo[3,4-b]pyridin-4-yl]methanon |
| 23 | (2,7-Diazaspiro[4.5]dec-2-yl)-[6-(4-hydroxyphenyl)-1H-pyrazolo[3,4-b]pyridin-4-yl]methanon |
| 24 | (2,7-Diazaspiro[4.5]dec-7-yl)-[6-(4-hydroxyphenyl)-1H-pyrazolo[3,4-b]pyridin-4-yl]methanon |
| 25 | (2,7-Diazaspiro[4.4]non-2-yl)-[6-(4-hydroxyphenyl)-1H-pyrazolo[3,4-b]pyridin-4-yl]methanon |
| 26 | [6-(4-Hydroxyphenyl)-1H-pyrazolo[3,4-b]pyridin-4-yl]-(1-oxa-4,9-diazaspiro[5.5]undec-9-yl)methanon |
| 27 | [6-(4-Hydroxyphenyl)-1H-pyrazolo[3,4-b]pyridin-4-yl]-(1-oxa-4,8-diazaspiro[5.5]undec-4-yl)methanon |
| 28 | [6-(4-Hydroxyphenyl)-1H-pyrazolo[3,4-b]pyridin-4-yl]-(1-oxa-4,8-diazaspiro[5.5]undec-8-yl)methanon |
| 29 | 6-(4-Hydroxyphenyl)-1H-pyrazolo[3,4-b]pyridin-4-carbonsäure(trans-4-methoxypyrrolidin-3-yl)amid |
| 30 | 6-(4-Hydroxyphenyl)-1H-pyrazolo[3,4-b]pyridin-4-carbonsäure(trans-3-aminocyclobutyl)amid |
| 31 | [6-(4-Hydroxyphenyl)-1H-pyrazolo[3,4-b]pyridin-4-yl]-(6-oxa-2,9-diazaspiro[4.5]dec-2-yl)methanon |
| 32 | 6-(4-Hydroxyphenyl)-1H-pyrazolo[3,4-b]pyridin-4-carbonsäure-(5-azaspiro[3.5]non-8-yl)amid |
| 33 | 6-(4-Hydroxyphenyl)-1H-pyrazolo[3,4-b]pyridin-4-carbonsäure-(3-aminocyclohexyl)amid |
| 34 | (Hexahydropyrrolo[3,4-b]pyrrol-1-yl)-[6-(4-hydroxyphenyl)-1H-pyrazolo[3,4-b]pyridin-4-yl]methanon |
| 35 | 6-(4-Hydroxyphenyl)-1H-pyrazolo[3,4-b]pyridin-4-carbonsäure-azetidin-3-ylamid |
| 36 | (3-Aminoazetidin-1-yl)-[6-(4-hydroxyphenyl)-1H-pyrazolo[3,4-b]pyridin-4-yl]methanon |
| 37 | [6-(4-Hydroxyphenyl)-1H-pyrazolo[3,4-b]pyridin-4-yl]-(3-methylpiperazin-1-yl)methanon |
| 38 | (4-Aminopiperidin-1-yl)-[6-(4-hydroxyphenyl)-1H-pyrazolo[3,4-b]pyridin-4-yl]methanon |
| 39 | [6-(4-Hydroxyphenyl)-1H-pyrazolo[3,4-b]pyridin-4-yl]-(3-methylaminopyrrolidin-1-yl)methanon |
| 40 | 6-(4-Hydroxyphenyl)-1H-pyrazolo[3,4-b]pyridin-4-carbonsäure-(S)-(1-azabicyclo[2.2.2]oct-3-yl)amid |
| 41 | 6-(4-Hydroxyphenyl)-1H-pyrazolo[3,4-b]pyridin-4-carbonsäure-(1-methylpiperidin-3-yl)amid |
| 42 | 6-(4-Hydroxyphenyl)-1H-pyrazolo[3,4-b]pyridin-4-carbonsäure-methylpiperidin-4-ylamid |
| 43 | (2,8-Diazaspiro[4.5]dec-8-yl)-[6-(4-hydroxyphenyl)-1H-pyrazolo[3,4-b]pyridin-4-yl]methanon |
| 44 | (3,9-Diazaspiro[5.5]undec-3-yl)-[6-(4-hydroxyphenyl)-1H-pyrazolo[3,4-b]pyridin-4-yl]methanon |
| 45 | (3-Aminopyrrolidin-1-yl)-[6-(4-hydroxyphenyl)-1H-pyrazolo[3,4-b]pyridin-4-yl]methanon |
| 46 | (Hexahydropyrrolo[3,4-c]pyrrol-2-yl)-[6-(4-hydroxyphenyl)-1H-pyrazolo[3,4-b]pyridin-4-yl]methanon |
| 47 | 2,5-Diazabicyclo[2.2.1]hept-2-yl-[6-(4-hydroxyphenyl)-1H-pyrazolo[3,4-b]pyridin-4-yl]methanon |
| 48 | 6-(4-Hydroxyphenyl)-1H-pyrazolo[3,4-b]pyridin-4-carbonsäure-(2,3-dihydrospiro[1H-inden-1,4'-piperidin]-3-yl)amid |
| 49 | 6-(4-Hydroxyphenyl)-1H-pyrazolo[3,4-b]pyridin-4-carbonsäure-(1,2-dihydrospiro[3H-indol-3,4'-piperidin]-1'-yl)amid |
| 50 | 6-(4-Hydroxyphenyl)-1H-pyrazolo[3,4-b]pyridin-4-carbonsäure-(2,3-dihydrospiro[1 H-inden-1,4'-piperidin]-3-yl)amid |
| 51 | 6-(4-Hydroxyphenyl)-1H-pyrazolo[3,4-b]pyridin-4-carbonsäure(trans-2-aminocyclopropyl)amid |
| 52 | [6-(4-Hydroxyphenyl)-1H-pyrazolo[3,4-b]pyridin-4-yl]-(4-methylpiperazin-1-yl)methanon |
| 53 | 1-{4-[6-(4-Hydroxyphenyl)-1H-pyrazolo[3,4-b]pyridin-4-carbonyl]piperazin-1-yl}ethanon |
| 54 | [6-(4-Hydroxyphenyl)-1H-pyrazolo[3,4-b]pyridin-4-yl]-((S)-2-pyrrolidin-1-ylmethylpyrrolidin-1-yl)methanon |
| 55 | ((S)-3-Dimethylaminopyrrolidin-1-yl)-[6-(4-hydroxyphenyl)-1H-pyrazolo[3,4-b]pyridin-4-yl]methanon |
| 56 | [6-(4-Hydroxyphenyl)-1H-pyrazolo[3,4-b]pyridin-4-yl]-(4-isopropylpiperazin-1-yl)methanon |
| 57 | (4-Cyclopropancarbonylpiperazin-1-yl)-[6-(4-hydroxyphenyl)-1H-pyrazolo[3,4-b]pyridin-4-yl]methanon |
| 58 | (4-Cycloheptylpiperazin-1-yl)-[6-(4-hydroxyphenyl)-1H-pyrazolo[3,4-b]pyridin-4-yl]methanon |
| 59 | 1-{4-[6-(4-Hydroxyphenyl)-1H-pyrazolo[3,4-b]pyridin-4-carbonyl]-[1,4]diazepan-1-yl}ethanon |
| 60 | 6-(4-Hydroxyphenyl)-1H-pyrazolo[3,4-b]pyridin-4-carbonsäure-(1-ethylpiperidin-3-yl)amid |
| 61 | [6-(4-Hydroxyphenyl)-1H-pyrazolo[3,4-b]pyridin-4-yl]-[4-(tetrahydrofuran-2-carbonyl)piperazin-1-yl]methanon |
| 62 | 6-(4-Hydroxyphenyl)-1H-pyrazolo[3,4-b]pyridin-4-carbonsäure-(1-methylazetidin-3-yl)amid |
| 63 | 6-(4-Hydroxyphenyl)-1H-pyrazolo[3,4-b]pyridin-4-carbonsäure-(5-oxopyrrolidin-2-ylmethyl)amid |
| 64 | (4-Ethyl-[1,4]diazepan-1-yl)-[6-(4-hydroxyphenyl)-1H-pyrazolo[3,4-b]pyridin-4-yl]methanon |
| 65 | [6-(4-Hydroxyphenyl)-1H-pyrazolo[3,4-b]pyridin-4-yl]-[3-(4-methylpiperidin-1-yl)pyrrolidin-1-yl]methanon |
| 66 | 6-(4-Hydroxyphenyl)-1H-pyrazolo[3,4-b]pyridin-4-carbonsäure-(1-methylpiperidin-4-yl)amid |
| 67 | (6-4-Hydroxyphenyl)-1H-pyrazolo[3,4-b]pyridin-4-carbonsäure-(1-cyclohexylpiperidin-4-yl)amid |
| 68 | 6-(4-Hydroxyphenyl)-1H-pyrazolo[3,4-b]pyridin-4-carbonsäure-(1-acetylpiperidin-4-yl)amid |
| 69 | 6-(4-Hydroxyphenyl)-1H-pyrazolo[3,4-b]pyridin-4-carbonsäure-((R)-6-oxopiperidin-3-yl)amid |
| 70 | (5-Ethyl-2,5-diazabicyclo[2.2.1]hept-2-yl)-[6-(4-hydroxyphenyl)-1H-pyrazolo[3,4-b]pyridin-4-yl]methanon |
| 71 | [6-(4-Hydroxyphenyl)-1H-pyrazolo[3,4-b]pyridin-4-yl]-[4-(2-methoxyethyl)piperazin-1-yl]methanon |
| 72 | 6-(4-Hydroxyphenyl)-1H-pyrazolo[3,4-b]pyridin-4-carbonsäure-(1-azabicyclo[2.2.2]oct-3-yl)amid |
| 73 | 6-(4-Hydroxyphenyl)-1H-pyrazolo[3,4-b]pyridin-4-carbonsäure-methyl-(1-methylpiperidin-4-yl)amid |
| 74 | [6-(4-Hydroxyphenyl)-1H-pyrazolo[3,4-b]pyridin-4-yl]-[4-(pyridin-3-carbonyl)piperazin-1-yl]methanon |
| 75 | 6-(4-Hydroxyphenyl)-1H-pyrazolo[3,4-b]pyridin-4-carbonsäure-(1-pyridin-4-ylmethylpiperidin-4-yl)amid |
| 76 | 4-[6-(4-Hydroxyphenyl)-1H-pyrazolo[3,4-b]pyridin-4-carbonyl]-1-methylpiperazin-2-on |
| 77 | 6-(4-Hydroxyphenyl)-1H-pyrazolo[3,4-b]pyridin-4-carbonsäure-((R)-1-benzylpiperidin-3-yl)amid |
| 78 | 6-(4-Hydroxyphenyl)-1H-pyrazolo[3,4-b]pyridin-4-carbonsäure-(1-pyridin-3-ylmethylpiperidin-4-yl)amid |

9. Pharmazeutische Zusammensetzung, umfassend mindestens eine Verbindung der Formel I nach einem der Ansprüche 1 bis 8 oder ein physiologisch akzeptables Solvat einer dieser, zur Verwendung als Pharmazeutikum.

10. Verbindung der Formel I nach einem der Ansprüche 1 bis 8 oder ein physiologisch akzeptables Salz davon, oder ein physiologisch akzeptables Solvat einer dieser, zur Verwendung als Pharmazeutikum.

11. Verbindung der Formel I nach einem der Ansprüche 1 bis 8 oder ein physiologisch akzeptables Salz davon, oder ein physiologisch akzeptables Solvat einer dieser, zur Verwendung bei der Behandlung von Krankheiten, die mit Diabetes und diabetischen Komplikationen assoziiert sind.

12. Verbindung der Formel I nach einem der Ansprüche 1 bis 8 oder ein physiologisch akzeptables Salz davon, oder ein physiologisch akzeptables Solvat einer dieser, zur Verwendung bei der Prävention und Behandlung von Nephropathie, Neuropathie, Retinopathie, Ischämie, Entzündung, Erkrankungen des zentralen Nervensystems, Herz-Kreislauf-Krankheiten, dermatologischen Krankheiten, Autoimmunkrankheiten und Krebs.

13. Verbindung der Formel I nach einem der Ansprüche 1 bis 8 oder ein physiologisch akzeptables Salz davon, oder ein physiologisch akzeptables Solvat einer dieser, zur Verwendung bei der Behandlung einer mit dem PKC-Rezeptor assoziierten Krankheit.

## Revendications

1. Composé de formule I 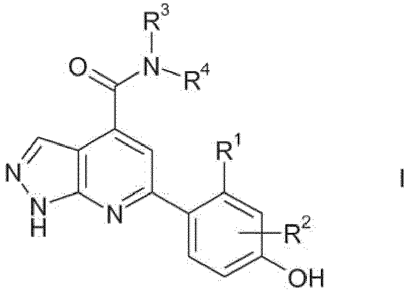 dans lequel
R¹ est H, halogène ou alkyle en C₁-C₄;
R² est H, halogène ou alkyle en C₁-C₄;
R³ est H ou alkyle en C₁-C₄;
R⁴ est
a) alkyle en C₀-C₆ qui est monosubstitué par
i) un hétérocycle monocyclique de 3 à 8 chaînons comprenant un atome d'azote cyclique et facultativement un hétéroatome cyclique supplémentaire choisi dans le groupe constitué de l'azote et de l'oxygène, qui est non substitué ou substitué par un à cinq substituants identiques ou différents choisis dans le groupe constitué de
ia)F,
ib)alkyle en C₁-C₄, qui est non substitué ou substitué une à cinq fois par F,
ic)O-(alkyle en C₁-C₄), qui est non substitué ou substitué une à cinq fois par F,
id)phényle, qui est non substitué ou substitué par un à deux substituants identiques ou différents choisis dans le groupe constitué d'halogène et alkyle en C₁-C₄, où l'alkyle en C₁-C₄ est non substitué ou substitué une à cinq fois par F,
ie)(alkylène en C₁-C₄)-phényle, qui est non substitué ou substitué une à cinq fois par F,
if) cycloalkyle en C₃-C₈,
ig)oxo (=O), et
ih)(CO)-(alkyle en C₁-C₄),
et où alkyle en C₀-C₆ peut en outre être monosubstitué par phényle ou pyridyle, où phényle ou pyridyle est non substitué ou substitué par un à deux substituants identiques ou différents choisis dans le groupe constitué d'halogène et alkyle en C₁-C₄, où alkyle en C₁-C₄ est non substitué ou substitué une à cinq fois par F ;
ii) cycloalkyle en C₃-C₈ qui est substitué par un à deux substituants identiques ou différents choisis dans le groupe constitué de NH₂, NH(alkyle en C₁-C₄) et N(alkyle en C₁-C₄)₂, et où cycloalkyle en C₃-C₈ peut en outre être substitué par un à trois substituants identiques ou différents choisis dans le groupe constitué de
iia) F,
iib) alkyle en C₁-C₄, où alkyle en C₁-C₄ est non substitué ou substitué une à cinq fois par F,
iic) O-(alkyle en C₁-C₄), qui est non substitué ou substitué une à cinq fois par F,
iid) phényle, qui est non substitué ou substitué par un à deux substituants identiques ou différents choisis dans le groupe constitué d'halogène et alkyle en C₁-C₄, où alkyle en C₁-C₄ est non substitué ou substitué une à cinq fois par F, et
iie) (alkylène en C₁-C₄)-phényle, qui est non substitué ou substitué une à cinq fois par F ;
ou
iii) NH₂, NH(alkyle en C₁-C₆), ou N(alkyle en C₁-C₆)₂,
et où alkyle en C₁-C₆ peut en outre être monosubstitué par phényle, phénylène-(alkyle en C₁-C₄) ou phénylène-O-(alkyle en C₁-C₄);
b) un groupe cycloalkyle en C₆-C₁₁ bicyclique, qui est monosubstitué par (alkylène en C₀-C₂)-NH₂, (alkylène en C₀-C₂)-NH-(alkyle en C₁-C₄), (alkylène en C₀-C₂)-N(alkyle en C₁-C₄)₂,
c) un groupe hétérocycloalkyle en C₆-C₁₀ bicyclique condensé contenant un atome d'azote, qui est lié par l'intermédiaire d'un atome de carbone et qui est non substitué ou substitué par un ou deux substituants identiques ou différents choisis dans le groupe constitué de F et alkyle en C₁-C₄, qui est non substitué ou substitué une à cinq fois par F ;
d) un groupe hétérocycloalkyle en C₇-C₁₁ spiro-bicyclique contenant un atome d'azote, qui est lié par l'intermédiaire d'un atome de carbone et qui est non substitué ou substitué par un ou deux substituants identiques ou différents choisis dans le groupe constitué de F et alkyle en C₁-C₄, qui est non substitué ou substitué une à cinq fois par F ;
e) un groupe hétérocycloalkyle en C₇-C₉ bicyclique ponté contenant un atome d'azote, qui est lié par l'intermédiaire d'un atome de carbone et qui est non substitué ou substitué par un ou deux substituants identiques ou différents choisis dans le groupe constitué de F et alkyle en C₁-C₄, qui est non substitué ou substitué une à cinq fois par F ; ou
f) un groupe hétérocycloalkyle en C₁₁-C₁₅ tricyclique contenant un atome d'azote, qui est lié par l'intermédiaire d'un atome de carbone et qui est constitué d'un anneau spiro-bicyclique avec un cycle phényle condensé additionnel, et qui est non substitué ou substitué par un ou deux substituants identiques ou différents choisis dans le groupe constitué de F et alkyle en C₁-C₄, qui est non substitué ou substitué une à cinq fois par F ;
ou
R³ et R⁴ conjointement avec l'atome N portant ceux-ci désignent
a) une 1,4-pipérazinyle de formule 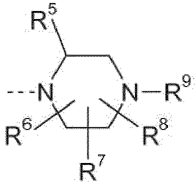 dans laquelle
R⁵ est H ou alkyle en C₀-C₆, qui est non substitué ou substitué une à cinq fois par F, ou monosubstitué par un substituant choisi dans le groupe constitué de
i) O-(alkyle en C₁-C₄), qui est non substitué ou substitué une à cinq fois par F,
ii) cycloalkyle en C₃-C₆,
iii) phényle, qui est non substitué ou substitué par un à deux substituants identiques ou différents choisis dans le groupe constitué d'halogène, CF₃, O-(alkyle en C₁-C₄), OCF₃, et alkyle en C₁-C₄,
iv) un cycle hétéroaromatique monocyclique de 5 à 6 chaînons comprenant un hétéroatome choisi dans le groupe constitué de l'azote, l'oxygène, et le soufre, qui est non substitué ou substitué par un à deux substituants identiques ou différents choisis dans le groupe constitué d'halogène, CF₃, O-(alkyle en C₁-C₄), OCF₃, et alkyle en C₁-C₄,
v) benzo[1,3]dioxole, et
vi) CO-O-(alkyle en C₁-C₄) ou CO- NH-(alkyle en C₁-C₆) ;
R⁶ est H ou alkyle en C₀-C₆, qui est non substitué ou substitué une à cinq fois par F ou monosubstitué par un substituant choisi dans le groupe constitué de
i) CO-O-(alkyle en C₁-C₄) ;
ii) cycloalkyle en C₃-C₆,
iii) phényle, qui est non substitué ou substitué par un à deux substituants identiques ou différents choisis dans le groupe constitué d'halogène, CF₃, O-(alkyle en C₁-C₄), OCF₃, et alkyle en C₁-C₄,
iv) O-(alkyle en C₁-C₄), qui est non substitué ou substitué une à cinq fois par F, et
v) un cycle hétéroaromatique monocyclique de 5 à 6 chaînons comprenant un hétéroatome choisi dans le groupe constitué de l'azote, l'oxygène et le soufre, qui est non substitué ou substitué par un à deux substituants identiques ou différents choisis dans le groupe constitué d'halogène, CF₃, O-(alkyle en C₁-C₄), OCF₃, et alkyle en C₁-C₄ ;
R⁷ est H, alkyle en C₁-C₆, qui est non substitué ou substitué une à cinq fois par F, ou phényle;
R⁸ est H, alkyle en C₁-C₆ ou oxo (=0) ;
R⁹ est H, alkyle en C₁-C₆, cycloalkyle en C₃-C₆ ou CO-R¹⁰, où alkyle en C₁-C₆ est non substitué ou substitué une à cinq fois par F, ou monosubstitué par un substituant choisi dans le groupe constitué de O-(alkyle en C₁-C₄), SO₂-(alkyle en C₁-C₄), phényle, un cycle monocyclique hétérocyclique de 5 à 6 chaînons comprenant un hétéroatome choisi dans le groupe constitué de l'azote et de l'oxygène, un cycle hétéroaromatique monocyclique de 5 à 6 chaînons comprenant un hétéroatome choisi dans le groupe constitué de l'azote et de l'oxygène; où R¹⁰ est alkyle en C₁-C₄, cycloalkyle en C₃-C₆, NH₂, phényle, un cycle monocyclique hétérocyclique de 5 à 6 chaînons comprenant un hétéroatome choisi dans le groupe constitué de l'azote et de l'oxygène, ou un cycle hétéroaromatique monocyclique de 5 à 6 chaînons comprenant un hétéroatome choisi dans le groupe constitué de l'azote et de l'oxygène, où le phényle peut en outre être monosubstitué par alkyle en C₁-C₄ ou O-(alkyle en C₁-C₄) ;
b) un groupe hétérocycloalkyle monocyclique de quatre à sept chaînons contenant un atome d'azote, qui est lié par l'intermédiaire dudit azote et qui est monosubstitué par (alkylène en C₀-C₆)-NH₂, (alkylène en C₀-C₆)-NH-(alkyle en C₁-C₄), (alkylène en C₀-C₆)-NH-phényle, (alkylène en C₀-C₆)-N(alkyle en C₁-C₄)₂, (alkylène en C₀-C₆)-N(alkyle en C₁-C₄)(phényle), (alkylène en C₀-C₂)-azétidinyle, (alkylène en C₀-C₂)-pyrrolidinyle, ou (alkylène en C₀-C₂)-pipéridyle ; où ledit groupe hétérocycloalkyle peut en outre être monosubstitué par alkyle en C₁-C₆, qui est non substitué ou substitué une à cinq fois par F, CO-O-(alkyle en C₁-C₄) ou phényle, et où ledit groupe azétidinyle, pyrrolidinyle et pipéridyle peut en outre être monosubstitué par alkyle en C₁-C₆, qui est non substitué ou substitué une à cinq fois par F ;
c) un 1,4-diazépanyle, qui est non substitué ou monosubstitué par un substituant choisi dans le groupe constitué de
i) alkyle en C₁-C₆, où alkyle en C₁-C₆ est non substitué ou substitué une à cinq fois par F,
ii) CO-(alkyle en C₁-C₆), où alkyle en C₁-C₆ est non substitué ou substitué une à cinq fois par F,
ii) CO-phényle, et
iv) CO-pyridyle ;
d) un groupe hétérocycloalkyle en C₆-C₁₀ bicyclique condensé contenant deux atomes d'azote, qui est lié par l'intermédiaire d'un atome d'azote et qui peut contenir un hétéroatome supplémentaire choisi dans le groupe constitué de l'azote, de l'oxygène et du soufre, où ledit groupe hétérocycloalkyle est non substitué ou substitué par un à deux substituants identiques ou différents choisis dans le groupe constitué de F, (alkylène en C₀-C₂)-phényle, oxo (=O), et alkyle en C₁-C₄, où alkyle en C₁-C₄ est non substitué ou substitué une à cinq fois par F ;
e) un groupe hétérocycloalkyle en C₇-C₁₁ spiro-bicyclique contenant deux atomes d'azote, qui est lié par l'intermédiaire d'un atome d'azote et qui peut contenir un hétéroatome supplémentaire choisi dans le groupe constitué de l'azote, de l'oxygène et du soufre, où ledit groupe hétérocycloalkyle est non substitué ou mono- ou di-substitué par F ou alkyle en C₁-C₄, qui est non substitué ou substitué une à cinq fois par F ;
f) un groupe hétérocycloalkyle en C₇-C₉ bicyclique ponté contenant un atome d'azote, qui est lié par l'intermédiaire dudit atome d'azote, qui est monosubstitué par (alkylène en C₀-C₂)-NH₂, (alkylène en C₀-C₂)-NH-(alkyle en C₁-C₄), (alkylène en C₀-C₂)-N(alkyle en C₁-C₄)₂
g) un groupe hétérocycloalkyle en C₇-C₉ bicyclique ponté contenant deux atomes d'azote, qui est lié par l'intermédiaire d'un atome d'azote; et qui est non substitué ou substitué par un à quatre substituants identiques ou différents choisis dans le groupe constitué de F, OH, et alkyle en C₁-C₄, qui est non substitué ou substitué une à cinq fois par F ; ou
h) un groupe hétérocycloalkyle en C₁₁-C₁₅ tricyclique contenant deux atomes d'azote, qui est lié par l'intermédiaire d'un atome d'azote et qui est constitué d'un anneau spiro-bicyclique avec un cycle phényle condensé additionnel, et qui est non substitué ou substitué par un ou deux substituants identiques ou différents choisis dans le groupe constitué de F et alkyle en C₁-C₄, qui est non substitué ou substitué une à cinq fois par F ; sous l'une quelconque de ses formes stéréoisomères, ou un mélange de formes stéréoisomères dans un rapport quelconque, ou un sel physiologiquement acceptable de celui-ci, ou un solvate physiologiquement acceptable de l'un quelconque de ceux-ci.

2. Composé de formule I selon la revendication 1,
dans lequel
R¹ est H, halogène ou alkyle en C₁-C₄;
R² est H ou halogène;
R³ est H ou alkyle en C₁-C₄;
R⁴est
a) alkyle en C₀-C₄ qui est monosubstitué par
i) un hétérocycle monocyclique de 3 à 8 chaînons comprenant un atome d'azote cyclique et facultativement un hétéroatome cyclique supplémentaire choisi dans le groupe constitué de l'azote et de l'oxygène, qui est non substitué ou substitué par un à cinq substituants identiques ou différents choisis dans le groupe constitué de
ia)F,
ib)alkyle en C₁-C₄, qui est non substitué ou substitué une à cinq fois par F,
ic)O-(alkyle en C₁-C₄), qui est non substitué ou substitué une à cinq fois par F,
id)phényle, qui est non substitué ou substitué par un à deux substituants identiques ou différents choisis dans le groupe constitué d'halogène et alkyle en C₁-C₄, où alkyle en C₁-C₄ est non substitué ou substitué une à cinq fois par F,
ie)(alkylène en C₁-C₄)-phényle, qui est non substitué ou substitué une à cinq fois par F,
if) cycloalkyle en C₃-C₈,
ig)oxo (=O), et
ih)(CO)-(alkyle en C₁-C₄),
et où alkyle en C₀-C₆ peut en outre être monosubstitué par phényle ou pyridyle, où phényle ou pyridyle est non substitué ou substitué par un à deux substituants identiques ou différents choisis dans le groupe constitué d'halogène et alkyle en C₁-C₄, où alkyle en C₁-C₄ est non substitué ou substitué une à cinq fois parF;
ii) cycloalkyle en C₃-C₈ qui est substitué par un à deux substituants identiques ou différents choisis dans le groupe constitué de NH₂, NH(alkyle en C₁-C₄) et N(alkyle en C₁-C₄)₂, et où cycloalkyle en C₃-C₈ peut en outre être substitué par un à trois substituants identiques ou différents choisis dans le groupe constitué de
iia) F,
iib) alkyle en C₁-C₄, où alkyle en C₁-C₄ est non substitué ou substitué une à cinq fois par F ;
b) un groupe hétérocycloalkyle en C₇-C₁₁ spiro-bicyclique contenant un atome d'azote, qui est lié par l'intermédiaire d'un atome de carbone et qui est non substitué ou substitué par un ou deux substituants identiques ou différents choisis dans le groupe constitué de F et alkyle en C₁-C₄, qui est non substitué ou substitué une à cinq fois par F ;
c) un groupe hétérocycloalkyle en C₇-C₉ bicyclique ponté contenant un atome d'azote, qui est lié par l'intermédiaire d'un atome de carbone et qui est non substitué ou substitué par un ou deux substituants identiques ou différents choisis dans le groupe constitué de F et alkyle en C₁-C₄, qui est non substitué ou substitué une à cinq fois par F ; ou
d) un groupe hétérocycloalkyle en C₁₁-C₁₅ tricyclique contenant un atome d'azote, qui est lié par l'intermédiaire d'un atome de carbone et qui est constitué d'un anneau spiro-bicyclique avec un cycle phényle condensé additionnel, et qui est non substitué ou substitué par un ou deux substituants identiques ou différents choisis dans le groupe constitué de F et alkyle en C₁-C₄, qui est non substitué ou substitué une à cinq fois par F ;
ou
R³ et R⁴ conjointement avec l'atome N portant ceux-ci désignent
a) une 1,4-pipérazinyle de formule 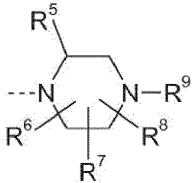 dans laquelle
R⁵ est H ou alkyle en C₁-C₆, qui est non substitué ou substitué une à cinq fois par F, ou monosubstitué par un substituant choisi dans le groupe constitué de
i) O-(alkyle en C₁-C₄),
ii) cycloalkyle en C₃-C₆,
iii) phényle,
iv) un cycle hétéroaromatique monocyclique de 5 à 6 chaînons comprenant un hétéroatome choisi dans le groupe constitué de l'azote, l'oxygène, et le soufre;
R⁶ est H ou alkyle en C₁-C₆, qui est non substitué ou substitué une à cinq fois par F ;
R⁷ est H, alkyle en C₁-C₆;
R⁸ est H, alkyle en C₁-C₆ ou oxo (=O);
R⁹ est H, alkyle en C₁-C₆, cycloalkyle en C₃-C₆ ou CO-R¹⁰, où alkyle en C₁-C₆ est non substitué ou substitué une à cinq fois par F, ou O-(alkyle en C₁-C₄) monosubstitué ; où
R¹⁰ est alkyle en C₁-C₄, cycloalkyle en C₃-C₆, NH₂, phényle, un cycle monocyclique hétérocyclique de 5 à 6 chaînons comprenant un hétéroatome choisi dans le groupe constitué de l'azote et de l'oxygène, ou un cycle hétéroaromatique monocyclique de 5 à 6 chaînons comprenant un hétéroatome choisi dans le groupe constitué de l'azote et de l'oxygène ;
b) un groupe hétérocycloalkyle monocyclique de quatre à sept chaînons contenant un atome d'azote, qui est lié par l'intermédiaire dudit azote et qui est monosubstitué par (alkylène en C₀-C₆)-NH₂, (alkylène en C₀-C₆)-NH-(alkyle en C₁-C₄), (alkylène en C₀-C₆)-N(alkyle en C₁-C₄)₂, (alkylène en C₀-C₂)-azétidinyle, (alkylène en C₀-C₂)- pyrrolidinyle, ou (alkylène en C₀-C₂)-pipéridyle ; où ledit groupe hétérocycloalkyle peut en outre être monosubstitué par alkyle en C₁-C₆, et où ledit groupe azétidinyle, pyrrolidinyle et pipéridyle peut en outre être monosubstitué par alkyle en C₁-C₆;
c) un 1,4-diazépanyle, qui est non substitué ou monosubstitué par un substituant choisi dans le groupe constitué de
i) alkyle en C₁-C₆, où alkyle en C₁-C₆ est non substitué ou substitué une à cinq fois par F,
ii) CO-(alkyle en C₁-C₆), où alkyle en C₁-C₆ est non substitué ou substitué une à cinq fois par F ;
d) un groupe hétérocycloalkyle en C₆-C₁₀ bicyclique condensé contenant deux atomes d'azote, qui est lié par l'intermédiaire d'un atome d'azote, où ledit groupe hétérocycloalkyle est non substitué ou substitué par un à deux substituants identiques ou différents choisis dans le groupe constitué de F, (alkylène en C₀-C₂)-phényle, oxo (=O), et alkyle en C₁-C₄, où alkyle en C₁-C₄ est non substitué ou substitué une à cinq fois par F ;
e) un groupe hétérocycloalkyle en C₇-C₁₁ spiro-bicyclique contenant deux atomes d'azote, qui est lié par l'intermédiaire d'un atome d'azote et qui peut contenir un atome d'oxygène supplémentaire, où ledit groupe hétérocycloalkyle est non substitué ou mono- ou di-substitué par F ou alkyle en C₁-C₄, qui est non substitué ou substitué une à cinq fois par F ;
ou
f) un groupe hétérocycloalkyle en C₁₁-C₁₅ tricyclique contenant deux atomes d'azote, qui est lié par l'intermédiaire d'un atome d'azote et qui est constitué d'un anneau spiro-bicyclique avec un cycle phényle condensé additionnel, et qui est non substitué ou substitué par un ou deux substituants identiques ou différents choisis dans le groupe constitué de F et alkyle en C₁-C₄, qui est non substitué ou substitué une à cinq fois par F.

3. Composé de formule I selon la revendication 1 ou 2,
dans lequel
R¹ est H ou halogène ;
R² est H ;
R³ est H ou alkyle en C₁-C₄;
R⁴ est
a) alkyle en C₀-C₄ qui est monosubstitué par
i) un hétérocycle monocyclique de 3 à 8 chaînons comprenant un azote cyclique, qui est non substitué ou substitué par un à quatre substituants identiques ou différents choisis dans le groupe constitué d'alkyle en C₁-C₄, O-(alkyle en C₁-C₄), (alkylène en C₁-C₄)-phényle, (C₁-C₄)- alkylène-pyridyle, cycloalkyle en C₃-C₈, oxo (=O), et (CO)-(alkyle en C₁-C₄) ;
ii) cycloalkyle en C₃-C₈ qui est monosubstitué par NH₂;
b) un groupe hétérocycloalkyle en C₉ spiro-bicyclique contenant un atome d'azote, qui est lié par l'intermédiaire d'un atome de carbone ;
c) un groupe hétérocycloalkyle en C₈ bicyclique ponté contenant un atome d'azote, qui est lié par l'intermédiaire d'un atome de carbone ;
d) un groupe hétérocycloalkyle en C₁₄ tricyclique contenant un atome d'azote, qui est lié par l'intermédiaire d'un atome de carbone et qui est constitué d'un anneau spiro-bicyclique avec un cycle phényle condensé additionnel ;
ou
R³ et R⁴ conjointement avec l'atome N portant ceux-ci désignent
a) une 1,4-pipérazinyle de formule 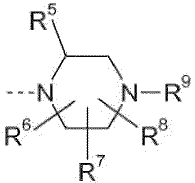 dans laquelle
R⁵ est H ou alkyle en C₁-C₆;
R⁶ est H ou alkyle en C₁-C₆;
R⁷ est H ou alkyle en C₁-C₆;
R⁸ est H ou oxo (=O);
R⁹ est H, alkyle en C₁-C₆, cycloalkyle en C₃-C₆ ou CO-R¹⁰, où alkyle en C₁-C₆ est non substitué ou monosubstitué par O-(alkyle en C₁-C₄);
R¹⁰ est alkyle en C₁-C₄, cycloalkyle en C₃-C₆, un cycle hétérocyclique aromatique ou aliphatique de 5 à 6 chaînons comprenant un hétéroatome choisi parmi l'azote ou l'oxygène;
b) 1-azétidinyle, qui est monosubstitué par NH₂;
c) 1-pyrrolidinyle, qui est monosubstitué par NH₂, NH-(alkyle en C₁-C₄), NH(alkyle en C₁-C₄)₂, (alkylène en C₀-C₂)-pyrrolidinyle ou (C₀-C₂)-pipéridinyle qui est non substitué ou monosubstitué par alkyle en C₁-C₄ ;
d) 1-pipéridyle, qui est monosubstitué par NH₂;
e) 1,4-diazépanyle de formule 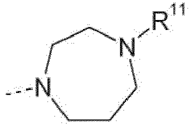 où
R¹¹ est H, alkyle en C₁-C₆ ou CO-(alkyle en C₁-C₄);
f) un groupe hétérocycloalkyle en C₈-C₁₀ bicyclique condensé contenant deux atomes d'azote, qui est lié par l'intermédiaire d'un azote;
g) un groupe hétérocycloalkyle en C₈-C₁₁ spiro-bicyclique contenant deux atomes d'azote, qui est lié par l'intermédiaire d'un atome d'azote et qui peut contenir un atome d'oxygène supplémentaire;
h) un groupe hétérocycloalkyle en C₁₄ tricyclique contenant deux atomes d'azote, qui est lié par l'intermédiaire d'un atome d'azote et qui est constitué d'un anneau spiro-bicyclique avec un cycle phényle condensé additionnel.

4. Composé de formule I selon l'une quelconque des revendications 1 à 3,
dans lequel
R¹ est Hou F;
R² est H ;
R³ est H ou CH₃;
R⁴ est
a) alkyle en C₀-C₁ qui est monosubstitué par
i) azétidyle, pyrrolidinyle ou pipéridyle, qui sont non substitués ou substitués par un à quatre substituants identiques ou différents choisis dans le groupe constitué de CH₃, C₂H₅, O-CH₃, méthylène-phényle, méthylène-pyridyle, cyclohexyle, oxo (=O), et (CO)-CH₃;
ii) cycloalkyle en C₃-C₆ qui est monosubstitué par NH₂;
b) un cycle spiro-bicyclique 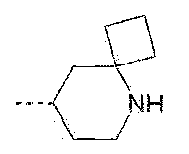
c) un anneau bicyclique ponté choisi dans le groupe constitué de 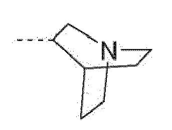
d) un anneau spiro-bicyclique avec un cycle condensé choisi dans le groupe constitué de 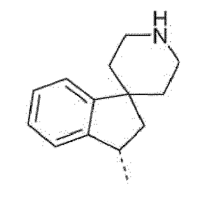
ou
R³ et R⁴ conjointement avec l'atome N portant ceux-ci désignent
a) un 1,4-pipérazinyle de formule 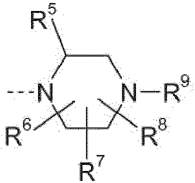 où
R⁵ est H ou CH₃;
R⁶ est H ou CH₃;
R⁷ est H ou alkyle en C₁-C₄;
R⁸ est H ou oxo (=O)
R⁹ est H, alkyle en C₁-C₃, cycloheptyle ou CO-R¹⁰, où alkyle en C₁-C₃ est non substitué ou monosubstitué par O-CH₃;
R¹⁰ est CH₃, cyclopropyle, 2-furyle ou 3-pyridyle;
b) 1-azétidinyle, qui est monosubstitué par NH_{2;}
c) 1-pyrrolidinyle, qui est monosubstitué par NH₂, NH(CH₃), NH(CH₃)₂, méthylène-1-pyrrolidinyle ou 1-pipéridinyl-4-méthyle ;
d) 1 -pipéridyle, qui est monosubstitué par NH₂;
e) un 1,4-diazépanyle de formule 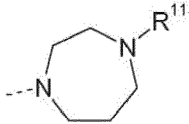 où
R¹¹ est H, éthyle ou CO-CH₃;
f) un anneau bicyclique condensé choisi dans le groupe constitué de 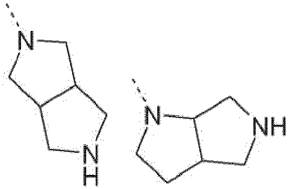
g) un anneau spiro-bicyclique choisi dans le groupe constitué de 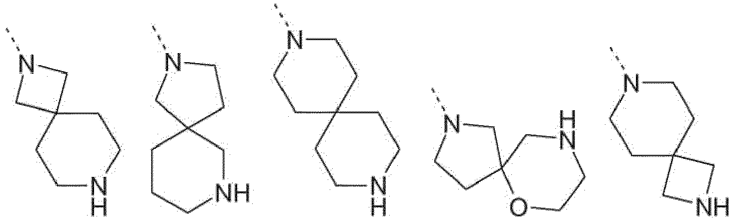 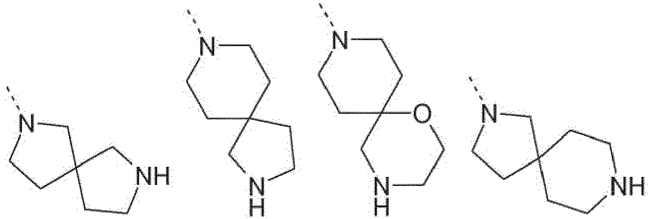 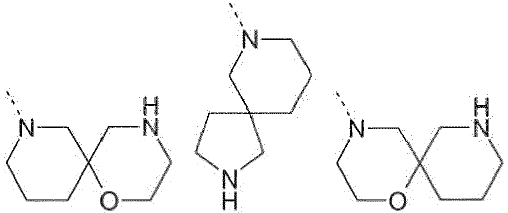
h) un anneau bicyclique ponté choisi dans le groupe constitué de 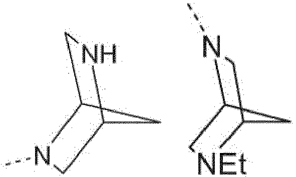
i) un anneau spiro-bicyclique avec un cycle condensé choisi dans le
groupe constitué de

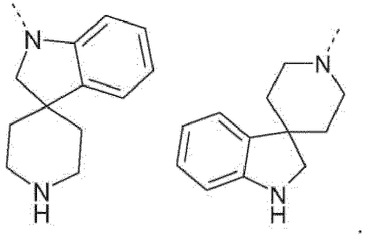

5. Composé de formule I selon l'une quelconque des revendications 1 à 4,
dans lequel
R¹ est H ou F;
R² est H;
R³ est H ou CH₃;
R⁴ est
a) méthylène qui est monosubstitué par
i) azétidyle ou pyrrolidinyle, qui sont liés par un atome de carbone et qui sont non substitués ou substitués par oxo (=O) ;
ii) cyclohexyle qui est monosubstitué par NH₂;
b) azétidyle qui est lié par un atome de carbone et qui est non substitué ou substitué par CH₃;
c) pyrrolidinyle, qui est lié par un atome de carbone et qui est non substitué ou substitué par O-CH₃;
d) pipéridyle, qui est lié par un atome de carbone et qui est non substitué ou substitué par un à quatre substituants identiques ou différents choisis dans le groupe constitué de CH₃, C₂H₅, méthylène-phényle, méthylène-pyridyle, cyclohexyle, oxo (=O), et (CO)-CH₃;
e) cycloalkyle en C₃-C₆ qui est monosubstitué par NH₂;
f) un anneau spiro-bicyclique 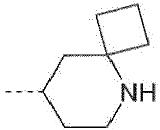
g) un anneau bicyclique ponté choisi dans le groupe constitué de 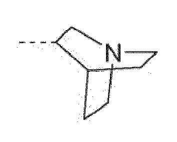
h) un anneau spiro-bicyclique avec un cycle condensé choisi dans le groupe constitué de 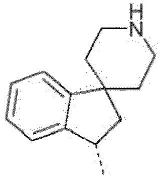
ou
R³ et R⁴ conjointement avec l'atome N portant ceux-ci désignent
a) un 1,4-pipérazinyle de formule 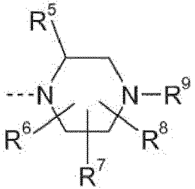 où
R⁵ est H ou CH₃;
R⁶ est H ou CH₃;
R⁷ est H ou alkyle en C₁-C₄;
R⁸ est H ou oxo (=O);
R⁹ est H, alkyle en C₁-C₃, cycloheptyle ou CO-R¹⁰, où alkyle en C₁-C₃ est non substitué ou monosubstitué par O-CH₃ ;
R¹⁰ est CH₃, cyclopropyle, 2-furyle ou 3-pyridyle;
b) 1-azétidinyle, qui est monosubstitué par NH₂;
c) 1-pyrrolidinyle, qui est monosubstitué par NH₂, NH(CH₃), NH(CH₃)₂, méthylène-1-pyrrolidinyle ou 1-pipéridinyl-4-méthyle ;
d) 1-pipéridyle, qui est monosubstitué par NH₂;
e) un 1,4-diazépanyle de formule 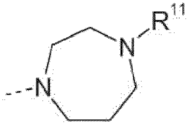 où
R¹¹ est H, éthyle ou CO-CH₃;
f) un anneau bicyclique condensé choisi dans le groupe constitué de 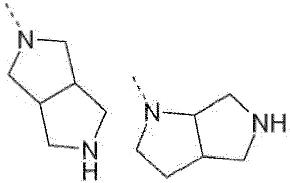
g) un anneau spiro-bicyclique choisi dans le groupe constitué de 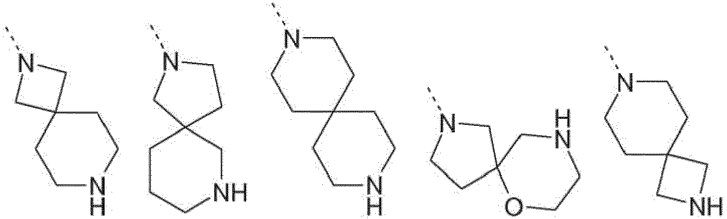 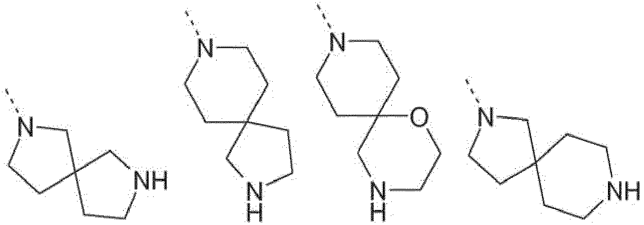 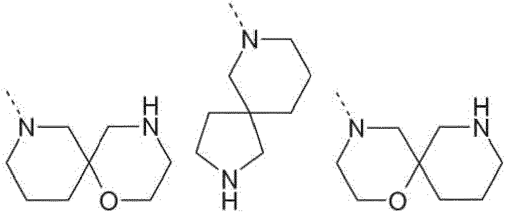
h) un anneau bicyclique ponté choisi dans le groupe constitué de 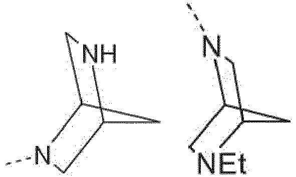
i) un anneau spiro-bicyclique avec un cycle condensé choisi dans le groupe constitué de

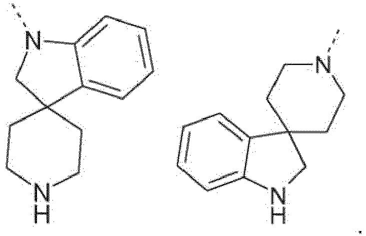

6. Composé de formule I selon l'une quelconque des revendications 1 à 5,
dans lequel
R¹ est H ou F;
R² est H;
R³ est H ou CH₃;
R⁴ est
a) méthylène qui est monosubstitué par
i) 3-azétidyle;
ii) cyclohexyle qui est monosubstitué par NH₂;
b) azétidyle qui est lié par un atome de carbone;
c) pyrrolidinyle, qui est lié par un atome de carbone;
d) pipéridyle, qui est lié par un atome de carbone et qui est non substitué ou substitué par un substituant choisi dans le groupe constitué de CH₃, C₂H₅, méthylène-phényle, méthylène-pyridyle, et cyclohexyle;
e) cyclohexyle qui est monosubstitué par NH₂;
f) un anneau spiro-bicyclique 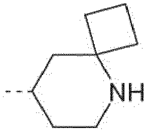
ou
R³ et R⁴ conjointement avec l'atome N portant ceux-ci désignent
a) un 1,4-pipérazinyle de formule 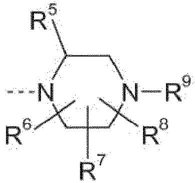 dans lequel
R⁵ est H ou CH₃;
R⁶ est H ou CH₃;
R⁷ est H ou alkyle en C₁-C_{4;}
R⁸ est H ;
R⁹ est H ou alkyle en C₁-C₃;
b) 1 -azétidinyle, qui est monosubstitué par NH₂;
c) 1-pyrrolidinyle, qui est monosubstitué par NH₂, NH(CH₃), NH(CH₃)₂, méthylène-1-pyrrolidinyle ou 1-pipéridinyl-4-méthyle ;
d) 1 -pipéridyle, qui est monosubstitué par NH₂;
e) un 1,4-diazépanyle de formule 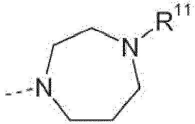 dans lequel
R¹¹ est H ou éthyle;
f) un anneau bicyclique condensé choisi dans le groupe constitué de 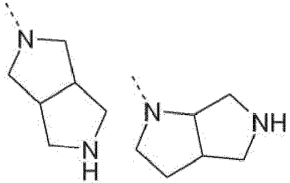
g) un anneau spiro-bicyclique choisi dans le groupe constitué de

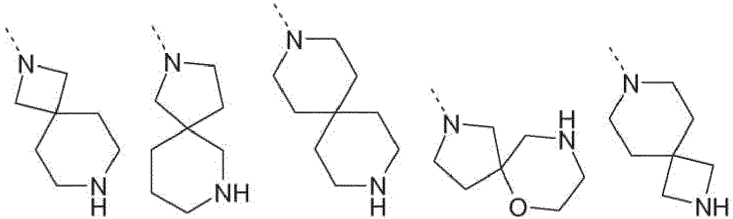

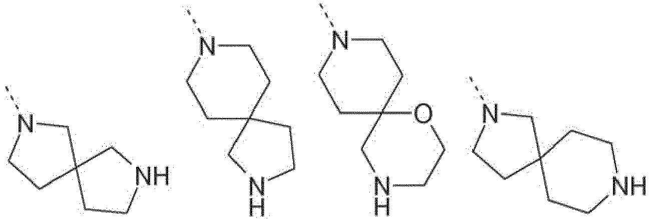

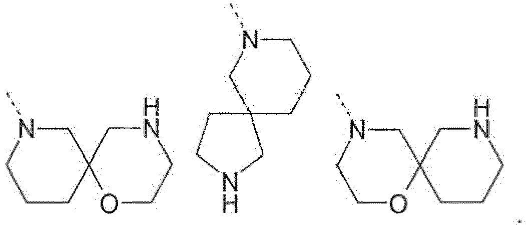

7. Composé de formule I selon l'une quelconque des revendications 1 à 6,
dans lequel
R¹ est H;
R² est H;
R³ est H;
R⁴ est
a) méthylène qui est monosubstitué par
i) 3-azétidyle;
ii) 4-amino-cyclohexyle;
b) 3-azétidyle;
c) 3-pyrrolidinyle;
d) 3-pipéridyle ou 4-pipéridyle, qui sont non substitués ou substitués à l'atome d'azote par un substituant choisi dans le groupe constitué de CH₃, C₂H₅, méthylène-phényle, méthylène-pyridyle, et cyclohexyle;
e) 3-amino-cyclohexyle ou 4-amino-cylohexyle;
f) un anneau spiro-bicyclique 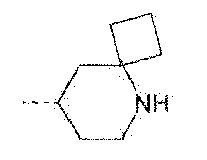
ou
R³ et R⁴ conjointement avec l'atome N portant ceux-ci désignent
a) un 1,4-pipérazinyle de formule 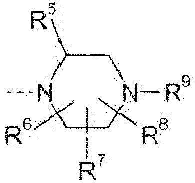 où
R⁵ est H ou CH₃;
R⁶ est H ou CH₃;
R⁷ est H ou iso-propyle;
R⁸ est H ; ,
R⁹ est H ou alkyle en C₁-C₃;
b) 3-amino-1-azétidinyle;
c) 1-pyrrolidinyle, qui est monosubstitué à la position 3 par NH₂, NH(CH₃), NH(CH₃)₂, ou 1-pipéridinyl-4-méthyle;
d) 4-amino-1-pipéridyle;
e) un 1,4-diazépanyle de formule 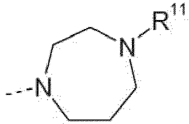 où
R¹¹ est H ou éthyle;
f) un anneau bicyclique condensé choisi dans le groupe constitué de 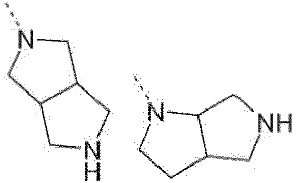
g) un anneau spiro-bicyclique choisi dans le groupe constitué de

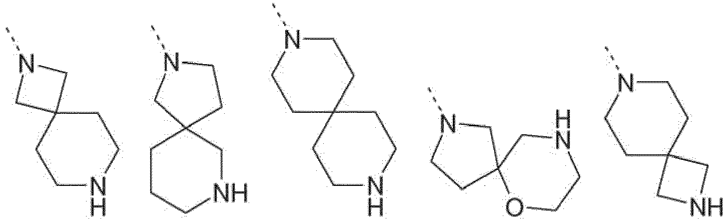

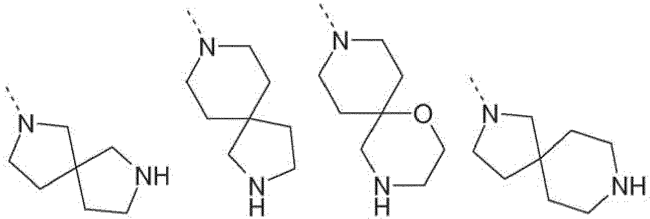

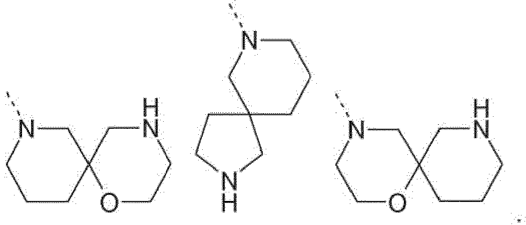

8. Composé selon la revendication 1 choisi dans le groupe constitué de
| | |
|---|---|
| 1 | (2,2,6,6-tétraméthyl-pipéridin-4-yl)-amide d'acide 6-(4-hydroxy-phényl)-1H-pyrazolo[3,4-b]pyridine-4-carboxylique |
| 2 | pipéridin-4-ylamide d'acide 6-(4-hydroxy-phényl)-1H-pyrazolo[3,4-b]pyridine-4-carboxylique |
| 3 | (3-amino-pipéridin-1-yl)-[6-(4-hydroxy-phényl)-1H-pyrazolo[3,4-b]pyridin-4-yl]-méthanone |
| 4 | [6-(4-hydroxy-phényl)-1H-pyrazolo[3,4-b]pyridin-4-yl]-pipérazin-1-yl-méthanone |
| 5 | pyrrolidin-3-ylamide d'acide 6-(4-hydroxy-phényl)-1H-pyrazolo[3,4-b]pyridine-4-carboxylique |
| 6 | ((3S,4S)-4-méthoxy-pyrrolidin-3-yl)-amide d'acide 6-(4-hydroxy-phényl)-1H-pyrazolo[3,4-b]pyridine-4-carboxylique |
| 7 | [1,4]diazépan-1 -yl-[6-(4-hydroxy-phényl)-1H-pyrazolo[3,4-b]pyridin-4-yl]-méthanone |
| 8 | (cis-4-amino-cyclohexyl)-amide d'acide 6-(4-hydroxy-phényl)-1H-pyrazolo[3,4-b]pyridine-4-carboxylique |
| 9 | (trans-4-amino-cyclohexyl)-amide d'acide 6-(4-hydroxy-phényl)-1H-pyrazolo[3,4-b]pyridine-4-carboxylique |
| 10 | (2,2-diméthyl-pipérazin-1-yl)-[6-(4-hydroxy-phényl)-1H-pyrazolo[3,4-b]pyridin-4-yl]-méthanone |
| 11 | (trans-4-amino-cyclohexylméthyl)-amide d'acide 6-(4-hydroxy-phényl)-1H-pyrazolo[3,4-b]pyridine-4-carboxylique |
| 12 | (cis-4-amino-cyclohexylméthyl)-amide d'acide 6-(4-hydroxy-phényl)-1H-pyrazolo[3,4-b]pyridine-4-carboxylique |
| 13 | (azétidin-3-ylméthyl)-amide d'acide 6-(4-hydroxy-phényl)-1H-pyrazolo[3,4-b]pyridine-4-carboxylique |
| 14 | (3-amino-cyclobutyl)-amide d'acide 6-(4-hydroxy-phényl)-1H-pyrazolo[3,4-b]pyridine-4-carboxylique |
| 15 | [6-(4-hydroxy-phényl)-1H-pyrazolo[3,4-b]pyridin-4-yl]-(5-isopropyl-2,2-diméthyl-pipérazin-1-yl)-méthanone |
| 16 | ((R)-3-amino-pyrrolidin-1-yl)-[6-(4-hydroxy-phényl)-1H-pyrazolo[3,4-b]pyridin-4-yl]-méthanone |
| 17 | ((S)-3-amino-pyrrolidin-1-yl)-[6-(4-hydroxy-phényl)-1H-pyrazolo[3,4-b]pyridin-4-yl]-méthanone |
| 18 | (R)-(1-aza-bicyclo[2.2.2]oct-3-yl)amide d'acide 6-(4-hydroxy-phényl)-1H-pyrazolo[3,4-b]pyridine-4-carboxylique |
| 19 | (R)-pipéridin-3-ylamide d'acide 6-(4-hydroxy-phényl)-1H-pyrazolo[3,4-b]pyridine-4-carboxylique |
| 20 | (2,7-diaza-spiro[3.5]non-2-yl)-[6-(4-hydroxy-phényl)-1H-pyrazolo[3,4-b]pyridin-4-yl]-méthanone |
| 21 | (2,7-diaza-spiro[3.5]non-7-yl)-[6-(4-hydroxy-phényl)-1H-pyrazolo[3,4-b]pyridin-4-yl]-méthanone |
| 22 | (2,8-diaza-spiro[4.5]dec-2-yl)-[6-(4-hydroxy-phényl)-1H-pyrazolo[3,4-b]pyridin-4-yl]-méthanone |
| 23 | (2,7-diaza-spiro[4.5]dec-2-yl)-[6-(4-hydroxy-phényl)-1H-pyrazolo[3,4-b]pyridin-4-yl]-méthanone |
| 24 | (2,7-diaza-spiro[4.5]déc-7-yl)-[6-(4-hydroxy-phényl)-1H-pyrazolo[3,4-b]pyridin-4-yl]-méthanone |
| 25 | (2,7-diaza-spiro[4.4]non-2-yl)-[6-(4-hydroxy-phényl)-1H-pyrazolo[3,4-b]pyridin-4-yl]-méthanone |
| 26 | [6-(4-hydroxy-phényl)-1H-pyrazolo[3,4-b]pyridin-4-yl]-(1-oxa-4,9-diaza-spiro[5.5]undéc-9-yl)-méthanone |
| 27 | [6-(4-hydroxy-phényl)-1H-pyrazolo[3,4-b]pyridin-4-yl]-(1-oxa-4,8-diaza-spiro[5.5]undéc-4-yl)-méthanone |
| 28 | [6-(4-hydroxy-phényl)-1H-pyrazolo[3,4-b]pyridin-4-yl]-(1-oxa-4,8-diaza-spiro[5.5]undéc-8-yl)-méthanone |
| 29 | (trans-4-méthoxy-pyrrolidin-3-yl)-amide d'acide 6-(4-hydroxy-phényl)-1H-pyrazolo[3,4-b]pyridine-4-carboxylique |
| 30 | (trans-3-amino-cyclobutyl)-amide d'acide 6-(4-hydroxy-phényl)-1H-pyrazolo[3,4-b]pyridine-4-carboxylique |
| 31 | [6-(4-hydroxy-phényl)-1H-pyrazolo[3,4-b]pyridin-4-yl]-(6-oxa-2,9-diaza-spiro[4.5]déc-2-yl)-méthanone |
| 32 | (5-aza-spiro[3.5]non-8-yl)-amide d'acide 6-(4-hydroxy-phényl)-1H-pyrazolo[3,4-b]pyridine-4-carboxylique |
| 33 | (3-amino-cyclohexyl)-amide d'acide 6-(4-hydroxy-phényl)-1H-pyrazolo[3,4-b]pyridine-4-carboxylique |
| 34 | (Hexahydro-pyrrolo[3,4-b]pyrrol-1-yl)-[6-(4-hydroxy-phényl)-1H-pyrazolo[3,4-b]pyridin-4-yl]-méthanone |
| 35 | azétidin-3-ylamide d'acide 6-(4-hydroxy-phényl)-1H-pyrazolo[3,4-b]pyridine-4-carboxylique |
| 36 | (3-amino-azétidin-1-yl)-[6-(4-hydroxy-phényl)-1H-pyrazolo[3,4-b]pyridin-4-yl]-méthanone |
| 37 | [6-(4-hydroxy-phényl)-1H-pyrazolo[3,4-b]pyridin-4-yl]-(3-méthyl-pipérazin-1-yl)-méthanone |
| 38 | (4-amino-pipéridin-1-yl)-[6-(4-hydroxy-phényl)-1H-pyrazolo[3,4-b]pyridin-4-yl]-méthanone |
| 39 | [6-(4-hydroxy-phényl)-1H-pyrazolo[3,4-b]pyridin-4-yl]-(3-méthylamino-pyrrolidin-1-yl)-méthanone |
| 40 | (S)-(1-aza-bicyclo[2.2.2]oct-3-yl)amide d'acide 6-(4-hydroxy-phényl)-1H-pyrazolo[3,4-b]pyridine-4-carboxylique |
| 41 | (1-méthyl-pipéridin-3-yl)-amide d'acide 6-(4-hydroxy-phényl)-1H-pyrazolo[3,4-b]pyridine-4-carboxylique |
| 42 | méthyl-pipéridin-4-yl-amide d'acide 6-(4-hydroxy-phényl)-1H-pyrazolo[3,4-b]pyridine-4-carboxylique |
| 43 | (2,8-diaza-spiro[4.5]dec-8-yl)-[6-(4-hydroxy-phényl)-1H-pyrazolo[3,4-b]pyridin-4-yl]-méthanone |
| 44 | (3,9-diaza-spiro[5.5]undec-3-yl)-[6-(4-hydroxy-phényl)-1H-pyrazolo[3,4-b]pyridin-4-yl]-méthanone |
| 45 | (3-amino-pyrrolidin-1-yl)-[6-(4-hydroxy-phényl)-1H-pyrazolo[3,4-b]pyridin-4-yl]-méthanone |
| 46 | (hexahydro-pyrrolo[3,4-c]pyrrol-2-yl)-[6-(4-hydroxy-phényl)-1H-pyrazolo[3,4-b]pyridin-4-yl]-méthanone |
| 47 | 2,5-diaza-bicyclo[2.2.1]hept-2-yl-[6-(4-hydroxy-phényl)-1H-pyrazolo[3,4-b]pyridin-4-yl]-méthanone |
| 48 | (2,3-dihydro-spiro[1H-indène-1,4'-pipéridin]-3-yl)-amide d'acide 6-(4-hydroxy-phényl)-1H-pyrazolo[3,4-b]pyridine-4-carboxylique |
| 49 | (1,2-dihydrospiro[3H-indole-3,4'-pipéridin]-1'-yl)-amide d'acide 6-(4-hydroxy-phényl)-1H-pyrazolo[3,4-b]pyridine-4-carboxylique |
| 50 | (2,3-dihydrospiro[1H-indène-1,4'-pipéridin]-3-yl)-amide d'acide 6-(4-hydroxy-phényl)-1H-pyrazolo[3,4-b]pyridine-4-carboxylique |
| 51 | (trans-2-amino-cyclopropyl)-amide d'acide 6-(4-hydroxy-phényl)-1H-pyrazolo[3,4-b]pyridine-4-carboxylique |
| 52 | [6-(4-hydroxy-phényl)-1H-pyrazolo[3,4-b]pyridin-4-yl]-(4-méthyl-pipérazin-1-yl)-méthanone |
| 53 | 1-{4-[6-(4-hydroxy-phényl)-1H-pyrazolo[3,4-b]pyridine-4-carbonyl]-pipérazin-1-yl}-éthanone |
| 54 | [6-(4-hydroxy-phényl)-1H-pyrazolo[3,4-b]pyridin-4-yl]-((S)-2-pyrrolidin-1-ylméthyl-pyrrolidin-1-yl)-méthanone |
| 55 | ((S)-3-diméthylamino-pyrrolidin-1-yl)-[6-(4-hydroxy-phényl)-1H-pyrazolo[3,4-b]pyridin-4-yl]-méthanone |
| 56 | [6-(4-hydroxy-phényl)-1H-pyrazolo[3,4-b]pyridin-4-yl]-(4-isopropyl-pipérazin-1-yl)-méthanone |
| 57 | (4-Cyclopropanecarbonyl-pipérazin-1-yl)-[6-(4-hydroxy-phényl)-1H-pyrazolo[3,4-b]pyridin-4-yl]-méthanone |
| 58 | (4-Cycloheptyl-pipérazin-1-yl)-[6-(4-hydroxy-phényl)-1H-pyrazolo[3,4-b]pyridin-4-yl]-méthanone |
| 59 | 1-{4-[6-(4-hydroxy-phényl)-1H-pyrazolo[3,4-b]pyridine-4-carbonyl]-[1,4]diazépan-1-yl}-éthanone |
| 60 | (1-éthyl-pipéridin-3-yl)-amide d'acide 6-(4-hydroxy-phényl)-1H-pyrazolo[3,4-b]pyridine-4-carboxylique |
| 61 | [6-(4-hydroxy-phényl)-1H-pyrazolo[3,4-b]pyridin-4-yl]-[4-(tétrahydro-furan-2-carbonyl)-pipérazin-1-yl]-méthanone |
| 62 | (1-méthyl-azétidin-3-yl)-amide d'acide 6-(4-hydroxy-phényl)-1H-pyrazolo[3,4-b]pyridine-4-carboxylique |
| 63 | (5-oxo-pyrrolidin-2-ylméthyl)-amide d'acide 6-(4-hydroxy-phényl)-1H-pyrazolo[3,4-b]pyridine-4-carboxylique |
| 64 | (4-Éthyl-[1,4]diazépan-1-yl)-[6-(4-hydroxy-phényl)-1H-pyrazolo[3,4-b]pyridin-4-yl]-méthanone |
| 65 | [6-(4-hydroxy-phényl)-1H-pyrazolo[3,4-b]pyridin-4-yl]-[3-(4-méthyl-pipéridin-1-yl)-pyrrolidin-1-yl]-méthanone |
| 66 | (1-méthyl-pipéridin-4-yl)-amide d'acide 6-(4-hydroxy-phényl)-1H-pyrazolo[3,4-b]pyridine-4-carboxylique |
| 67 | (1-cyclohexyl-pipéridin-4-yl)-amide d'acide 6-(4-hydroxy-phényl)-1H-pyrazolo[3,4-b]pyridine-4-carboxylique |
| 68 | (1-acétyl-pipéridin-4-yl)-amide d'acide 6-(4-hydroxy-phényl)-1H-pyrazolo[3,4-b]pyridine-4-carboxylique |
| 69 | ((R)-6-oxo-pipéridin-3-yl)-amide d'acide 6-(4-hydroxy-phényl)-1H-pyrazolo[3,4-b]pyridine-4-carboxylique |
| 70 | (5-Éthyl-2,5-diaza-bicyclo[2.2.1]hept-2-yl)-[6-(4-hydroxy-phényl)-1H-pyrazolo[3,4-b]pyridin-4-yl]-méthanone |
| 71 | [6-(4-hydroxy-phényl)-1H-pyrazolo[3,4-b]pyridin-4-yl]-[4-(2-méthoxy-éthyl)-pipérazin-1-yl]-méthanone |
| 72 | (1-aza-bicyclo[2.2.2]oct-3-yl)-amide d'acide 6-(4-hydroxy-phényl)-1H-pyrazolo[3,4-b]pyridine-4-carboxylique |
| 73 | méthyl-(1-méthyl-pipéridin-4-yl)-amide d'acide 6-(4-hydroxy-phényl)-1H-pyrazolo[3,4-b]pyridine-4-carboxylique |
| 74 | [6-(4-hydroxy-phényl)-1H-pyrazolo[3,4-b]pyridin-4-yl]-[4-(pyridine-3-carbonyl)-pipérazin-1-yl]-méthanone |
| 75 | (1-pyridin-4-ylméthyl-pipéridin-4-yl)-amide d'acide 6-(4-hydroxy-phényl)-1H-pyrazolo[3,4-b]pyridine-4-carboxylique |
| 76 | 4-[6-(4-hydroxy-phényl)-1H-pyrazolo[3,4-b]pyridine-4-carbonyl]-1-méthyl-pipérazin-2-one |
| 77 | ((R)-1-benzyl-pipéridin-3-yl)-amide d'acide 6-(4-hydroxy-phényl)-1H-pyrazolo[3,4-b]pyridine-4-carboxylique |
| 78 | (1-pyridin-3-ylméthyl-pipéridin-4-yl)-amide d'acide 6-(4-hydroxy-phényl)-1H-pyrazolo[3,4-b]pyridine-4-carboxylique |

9. Composition pharmaceutique comprenant au moins un composé de formule I selon l'une quelconque des revendications 1 à 8 ou un solvate physiologiquement acceptable de l'un quelconque de ceux-ci, pour utilisation en tant qu'agent pharmaceutique.

10. Composé de formule I selon l'une quelconque des revendications 1 à 8 ou un sel physiologiquement acceptable de celui-ci, ou un solvate physiologiquement acceptable de l'un quelconque de ceux-ci, pour utilisation en tant qu'agent pharmaceutique.

11. Composé de formule I selon l'une quelconque des revendications 1 à 8 ou un sel physiologiquement acceptable de celui-ci, ou un solvate physiologiquement acceptable de l'un quelconque de ceux-ci, pour utilisation dans le traitement de maladies associées au diabète et de complications diabétiques.

12. Composé de formule I selon l'une quelconque des revendications 1 à 8 ou un sel physiologiquement acceptable de celui-ci, ou un solvate physiologiquement acceptable de l'un quelconque de ceux-ci, pour utilisation dans la prévention et le traitement d'une néphropathie, une neuropathie, une rétinopathie, une ischémie, une inflammation, des troubles du système nerveux central, des maladies cardiovasculaires, des maladies dermatologiques, des maladies auto-immunes et le cancer.

13. Composé de formule I selon l'une quelconque des revendications 1 à 8 ou un sel physiologiquement acceptable de celui-ci, ou un solvate physiologiquement acceptable de l'un quelconque de celui-ci, pour utilisation dans le traitement d'une maladie associée au récepteur PKC.
